# EUROPEAN PATENT SPECIFICATION

(11) **EP 1 735 332 B1**
(45) Date of publication and mention of the grant of the patent: **19.10.2011**
(21) Application number: 05738987.6
(22) Date of filing: 06.04.2005
(51) Int. Cl.: C07J 71/00, A61K 31/58, A61P 25/28, A61P 29/00

(54) **AZACYCLOSTEROID HISTAMINE-3 RECEPTOR LIGANDS**
AZAZYKLOSTEROID ALS LIGANDE DER HISTAMIN-3 REZEPTOREN
LIGANDS DE RECEPTEUR H3 DE L'HISTAMINE AZACYCLOSTEROIDE

(30) Priority: 07.04.2004 US 819849
(43) Date of publication of application: 27.12.2006
(73) Proprietor: ABBOTT LABORATORIES, Abbott Park IL 60064-3500 (US)
(72) Inventor: ZHAO, Chen, Libertyville, Illinois 60048 (US); SUN, Minghua, Libertyville, Illinois 60048 (US); COWART, Marlon, D., Round Lake Beach, Illinois 60073 (US); BENNANI, Youssef, L., Shaker Heights, Illinois 44122 (US)
(74) Representative: Modiano, Micaela Nadia
(86) International application number: PCT/US2005/014019
(87) International publication number: WO 2005/100377

(56) References cited:
- GB-A- 1 170 945
- GB-A- 1 230 681
- US-A- 3 485 825
- H. KAPNANG ET AL: "Desamination directe d'amines tertiares N,N-Dimethylés" TETRAHEDRON LETTERS., vol. 21, 1980, pages 2951-2954, XP002340701 NLELSEVIER SCIENCE PUBLISHERS, AMSTERDAM.
- G. VAN DE WOUDE ET AL: "Amino steroids - Conanine and heteroconanine derivatives" BULLETIN DES SOCIETES CHIMIQUES BELGES., vol. 82, 1973, pages 49-62, XP008050836 GBPERGAMON PRESS LTD. OXFORD.
- DATABASE CA [Online] CHEMICAL ABSTRACTS SERVICE, COLUMBUS, OHIO, US; SIDDIQUI, SALIMUZZAMAN ET AL: "Some extensions of von Braun BrCN reaction on organic bases" XP002340726 retrieved from STN Database accession no. 1983:405897 & PROCEEDINGS OF THE PAKISTAN ACADEMY OF SCIENCES , 18(2), 89-108 CODEN: PKSPAW; ISSN: 0377-2969, 1981,
- A. CAVÉ ET AL: "No 130. - Alcaloïdes stéroïdiques. LVIII. - Influence de la nature des solvants polaires aprotoniques sur la stéréochimie et le mécanisme de l'azidolyse des tosyloxy-3.beta.-delta-5-stéroïdes. Application à la synthèse d'amino-3.alpha.,delta-5 stéroïdes" BULLETIN DE LA SOCIETE CHIMIQUE DE FRANCE., 1967, pages 701-706, XP002340702 FRSOCIETE FRANCAISE DE CHIMIE. PARIS.
- M-M JANOT ET AL: "No 122.- Alcaloïdes stéroïdiques XVI. La malouphyllamine, alcaloïde du Malouetia bequaertiana E. Woodson (Apocynacées)" BULLETIN DE LA SOCIETE CHIMIQUE DE FRANCE., 1963, pages 641-646, XP002340703 FRSOCIETE FRANCAISE DE CHIMIE. PARIS.
- DATABASE CA [Online] CHEMICAL ABSTRACTS SERVICE, COLUMBUS, OHIO, US; CARDELL, LARS OLAF ET AL: "Characterization of the histamine receptors in the guinea-pig lung: evidence for relaxant histamine H3 receptors in the trachea Characterization of the histamine receptors in the guinea-pig lung: evidence for relaxant histamine H3 receptors in the trachea" XP002340727 retrieved from STN Database accession no. 1994:209161 & BRITISH JOURNAL OF PHARMACOLOGY , 111(2), 445-54 CODEN: BJPCBM; ISSN: 0007-1188, 1994,
- DATABASE CA [Online] CHEMICAL ABSTRACTS SERVICE, COLUMBUS, OHIO, US; BAKHSH, ILAHI: "Pharmacological action of conessine and isoconessine" XP002340728 retrieved from STN Database accession no. 1937:8393 & J. PHARMACOL. , 58, 373-92, 1936,
- DATABASE CA [Online] CHEMICAL ABSTRACTS SERVICE, COLUMBUS, OHIO, US; CHOPRA, R. N. ET AL: "Pharmacological action of conessine, the alkaloid of Holarrhena anti-dysenterica" XP002340729 retrieved from STN Database accession no. 1927:14950 & INDIAN MEDICAL GAZETTE , 62, 132-40 CODEN: IMGAAY; ISSN: 0019-5863, 1927,
- DATABASE CA [Online] CHEMICAL ABSTRACTS SERVICE, COLUMBUS, OHIO, US; BURN, J. H.: "The action of conessine and holarrhenine, the alkaloids of Holarrhena congolensis, and also of oxyconessine" XP002340730 retrieved from STN Database accession no. 1915:4281 & J. PHARMACOL. , 6, 305-21, 1915,
- DATABASE CA [Online] CHEMICAL ABSTRACTS SERVICE, COLUMBUS, OHIO, US; AUFFRET, CH. ET AL: "Distribution and fixing of conessine in organs of the monkey" XP002340731 retrieved from STN Database accession no. 1951:9396 & MEDECINE TROPICALE (MARSEILLE) , 10, 530-6 CODEN: METRA2; ISSN: 0025-682X, 1950,
- DATABASE EMBASE [Online] ELSEVIER SCIENCE PUBLISHERS, AMSTERDAM, NL; 1 June 2003 (2003-06-01), STARK H: "Recent advances in histamine H3/H4 receptor ligands" XP002340732 Database accession no. EMB-2003250038 & EXPERT OPINION ON THERAPEUTIC PATENTS 01 JUN 2003 UNITED KINGDOM, vol. 13, no. 6, 1 June 2003 (2003-06-01), pages 851-865, ISSN: 1354-3776

## Description

### BACKGROUND OF THE INVENTION

### Technical Field

The invention relates to azacyclosteroid compounds, compositions comprising such compounds, and methods of treating conditions and disorders using such compounds and compositions.

### Description of Related Technology

Histamine is a well-known modulator of neuronal activity. At least four types of histamine receptors have been reported in the literature, typically referred to as histamine-1, histamine-2, histamine-3, and histamine-4. The class of histamine receptor known as histamine-3 receptors is believed to play a role in neurotransmission in the central nervous system.

The histamine-3 (H₃) receptor was first characterized pharmacologically on histaminergic nerve terminals (Nature, 302:832-837 (1983)), where it regulates the release of neurotransmitters in both the central nervous system and peripheral organs, particularly the lungs, cardiovascular system and gastrointestinal tract. H₃ receptors are thought to be disposed presynaptically on histaminergic nerve endings, and also on neurons possessing other activity, such as adrenergic, cholinergic, serotoninergic, and dopaminergic activity. The existence of H₃ receptors has been confirmed by the development of selective H₃ receptor agonists and antagonists ((Nature, 327:117-123 (1987); Leurs and Timmerman, ed. "The History of H3 Receptor: a Target for New Drugs," Elsevier (1998)).

The activity at the H₃ receptors can be modified or regulated by the administration of H₃ receptor ligands. The ligands can exhibit antagonist, agonist, partial agonist, or inverse agonist properties. For example, H₃ receptors have been linked to conditions and disorders related to memory and cognition processes, neurological processes, cardiovascular function, and regulation of blood sugar, among other systemic activities. Although various classes of compounds demonstrating H₃ receptor-modulating activity exist, it would be beneficial to provide additional compounds demonstrating activity at the H₃ receptors that can be incorporated into pharmaceutical compositions useful for therapeutic methods. GB-A-1,170 945 discloses azacyclosteroids and their anti-inflammatory, anti-atheromatous and anti-cholesterolemiant activities but makes no mention of any effects on histamine receptors.

### SUMMARY OF THE INVENTION

The invention is directed to azacyclosteroid compounds, compositions comprising the compounds, and methods of using such compounds and compositions. The compounds of the invention have the formula: 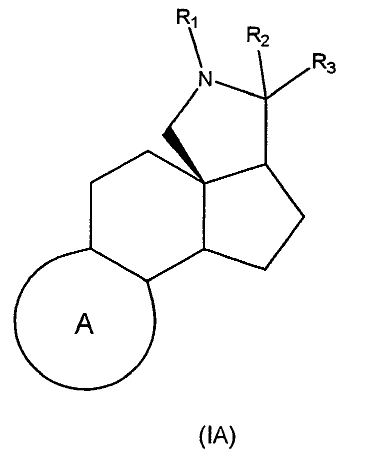 or a pharmaceutically acceptable salt wherein
R₁ is selected from the group consisting of hydrogen, acetyl, alkyl, fluoroalkyl, and cycloalkyl;
R₂ and R₃ are each independently selected from the group consisting of hydrogen and alkyl, or R₂ and R₃ taken together form a 3- to 6-membered ring;
Ring A of the formula: 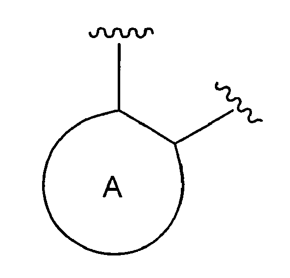 is selected from the following: 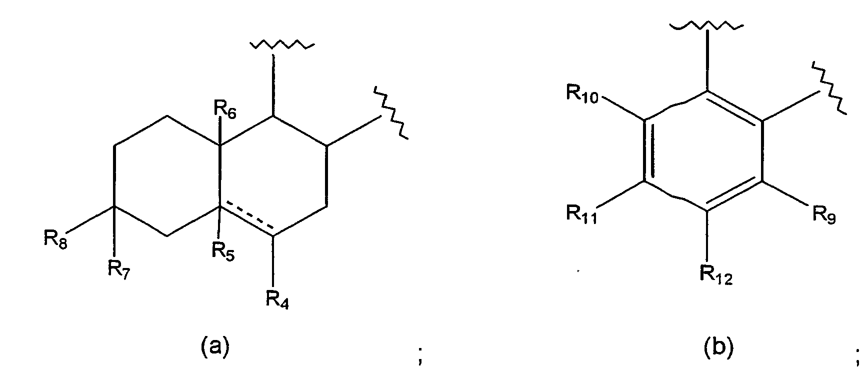 and 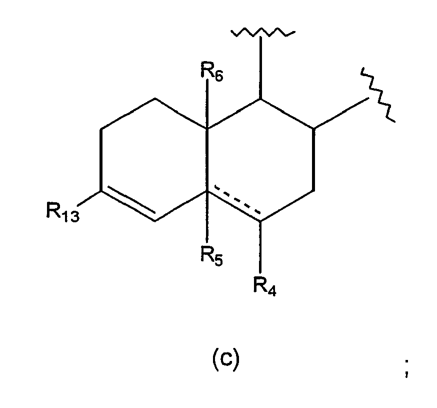 wherein:
the dotted line represents an optional bond;
R₄ and R₅ are each independently selected from the group co nsisting of hydrogen and fluorine, provided that R₅ is present only when the bond represented by the dotted line is absent;
R₆ is selected from the group consisting of hydrogen, alkyl, and fluorine;
one of R₇ and R₈ is hydrogen; and the other of R₇ and R₈ is selected from the group consisting of:
   a) NR₁₈R₁₉; or
   b) SR₂₀ or O(C=O)N(R₂₀)(R₂₁)
      and
   c) NR₂₂(C=O)NR₂₆R₂₇, NR₂₂(C=O)CH(NR₂₈R₂₉)R₃₀, or NR₂₂(C=O)C(OR₂₃)R₃₀R_{30b}; and
   d) NR₂₂SO₂R₃₁ or NR₂₂SO₂N(R₂₂)(R₂₃);
   or R₇ and R₈ taken together with the carbon atom to which each is attached forms a group of the formula -C=C(Rₐ)(R_{b}), wherein Rₐ and R_{b} are each inde pendently selected from the group consisting of hydrogen, alkyl, and cycloalkyl;
R₉ and R₁₀ are each independently selected from the group consisting of hydrogen, halogen, alkyl, and cyano;
R₁₁ and R₁₂ are each independently selected from the group consisting of hydrogen, heteroaryl, heterocycle, aryl, arylalkyl, aryloxy, arylcarbonyl, arylcarbonyloxy, arylalkoxy, alkylsulfonyl, arylsulfonyl, and trifluoromethylsulfonyl; orone of R₁₀ and R₁₁ or R₁₁ and R₁₂ taken together with the atoms to which each is attach ed form a 5- to 6-membered aromatic or heteroaromatic ring;
R₁₃ is selected from the group consisting of alkyl, cycloalkyl, aryl, and heteroaryl;
R₁₈ is hydrogen or C₁-C₆ alkyl and R₁₉ is selected from the group consisting of aryl and heteroaryl, or R₁₈ and R₁₉ at each occurrence is taken together form a 3- to 8-membered heterocycle;
R₂₀ and R₂₁ at each occurrence are each independently selected from the group consisting of hydrogen, alkyl, cycloalkyl, alkoxyalkyl, cyanoalkyl, hydroxyalkyl, haloalkyl, aryl, aryloxy, arylalkyl, arylsulfonyl, heteroaryl, heteroarylalkyl, heteroarylcarbonyl, heterocycle, and heterocyclealkyl.
R₂₂ at each occurrence is selected from the group consisting of hydrogen and alkyl;
R₂₃ at each occurrence is selected from the group consisting of hydrogen, alkyl, and alkylcarbonyl;
R₂₆ and R₂₇ are each independently selected from the group consisting of alkyl, cycloalkyl, aryl, heteroaryl, and heterocycle, provided that R₂₆ and R₂₇ are not both alkyl, or R₂₆ and R₂₇ taken together with the nitrogen atom to which each is attached forms an aromatic or non-aromatic 5- to 6-membered ring, wherein 0, 1, or 2 carbon atoms in the ring is substituted with a hetero atom selected from O, S, or NR₂₃;
R₂₈ and R₂₉ at each occurrence are each independently selected from the group consisting of hydrogen, alkyl, alkylcarbonyl, alkoxycarbonyl, cycloalkyl, aryl, heteroaryl, and heterocycle, or R₂₈ and R₂₉ taken together with the nitrogen atom to which each is attached forms an aromatic or non-aromatic 5- to 6-membered ring, wherein 0, 1, or 2 carbon atoms in the ring is substituted with a heteroatom selected from O, S, or NR₂₃;
R₃₀ at each occurrence is independently selected from the group consisting of hydrogen, alkyl, alkoxyalkyl, hydroxyalkyl, aryl, arylalkyl, cycloalkyl, cycloalkylalkyl, cyanoalkyl, haloalkyl, heteroaryl, heteroarylalkyl, heteroarylcarbonyl, heterocycle, and heterocyclealkyl;
R_{30b} is selected from the group consisting of hydrogen and alkyl, or when R₃₀ is alkyl and R_{30b} is alkyl, the alkyl groups can be bonded together to form a C₃-C₄ cycloalkyl group; and
R₃₁ is selected from the group consisting of alkyl, cycloalkyl, aryl, heterocycle and heteroaryl.

The invention also relates to compounds according to formula IB, as defined below, or the compound benzoic acid 2,3,11a- trimethyl- 2,3,3a,4,5,5a,5b,6,8,9,10,11,11a,11b,12,13- hexadecahydro-1H-2-aza-pentaleno[1,6a- a]phenantren-9-yl ester for use in a method of treating a condition modulated by the histamine-3 receptors in a mammal comprising administering an effective amount of said compound, wherein the condition is related to function in a mammal selected from the group consisting of memory and cognition processes, neurological processes and regulation of blood sugar 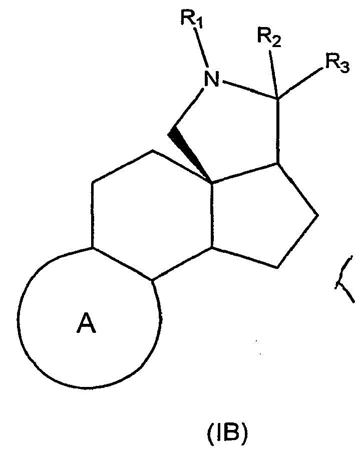 or a pharmaceutically acceptable salt thereof, wherein
R₁ is selected from the group consisting of hydrogen, acetyl, alkyl, fluoroalkyl, and cycloalkyl;
R₂ and R₃ are each independently selected from the group consisting of hydrogen and alkyl, or R₂ and R₃ taken together form a 3- to 6-membered ring;
Ring A of the formula: 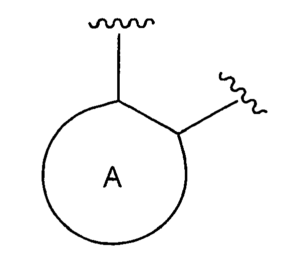 is selected from the following: 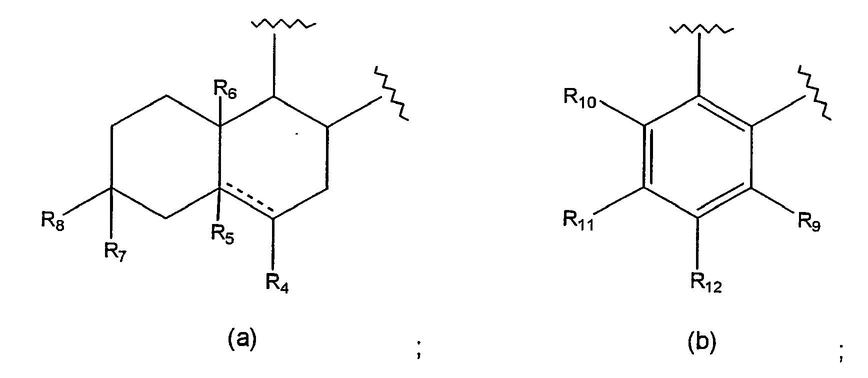 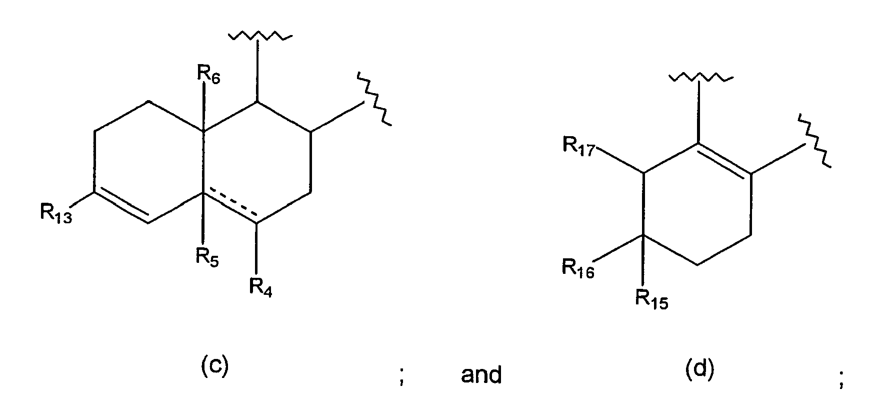 wherein:
the dotted line represents an optional bond;
R₄ and R₅ are each independently selected from the group consisting of hydrogen and fluorine, provided that R₅ is present only when the bond represented by the dotted line is absent;
R₆ is selected from the group consisting of hydrogen, alkyl, and fluorine;
one of R₇ and R₈ is hydrogen; and the other of R₇ and R₈ is selected from the group consisting of:
   a) NR₁₈R₁₉;
   b) OR₂₀, SR₂₀, O(C=O)OR₂₀, O(C=O)N(R₂₀)(R₂₁), O(C=O)C(R₂₃)(R₂₄)(R_{24b}), or O(C=O)CH(NR₂₈R₂₉)R₂₅; and
   c) NR₂₂(C=O)R₂₅, NR₂₂(C=O)NR₂₆R₂₇, NR₂₂(C=O)CH(NR₂₈R₂₉)R₃₀, N(R₂₂)(C=O)OR₂₀, or NR₂₂(C=O)C(OR₂₃)R₃₀R_{30b}; and
   d) NR₂₂SO₂R₃₁ or NR₂₂SO₂N(R₂₂)(R₂₃);
   or R₇ and R₈ taken together with the carbon atom to which each is attached forms a group of the formula -C=C(Rₐ)(R_{b}), wherein Rₐ and R_{b} are each independently selected from the group consisting of hydrogen, alkyl, and cycloalkyl;
R₉ and R₁₀ are each independently selected from the group consisting of hydrogen, halogen, alkyl, and cyano;
R₁₁ and R₁₂ are each independently selected from the group consisting of hydrogen, heteroaryl, heterocycle, aryl, arylalkyl, aryloxy, arylcarbonyl, arylcarbonyloxy, arylalkoxy, alkylsulfonyl, arylsulfonyl, and trifluoromethylsulfonyl; or one of R₁₀ and R₁₁ or R₁₁ and R₁₂ taken together with the atoms to which each is attached form a 5- to 6-membered aromatic or heteroaromatic ring;
R₁₃ is selected from the group consisting of alkyl, cycloalkyl, aryl, and heteroaryl;
R₁₅ and R₁₆ taken together with the atom to which each is attached forms a 5- to 6-membered ring optionally substituted with an alkylamino group;
R₁₇ is selected from the group consisting of hydrogen, alkyl, and fluoro;
R₁₈ is hydrogen or C₁-C₆ alkyl and R₁₉ is selected from the group consisting of aryl and heteroaryl, or R₁₈ and R₁₉ at each occurrence is taken together form a 3- to 8-membered heterocycle;
R₂₀ and R₂₁ at each occurrence are each independently selected from the group consisting of hydrogen, alkyl, cycloalkyl, alkoxyalkyl, cyanoalkyl, hydroxyalkyl, haloalkyl, aryl, aryloxy, arylalkyl, arylsulfonyl, heteroaryl, heteroarylalkyl, heteroarylcarbonyl, heterocycle, and heterocyclealkyl;
R₂₂ at each occurrence is selected from the group consisting of hydrogen and alkyl;
R₂₃ at each occurrence is selected from the group consisting of hydrogen, alkyl, and alkylcarbonyl;
R₂₄ and R_{24b} are each independently selected from the group consisting of hydrogen, alkyl, aryl, arylalkyl, and cycloalkyl;
R₂₅ at each occurrence is independently selected from the group consisting of C₁-C₆ alkyl, alkoxyalkyl, hydroxyalkyl, aryl, arylalkyl, cycloalkyl, cycloalkylalkyl, cyanoalkyl, haloalkyl, heteroaryl, heteroarylalkyl, heteroarylcarbonyl, heterocycle, and heterocyclealkyl;
R₂₆ and R₂₇ are each independently selected from the group consisting of alkyl, alkylcarbonyl, alkoxycarbonyl, cycloalkyl, aryl, heteroaryl, and heterocycle, or R₂₆ and R₂₇ taken together with the nitrogen atom to which each is attached forms an aromatic or non-aromatic 5- to 6-membered ring, wherein 0, 1, or 2 carbon atoms in the ring is substituted with a heteroatom selected from O, S, or NR₂₃;
R₂₈ and R₂₉ at each occurrence are each independently selected from the group consisting of hydrogen, alkyl, alkylcarbonyl, alkoxycarbonyl, cycloalkyl, aryl, heteroaryl, and heterocycle, or R₂₈ and R₂₉ taken together with the nitrogen atom to which each is attached forms an aromatic or non-aromatic 5- to 6-membered ring, wherein 0, 1, or 2 carbon atoms in the ring is substituted with a heteroatom selected from O, S, or NR₂₃;
R₃₀ at each occurrence is independently selected from the group consisting of hydrogen, alkyl, alkoxyalkyl, hydroxyalkyl, aryl, arylalkyl, cycloalkyl, cycloalkylalkyl, cyanoalkyl, haloalkyl, heteroaryl, heteroarylalkyl, heteroarylcarbonyl, heterocycle, and heterocyclealkyl;
R_{30b} is selected from the group consisting of hydrogen and alkyl, or when R₃₀ is alkyl and R_{30b} is alkyl, the alkyl groups can be bonded together to form a C₃-C₄ cycloalkyl group; and
R₃₁ is selected from the group consisting of alkyl, cycloalkyl, aryl, heterocycle, and heteroaryl.
Another aspect of the invention relates to pharmaceutical compositions comprising compounds of fromula IA of the invention. Such compositions can be administered in accordance with a method of the invention, typically as part of a therapeutic regimen for treatment or prevention of conditions and disorders related to H₃ receptor activity.
Yet another aspect of the invention relates to compounds of formula IA for use in a method of selectively modulating H₃ receptor activity in particular for treating and/or preventing conditions and disorders related to H₃ receptor modulation in mammals. More particularly, the invention concerns compounds of formula IA for use in a method of treatment of conditions and disorders related to memory and cognition processes, neurological processes, cardiovascular function, and body weight and to compounds of formula IB, for use in the treatment of these specific conditions and disorders.
The compounds, compositions comprising the compounds, and methods for treating or preventing conditions and disorders by administering the compounds are further described herein.

### DETAILED DESCRIPTION OF THE INVENTION

### Definition of Terms

Certain terms as used in the specification are intended to refer to the following definitions, as detailed below.

The term "acyl" as used herein, means an alkyl group, as defined herein, appended to the parent molecular moiety through a carbonyl group, as defined herein. Representative examples of acyl include, but are not limited to, acetyl, 1-oxopropyl, 2,2-dimethyl-1-oxopropyl, 1-oxobutyl, and 1-oxopentyl.

The term "acyloxy" as used herein, means an acyl group, as defined herein, appended to the parent molecular moiety through an oxygen atom. Representative examples of acyloxy include, but are not limited to, acetyloxy, propionyloxy, and isobutyryloxy.

The term "alkenyl" as used herein, means a straight or branched chain hydrocarbon containing from 2 to 10 carbons and containing at least one carbon-carbon double bond formed by the removal of two hydrogens. Representative examples of alkenyl include, but are not limited to, ethenyl, 2-propenyl, 2-methyl-2-propenyl, 3-butenyl, 4-pentenyl, 5-hexenyl, 2-heptenyl, 2-methyl-1-heptenyl, and 3-decenyl.

The term "alkoxy" as used herein, means an alkyl group, as defined herein, appended to the parent molecular moiety through an oxygen atom. Representative examples of alkoxy include, but are not limited to, methoxy, ethoxy, propoxy, 2-propoxy, butoxy, tert-butoxy, pentyloxy, and hexyloxy.

The term "alkoxyalkoxy" as used herein, means an alkoxy group, as defined herein, appended to the parent molecular moiety through another alkoxy group, as defined herein. Representative examples of alkoxyalkoxy include, but are not limited to, tert-butoxymethoxy, 2-ethoxyethoxy, 2-methoxyethoxy, and methoxymethoxy.

The term "alkoxyalkyl" as used herein, means an alkoxy group, as defined herein, appended to the parent molecular moiety through an alkyl group, as defined herein. Representative examples of alkoxyalkyl include, but are not limited to, tertbutoxymethyl, 2-ethoxyethyl, 2-methoxyethyl, and methoxymethyl.

The term "alkoxycarbonyl" as used herein, means an alkoxy group, as defined herein, appended to the parent molecular moiety through a carbonyl group, as defined herein. Representative examples of alkoxycarbonyl include, but are not limited to, methoxycarbonyl, ethoxycarbonyl, and tert-butoxycarbonyl.

The term "alkoxyimino" as used herein, means an alkoxy group, as defined herein, appended to the parent molecular moiety through an imino group, as defined herein. Representative examples of alkoxyimino include, but are not limited to, ethoxy(imino)methyl and methoxy(imino)methyl.

The term "alkoxysulfonyl" as used herein, means an alkoxy group, as defined herein, appended to the parent molecular moiety through a sulfonyl group, as defined herein. Representative examples of alkoxysulfonyl include, but are not limited to, methoxysulfonyl, ethoxysulfonyl, and propoxysulfonyl.

The term "alkyl" as used herein, means a straight or branched chain hydrocarbon containing from 1 to 10 carbon atoms. Representative examples of alkyl include, but are not limited to, methyl, ethyl, n-propyl, iso-propyl, n-butyl, sec-butyl, iso-butyl, tert-butyl, n-pentyl, isopentyl, neopentyl, n-hexyl, 3-methylhexyl, 2,2-dimethylpentyl, 2,3-dimethylpentyl, n-heptyl, n-octyl, n-nonyl, and n-decyl.

The term "alkylcarbonyl" as used herein, means an alkyl group, as defined herein, appended to the parent molecular moiety through a carbonyl group, as defined herein. Representative examples of alkylcarbonyl include, but are not limited to, methylcarbonyl and ethylcarbonyl.

The term "alkylsulfonyl" as used herein, means an alkyl group, as defined herein, appended to the parent molecular moiety through a sulfonyl group, as defined herein. Representative examples of alkylsulfonyl include, but are not limited to, methylsulfonyl and ethylsulfonyl.

The term "alkynyl" as used herein, means a straight or branched chain hydrocarbon group containing from 2 to 10 carbon atoms and containing at least one carbon-carbon triple bond. Representative examples of alkynyl include, but are not limited, to acetylenyl, 1-propynyl, 2-propynyl, 3-butynyl, 2-pentynyl, and 1-butynyl.

The term "amido" as used herein, means an amino, alkylamino, or dialkylamino group appended to the parent molecular moiety through a carbonyl group, as defined herein. Representative examples of amido include, but are not limited to, aminocarbonyl, methylaminocarbonyl, dimethylaminocarbonyl, and ethylmethylaminocarbonyl.

The term "amino" as used herein, means a -NH₂ group.

The term "aryl" as used herein, means a monocyclic or bicyclic aromatic ring system. Representative examples of aryl include, but are not limited to, phenyl and naphthyl.

The aryl groups of this invention are substituted with 0, 1, 2, 3, 4, or 5 substituents independently selected from acyl, acyloxy, alkanoyl, alkenyl, alkoxy, alkoxyalkoxy, alkoxyalkyl, alkoxycarbonyl, alkoxyimino, alkoxysulfonyl, alkyl, alkylsulfonyl, alkynyl, amido, carbonyl, carboxy, cyano, formyl, haloalkoxy, haloalkyl, halo, hydroxy, hydroxyalkyl, hydroxyimino, mercapto, nitro, thioalkoxy, -NR_{A}R_{B}, and (NR_{A}R_{B})sulfonyl.

The term "arylalkyl" as used herein, means at least one aryl group, as defined herein, appended to the parent molecular moiety through an alkyl group, as defined herein. Representative examples of arylalkyl include phenylmethyl, naphthylethyl, and the like.

The term "aryloxy" as used herein, means an aryl group, as defined herein, appended to the parent molecular moiety through an oxygen atom, as defined herein. Representative examples of aryloxy include, but are not limited to, phenoxy and naphthoxy.

The term "arylalkoxy" as used herein, means an aryl group, as defined herein, appended to the parent molecular moiety through an alkoxy group, as defined herein. Representative examples of arylalkoxy include, but are not limited to, phenylmethoxy, 2-phenylethoxy, 2-naphthylethoxy, and naphthylmethoxy.

The term "arylsulfonyl" as used herein, means an aryl group, as defined herein, appended to the parent molecular moiety through a sulfonyl group, as defined herein. Representative examples of arylsulfonyl include, but are not limited to, phenylsulfonyl and naphthylsulfonyl.

The term "carbonyl" as used herein, means a -C(O)- group.

The term "carboxy" as used herein, means a -CO₂H group, which may be protected as an ester group -CO₂-alkyl.

The term "cyano" as used herein, means a -CN group.

The term "cyanoalkyl" as used herein, means at least one cyano group, as defined herein, appended to the parent molecular moiety through an alkyl group, as defined herein. Representative examples of cyanoalkyl include, but are not limited to, cyanomethyl.

The term "cycloalkyl" as used herein, means a saturated cyclic hydrocarbon group containing from 3 to 8 carbons. Examples of cycloalkyl include cyclopropyl, cyclobutyl, cyclopentyl, cyclohexyl, cycloheptyl, and cyclooctyl.

The cycloalkyl groups of the invention are substituted with 0, 1, 2, 3, or 4 substituents selected from acyl, acyloxy, alkenyl, alkoxy, alkoxyalkoxy, alkoxyalkyl, alkoxycarbonyl, alkoxyimino, alkyl, alkynyl, amido, carboxy, cyano, ethylenedioxy, formyl, haloalkoxy, haloalkyl, halo, hydroxy, hydroxyalkyl, methylenedioxy, thioalkoxy, and -NR_{A}R_{B}.

The term "cycloalkylalkyl" as used herein, means a cycloalkyl group, as defined herein, appended to the parent molecular moiety through an alkyl group, as defined herein. Representative examples of cycloalkylalkyl include, but are not limited to, cyclopropylmethyl, 2-cyclobutylethyl, cyclopentylmethyl, cyclohexylmethyl, and 4-cycloheptylbutyl.

The term "ethylenedioxy" as used herein, means a -O(CH₂)₂O- group wherein the oxygen atoms of the ethylenedioxy group are attached to the parent molecular moiety through one carbon atom forming a five-membered ring or the oxygen atoms of the ethylenedioxy group are attached to the parent molecular moiety through two adjacent carbon atoms forming a six-membered ring.

The term "fluoro" as used herein means -F.

The term "fluoroalkyl" as used herein, means at least one fluoro group, as defined herein, appended to the parent molecular moiety through an alkyl group, as defined herein. Representative examples of fluoroalkyl include, but are not limited to, fluoromethyl, difluoro methyl, trifluoromethyl, pentafluoroethyl, and 2,2,2-trifluoroethyl.

The term "formyl" as used herein, means a -C(O)H group.

The term "halo" or "halogen" as used herein, means -Cl, -Br, -I or -F.

The term "haloalkoxy" as used herein, means at least one halogen, as defined herein, appended to the parent molecular moiety through an alkoxy group, as defined herein. Representative examples of haloalkoxy include, but are not limited to, chloromethoxy, 2-fluoroethoxy, trifluoromethoxy, and pentafluoroethoxy.

The term "haloalkyl" as used herein, means at least one halogen, as defined herein, appended to the parent molecular moiety through an alkyl group, as defined herein. Representative examples of haloalkyl include, but are not limited to, chloromethyl, 2-fluoroethyl, trifluoromethyl, pentafluoroethyl, and 2-chloro-3-fluoropentyl.

The term "heteroaryl," as used herein, refers to an aromatic five- or six-membered ring wherein 1, 2, 3, or 4 heteroatoms are independently selected from nitrogen, oxygen, or sulfur. Heteroaryl also refers to fused aromatic nine- and tenmembered bicyclic rings containing 1, 2, 3, or 4 heteroatoms independently selected from nitrogen, oxygen, or sulfur, or a tautomer thereof. Examples of such rings include, but are not limited to, a ring wherein one carbon is replaced with an O or S atom; one, two, or three N atoms arranged in a suitable manner to provide an aromatic ring, or a ring wherein two carbon atoms in the ring are replaced with one O or S atom and one N atom. The heteroaryl groups are connected to the parent molecular moiety through a carbon or nitrogen atom. Representative examples of heteroaryl include, but are not limited to, furyl, imidazolyl, isoxazolyl, isothiazolyl, oxadiazolyl, oxazolyl, pyrazinyl, pyrazolyl, pyridazinyl, pyridazinonyl, pyridinyl, pyrimidinyl, pyrrolyl, tetrazolyl, thiadiazolyl, thiazolyl, thienyl, triazinyl, triazolyl, indolyl, benzothiazolyl, benzofuranyl, isoquinolinyl, and quinolinyl.

The heteroaryl groups of the invention are substituted with 0, 1, 2, 3, or 4 substituents independently selected from acyl, acyloxy, alkenyl, alkoxy, alkoxyalkoxy, alkoxyalkyl, alkoxycarbonyl, alkoxyimino, alkoxysulfonyl, alkyl, alkylsulfonyl, alkynyl, amido, carboxy, cyano, formyl, haloalkoxy, haloalkyl, halo, hydroxy, hydroxyalkyl, mercapto, nitro, thioalkoxy, -NR_{A}R_{B}, and (NR_{A}R_{B})sulfonyl.

The term "heteroarylalkyl" as used herein, means at least one heteroaryl group, as defined herein, appended to the parent molecular moiety through an alkyl group, as defined herein. Representative examples of heteroarylalkyl include thienylmethyl, triazinylethyl, triazolylethyl, indolylmethyl, and the like.

The term "heterocycle," as used herein, refers to a three-, four-, five-, six-, seven-, or eight-membered ring containing one, two, or three heteroatoms independently selected from the group consisting of nitrogen, oxygen, and sulfur. Rings containing at least four members can be saturated or unsaturated. For example, the four- and five- membered ring has zero or one double bond. The six-membered ring has zero, one, or two double bonds. The seven-and eight- membered rings have zero, one, two, or three double bonds. The heterocycle groups of the invention can be attached to the parent molecular moiety through a carbon atom or a nitrogen atom. Representative examples of nitrogen-containing heterocycles include, but are not limited to, azepanyl, azetidinyl, aziridinyl, azocanyl, morpholinyl, piperazinyl, piperidinyl, pyrrolidinyl, pyrrolinyl, and thiomorpholinyl. Representative examples of non-nitrogen containing heterocycles include, but are not limited to, tetrahydrofuranyl and tetrahydropyranyl.

The heterocycles of the invention are substituted with 0, 1, 2, 3, or 4 substituents independently selected from acyl, acyloxy, alkenyl, alkoxy, alkoxyalkoxy, alkoxyalkyl, alkoxycarbonyl, alkoxyimino, alkoxysulfonyl, alkyl, alkylsulfonyl, alkynyl, amido, arylalkyl, arylalkoxycarbonyl, carboxy, cyano, formyl, haloalkoxy, haloalkyl, halo, hydroxy, hydroxyalkyl, mercapto, nitro, oxo, thioalkoxy, -NR_{A}R_{B}, and (NR_{A}R_{B})sulfonyl.

The term "heterocyclealkyl" as used herein, means at least one heteroaryl group, as defined herein, appended to the parent molecular moiety through an alkyl group, as defined herein. Representative examples of heterocyclealkyl include morpholinylmethyl, piperazinylmethyl, piperidinylethyl, pyrrolidinylethyl, and pyrrolinylethyl.

The term "hydroxy" as used herein means an -OH group.

The term "hydroxyalkyl" as used herein, means at least one hydroxy group, as defined herein, appended to the parent molecular moiety through an alkyl group, as defined herein. Representative examples of hydroxyalkyl include, but are not limited to, hydroxymethyl, 2-hydroxyethyl, 3-hydroxypropyl, 2,3-dihydroxypentyl, and 2-ethyl-4-hydroxyheptyl.

The term "hydroxy-protecting group" means a substituent which protects hydroxyl groups against undesirable reactions during synthetic procedures. Examples of hydroxy-protecting groups include, but are not limited to, methoxymethyl, benzyloxymethyl, 2-methoxyethoxymethyl, 2-(trimethylsilyl)ethoxymethyl, benzyl, triphenylmethyl, 2,2,2-trichloroethyl, t-butyl, trimethylsilyl, t-butyldimethylsilyl, t-butyldiphenylsilyl, methylene acetal, acetonide benzylidene acetal, cyclic ortho esters, methoxymethylene, cyclic carbonates, and cyclic boronates. Hydroxy-protecting groups are appended onto hydroxy groups by reaction of the compound that contains the hydroxy group with a base, such as triethylamine, and a reagent selected from an alkyl halide, alkyl triflate, trialkylsilyl halide, trialkylsilyl triflate, aryldialkylsilyltriflate, or an alkylchloroformate, CH₂I₂, or a dihaloboronate ester, for example with methyliodide, benzyl iodide, triethylsilyltriflate, acetyl chloride, benzylchloride, or dimethylcarbonate. A protecting group also may be appended onto a hydroxy group by reaction of the compound that contains the hydroxy group with acid and an alkyl acetal.

The term "mercapto" as used herein, means a -SH group.

The term "methylenedioxy" as used herein, means a -OCH₂O- group wherein the oxygen atoms of the methylenedioxy are attached to the parent molecular moiety through two adjacent carbon atoms.

The term "-NR_{A}R_{B}" as used herein, means two groups, R_{A} and R_{B}, which are appended to the parent molecular moiety through a nitrogen atom. R_{A} and R_{B} are independently selected from hydrogen, alkyl, acyl and formyl. Representative examples of -NR_{A}R_{B} include, but are not limited to, amino, methylamino, acetylamino, and acetylmethylamino.

The term "(NR_{A}R_{B})sulfonyl" as used herein, means a -NR_{A}R_{B} group, as defined herein, appended to the parent molecular moiety through a sulfonyl group, as defined herein. Representative examples of (NR_{A}R_{B})sulfonyl include, but are not limited to, aminosulfonyl, (methylamino)sulfonyl, (dimethylamino)sulfonyl and (ethylmethylamino)sulfonyl.

The term "nitro" as used herein means a -N(O)₂- group.

The term "oxo" as used herein means a -O- group.

The term "sulfonyl" as used herein means a -S(O)₂- group.

The term "thioalkoxy" as used herein means an alkyl group, as defined herein, appended to the parent molecular moiety through a sulfur atom. Representative examples of thioalkoxy include, but are not limited to, methylthio, ethylthio, and propylthio.

As used herein, the term "antagonist" encompasses and describes compounds that prevent receptor activation by an H₃ receptor agonist alone, such as histamine, and also encompasses compounds known as "inverse agonists". Inverse agonists are compou nds that not only prevent receptor activation by an H₃ receptor agonist, such as histamine, but inhibit intrinsic receptor activity.

### Compounds of the Invention

More particularly, the invention can comprise compounds of formula (II), having the form ula: 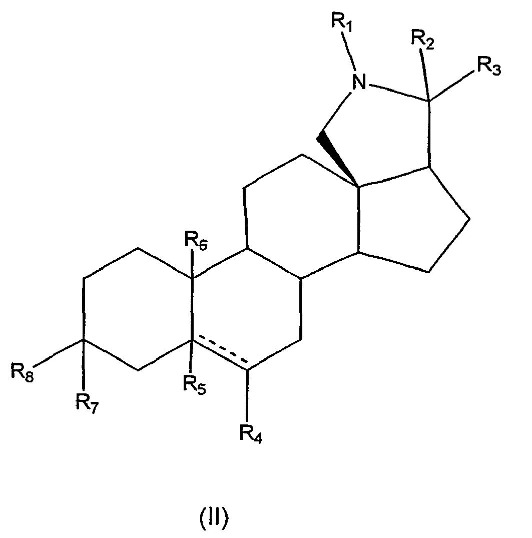 wherein:
the dotted line represents an optional bond;
R₁ is selected from the group consisting of hydrogen, acetyl, alkyl, fluoroalkyl, and cycloalkyl;
R₂ and R₃ are each independently selected from the group consisting of hydrogen and alkyl, or R₂ and R₃ taken together form a 3- to 6-membered ring;
R₄ and R₅ are each independently selected from the group consisting of hydrogen and fluorine, provided that R₅ is present only when the bond represented by the dotted line is absent;
R₆ is selected from the group consisting of hydrogen, alkyl, and fluorine;
one of R₇ and R₈ is hydrogen; and the other of R₇ and R₈ is selected from the group consisting of:
   a) NR₁₈R₁₉;
   b) SR₂₀ or O(C=O)N(R₂₀)(R₂₁)
      ; and
   c) NR₂₂(C=O)NR₂₆R₂₇, NR₂₂(C=O)CH(NR₂₈R₂₉)R₃₀, or NR₂₂(C=O)C(OR₂₃)R₃₀R_{30b}; and
   d) NR₂₂SO₂R₃₁ or NR₂₂SO₂N(R₂₂)(R₂₃);
   or R₇ and R₈ taken together with the carbon atom to which each is attached forms a
   group of the formula -C=C(Rₐ)(R_{b}), wherein Rₐ and R_{b} are each independently selected from the group consisting of hydrogen, alkyl, and cycloalkyl;
R₁₈ is hydrogen or C₁-C₆ alkyl and R₁₉ is selected from the group consisting of aryl and heteroaryl, or R₁₈ and R₁₉ at each occurrence is taken together form a 3- to 8-membered heterocycle;
R₂₀ and R₂₁ at each occurrence are each independently selected from the group consisting of hydrogen, alkyl, cycloalkyl, alkoxyalkyl, cyanoalkyl, hydroxyalkyl, haloalkyl, aryl, aryloxy, arylalkyl, arylsulfonyl, heteroaryl, heteroarylalkyl, heteroarylcarbonyl, heterocycle, and heterocyclealkyl.
R₂₂ at each occurrence is selected from the group consisting of hydrogen and alkyl;
R₂₃ at each occurrence is selected from the group consisting of hydrogen, alkyl, and alkylcarbonyl;
R₂₆ and R₂₇ are each inde pendently selected from the group consisting of alkyl, cycloalkyl, aryl, heteroaryl, and heterocycle, provided that R₂₆ and R₂₇ are not both alkyl, or R₂₆ and R₂₇ taken together with the nitrogen atom to which each is attached forms an aromatic or non-aromatic 5- to 6-membered ring, wherein 0, 1, or 2 carbon atoms in the ring is substituted with a heteroatom selected from O, S, or NR₂₃;
R₂₈ and R₂₉ at each occurrence are each independently selected from the group consisting of hydrogen, alkyl, alkylcarbonyl, alkoxycarbonyl, cycloalkyl, aryl, heteroaryl, and heterocycle, or R₂₈ and R₂₉ taken together with the nitrogen atom to which each is attached forms an aromatic or non-aromatic 5- to 6-membered ring, wherein O, 1, or 2 carbon atoms in the ring is substituted with a heteroatom selected from O, S, or NR₂₃;
R₃₀ at each occurrence is independently selected from the group consisting of hydrogen, alkyl, alkoxyalkyl, hydroxyalkyl, aryl, arylalkyl, cycloalkyl, cycloalkylalkyl, cyanoalkyl, haloalkyl, heteroaryl, heteroarylalkyl, heteroarylcarbonyl, heterocycle, and heterocyclealkyl;
R_{30b} is selected from the group consisting of hydrogen and alkyl, or when R₃₀ is alkyl and R_{30b} is alkyl, the alkyl groups can be bonded together to form a C₃-C₄ cycloalkyl group; and
R₃₁ is selected from the group consisting of alkyl, cycloalkyl, aryl, heterocycle, and heteroaryl, also are specifically contemplated. Such compounds are preferred for the method of the invention.

Preferred compounds of formula (1A), (1 B) and (II) are those wherein one of R₇ and R₈ is hydrogen; and the other of R₇ and R₈ is selected from the group consisting of NR₂₂(C=O)R₂₅ (formula IB only), NR₂₂(C=O)CH(NR₂₈R₂₉)R₃₀, and NR₂₂(C=O)C(OR₂₃)R₃₀R_{30b}.

The preferred group for R₇ or R₈ in compounds of formula (IA), (1 B), or (II) is NR₂₂(C=O)CH(NR₂₈R₂₉)R₃₀, wherein R₂₂ is alkyl, R₂₈ and R₂₉ are each hydrogen, and R₃₀ is selected from the group consisting of alkyl and aryl, particularly phenyl. Particularly, the compounds wherein R₂₂ is methyl are preferred.

Specific examples of compounds contemplated as part of the invention, include, but are not limited to, those named and shown in the Examples, not including the Reference Examples.

Unless otherwise indicated, compounds of the invention may exist as stereoisomers wherein, asymmetric or chiral centers are present. These stereoisomers are "R" or "S" depending on the configuration of substituents arou nd the chiral carbon atom. The terms "R" and "S" used herein are configurations as defined in IUPAC 1974 Recommendations for Section E, Fundamental Stereochemistry, Pure Appl. Chem., 1976, 45: 13-30. The invention contemplates various stereoisomers and mixtures thereof and are specifically included within the scope of this invention. Stereoisomers include enantiomers and diastereomers, and mixtures of enantiomers or diastereomers. Individual stereoisomers of compounds of the invention may be prepared synthetically from commercially available starting materials containing asymmetric or chiral centers or by preparation of racemic mixtures followed by resolution well-known to those of ordinary skill in the art. These methods of resolution are exemplified by (1) attachment of a mixture of enantiomers to a chiral auxiliary, separation of the resulting mixture of diastereomers by recrystallization or chromatography and optional liberation of the optically pure product from the auxiliary as described in Fumiss, Hannaford, Smith, and Tatchell, "Vogel's Textbook of Practical Organic Chemistry", 5th edition (1989), Longman Scientific & Technical, Essex CM20 2JE, Eng land, or (2) direct separation of the mixture of optical enantiomers on chiral chromatographic columns or (3) fractional recrystallization methods.

### Methods for Preparing Compounds of the Invention

The compounds of the invention can be better understood in connection with the following synthetic schemes and methods. Such description illustrates a means by which the compounds can be prepared.

As used in the descriptions of the schemes and the examples, certain abbreviations are intended to have the following meanings: Ac for acetyl; BINAP for 2,2'-bis(diphenylphosphino)-1,1'-binaphthyl; Boc (or BOC) for butyloxycarbonyl; Bu for butyl; DCM for dichloromethane; DMF for N,N-dimethylformamide; DMSO for dimethylsulfoxide; EDC for 1-[3-(dimethylamino)propyl]-3-ethyl carbodiimide; EDTA for ethylenediaminetetraacetic acid; Et for ethyl; EtOH for ethanol; EtOAc for ethyl acetate; HOBt for hydroxybenzotriazole; HPLC for high pressure liquid chromatography; Me for methyl; MeOH for methanol; Ms for methanesulfonyl; OAc for acetate; Pb/Cd for lead on cadmium; Pd/C for palladium on carbon; Ph for phenyl; PNP for paranitrophenol; tBu for tert-butyl; TEA for triethylamine; TFA for trifluoroacetic acid; THF for tetrahydrofuran; Tf for trifluoromethanesulfonyl; and Troc for 2,2,2-trichloroethylcarbonyl.

The compounds of this invention can be pre pared by a variety of synthetic procedures. Representative procedures are shown in, but are not limited to, Schemes 1-5.

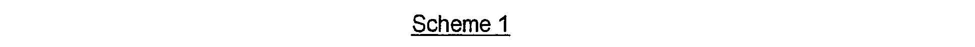

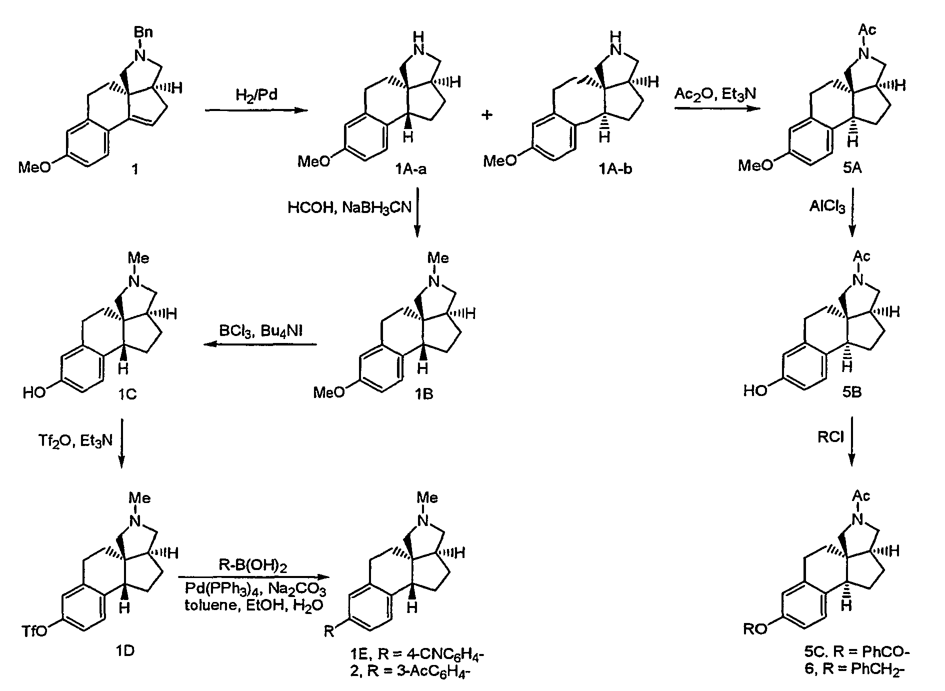

Compounds of formula 1 E, 2, 5C and 6 can be prepared as described in Scheme 1. A compound of formula 1 is prepared according to the procedure described in Kopach, M. E.; Fray, A. H. & Meyers A.I., J. Am. Chem. Soc. 1996, 118, 9876. Compound 1 can be hydrogenated under hydrogenation conditions well-known to those with skill in the art, for example hydrogen gas in the presence of a palladium catalyst. The two products 1A-a and 1A-b can be separated by column chromatography. A compound of formula 1A-a is treated with paraformaldehyde and sodium cyanoborohydride to provide a compound of formula 1B. Compound 1B is demethylated with tetrabutylammonium iodide and trich loroborane to afford a compound of formula 1C. Compound 1C is treated wth trifluoromethane sulfonic anhydride in an amine to afford a compound of formula 1 D, which is coupled with R-boronic acid, wherein R is 4-CHC₆H₄- or 3-AcC₆H₄- in the presence of tetrakis(triphenylphosphine)palladium catalyst to give compounds of formula 1 E and 2, respectively. A compound of formula 1A-b is treated with acetic anhydride and triethyl amine to provide a compound of formula 5A. A compound of formula 5A is demethylated with aluminum chloride to afford a compound of formula 5B. Compound 5B is treated with R-Cl, wherein R is Ph-CH₂- or Ph-CO- to give compounds of formula 5C and 6, respectively.

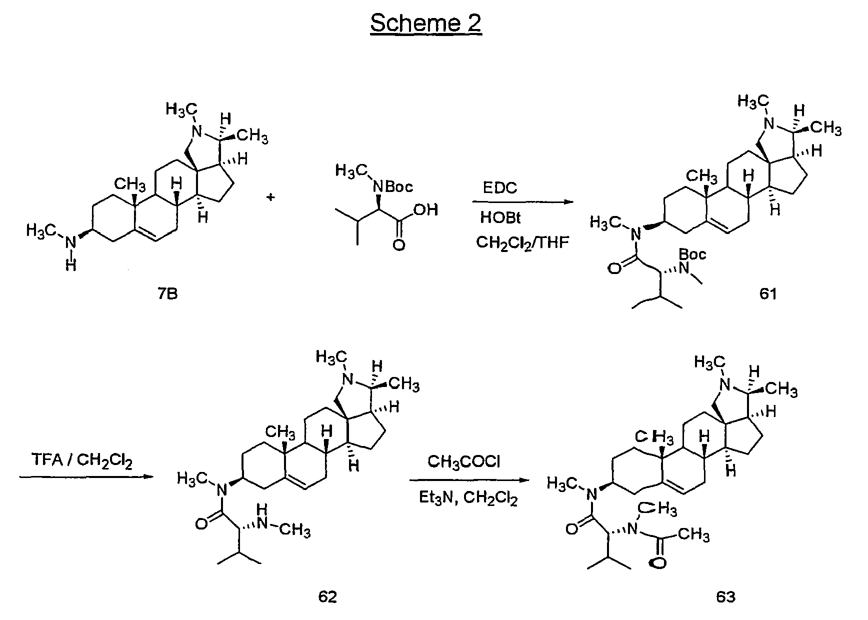

Compounds of formulas 61, 62, and 63 are prepared as described above in Scheme 2. A compound of formula 7B are treated with BOC-N-methyl-D-valine in the presence of 1-[3-(dimethylamino)propyl]-3-ethylcarbodiimide hydrochloride and 1-hydroxybenzotriazole to provide a compound of formula 61. Compound 61 is deprotected with trifluoroacetic acid to provide a compound of formula 62. Compound 62 undergoes addition by treatment with acetyl chloride to provide a compound of formula 63.

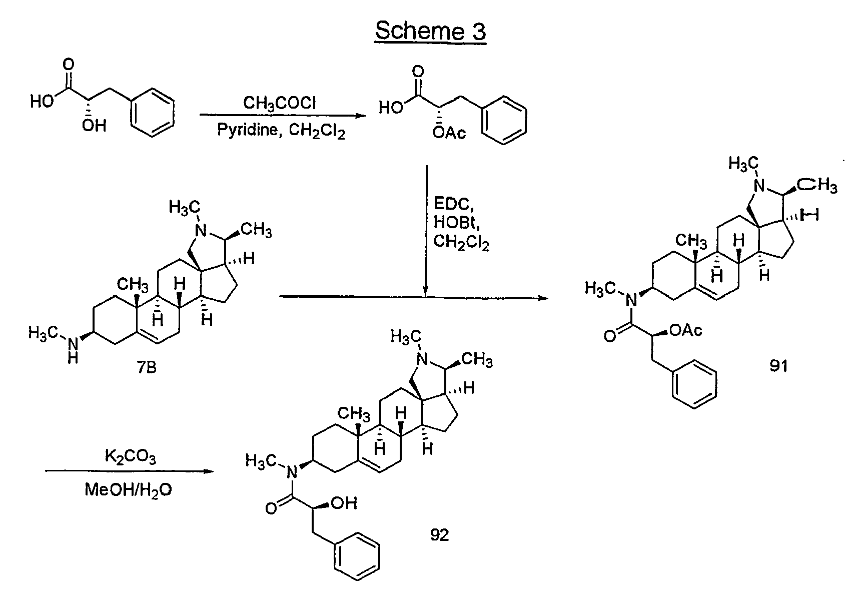

Compounds of formula 91 and 92 are prepared as described in Scheme 3. A compound of formula 7B are reacted with L-2-acetoxy-3-phenyl-propionic acid, which can be prepared from L-3-phenyllactic acid in a suspension with acetyl chloride and pyridine in a suitable solvent, for example dichloromethane, to afford a compound of formula 91. Compound 91 is hydrolyzed under basic conditions using potassium carbonate and methanol to provide a compound of formula 92.

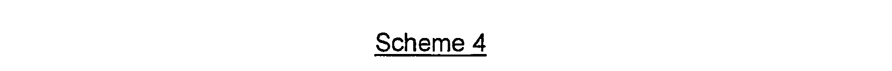

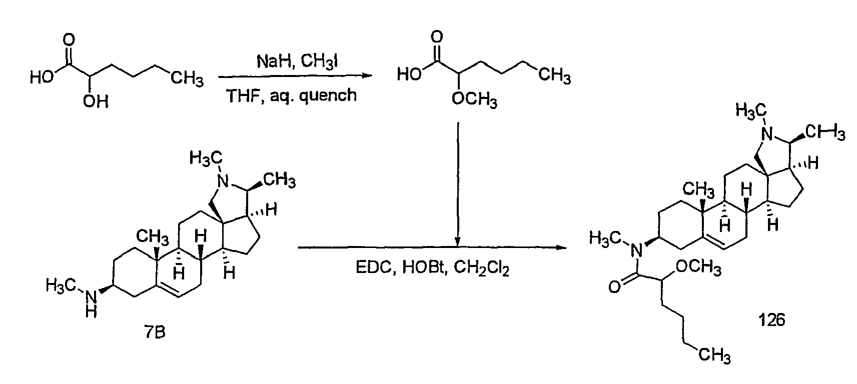

A compound of formula 126 is prepared as shown in Scheme 4. A compound of formula 7B is reacted with 2-methoxy caproic acid in the presence of 1-[3-(dimethylamino)propyl]-3-ethylcarbodiimide hydrochloride and 1-hydroxybenzotriazole to afford a compound of formula 126.

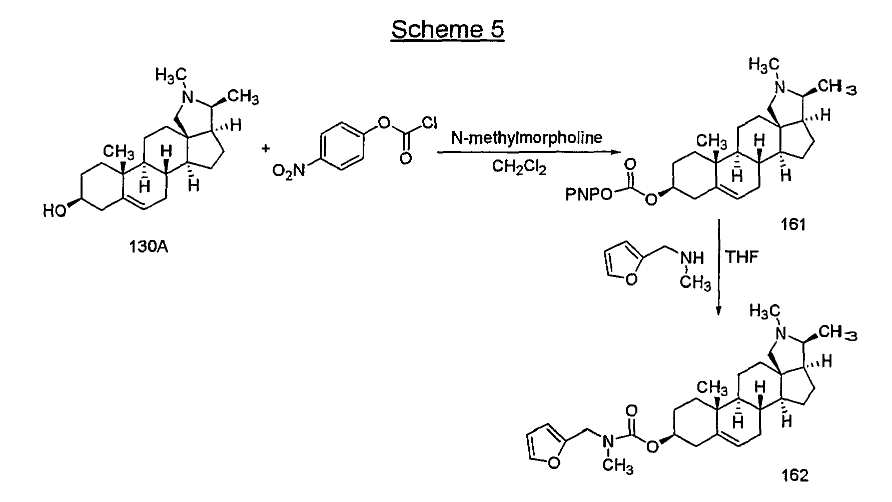

Compounds of formulas 161 and 162 are prepared as described in Scheme 6. A compound of formula 130A, which is prepared according to procedures described in Hora and Cerny; Collect. Czech. Chem. Commun. 26, 2217 (1961) and Labler et al. Collect. Czech. Chem. Commun. 28; 2015 (1963), are reacted with 4-nitrophenylchloroformate in the presence of N-methylmorpholine to provide compounds of formula 161. A compound of formula 161 is treated with N-methylfurylamine to provide a compound of formula 162.

The processes for making compounds described herein can be used in conjunction with the individual Examples to provide a variety of compounds within the scope of the claimed compounds and compounds useful for the claimed methods. In particular, suitable starting materials can be substituted in the Schemes and Examples described to provide compounds not specifically described in the Schemes and/or Examples without undue experimentation.

The compounds and intermediates of the invention may be isolated and purified by methods well-known to those skilled in the art of organic synthesis. Examples of conventional methods for isolating and purifying compounds can include, but are not limited to, chromatography on solid supports such as silica gel, alumina, or silica derivatized with alkylsilane groups, by recrystallization at high or low temperature with an optional pretreatment with activated carbon, thin-layer chromatography, distillation at various pressures, sublimation under vacuum, and trituration, as described for instance in "Vogel's Textbook of Practical Organic Chemistry", 5th edition (1989), by Fumiss, Hannaford, Smith, and Tatchell, pub. Longman Scientific & Technical, Essex CM20 2JE, England.

The compounds of the invention have at least one basic nitrogen whereby the compound can be treated with an acid to form a desired salt. For example, a compound may be reacted with an acid at or above room temperature to provide the desired salt, which is deposited, and collected by filtration after cooling. Examples of acids suitable for the reaction include, but are not limited to tartaric acid, lactic acid, succinic acid, as well as mandelic, atrolactic, methanesulfonic, ethanesulfonic, toluenesulfonic, naphthalenesulfonic, carbonic, fumaric, gluconic, acetic, propionic, salicylic, hydrochloric, hydrobromic, phosphoric, sulfuric, citric, or hydroxybutyric acid, camphorsulfonic, malic, phenylacetic, aspartic, glutamic, and the like.

### Compositions of the Invention

The invention also provides pharmaceutical compositions comprising a therapeutically effective amount of a compound of formula (IA) in combination with a pharmaceutically acceptable carrier. The compositions comprise compounds of the invention formulated together with one or more non-toxic pharmaceutically acceptable carriers. The pharmaceutical compositions can be formulated for oral administration in solid or liquid form, for parenteral injection or for rectal administration.

The term "pharmaceutically acceptable carrier," as used herein, means a non-toxic, inert solid, semi-solid or liquid filler, diluent, encapsulating material or formulation auxiliary of any type. Some examples of materials which can serve as pharmaceutically acceptable carriers are sugars such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; cocoa butter and suppository waxes; oils such as peanut oil, cottonseed oil, safflower oil, sesame oil, olive oil, corn oil and soybean oil; glycols; such a propylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol, and phosphate buffer solutions, as well as other non-toxic compatible lubricants such as sodium lauryl sulfate and magnesium stearate, as well as coloring agents, releasing agents, coating agents, sweetening, flavoring and perfuming agents, preservatives and antioxidants can also be present in the composition, according to the judgment of one skilled in the art of formulations.

The pharmaceutical compositions of this invention can be administered to humans and other mammals orally, rectally, parenterally, intracisternally, intravaginally, intraperitoneally, topically (as by powders, ointments or drops), bucally or as an oral or nasal spray. The term "parenterally," as used herein, refers to modes of administration, including intravenous, intramuscular, intraperitoneal, intrasternal, subcutaneous, intraarticular injection and infusion.

Pharmaceutical compositions for parenteral injection comprise pharmaceutically acceptable sterile aqueous or nonaqueous solutions, dispersions, suspensions or emulsions and sterile powders for reconstitution into sterile injectable solutions or dispersions. Examples of suitable aqueous and nonaqueous carriers, diluents, solvents or vehicles include water, ethanol, polyols (propylene glycol, polyethylene glycol, glycerol, and the like, and suitable mixtures thereof), vegetable oils (such as olive oil) and injectable organic esters such as ethyl oleate, or suitable mixtures thereof. Suitable fluidity of the composition may be maintained, for example, by the use of a coating such as lecithin, by the maintenance of the required particle size in the case of dispersions, and by the use of surfactants.

These compositions may also contain adjuvants such as preservative agents, wetting agents, emulsifying agents, and dispersing agents. Prevention of the action of microorganisms may be ensured by various antibacterial and antifungal agents, for example, parabens, chlorobutanol, phenol, sorbic acid, and the like. It may also be desirable to include isotonic agents, for example, sugars, sodium chloride and the like. Prolonged absorption of the injectable pharmaceutical form may be brought about by the use of agents delaying absorption, for example, aluminum monostearate and gelatin.

In some cases, in order to prolong the effect of a drug, it is often desirable to slow the absorption of the drug from subcutaneous or intramuscular injection. This may be accomplished by the use of a liquid suspension of crystalline or amorphous material with poor water solubility. The rate of absorption of the drug can depend upon its rate of dissolution, which, in turn, may depend upon crystal size and crystalline form. Alternatively, a parenterally administered drug form can be administered by dissolving or suspending the drug in an oil vehicle.

Suspensions, in addition to the active compounds, may contain suspending agents, for example, ethoxylated isostearyl alcohols, polyoxyethylene sorbitol and sorbitan esters, microcrystalline cellulose, aluminum metahydroxide, bentonite, agar-agar, tragacanth, and mixtures thereof.

If desired, and for more effective distribution, the compounds of the invention can be incorporated into slow-release or targeted-delivery systems such as polymer matrices, liposomes, and microspheres. They may be sterilized, for example, by filtration through a bacteria-retaining filter or by incorporation of sterilizing agents in the form of sterile solid compositions, which may be dissolved in sterile water or some other sterile injectable medium immediately before use.

Injectable depot forms are made by forming microencapsulated matrices of the drug in biodegradable polymers such as polylactide-polyglycolide. Depending upon the ratio of drug to polymer and the nature of the particular polymer employed, the rate of drug release can be controlled. Examples of other biodegradable polymers include poly(orthoesters) and poly(anhydrides) Depot injectable formulations also are prepared by entrapping the drug in liposomes or microemulsions which are compatible with body tissues.

The injectable formulations can be sterilized, for example, by filtration through a bacterial-retaining filter or by incorporating sterilizing agents in the form of sterile solid compositions which can be dissolved or dispersed in sterile water or other sterile injectable medium just prior to use.

Injectable preparations, for example, sterile injectable aqueous or oleaginous suspensions may be formulated according to the known art using suitable dispersing or wetting agents and suspending agents. The sterile injectable preparation may also be a sterile injectable solution, suspension or emulsion in a nontoxic, parenterally acceptable diluent or solvent such as a solution in 1,3-butanediol. Among the acceptable vehicles and solvents that may be employed are water, Ringer's solution, U.S. P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

Solid dosage forms for oral administration include capsules, tablets, pills, powders, and granules. In such solid dosage forms, one or more compounds of the invention is mixed with at least one inert pharmaceutically acceptable carrier such as sodium citrate or dicalcium phosphate and/or a) fillers or extenders such as starches, lactose, sucrose, glucose, mannitol, and salicylic acid; b) binders such as carboxymethylcellulose, alginates, gelatin, polyvinylpyrrolidinone, sucrose, and acacia; c) humectants such as glycerol; d) disintegrating agents such as agar-agar, calcium carbonate, potato or tapioca starch, alginic acid, certain silicates, and sodium carbonate; e) solution retarding agents such as paraffin; f) absorption accelerators such as quaternary ammonium compounds; g) wetting agents such as cetyl alcohol and glycerol monostearate; h) absorbents such as kaolin and bentonite clay; and i) lubricants such as talc, calcium stearate, magnesium stearate, solid polyethylene glycols, sodium lauryl sulfate, and mixtures thereof. In the case of capsules, tablets and pills, the dosage form may also comprise buffering agents.

Solid compositions of a similar type may also be employed as fillers in soft and hard-filled gelatin capsules using lactose or milk sugar as well as high molecular weight polyethylene glycols.

The solid dosage forms of tablets, dragees, capsules, pills, and granules can be prepared with coatings and shells such as enteric coatings and other coatings well known in the pharmaceutical formulating art. They may optionally contain opacifying agents and can also be of a composition that they release the active ingredient(s) only, or preferentially, in a certain part of the intestinal tract in a delayed manner. Examples of materials useful for delaying release of the active agent can include polymeric substances and waxes.

Compositions for rectal or vaginal administration are preferably suppositories which can be prepared by mixing the compounds of this invention with suitable nonirritating carriers such as cocoa butter, polyethylene glycol or a suppository wax which are solid at ambient temperature but liquid at body temperature and therefore melt in the rectum or vaginal cavity and release the active compound.

Liqu id dosage forms for oral administration include pharmaceutically acceptable emulsions, microemulsions, solutions, suspensions, syrups and elixirs. In addition to the active compounds, the liquid dosage forms may contain inert diluents commonly used in the art such as, for example, water or other solvents, solubilizing agents and emulsifiers such as ethyl alcohol, isopropyl alcohol, ethyl carbonate, ethyl acetate, benzyl alcohol, benzyl benzoate, propylene glycol, 1,3-butylene glycol, dimethylformamide, oils (in particular, cottonseed, groundnut, corn, germ, olive, castor, and sesame oils), glycerol, tetrahydrofurfuryl alcohol, polyethylene glycols and fatty acid esters of sorbitan, and mixtures thereof.

Besides inert diluents, the oral compositions can also include adjuvants such as wetting agents, emulsifying and suspending agents, sweetening, flavoring, and perfuming agents.

Dosage forms for topical or transdermal administration of a compound of this invention include ointments, pastes, creams, lotions, gels, powders, solutions, sprays, inhalants or patches. A desired compound of the invention is admixed under sterile conditions with a pharmaceutically acceptable carrier and any needed preservatives or buffers as may be required. Ophthalmic formulation, eardrops, eye ointments, powders and solutions are also contemplated as being within the scope of this invention.

The ointments, pastes, creams and gels may contain, in addition to an active compound of this invention, animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, lactose, talc, silicic acid, aluminum hydroxide, calcium silicates and polyamide powder, or mixtures of these substances. Sprays can additionally contain customary propellants such as chlorofluorohydrocarbons.

Compounds of the invention may also be administered in the form of liposomes. As is known in the art, liposomes are generally derived from phospholipids or other lipid substances. Liposomes are formed by mono- or multi-lamellar hydrated liquid crystals that are dispersed in an aqueous medium. Any non-toxic, physiologically acceptable and metabolizable lipid capable of forming liposomes may be used. The present compositions in liposome form may contain, in addition to the compounds of the invention, stabilizers, preservatives, and the like. The preferred lipids are the natural and synthetic phospholipids and phosphatidylcholines (lecithins) used separately or together.

Methods to form liposomes are known in the art. See, for example, Prescott, Ed., Methods in Cell Biology, Volume XIV, Academic Press, New York, N. Y., (1976), p 33 et seq.

Dosage forms for topical administration of a compound of this invention include powders, sprays, ointments and inhalants. The active compound is mixed under sterile conditions with a pharmaceutically acceptable carrier and any needed preservatives, buffers or propellants which can be required. Opthalmic formulations, eye ointments, powders and solutions are also contemplated as being within the scope of this invention. Aqueous liquid compositions of the invention also are particularly useful.

The compounds of the invention can be used in the form of pharmaceutically acceptable salts, esters, or amides derived from inorganic or organic acids. The term "pharmaceutically acceptable salts, esters and amides," as used herein, refer to carboxylate salts, amino acid addition salts, zwitterions, esters and amides of compounds of formula (I) which are, within the scope of sound medical judgment, suitable for use in contact with the tissues of humans and lower animals without undue toxicity, irritation, allergic response, and the like, are commensurate with a reasonable benefit/risk ratio, and are effective for their intended use.

The term "pharmaceutically acceptable salt" refers to those salts which are, within the scope of sound medical judgment, suitable for use in contact with the tissues of humans and lower animals without undue toxicity, irritation, allergic response, and the like, and are commensurate with a reasonable benefit/risk ratio. Pharmaceutically acceptable salts are well-known in the art. The salts can be prepared in situ during the final isolation and purification of the compounds of the invention or separately by reacting a free base function with a suitable organic acid.

Representative acid addition salts include, but are not limited to acetate, adipate, alginate, citrate, aspartate, benzoate, benzenesulfonate, bisulfate, butyrate, camphorate, camphorsulfonate, digluconate, glycerophosphate, hemisulfate, heptanoate, hexanoate, fumarate, hydrochloride, hydrobromide, hydroiodide, 2-hydroxyethansulfonate (isethionate), lactate, maleate, methanesulfonate, nicotinate, 2-naphthalenesulfonate, oxalate, pamoate, pectinate, persulfate, 3-phenylpropionate, picrate, pivalate, propionate, succinate, tartrate, thiocyanate, phosphate, glutamate, bicarbonate, p-toluenesulfonate and undecanoate.

Also, the basic nitrogen-containing groups can be quaternized with such agents as lower alkyl halides such as methyl, ethyl, propyl, and butyl chlorides, bromides and iodides; dialkyl sulfates such as dimethyl, diethyl, dibutyl and diamyl sulfates; long chain halides such as decyl, lauryl, myristyl and stearyl chlorides, bromides and iodides; arylalkyl halides such as benzyl and phenethyl bromides and others. Water or oil-soluble or dispersible products are thereby obtained.

Examples of acids which can be employed to form pharmaceutically acceptable acid addition salts include such inorganic acids as hydrochloric acid, hydrobromic acid, sulphuric acid and phosphoric acid and such organic acids as oxalic acid, maleic acid, succinic acid, and citric acid.

Basic addition salts can be prepared in situ during the final isolation and purification of compounds of this invention by reacting a carboxylic acid-containing moiety with a suitable base such as the hydroxide, carbonate or bicarbonate of a pharmaceutically acceptable metal cation or with ammonia or an organic primary, secondary or tertiary amine. Pharmaceutically acceptable salts include, but are not limited to, cations based on alkali metals or alkaline earth metals such as lithium, sodium, potassium, calcium, magnesium, and aluminum salts, and the like, and nontoxic quaternary ammonia and amine cations including ammonium, tetramethylammonium, tetraethylammonium, methylamine, dimethylamine, trimethylamine, triethylamine, diethylamine, ethylamine and the such as. Other representative organic amines useful for the formation of base addition salts include ethylenediamine, ethanolamine, diethanolamine, piperidine, and piperazine.

### Methods of the Invention

Compounds and compositions of formulas (IA), and (II) as described for the invention are useful for modulating the effects of histam ine-3 receptors. In particular, the compounds and compositions of the invention can be used for treating and preventing disorders modulated by the histamine-3 receptors. Typically, such disorders can be ameliorated by selectively modulating the histamine-3 receptors in a mammal, preferably by administering a compound or composition of the invention, either alone or in combination with another active agent as part of a therapeutic regimen. The compounds of formula IB are disclosed for use in the treatment of a condition related to function in a mammal selected from the group consisting of memory and cogniton processes, neurological processes and regulation of blood sugar.

The compounds of the invention, including but not limited to those specified in the examples, possess an affinity for the histamine-3 receptors. As histamine-3 receptor ligands, the compounds of the invention may be useful for the treatment and prevention of diseases or conditions such as acute myocardial infarction, Alzheimer's disease, asthma, attention-deficit hyperactivity disorder, bipolar disorder, cognitive enhancement, cognitive deficits in psychiatric disorders, deficits of memory, deficits of learning, dementia, cutaneous carcinoma, drug abuse, diabetes, type II diabetes, dep ression, epilepsy, gastrointestinal disorders, inflammation, insulin resistance syndrome, jet lag, medullary thyroid carcinoma, melanoma, Meniere's disease, metabolic syndrome, mild cognitive impairment, migraine, mood and attention alteration, motion sickness, narcolepsy, neurogenic inflammation, obesity, obsessive compulsive disorder, pain, Parkinson's disease, polycystic ovary syndrome, schizophrenia, seizures, septic shock, sleep disorders, Syndrome X, Tourette's syndrome, vertigo, and wakefulness.

The ability of the compounds of the invention, including, but not limited to, those specified in the examples, to treat septic shock and cardiovascular disorders, in particular, acute myocardial infarction may be demonstrated by Imamura et al., Circ.Res., 78:475-481 (1996); Imamura et. al., Circ.Res., 78:863-869 (1996); R. Levi and N.C.E. Smith, "Histamine H3-receptors: A new frontier in myocardial ischemia", J. Pharm. Exp. Ther., 292:825-830 (2000); and Hatta, E., K. Yasuda and R. Levi, "Activation of histamine H3 receptors inhibits carrier-mediated norepinephrine release in a human model of protracted myocardial ischemia", J. Pharm. Exp. Ther., 283:494-500 (1997).

The ability of the compounds of the invention, including, but not limited to, those specified in the examples, to treat sleep disorders, in particular, narcolepsy may be demonstrated by Lin et al., Brain Res., 523:325-330 (1990); Monti, et al., Neuropsychopharmacology 15:31-35 (1996); Sakai, et al., Life Sci., 48:2397-2404 (1991); Mazurkiewicz-Kwilecki and Nsonwah, Can. J. Physiol. Pharmacol., 67:75-78 (1989); Wada, et al., Trends in Neuroscience 14:415 (1991); and Monti, et al., Eur. J. Pharmacol. 205:283 (1991).

The ability of the compounds of the invention, including, but not limited to, those specified in the examples, to treat cognition and memory process disorders may be demonstrated by Mazurkiewicz-Kwilecki and Nsonwah, Can. J. Physiol. Pharmacol., 67:75-78 (1989); P. Panula, et al., Neuroscience, 82:993-997 (1997); Haas, et al., Behav. Brain Res., 66:41-44 (1995); De Almeida and Izquierdo, Arch. Int. Pharmacodyn., 283:193-198 (1986); Kamei et al., Psychopharmacology, 102:312-318 (1990); Kamei and Sakata, Jpn. J. Pharmacol., 57:437-482 (1991); Schwartz et al., Psychopharmacology, The fourth Generation of Progress. Bloom and Kupfer (eds). Raven Press, New York, (1995) 397; and Wada, et al., Trends in Neurosci., 14:415 (1991).

The ability of the compounds of the invention, including, but not limited to, those specified in the examples, to treat attention-deficit hyperactivity disorder (ADHD) may be demonstrated by Shaywitz et al., Psychopharmacology, 82:73-77 (1984); Dumery and Blozovski, Exp. Brain Res., 67:61-69 (1987); Tedford et al., J. Pharmacol. Exp. Ther., 275:598-604 (1995); Tedford et al., Soc. Neurosci. Abstr., 22:22 (1996); and Fox, et al., Behav. Brain Res., 131:151-161 (2002).

The ability of the compounds of the invention, including, but not limited to, those specified in the examples, to treat seizures, in particular, epilepsy may be demonstrated by Yokoyama, et al., Eur. J. Pharmacol., 234:129 (1993); Yokoyama and linuma, CNS Drugs 5:321 (1996); Onodera et al., Prog. Neurobiol., 42:685 (1994); R. Leurs, R.C. Vollinga and H. Timmerman, "The medicinal chemistry and therapeutic potential of ligands of the histamine H3 receptor", Progress in Drug Research 45:107-165, (1995); Leurs and Timmerman, Prog. Drug Res., 39:127 (1992); The Histamine H3 Receptor, Leurs and Timmerman (eds), Elsevier Science, Amsterdam, The Netherlands (1998); H. Yokoyama and K. linuma, "Histamine and Seizures: Implications for the treatment of epilepsy", CNS Drugs, 5(5):321-330 (1995); and K. Hurukami, H. Yokoyama, K. Onodera, K. linuma and T. Watanabe, "AQ-0145, A newly developed histamine H3 antagonist, decreased seizure susceptibility of electrically induced convulsions in mice", Meth. Find. Exp. Clin. Pharmacol., 17(C):70-73 (1995).

The ability of the compounds of the invention, including, but not limited to, those specified in the examples, to treat motion sickness, Alzheimer's disease, and Parkinson's disease may be demonstrated by Onodera, et al., Prog. Neurobiol., 42:685 (1994); Leurs and Timmerman, Prog. Drug Res., 39:127 (1992); and The Histamine H3 Receptor, Leurs and Timmerman (eds), Elsevier Science, Amsterdam, The Netherlands (1998).

The ability of the compounds of the invention, including, but not limited to, those specified in the examples, to treat narcolepsy, schizophrenia, depression, and dementia may be demonstrated by R. Leurs, R.C. Vollinga and H. Timmerman, "The medicinal chemistry and therapeutic potential of ligands of the histamine H3 receptor", Progress in Drug Research 45:107-165 (1995); The Histamine H3 Receptor, Leurs and Timmerman (eds), Elsevier Science, Amsterdam, The Netherlands (1998); and Perez-Garcia C, et. al., and Psychopharmacology (Berlin) 142(2):215-20 (Feb, 1999).

The ability of the compounds of the invention, including, but not limited to, those specified in the examples, to treat wakefulness, cognitive enhancement, mood and attention alteration, vertigo and motion sickness, and treatment of cognitive deficits in psychiatric disorders may be demonstrated by Schwartz, Physiol. Review 71:1-51 (1991).

The ability of the compounds of the invention, including, but not limited to, those specified in the examples, to treat mild cognitive impairment, deficits of memory, deficits of learning and dementia may be demonstrated by C. E. Tedford, in "The Histamine H3 Receptor: a target for new drugs", the Pharmacochemistry Library, vol. 30 (1998) edited by R. Leurs and H. Timmerman, Elsevier (New York). p. 269 and references also contained therein.

The ability of the compounds of the invention, including, but not limited to, those specified in the examples, to treat obesity may be demonstrated by Leurs, et al., Trends in Pharm. Sci., 19:177-183 (1998); E. Itoh, M. Fujimiay, and A. Inui, "Thioperamide, A histamine H3 receptor antagonist, powerfully suppresses peptide YY-induced food intake in rats," Biol. Psych., 45(4):475-481 (1999); S.I. Yates, et al., "Effects of a novel histamine H3 receptor antagonist, GT-2394, on food intake and weight gain in Sprague-Dawley rats," Abstracts, Society for Neuroscience, 102.10:219 (November, 2000); and C. Bjenning, et al., "Peripherally administered ciproxifan elevates hypothalamic histamine levels and potently red uces food intake in the Sprague Dawley rat," Abstracts, International Sendai Histamine Symposium, Sendai, Japan, #P39 (November, 2000).

The ability of the compounds of the invention, including, but not limited to, those specified in the examples, to treat inflammation and pain may be demonstrated by Phillips, et al., Annual Reports in Medicinal Chemistry 33:31-40 (1998).

The ability of the compounds of the invention, including, but not limited to, those specified in the examples, to treat migraine may be demonstrated by R. Leurs, R.C. Vollinga and H. Timmerman, "The medicinal chemistry and therapeutic potential of ligands of the histamine H3 receptor," Progress in Drug Research 45:107-165 (1995); Matsubara, et al., Eur. J. Pharmacol., 224: 145 (1992); and Rouleau, et al., J. Pharmacol. Exp. Ther., 281:1085 (1997).

The ability of the compounds of the invention, including, but not limited to, those specified in the examples, to treat cancer, in particular, melanoma, cutaneous carcinoma and medullary thyroid carcinoma may be demonstrated by Polish Med. Sci. Mon., 4(5):747 (1998); Adam Szelag, "Role of histamine H3-receptors in the proliferation of neoplastic cells in vitro," Med. Sci. Monit., 4(5):747-755 (1998); and C.H. Fitzsimons, et al., "Histamine receptors signalling in epidermal tumor cell lines with H-ras gene alterations," Inflammation Res., 47 (Suppl 1):S50-S51 (1998).

The ability of the compounds of the invention, including, but not limited to, those specified in the examples, to treat vestibular dysfunctions, in particular, Meniere's disease may be demonstrated by R. Leurs, R.C. Vollinga and H. Timmerman, "The medicinal chemistry and therapeutic potential of ligands of the histamine H3 receptor," Progress in Drug Research 45:107-165 (1995).

The ability of the compounds of the invention, including, but not limited to, those specified in the examples, to treat asthma may be demonstrated by A. Delaunois A., et al., "Modulation of acetylcholine, capsaicin and substance P effects by histamine H3 receptors in isolated perfused rabbit lungs," European Journal of Pharmacology 277(2-3):243-250 (1995); and Dimitriadou, et al., "Functional relationship between mast cells and C-sensitive nerve fibres evidenced by histamine H3-receptor modulation in rat lung and spleen," Clinical Science 87(2):151-163 (1994).

The ability of the compounds of the invention, including, but not limited to, those specified in the examples, to allergic rhinitis may be demonstrated by McLeod, et al., Progress in Resp. Research 31:133 (2001).

Compounds of the invention are particularly useful for treating and preventing a condition or disorder affecting the memory or cognition.

Actual dosage levels of active ingredients in the pharmaceutical compositions of this invention can be varied so as to obtain an amount of the active compound(s) which is effective to achieve the desired therapeutic response for a particular patient, compositions and mode of administration. The selected dosage level will depend upon the activity of the particular compound, the route of administration, the severity of the condition being treated and the condition and prior medical history of the patient being treated. However, it is within the skill of the art to start doses of the compound at levels lower than required to achieve the desired therapeutic effect and to gradually increase the dosage until the desired effect is achieved.

When used in the above or other treatments, a therapeutically effective amount of one of the compounds of the invention can be employed in pure form or, where such forms exist, in pharmaceutically acceptable salt, ester, amide or prodrug form. Alternatively, the compound can be administered as a pharmaceutical composition containing the compound of interest in combination with one or more pharmaceutically acceptable carriers. The phrase "therapeutically effective amount" of the compound of the invention means a sufficient amount of the compound to treat disorders, at a reasonable benefit/risk ratio applicable to any medical treatment. It will be understood, however, that the total daily usage of the compounds and compositions of the invention will be decided by the attending physician within the scope of sound medical judgment. The specific therapeutically effective dose level for any particular patient will depend upon a variety of factors including the disorder being treated and the severity of the disorder; activity of the specific compound employed; the specific composition employed; the age, body weight, general health, sex and diet of the patient; the time of administration, route of administration, and rate of excretion of the specific compound employed; the duration of the treatment; drugs used in combination or coincidental with the specific compound employed; and like factors well known in the medical arts. For example, it is well within the skill of the art to start doses of the compound at levels lower than required to achieve the desired therapeutic effect and to gradually increase the dosage until the desired effect is achieved.

The total daily dose of the compounds of this invention administered to a human or lower animal may range from about 0.003 to about 30 mg/kg/day. For purposes of oral administration, more preferable doses can be in the range of from about 0.1 to about 15 mg/kg/day. If desired, the effective daily dose can be divided into multiple doses for purposes of administration; consequently, single dose compositions may contain such amounts or submultiples thereof to make up the daily dose.

The compounds and processes of the invention will be better understood by reference to the following examples and reference examples, which are intended as an illustration of and not a limitation upon the scope of the invention.

### EXAMPLES

### Example 1

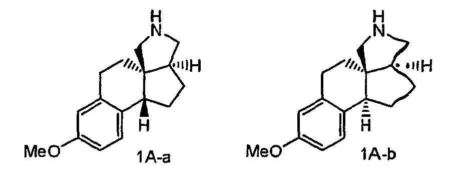

### 1A. 2-Methoxy-5,6,6a,7,8.9,10,11-octahydro-4bH-benzo[4,5]indeno[1,7a-c]pyrrole (1A-a and 1A-b)

8-Benzyl-2-methoxy-6a,7,8,9,10,11-hexahydro-6H-benzo[4,5]indeno[1,7a-c]pyrrole (prepared according to the procedure of Kopach, M. E.; Fray, A. H. and Meyers A. I., J. Am. Chem. Soc. 1996, 118, 9876)(1.0 g, 3.0 mmol) and 10% Pd/C (200 mg) were stirred in ethanol (30 mL) under hydrogen for 2h. The reaction mixture was filtered and concentrated *in vacuo* to provide 617.8 mg (84.2 %) of the title compounds 1A-a and 1A-b as a 3:1 mixture.
MS: (M+H)⁺= 244.

The mixture was separated by column chromatography on silica gel using 5% methanol and 0.5% ammonium hydroxide in dichloromethane provided pure 1A-a and 1 A-b.

1A-a: ¹H NMR (CDCl₃): δ 7.08 (d, 1H, J = 14.0 Hz), 6.73 (dd, 1H, J = 14.0, 4.5 Hz), 6.62 (d, J = 4.5 Hz), 3.77 (s, 3H), 3.01 (dd, 1H, J = 18.0, 12 Hz), 2.89 (d, 1H, J = 18 Hz), 2.74 (dd, 1H, J = 19, 5.5 Hz), 2.6- 2.83 (m, 2H), 2.60 (d, 1H, J = 19 Hz), 2.13 - 2.21 (m, 2H), 1.79 - 1.98 (m, 4H), 1.50 -1.68 (m, 2H), 1.33 (m, 1H).
MS: (M+H)⁺= 244.

1A-b: ¹H NMR (CDCl₃): δ 6.98 (dd, 1H, J = 8.7, 1.2 Hz), 6.66 (s, 1H), 6.64 (d, 1H, J = 3.0 Hz), 3.76 (s, 3H), 3.16 (dd, 1H, J = 11.5, 6.0 Hz), 3.00 (dd, 1H, J = 19, 9.0 Hz), 2.92 (dd, 1H, J = 18, 9.0 Hz), 2.89 (m, 1H), 2.84 (dd, 1H, J= 11.0, 2.0 Hz), 2.55 (d, 1H, J = 12.0 Hz), 2.45 (d, J = 12.0 Hz), 2.24 (m, 1H), 2.13 (m, 2H), 2.04 (ddd, 1H, J = 12.7, 8.2, 2.5 Hz). 1.91 (m. 1H), 1.58 (m, 1H), 1.56 (m, 1H).
MS: (M+H)⁺= 244.

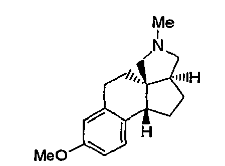

### 1B. 2-Methoxy-8-methyl-5,6,6a,7,8,9,10,11-octahydro-4bH-benzo[4,5]indeno[1,7a-c]pyrrole

2-Methoxy-5,6,6a,7,8,9,10,11-octahydro-4bH-benzo[4,5]indeno[1,7a-c]pyrrole (Example 1A, compound 1A-a) (220.0 mg, 0.90 mmol) and paraformaldehyde (810 mg, 2.7 mmol) were stirred in methanol (10 mL) at rt for 30 min. NaBH₃CN was added and stirring was continued for 30 min. The mixture was quenched with 1 N NaOH (10 mL), extracted with CH₂Cl₂ (20 mL x 4). The combined organic layers were dried over sodium sulfate, filtered and concentrated *in vacuo,* providing 230.1 mg (98.9%) of the title compound 1B as a white solid.

¹H NMR (CDCl₃): δ 7.08 (d, 1H, J = 8.4 Hz), 6.72 (dd, 1H, J = 10.5, 2.1 Hz). 6.62 (s, 1H), 3.77 (s, 3H), 2.92 (m, 1H), 2.73 (m, 1H), 2.64 (m. 2H), 2.44 (m. 1H), 2.34 (s, 3H), 2.15 (m, 2H), 2.04 (m, 1H), 1.95 (m, 1H), 1.79 (m, 1H), 1.68 (m, 1H), 1.55 (m, 3H).
MS: (M+H)⁺= 258.

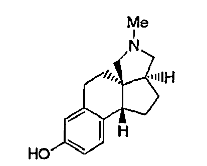

### 1C. 8-Methyl-5,6,6a,7,8,9,10,11-octahydro-4bH-benzo[4,5]indeno[1,7a-c]pyrrol-2-ol

2-Methoxy-8-methyl-5,6,6a,7,8,9,10,11-octahydro-4bH-benzo[4,5]indeno[1,7a-c]pyrrole (Example 1 B) (190.0 mg, 0.74 mmol) and tetrabutylammonium iodide (300 mg, 0.81 mmol) were dissolved in CH₂Cl₂ (20 mL) and cooled to -78°C. BCl₃ (1.85 mL, 1 M in CH₂Cl₂) was added dropwise. The mixture was stirred at 0 °C for 5 hrs. It was th en quenched with aq. satd. NaHCO₃ extracted with CH₂Cl₂ (15 mL x 4). The combine d organic layers were dried over sodium sulfate, filtered and concentrated *in vacuo.* The residue was purified by column chromatography on silica gel using 5% methanol in dichloromethane, providing 57.1 mg (31.8%) of the title compound 1C.

¹H NMR (DMSO-d₆): δ 9.10 (s, 1H), 6.96 (d, 1H, J = 18.5 Hz), 6.57 (dd, 1H, J = 14, 3.5 Hz), 6.48 (s, 3H), 1.29-3.50 (m, 14H).
MS: (M+H)⁺= 244.

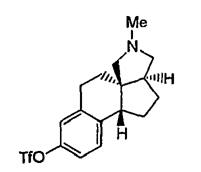

### 1D. Trifluoro-methanesulfonic acid 8-methyl-5,6,6a,7,8,9,10,11-octahydro-4bH-benzo[4,5]indeno[1,7a-c]pyrrol-2-yl ester

8-Methyl-5,6,6a,7,8,9,10,11-octahydro-4bH-benzo[4,5]indeno[1,7a-c]pyrrol-2-ol (Example 1C) (54.0 mg, 0.22 mmol) and triethylamine (42 µL, 0.30 mmol) were dissolved in CH₂Cl₂ (10 mL) and cooled to -78 °C. Trifluoromethane sulfonic anhydride (42 µL, 0.24 mmol) was added dropwise. The mixture was stirred at 0 °C for 1 hr. It was then quenched with water (2 mL), extracted with CH₂Cl₂ (10 mL x 3). The combined organic layers were dried over sodium sulfate, filtered and concentrated *in vacuo.* The residue was purified by column chromatography on silica gel using 10% methanol in dichloromethane, providing (70.0 mg (85.0%) of the title compound 1 D.
MS: (M+H)⁺= 376.

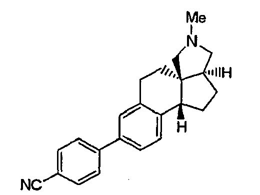

### 1E. 4-(8-Methyl-5,6,6a,7,8,9,10,11-octahydro-4bH-benzo[4,5]indeno[1,7a-]pyrrol-2-yl)-benzonitrile

Trifluoro-methanesulfonic acid 8-methyl-5,6,6a,7,8,9,10,11-octahydro-4bH-benzo[4,5]indeno[1,7a-c]pyrrol-2-yl ester (Example 1 D) (23.0 mg, 0.061 mmol) and 4-cyanophenylboronic acid (18 mg, 0.122 mmol) were dissolved in toluene (2 mL) and ethanol (0.5 mL). 0.15 mL of 1M Na₂CO₃ solution was added. Nitrogen was bubbled through the reaction mixture for 10 min. Tetrakis(triphenylphosphine)palladium (7.1 mg, 0.006 mmol) was then added. The mixture was stirred at 90 °C for 20 h. The reaction mixture was quenched with water (2 mL), extracted with CH₂Cl₂ (5 mL x 4). The combined organic layers were dried over sodium sulfate, filtered and concentrated *in vacuo.* The residue was purified by column chromatography on silica gel twice using 10% methanol in dichloromethane and 0.5% ammonium hydroxide and 5% methanol in dichloromethane, providing 6.2 mg (30.8%) of the title compound 1 E.

¹H NMR (CDCl₃): δ 7.68 (q, 4H), 7.37 (dd, 1H), 7.29 - 7.31 (m, 2H), 3.33 - 1.25 (m, 17H).
MS: (M+H)⁺= 329.

### Example 2

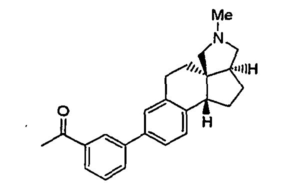

### 1-[3-(8-Methyl-5,6,6a,7,8,9,10,11-octahydro-4bH-benzo[4,5]indeno[1,7a-c]pyrrol-2-yl)-phenyl]-ethanone

Using the procedure of Example 1E, but replacing 4-cyanophenylboronic acid with 3-acetylphenylboronic acid, provided, after silica gel chromatography using 5% methanol in dichloromethane, 3.9 mg (14.1 %) of the title compound as a yellow oil.

1H NMR (CDCl₃): δ 1.53-3.05 (m, 14 H) 2.34 (s, 3 H) 2.65 (s, 3 H) 7.00 (m, 1H) 7.34 (d, *J*=1.50 Hz, 1H) 7.40 (dd, *J*=8.48, 1.50 Hz, 1H) 7.51 (t, *J*=7.80 Hz, 1H) 7.77 (d, *J*=8.48 Hz, 1 H) 7.91 (d, *J*=7.80 Hz, 1H) 8.16 (m, 1H)
MS: (M+H)⁺= 346.

### Example 3

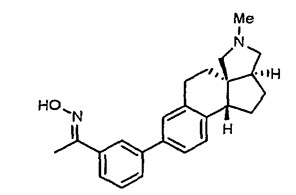

### 1-[3-(8-Methyl-5,6,6a,7,8,9,10,11-octahydro-4bH-benzo[4.5]indeno[1,7a-c]pyrrol-2-yl)-phenyl]-ethanone oxime

1-[3-(8-Methyl-5,6,6a,7,8,9,10,11-octahydro-4bH-benzo[4,5]indeno[1,7a-c]pyrrol-2-yl)-phenyl]-ethanone (Example 2) (10.0 mg, 0.028 mmol), hydroxylamine hydrochloride (2.9 mg, 0.042 mmol), and pyridine (226 µL, 0.28 mmol) were heated in ethanol (1 mL) at 80 °C for 3 h. The mixture was quenched with water (1 mL), extracted with CH₂Cl₂ (2 mL x 4). The combined organic layers were dried over sodium sulfate, filtered and concentrated *in vacuo.* The residue was purified by column chromatography on silica gel using 5% methanol in dichloromethane, providing 1.9 mg (18.3%) of the title compound.

¹H NMR (CDCl₃): δ 7.83 (s, 1H), 7.58 (m, 2H), 7.44-7.36 (m, 2 H), 7.32 (s, 1H), 7.23 (m, 1H), 3.02 (m, 1H), 2.74 (s, 3H), 2.32 (s, 3H) 2.82-1.48 (m, 13H).
MS: (M+H)⁺= 361.

### Example 4

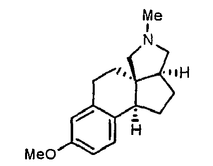

### 4A. 2-Methoxy-8-methyl-5,6,6a,7,8,9,10,11-octahydro-4bH-benzo[4,5]indeno[1,7a-c]pyrrole

Using the procedure of Example 1B but replacing the resultant compound of Example 1A (Compound 1A-a) with the resultant compound of Example 1A (Compound 1A-b), provided, after silica gel chromatography using 5% methanol and 0.5% ammonium hydroxide in dichloromethane, 102.0 mg (88.5%) of the title compound as a white solid.
¹H NMR (CDCl₃): δ 7.00 (d, 1H, *J*=9.15 Hz, 1H), 6.67 (s, 1H). 6.66 (d, *J*=6.44 Hz, 1H), 3.77 (s, 3H), 2.95 (m, 2H), 2.72 (m, 2H), 2.44 (m. 1H), 2.22 (s. 3H), 2.07-1.55 (m, 9H).
MS: (M+H)⁺= 258.

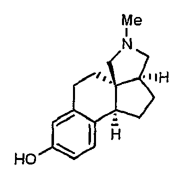

### 4B. 8-Methyl-5,6,6a,7,8,9,10,11-octahydro-4bH-benzo[4.5]indeno[1,7a-c]pyrrol-2-ol

Using the procedure of Example 1C, but replacing the resultant compound of Example 1B with the resultant compound of Example 4A, provided, after silica gel chromatography using 5% methanol and 0.5% ammonium hydroxide in dichloromethane, 26.0 mg (27.5%) of the title compound as a colorless oil.

¹H NMR (DMSO-d₆): δ 9.10 (s, 1H), 6.96 (d, 1H, J = 18.5 Hz), 6.57 (dd, 1H, J = 14, 3.5 Hz), 6.48 (s, 3H), 1.29-3.50 (m, 14H).
MS: (M+H)⁺= 244.

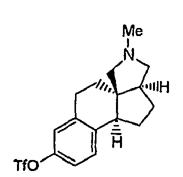

### 4C. Trifluoro-methanesulfonic acid 8-methyl-5,6,6a,7,8,9,10,11-octahydro-4bH-benzo[4.5]indeno[1,7a-c]pyrrol-2-yl ester

Using the procedure of Example 1 D, but replacing the resultant compound of Example 1C with the resultant compound of Example 4B, provided, after silica gel chromatography using 10% methanol in dichloromethane, 50.2 mg (100%) of the title compound as a colorless oil.
MS: (M+H)⁺= 376.

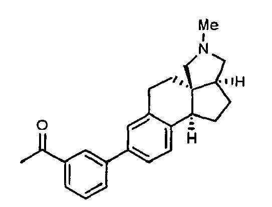

### 4D. 1-[3-(8-Methyl-5,6,6a,7,8,9,10,11-octahydro-4bH-benzo[4,5]indeno[1,7a-c]pyrrol-2-yl)-phenyl]-ethanone

Using the procedure of Example 1 E, but replacing 4-cyanophenylboronic acid with 3-acetylphenylboronic acid and replacing the resultant compound of Example 1 D with the resultant compound of Example 4C, provided, after silica gel chromatography using 5% methanol in dichloromethane, 5.3 mg (15.3%) of the title compound as a yellow oil.

1H NMR (CDCl₃): δ 2.47 (s, 3 H) 2.65 (s, 3 H) 0.85-3.14 (m, 14 H) 7.00 (m, 2 H) 7.13 (m, 1H) 7.48 (m, 1H) 7.98 (m, 1H) 8.14 (m, 1H) 8.53 (m, 1H)
MS: (M+H)⁺= 346.

### Example 5

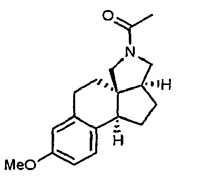

### 5A. 1-(2-Methoxy-5,6,6a,7,10,11-hexahydro-4bH-benzo[4,5]indeno[1,7a-c]pyrrol-8-yl)-ethanone

2-Methoxy-5,6,6a,7,8,9,10,11-octahydro-4bH-benzo[4,5]indeno[1,7a-c]pyrrole (Example 1A, compound 1A-b) (50.0 mg, 0.21 mmol) and triethylamine (86 µL, 0.61 mmol) were dissolved in CH₂Cl₂ (2 mL). Acetic anhydride (24 µL, 0.25 mmol) was added dropwise. The mixture was stirred at rt for 2 hr. It was then quenched with water (2 mL), and extracted with CH₂Cl₂ (5 mL x 3). The combined organic layers were dried over sodium sulfate, filtered, and concentrated *in vacuo.* The residue was purified by column chromatography on silica gel using 5% methanol in dichloromethane, providing 59.1 mg (100%) of the title compound.

1H NMR (CDCl₃): δ 1.59 (s, 3 H) 3.68 (d, *J*=12.21 Hz, 14 H) 3.77 (m, 3 H) 6.68 (m, 2 H) 6.99 (m, 1 H)
MS: (M+H)⁺= 286

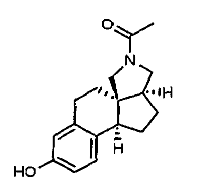

### 5B. 1-(2-Hydroxy-5,6,6a,7,10,11-hexahydro-4bH-benzo[4,5]indeno[1.7a-c]pyrrol-8-yl)-ethanone

1-(2-Methoxy-5,6,6a,7,10,11-hexahydro-4bH-benzo[4,5]indeno[1,7a-c]pyrrol-8-yl)-ethanone (Example 5A) (55.0 mg, 0.19 mmol) was dissolved in ethanethiol (4 mL) . Aluminum chloride powder (128 mg, 0.96 mmol) was added. The mixture was stirred at 0 °C for 1 h, quenched with NaHCO₃ (satd.aq., 4 mL), and extracted with CH₂Cl₂ (5 mL x 3). The combined organic layers were dried over sodium sulfate, filtered and concentrated *in vacuo.* The residue was purified by column chromatography on silica gel using 5% methanol in dichloromethane, providing 46.2 mg (88.3%) of the title compound.
MS: (M+H)⁺= 272

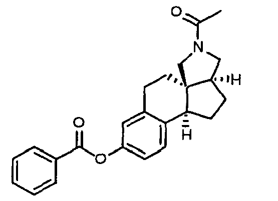

### 5C. Benzoic acid 8-acetyl-5,6,6a,7,8,9,10,11-octahydro-4bH-benzo[4,5]indeno[1,7a-c]pyrrol-2-yl ester

1-(2-H ydroxy-5,6, 6a, 7,10,11-hexahydro-4bH-benzo[4,5]indeno[1, 7a-c]pyrrol-8-yl)-ethanone (Example 5B) (10.0 mg, 0.037 mmol) and triethylamine (15.4 µL, 0.11 mmol) were dissolved in CH₂Cl₂ (2 mL). Benzoyl chloride (5.1 µL, 0.044 mmol) was added dropwise. The mixture was stirred at rt for 12 hr, quenched with water (2 mL), and extracted with CH₂Cl₂ (5 mL x 3). The combined organic layers were dried over sodium sulfate, filtered, and concentrated *in vacuo.* The residue was purified by column chromatography on silica gel using 5% methanol in dichloromethane, providing 11.1 mg (80.4%) of the title compound.

1H NMR (CDCl₃): δ 1.58 (s, 3 H) 1.72-3.43 (m, 13 H) 3.73 (m, 1 H) 6.97 (m, 2 H) 7.14 (m, 1 H) 7.52 (m, 2 H) 7.63 (m, 1 H) 8.19 (m, 2 H)
MS: (M+H)⁺= 376

### Example 6

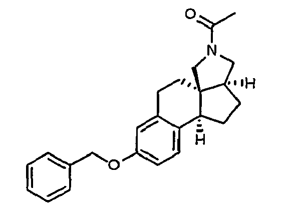

### 1-(2-Benzyloxy-5,6,6a,7,10,11-hexahydro-4bH-benzo[4,5]indeno[1,7a-c]pyrrol-8-yl)-ethanone

1-(2-Hydroxy-5,6,6a,7,10,11-hexahydro-4bH-benzo[4,5]indeno[1,7a-c]pyrrol-8-yl)-ethanone (Example 5B) (10.0 mg, 0.037 mmol) and potassium carbonate (25 mg, 0.18 mmol) were dissolved in DMF (0.5 mL). Benzyl chloride (10.2 µL, 0.088 mmol) and sodium iodide (5 mg) were added. The mixture was stirred at rt for 24 hr, quenched with water (2 mL), and extracted with CH₂Cl₂ (5 mL x 3). The combined organic layers were dried over sodium sulfate, filtered, and concentrated *in vacuo.* The residue was purified by column chromatography on silica gel using 5% methanol in dichloromethane, providing 5.5 mg (41.3%) of the title compound.

1 H NMR (CDCl₃): δ 1.57 (s, 3 H) 1.68-2.07 (m, 3 H) 2.31 (m, 2 H) 2.69 (dd, *J*=7.80, 6.44 Hz, 1 H) 2.96 (m, 3 H) 3.24 (m, 1 H) 3.46 (m, 1 H) 3.70 (m, 1 H) 5.03 (d, *J*=4.75 Hz, 2 H) 6.74 (m, 2 H) 6.99 (m, 1 H) 7.36 (m, 5 H)
MS: (M+H)⁺= 362.

### Reference Example 7

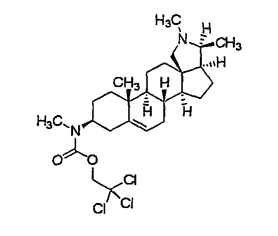

### 7A. Methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid 2,2,2-trichloro-ethyl ester

2,2,2-Trichloroethylchloroformate (5.94 g, 28 mmol) was added dropwise to a stirred solution of conessine (10 g, 28 mmol) in 200 mL benzene. Very thick gel resulted. The mixture was heated at reflux for 4 h (oil bath temperature 90 °C), then cooled down to room temperature, quenched with water, the pH adjusted with 25 mL saturated sodium bicarbonate, and extracted with dichloromethane 3X. Organic layers were dried over Na₂SO₄, filtered, and evaporated to give crude product, which was purified by flash chromatography to give desired product (8.8 g, 61 % yield).

¹H NMR (CDCl₃): δ 5.35 (m, 1 H), 4.74 (m, 2H), 3.90 (m, 1 H), 3.75 (m, 2H), 3.34 (m, 1 H), 2.92(s, 3H), 2.80 (d, 3H), 1.52 (s, 3H), 0.95 (s, 3H), 2.60-1.10 (m, 20H).
MS (DCI): (M+H)⁺= 517/519

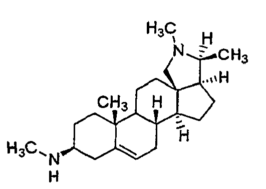

### 7B. Methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-amine

10% Pb/Cd couple* (1.2 g, 9 mmol Cd) was added to a rapidly stirred mixture of Troc-conessine (Reference Example 7A) (800mg, 1.54 mmol), THF (6 mL) and aq. 1 N NH₄OAc (6 mL). The mixture was stirred for 5 h, then another portion of 10% Pb/Cd couple (1.0g) was added, and stirred overnight. The solid was filtered, the filtrate was diluted with water, the pH adjusted with saturated sodium bicarbonate, and then extracted with dichloromethane 3X. The combined organic layer was dried over Na₂SO₄, filtered, and evaporated to give crude product, which was purified by flash chromatography using 0.5% ammonium hydroxide and 5% methanol in dichloromethane to give the desired product (397 mg, 75% yield).

### * Preparation of 10% Pb/Cd couple:

Yellow lead oxide (PbO, 108 mg, 0.49mmol) was dissolved in 5 mL 50% aq. AcOH, and the solution was slowly added to a vigorously stirred suspension of Cd dust (100 mesh, 546 mg, 4.9 mmol) in deionized water (10mL). The Cd darkened as Pb deposited on its surface, and formed clumps that were gently broken up with a glass rod. The dark, non-pyrophoric Pb/Cd couple was filtered, washed with water, then acetone, vacuum dried, crushed and stored in a closed vessel. This gives the 10% Pb/Cd couple (4.9 mmol Cd in 654 mg couple).

¹H NMR (CDCl₃): δ 5.35 (m, 1 H), 3.0 (d, 1 H), 2.45 (s, 3H), 2.22 (s, 3H), 1.05 (d, 3H), 0.93 (s, 3H), 2.40-1.00 (m, 23H).
MS (DCI): (M+H)⁺= 343

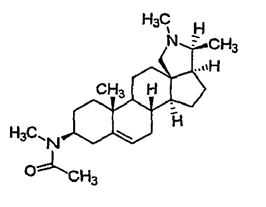

### 7C. N-Methyl-N-(2,3,11a-trimethyl-2,3,3a,4,5,5a,5b,6,8,910,11,11a,11b,12,13-hexadecahydro-1H-2-aza-pentaleno[1,6a-a]phenanthren-9-yl)-acetamide

A mixture of compound 7B (15 mg, 0.044 mmol), acetyl chloride (2.8 µL, 1.1 eq), triethylamine (15 µL, 3.0 eq) and dichloromethane (1 mL) was stirred at room temperature for 2h. The clear solution was directly loaded on silica gel column and eluted with 0.5 % ammonium hydroxide and 5% methanol in dichloromethane to give the desired product (10.5 mg, 78% yield)

¹H NMR (CDCl₃): δ 0.93 (m, 3 H) 1.08 (m, 3 H) 1.31 (m, 4 H) 1.57 (m, 3 H) 1.68 (m, 3 H) 1.87 (m, 7 H) 2.10 (m, 3 H) 2.23 (m, 3 H) 2.37 (m, 1 H) 2.52 (m, 1 H) 2.80 (m, 3 H) 2.87 (m, 3 H) 3.35 (m, 1 H) 3.73 (m, 1 H) 5.36 (m, 1 H)
MS: (M+H)⁺=385

### Reference Example 8

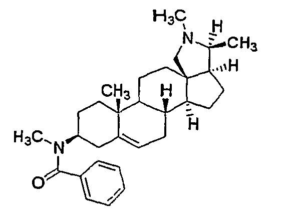

### N-Methyl-N-(2,3,11a-trimethyl-2,3,3a,4,5,5a,5b,6,8,9,10,11,11a,11b,12,13-hexadeca hydro-1H-2-aza-pentaleno[1,6a-a]phenanthren-9-yl)-benzamide

The title compound was prepared according to the procedures described in Reference Example 7C, except substituting benzoyl chloride for acetyl chloride. MS: (M+H)⁺= 447

### Example 9

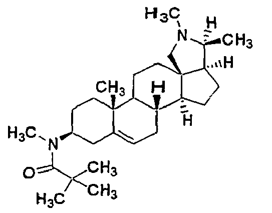

### 2,2,N-Trimethyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-propionamide

The title compound was prepared according to the procedures described in Reference Example 7C, except substituting trimethylacetyl chloride for acetyl chloride. ¹H NMR (CDCl₃): δ 0.93 (m, 3 H) 1.08 (m, 3 H) 1.26 (s, 9H) 1.31 (m, 4 H) 1.57 (m, 3 H) 1.68 (m, 3 H) 1.87 (m, 6 H) 2.10 (m, 3 H) 2.23 (m, 3 H) 2.37 (m, 1 H) 2.52 (m, 1 H) 2.86 (s, 3 H) 3.35 (m, 1 H) 3.73 (m, 1 H) 4.02 (m, 1 H) 5.36 (m, 1 H); MS: (M+H)⁺= 427.

### Example 10

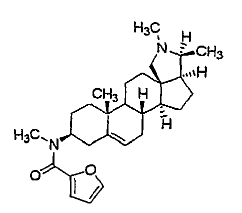

### Furan-2-carboxylic acid methyl-(2,3,11a-trimethyl-2,3,3a,4,5,5a,5b,6,8,9,10,11, 11a,11b,12,13-hexadecahydro-1H-2-aza-pentaleno[1,6a-a]phenanthren-9-yl)-amide

The title compound was prepared according to the procedures described in Reference Example 7C, except substituting 2-furoyl chloride for acetyl chloride. MS: (M+H)⁺= 437.

### Example 11

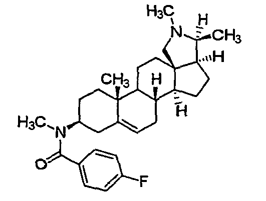

### 4-Fluoro-N-methyl-N-(2,3,11a-trimethyl-2,3,3a,4,5.5a,5b,6,8,9,10,11,11a,11b,12,13-hexadecahydro-1H-2-aza-pentaleno[1,6a-a]phenanthren-9-yl)-benzamide

The title compound was prepared according to the procedures described in Reference Example 7C, except substituting 4-fluorobenzoyl chloride for acetyl chloride. MS: (M+H)⁺= 465.

### Example 12

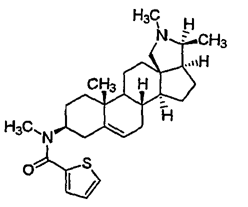

### Thiophene-2-carboxylic acid methyl-(2,3,11a-trimethyl-2,3,3a,4,5,5a,5b,6,8,9,10,11, 11a, 11b,12,13-hexadecahydro-1H-2-aza-pentaleno[1,6a-a]phenanthren-9-yl)-amide

The title compound was prepared according to the procedures described in Reference Example 7C, except substituting 2-thiophene carbonyl chloride for acetyl chloride. ¹H NMR (CDCl₃): δ 0.96 (s, 3 H) 1.23 (m, 4 H) 1.50 (d, J=6.44 Hz, 3 H) 1.79 (m, 7 H) 2.13 (m, 8 H) 2.58 (m, 2 H) 2.83 (d, J=4.07 Hz, 3 H) 3.07 (s, 3 H) 3.32 (m, 1 H) 3.88 (m, 1 H), 4.20 (m, 1 H) 5.37 (d, J=5.09 Hz, 1 H) 7.04 (m, 1H) 7.31 (m, 1 H) 7.44 (d, J=5.09 Hz, 1 H); MS: (M+H)⁺= 453.

### Example 13

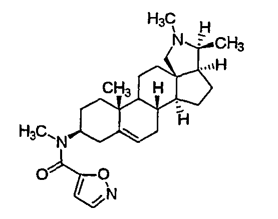

### Isoxazole-5-carboxylic acid methyl-(2,3,11a-trimethyl-2,3,3a,4,5,5a,5b,6,8,9,10,11, 11a,11b,12,13-hexadecahydro-1H-2-aza-pentaleno[1,6a-a]phenanthren-9-yl)-amide

The title compound was prepared according to the procedures described in Reference Example 7C, except substituting isoxazole-5-carbonyl chloride for acetyl chloride. MS: (M+H)⁺= 438.

### Example 14

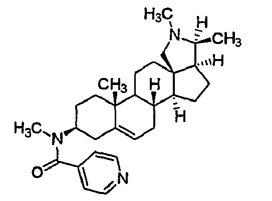

### N-Methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-isonicotinamide

The title compound was prepared according to the procedures described in Reference Example 7C, except substituting isonicotinoyl chloride hydrochloride for acetyl chloride and using 5 equivalents of triethylamine. ¹H NMR (CDCl₃): δ 0.91 (s, 3 H) 0.96 (s, 3 H) 1.00-2.75 (m, 21 H) 2.80 (m, 3 H) 3.03 (s, 3 H) 3.27 (m, 1 H) 3.71 (m, 1 H) 4.47 (m, 1 H) 5.30 (m, 1 H) 7.39 (d, J=4.07 Hz, 2 H) 8.71 (d, *J*=4.07 Hz, 2 H); MS:
(M+H)⁺= 448

### Example 15

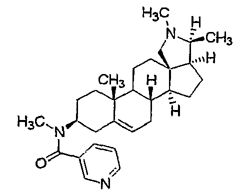

### N-Methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-nicotinamide

The title compound was prepared according to the procedures described in Reference Example 7C, except substituting nicotinoyl chloride hydrochloride for acetyl chloride and using 5 equivalents of triethylamine. MS: (M+H)⁺= 448.

### Example 16

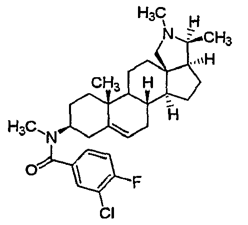

### 3-Chloro-4-fluoro-N-methyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-benzamide

The title compound was prepared according to the procedures described in Reference Example 7C, except substituting 3-chloro-4-fluorobenzoyl chloride for acetyl chloride. ¹H NMR (CDCl₃): δ 0.92 (s, 3 H) 1.18 (m, 6 H) 1.49 (s, 3 H) 1.82 (m, 7H) 2.15 (m, 7 H) 2.53 (m, 2H) 2.79 (m, 3 H) 2.92 (m, 3 H) 3.36 (m, 1 H) 3.73 (m, 1 H) 5.37 (m, 1 H) 7.15 (m, 2 H) 7.45 (m, 1 H); MS: (M+H)⁺= 499.

### Example 17

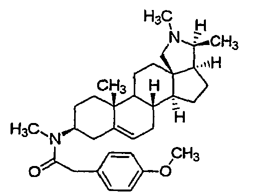

### 2-(4-Methoxy-phenyl)-N-methyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-acetamide

The title compound was prepared according to the procedures described in Reference Example 7C, except substituting 4-methoxyphenylacetyl chloride for acetyl chloride. MS: (M+H)⁺= 491.

### Example 18

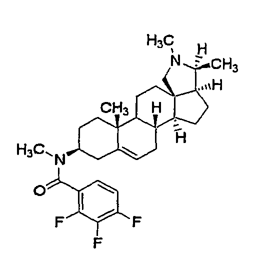

### 2,3,4-Trifluoro-N-methyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-benzamide

The title compound was prepared according to the procedures described in Reference Example 7C, except substituting 2,3,4-trifluorobenzoyl chloride for acetyl chloride. MS: (M+H)⁺= 491.

### Example 19

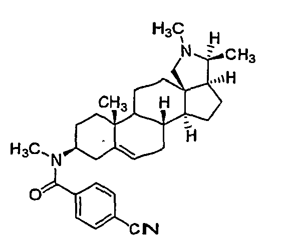

### 4-Cyano-N-methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-benzamide

The title compound was prepared according to the procedures described in Reference Example 7C, except substituting 4-cyanobenzoyl chloride for acetyl chloride. ¹H NMR (CDCl₃): δ 0.95 (s, 6 H) 1.00-2.65 (m, 22 H) 2.79 (d, *J*=4.41 Hz, 6 H) 3.02 (m, 1 H) 3.72 (m, 1 H) 5.33 (m, 1 H) 7.46 (m, 2 H) 7 - 70 (d, *J=*8.14 Hz, 2 H); MS: (M+H)⁺= 472.

### Example 20

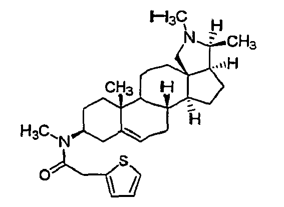

### N-Methyl-2-thiophen-2-yl-N-(2,3,11a-trimethyl-2,3,3a,4,5,5a,5b,6,8,9,10,11,11a, 11b, 12,13-hexadecahydro-1H-2-aza-pentaleno[1,6a-a]phenanthren-9-yl)-acetamide

The title compound was prepared according to the procedures described in Reference Example 7C, except substituting 2-thiopheneacetyl chloride for acetyl chloride. ¹H NMR (CDCl₃): δ 0.93 (s, 3 H) 1.04 (d, 3 H) 1.05-2.15 (m, 18 H) 2.20 (s, 3 H) 2.38 (m, 2 H) 2.50 (m, 1 H) 2.90 (d, 3 H) 3.01 (m, 1 H) 3.66 (m, 1 H) 3.90 (d, 2 H) 4.42 (m, 1 H) 5.34 (m, 1 H) 6.87 (m, 1 H) 6.95 (m, 1 H) 7.18 (m, 1 H); MS: (M+H)⁺= 467.

### Example 21

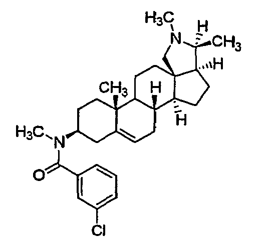

### 3-Chloro-N-methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)benzamide

The title compound was prepared according to th e procedures described in Reference Example 7C, except substituting 3-chlorobenzoyl chloride for acetyl chloride. ¹H NMR (CDCl₃): δ 0.93 (s, 3 H) 1.00-2.65 (m, 23 H) 2.80 (d, *J=*4.75 Hz, 6 H) 3.00 (m, 2 H) 3.35 (m, 1 H) 3.72 (m, 1 H) 5.30 (m, 1 H) 7.28 (m, 1 H) 7.36 (m, 3 H); MS: (M+H)⁺= 481.

### Example 22

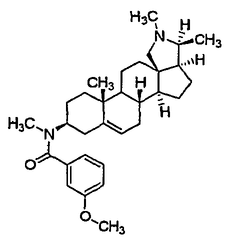

### 3-Methoxy-N-methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-benzamide

The title compound was prepared according to the procedures described in Reference Example 7C, except substituting *m*-anisoyl chloride for acetyl chloride.
MS: (M+H)⁺= 477.

### Example 23

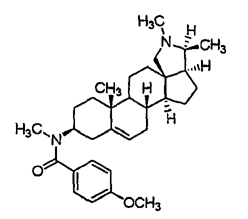

### 4-Methoxy-N-methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-benzamide

The title compound was prepared according to the procedures described in Reference Example 7C, except substituting p-anisoyl chloride for acetyl chloride. MS: (M+H)⁺= 477.

### Example 24

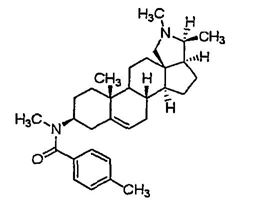

### 4,N-Dimethyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-benzamide

The title compound was prepared according to the procedures described in Reference Example 7C, except substituting p-toluoyl chlo ride for acetyl chloride. ¹H NMR (CDCl₃): δ 0.93 (s, 3 H) 1.00-2.30 (m, 21 H) 2.37 (s, 3 H) 2.54 (m, 2 H) 2.79 (s, 3 H) 2.80 (s, 3 H) 2.95 (m, 2 H) 3.31 (m, 1 H) 3.72 (m, 1 H) 5.30 (m, 1 H) 7.18 (d, *J*=7.80 Hz, 2 H) 7.27 (d, *J=*7.80 Hz, 2 H); MS: (M+H)⁺= 461.

### Example 25

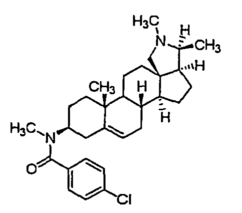

### 4-Chloro-N-methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-benzamide

The title compound was prepared according to the procedures described in Reference Example 7C, except substituting 4-chlorobenzoyl chloride for acetyl chloride. ¹H NMR (CDCl₃): δ 0.93 (s, 3 H) 1.00-2.60 (m, 23 H) 2.79 (s, 3 H) 2.80 (s, 3 H) 2.96 (m, 2 H) 3.30 (m, 1 H) 3.73 (m, 1 H) 5.30 (m, 1 H) 7.36 (m, 4 H); MS: (M+H)⁺= 482.

### Example 26

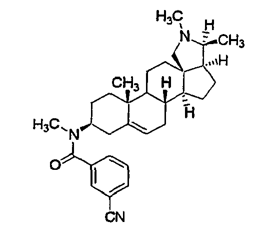

### 3-Cyano-N-methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-benzamide

The title compound was prepared according to the procedures described in Reference Example 7C, except substituting 3-cyanobenzoyl chloride for acetyl chloride. ¹H NMR (CDCl₃): δ 0.95 (s, 6 H) 1.00-2.60 (m, 20 H) 2.84 (s, 3 H) 3.02 (s, 3 H) 3.49 (m, 3 H) 4.46 (m, 1 H) 5.41 (m, 1 H) 7.53 (t, *J=*7.97 Hz, 1 H) 7.68 (m, 3 H); MS: (M+H)⁺= 471.

### Example 27

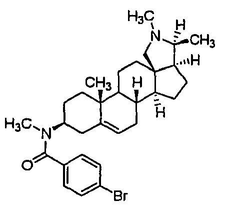

### 4-Bromo-N-methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-benzamide

The title compound was prepared according to the procedures described in Reference Example 7C, except substituting 4-bromobenzoyl chloride for acetyl chloride. ¹H NMR (CDCl₃): δ 0.93 (s, 3 H) 1.00-2.60 (m, 23 H) 2.79 (s, 3 H) 2.99 (s, 3 H) 3.35 (m, 2 H) 3.72 (m, 1 H) 4.44 (m, 1 H) 5.30 (m, 1 H) 7.23 (d, *J*=8.48 Hz, 2 H) 7.53 (d, *J*=8.48 Hz, 2 H); MS: (M+H)⁺= 527.

### Example 28

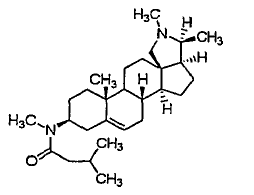

### 3,N-Dimethyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-butyramide

The title compound was prepared according to the procedures describ ed in Reference Example 7C, except substituting isovaleryl chloride for acetyl chloride. ¹H NMR (CDCl₃): δ 0.98 (m, 12 H) 1.00-2.60 (m, 23 H) 2.84 (s, 3 H) 2.87 (s, 3 H) 3.04 (m, 2 H) 3.58 (m, 1 H) 4.43 (m, 1 H) 5.38 (m, 1 H); MS: (M+H)⁺= 427.

### Example 29

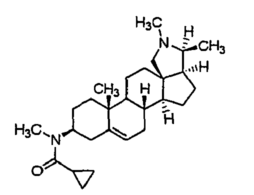

### Cyclopropanecarboxylic acid methyl-(2,3,11a-trimethyl-2,3,3a,4,5,5a,5b,6,8,9,10, 11,11a,11b,12,13-hexadecahydro-1H-2-aza-pentaleno[1,6a-a]phenanthren-9-yl)-amide

The title compound was prepared according to the procedures described in Reference Example 7C, except substituting cyclopropane carbonyl chloride for acetyl chloride. ¹H NMR (CDCl₃): δ 0.75 (m, 4 H) 0.96 (s, 3 H) 0.98 (s, 3 H) 1.00-2.60 (m, 23 H) 2.87 (s, 3 H) 3.04 (s, 3 H) 3.94 (m, 1 H) 4.40 (m, 1 H) 5.38 (m, 1 H); MS: (M+H)⁺= 411.

### Example 30

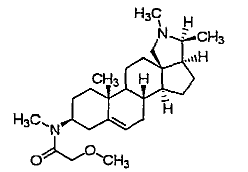

### 2-Methoxy-N-methyl-N-(2,3,11a-trimethyl-2,3,3a,4,5,5a,5b,6,8,9,10,11,11a,11b,12, 13-hexadecahydro-1H-2-aza-pentaleno[1,6a-a]phenanthren-9-yl)-acetamide

The title compound was prepared according to the procedures described in Reference Example 7C, except substituting methoxyacetyl chloride for acetyl chloride. ¹H NMR (CDCl₃): δ 0.95 (s, 3 H) 1.00-2.60 (m, 28 H) 2.87 (s, 3 H) 3.01 (m, 1 H) 3.43 (s, 3 H) 4.09 (m, 2 H) 4.38 (m, 1 H) 5.36 (m, 1 H); MS: (M+H)⁺= 415.

### Example 31

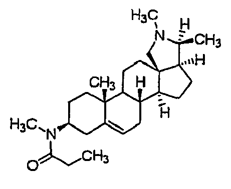

### N-Methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-propionamide

The title compound was prepared according to the procedures described in Reference Example 7C, except substituting propionyl chloride for acetyl chloride. ¹H NMR (CDCl₃): δ 0.95 (d, *J*=3.73 Hz, 3 H) 1.03 (m, 2 H) 1.14 (t, *J=*7.46 Hz, 3 H) 1.20-2.60 (m, 28 H) 2.85 (d, *J*=3.39 Hz, 3 H) 3.54 (m, 1 H) 4.41 (m, 1 H) 5.38 (m, 1 H); MS: (M+H)⁺= 399.

### Example 32

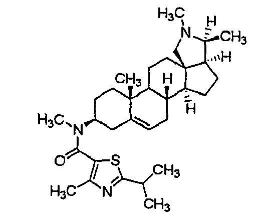

### 2-Isopropyl-4-methyl-thiazole-5-carboxylic acid methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-amide

A mixture of compound 7B (15 mg, 0.044 mmol), 2-isopropyl-4-methyl-thiazole-5-carboxylic acid (9.0 mg, 0.048 mmol), 1-[3-(dimethylamino)propyl]-3-ethylcarbodiimide hydrochloride (13 mg, 0.066 mmol), 1-hydroxybenzotriazole (9.0 mg, 0.066 mmol) and dichloromethane (1 mL) was stirred at room temperature overnight. The solvent was evaporated under reduced pressure and the residue was dissolved in minimum amount of dichloromethane and purified on silica gel column, which was eluted with 0.5% ammonium hydroxide and 5% methanol in dichloromethane to give the desired product (13 mg, 59% yield). ¹H NMR (CDCl₃): δ 0.96 (s, 3 H) 1.00-1.35 (m, 6 H) 1.38 (d, *J*=6.78 Hz, 6 H) 1.55-2.35 (m, 21 H) 2.39 (s, 3 H) 2.59 (m, 2 H) 2.95 (s, 3 H) 3.26 (m, 2 H) 5.37 (m, 1 H); MS: (M+H)⁺= 510.

### Example 33

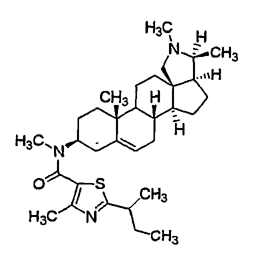

### 2-sec-Butyl-4-methyl-thiazole-5-carboxylic acid methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-amide

The title compound was prepared according to the procedures described in Example 32, except substituting 2-sec-butyl-4-methyl-thiazole-5-carboxylic acid for 2-isopropyl-4-methyl-thiazole-5-carboxylic acid. ¹H NMR (CDCl₃): δ 0.95 (m, 9 H) 1.16 (m, 9 H) 1.39 (d, *J*=6.78 Hz, 3 H) 1.60-2.60 (m, 21 H) 3.08 (m, 6 H) 5.40 (m, 1 H); MS: (M+H)⁺= 524.

### Example 34

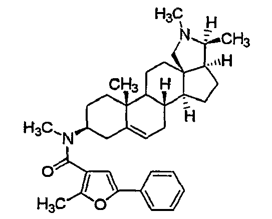

### 2-Methyl-5-phenyl-furan-3-carboxylic acid methyl-2,3,11a-trimethyl-2,3,3a,4,5, 5a,5b,6,8,9,10,11,11a,11b,12,13-hexadecahydro-1H-2-aza-pentaleno[1,6a-a]phenanthren-9-yl)-amide

The title compound was prepared according to the procedures described in Example 32, except substituting 2-methyl-5-phenyl-furan-3-carboxylic acid for 2-isopropyl-4-methyl-thiazole-5-carboxylic acid. ¹H NMR (CDCl₃): δ 0.97 (s, 3 H) 1.00-2.65 (m, 26 H) 2.44 (s, 3 H) 2.67 (m, 1 H) 2.98 (s, 3 H) 3.22 (m, 1 H) 3.79 (m, 1 H) 4.45 (m, 1 H) 5.37 (m, 1 H) 7.37 (m, 3 H) 7.62 (m, 3 H); MS: (M+H)⁺= 527.

### Example 35

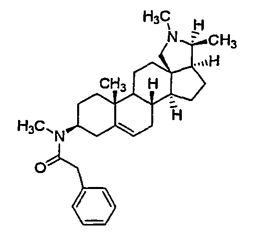

### N-Methyl-2-phenyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-acetamide

The title compound was prepared according to the procedures described in Example 32, except substituting phenylacetic acid for 2-isopropyl-4-methyl-thiazole-5-carboxylic acid. MS: (M+H)⁺= 461.

### Example 36

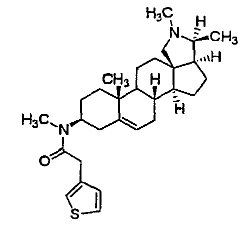

### N-Methyl-2-thiophen-3-yl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-acetamide

The title compound was prepared according to the procedures described in Example 32, except substituting 3-thiopheneacetic acid for 2-isopropyl-4-methylthiazole-5-carboxylic acid. ¹H NMR (CDCl₃): δ 0.92 (s, 3 H) 0.94 (s, 3 H) 1.00-2.60 (m, 19 H) 2.85 (d, *J*=4.07 Hz, 3 H) 3.05 (m, 1 H) 3.25 (m, 1 H) 3.59 (m, 2 H) 3.72 (d, *J*=9.49 Hz, 3 H) 4.44 (m, 1 H) 5.28 (m, 2 H) 5.35 (m, 1 H) 7.01 (d, *J*=4.75 Hz, 1 H) 7.06 (s, 1 H) 7.28 (m, 1 H); MS: (M+H)⁺= 467.

### Example 37

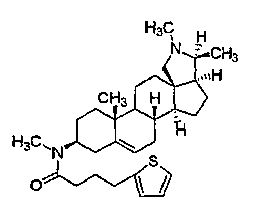

### N-Methyl-4-thiophen-2-yl-N-(2,3,11a-trimethyl-2,3,3a,4,5,5a,5b,6,8,9,10,11,11a,11b, 12,13-hexadecahydro-1H-2-aza-pentaleno[1,6a-a]phenanthren-9-yl)-butyramide

The title compound was prepared according to the procedures described in Example 32, except substituting 4-(2-thienyl)butyric acid for 2-isopropyl-4-methylthiazole-5-carboxylic acid. ¹H NMR (CDCl₃): δ 0.94 (s, 3 H) 1.00-2.60 (m, 29 H) 2.81 (s, 3 H) 2.84 (s, 3 H) 2.91 (t, *J*=7.29 Hz, 2 H) 3.44 (m, 1 H) 4.38 (m, 1 H) 5.35 (m, 1 H) 6.80 (d, *J=*3.39 Hz, 1 H) 6.92 (m, 1 H) 7.11 (d, *J*=5.09 Hz, 1 H); MS: (M+H)⁺= 495.

### Example 38

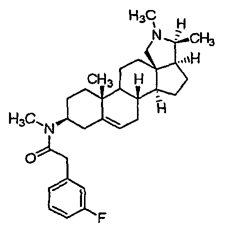

### 2-(3-Fluoro-phenyl)-N-methyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-acetamide

The title compound was prepared according to the procedures described in Example 32, except substituting 3-fluorophenylacetic acid for 2-isopropyl-4-methylthiazole-5-carboxylic acid. ¹H NMR (CDCl₃): δ 7.25 (m, 1 H), 6.95 (m, 3H), 5.30 (m, 1 H), 4.40 (m, 1H), 3.68 (d, 3H), 2.80 (s, 3H), 0.90 d, 3H), 2.70-1.00 (m, 28H); MS: (M+H)⁺= 479.

### Example 39

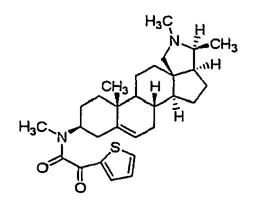

### N-Methyl-2-oxo-2-thiophen-2-yl-N-(2,3,11a-trimethyl-2,3,3a,4,5,5a,5b,6,8,9,10,11, 11a,11b,12,13-hexadecahydro-1H-2-aza-pentaleno[1,6a-a]phenanthren-9-yl)-acetamide

The title compound was prepared according to the procedures described in Example 32, except substituting 2-thiopheneglyoxylic acid for 2-isopropyl-4-methylthiazole-5-carboxylic acid. ¹H NMR (CDCl₃): δ 7.79 (m, 2H), 7.20 (m, 1 H), 5.35 (m, 1 H), 4.40 (m, 1 H), 3.02 (s, 3H), 2.90 (s, 3H), 0.93 (d, 3H), 2.60-1.00 (m, 26H); MS: (M+H)⁺= 481.

### Example 40

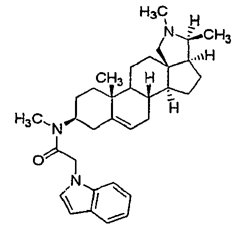

### 2-Indol-1-yl-N-methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-acetamide

The title compound was prepared according to the procedures described in Example 32, except substituting indoleacetic acid for 2-isopropyl-4-methyl-thiazole-5-carboxylic acid. ¹H NMR (CDCl₃): δ 0.90 (s, 3 H) 0.93 (s, 3 H) 1.00-2.50 (m, 24 H) 2.86 (d, *J*=2.03 Hz, 3 H) 3.00 (m, 1 H) 3.52 (m, 1 H) 4.36 (m, 1 H) 4.91 (d, *J*=10.17 Hz, 2 H) 5.30 (m, 1 H) 6.56 (s, 1 H) 7.11 (m, 2 H) 7.22 (m, 2 H) 7.61 (m, 1 H); MS: (M+H)⁺= 500.

### Example 41

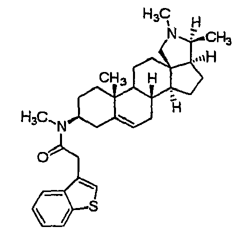

### 2-Benzo[b]thiophen-3-yl-N-methyl-N-2,3,11a-trimethyl-2,3,3a,4,5,5a,5b,6,8,9,10,11, 11a,11b,12,13-hexadecahydro-1H-2-aza-pentaleno[1,6a-a]phenanthren-9-yl)-acetamide

The title compound was prepared according to the procedures described in Example 32, except substituting benzo[b]thiophene-3-acetic acid for 2-isopropyl-4-methyl-thiazole-5-carboxylic acid. ¹H NMR (CDCl₃): δ 0.88 (s, 3 H) 0.94 (s, 3 H) 1.00-2.50 (m, 22 H) 2.87 (d, *J*=4.41 Hz, 6 H) 3.64 (m, 1 H) 3.92 (d, *J*=12.54 Hz, 2 H) 4.49 (m, 1 H) 5.30 (m, 1 H) 7.22 (m, 1 H) 7.38 (m, 2 H) 7.85 (m, 2 H); MS: (M+H)⁺= 517.

### Example 42

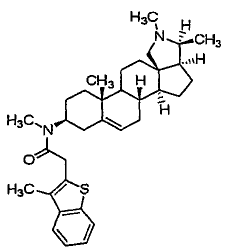

### N-Methyl-2-(3-methyl-benzo[b]thiophen-2-yl)-N-(2,3,11a-trimethyl-2,3,3a,4,5,5a,5b,6, 8,9,10,11,11a,11b,12,13-hexadecahydro-1H-2-aza-pentaleno[1,6a-a]phenanthren-9-yl)-acetamide

The title compound was prepared according to the procedures described in Example 32, except substituting 3-methylbenzo[b]thiopheneacetic acid for 2-isopropyl-4-methyl-thiazole-5-carboxylic acid. ¹H NMR (CDCl₃): δ 0.92 (d, J=8.14 Hz, 3 H) 1.00-2.50 (m, 25 H) 2.36 (d, J=6.44 Hz, 3 H) 2.86 (s, 3 H) 2.93 (s, 3 H) 3.65 (m, 1 H) 3.94 (m, 2 H) 4.37 (m, 1 H) 5.30 (m, 1 H) 7.32 (m, 2 H) 7.62 (m, 1 H) 7.74 (m, 1 H); MS: (M+H)⁺= 531.

### Example 43

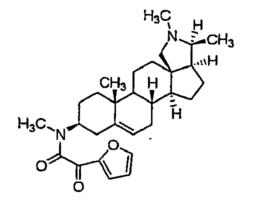

### 2-Furan-2-yl-N-methyl-2-oxo-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-acetamide

The title compound was prepared according to the procedures described in Example 32, except substituting 2-oxo-2-furanacetic acid for 2-isopropyl-4-methylthiazole-5-carboxylic acid. ¹H NMR (CDCl₃): δ 0.95 (d, *J*=11.87 Hz, 3 H) 1.00-2.60 (m, 26 H) 2.91 (s, 3 H) 3.00 (s, 3 H) 4.38 (m, 1 H) 5.30 (m, 1 H) 6.60 (dd, *J*=3.73, 1.70 Hz, 1 H) 7.33 (t, *J*=3.39 Hz, 1 H) 7.71 (s, 1 H); MS: (M+H)⁺= 465.

### Example 44

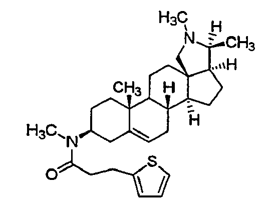

### N-Methyl-3-thiophen-2-yl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-propionamide

The title compound was prepared according to the procedures described in Example 32, except substituting 3-(2-thienyl)propanoic acid for 2-isopropyl-4-methylthiazole-5-carboxylic acid. ¹H NMR (CDCl₃): δ 0.94 (s, 3 H) 1.00-2.60 (m, 25 H) 2.66 (m, 2 H) 2.83 (s, 3 H) 2.86 (s, 3 H) 3.20 (m, 2 H) 3.53 (m, 1 H) 4.40 (m, 1 H) 5.34 (m, 1 H) 6.83 (d, *J*=3.39 Hz, 1 H) 6.92 (m, 1 H) 7.12 (d, *J*=5.42 Hz, 1 H); MS: (M+H)⁺= 481.

### Example 45

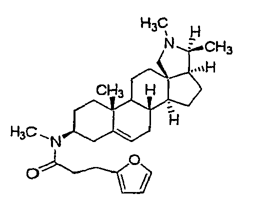

### 3-Furan-2-yl-N-methyl-N-(2,3,11a-trimethyl-2,3,3a,4,5,5a,5b,6,8,9,10,11,11a,11b,12,13-hexadecahydro-1H-2-aza-pentaleno[16,a-a]phenanthren-9-yl-propionamide

The title compound was prepared according to the procedures described in Example 32, except substituting (2-furyl)propanoic acid for 2-isopropyl-4-methylthiazole-5-carboxylic acid. ¹H NMR (CDCl₃): δ 0.95 (s, 3 H) 1.00-2.55 (m, 25 H) 2.64 (m, 2 H) 2.83 (s, 3 H) 2.86 (s, 3 H) 3.00 (m, 2 H) 3.54 (m, 1 H) 4.40 (m, 1 H) 5.35 (m, 1 H) 6.03 (t, *J*=2.88 Hz, 1 H) 6.28 (dd, *J*=3.22, 1.86 Hz, 1 H) 7.30 (s, 1 H); MS: (M+H)⁺= 465.

### Example 46

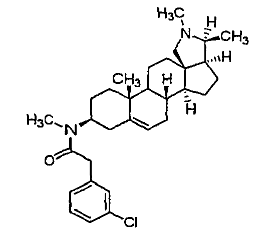

### 2-(3-Chloro-phenyl)-N-methyl-N-(2,3,11a-trimethyl-2,3,3a,4,5,5a,5b,6,8,9,10,11,11a, 11 b,12,13-hexadecahydro-1H-2-aza-pentaleno[1,6a-a]phenanthren-9-yl)-acetamide

The title compound was prepared according to the procedures described in Example 32, except substituting 3-chlorophenylacetic acid for 2-isopropyl-4-methylthiazole-5-carboxylic acid. MS: (M+H)⁺= 495/497.

### Example 47

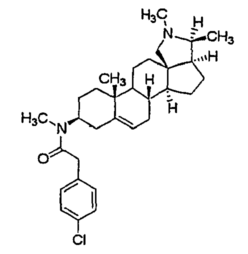

### 2-(4-Chloro-phenyl)-N-methyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-acetamide

The title compound was prepared according to the procedures described in Example 32, except substituting 4-chlorophenylacetic acid for 2-isopropyl-4-methylthiazole-5-carboxylic acid. MS: (M+H)⁺= 495/497.

### Example 48

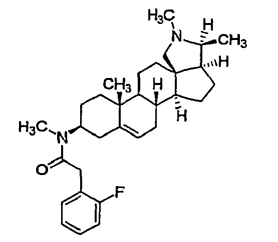

### 2-(2-Fluorophenyl)-N-methyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-acetamide

The title compound was prepared according to the procedures described in Example 32, except substituting 2-fluorophenylacetic acid for 2-isopropyl-4-methylthiazole-5-carboxylic acid. MS: (M+H)⁺= 479.

### Example 49

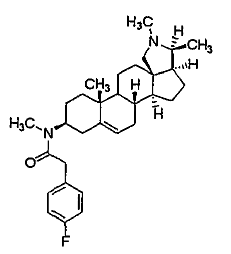

### 2-(4-Fluoro-phenyl)-N-methyl-N-(2,3,11a-trimethyl-2,3,3a,4,5,5a,5b,6,8,9,10,11,11a 11b,12,13-hexadecahydro-1H-2-aza-pentaleno[1,6a-a]phenanthren-9-yl)-acetamide

The title compound was prepared according to the procedures described in Example 32, except substituting 4-fluorophenylacetic acid for 2-isopropyl-4-methylthiazole-5-carboxylic acid. MS: (M+H)⁺= 479.

### Example 50

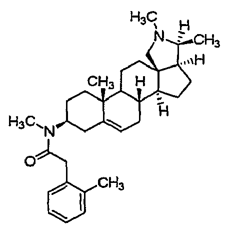

### N-Methyl-2-o-tolyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-acetamide

The title compound was prepared according to the procedures described in Example 32, except substituting o-tolylacetic acid for 2-isopropyl-4-methyl-thiazole-5-carboxylic acid. MS: (M+H)⁺= 475.

### Example 51

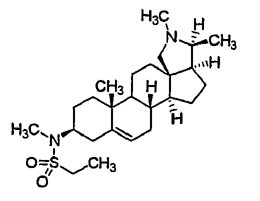

### Ethanesulfonic acid methyl-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-amide

A mixture of compound 7B (15 mg, 0.044 mmol), ethanesulfonyl chloride (5 µL, 1.5 eq), triethylamine (15 µL, 3.0 eq) and dichloromethane (1 mL) was stirred at room temperature overnight. The clear solution was directly loaded on silica gel column and eluted with 0.5% ammonium hydroxide and 5% methanol in dichloromethane to give the desired product (12.5 mg, 82% yield)

¹H NMR (CDCl₃): δ 5.35 (m, 1 H), 3.37 (m, 1 H), 2.95 (q, 2H), 2.85 (s, 3H), 2.80 (d, 3H), 1.35 (t, 3H), 0.90 (s, 3H), 2.60-1.00 (m, 20H).
MS (DCI): (M+H)⁺= 435, (M+NH₄)⁺ = 452

### Example 52

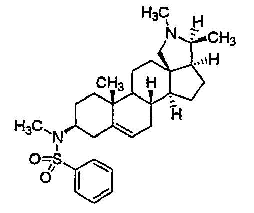

### N-Methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-benzenesulfonamide

The title compound was prepared according to the procedures described in Example 51, except substituting benzenesulfonyl chloride for ethanesulfonyl chloride. ¹H NMR (CDCl₃): δ 0.85 (s, 3 H) 0.95 (m, 1 H) 1.03 (d, 3 H) 1.10-1.45 (m, 9 H) 1.50-1.90 (m, 9 H) 2.03 (m, 1 H) 2.18 (s, 3 H) 2.34 (m, 2 H) 2.78 (s, 3 H) 2.95 (m, 1 H) 3.76 (m, 1 H) 5.22 (m, 1 H) 7.48 (m, 2 H) 7.57 (m, 1 H) 7.81 (m, 2 H); MS: (M+H)⁺= 483, (M+NH₄)⁺ = 500.

### Example 53

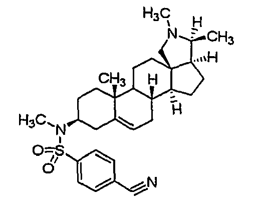

### 4-Cyano-N-methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-benzenesulfonamide

The title compound was prepared according to the procedures described in Example 51, except substituting 4-cyanobenzenesulfonyl chloride for ethanesulfonyl chloride. ¹H NMR (CDCl₃): δ 0.88 (s, 3 H) 1.00-2.40 (m, 28 H) 2.81 (s, 3 H) 2.99 (m, 1 H) 3.75 (m, 1 H) 5.25 (m, 1 H) 7.80 (m, 2 H) 7.93 (m, 2 H); MS: (M+H)⁺= 508.

### Example 54

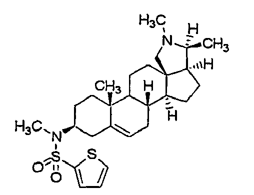

### Thiophene-2-sulfonic acid methyl-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-amide

The title compound was prepared according to the procedures described in Example 51, except substituting thiophene-2-sulfonyl chloride for ethanesulfonyl chloride. ¹H NMR (CDCl₃): δ 0.88 (s, 3 H) 1.00-2.40 (m, 29 H) 2.82 (s, 3 H) 3.77 (m, 1 H) 5.24 (m, 1 H) 7.09 (m, 1 H) 7.55 (m, 2 H); MS: (M+H)⁺= 489.

### Example 55

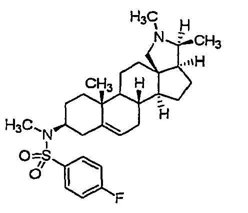

### 4-Fluoro-N-methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-benzenesulfonamide

The title compound was prepared according to the procedures described in Example 51, except substituting 4-fluorobenzenesulfonyl chloride for ethanesulfonyl chloride. ¹H NMR (CDCl₃): δ 0.88 (s, 3 H) 1.00-2.40 (m, 28 H) 2.80 (s, 3 H) 2.99 (m, 1 H) 3.73 (m, 1 H) 5.23 (m, 1 H) 7.50 (m, 2 H) 7.61 (m, 2 H); MS: (M+H)⁺= 501.

### Example 56

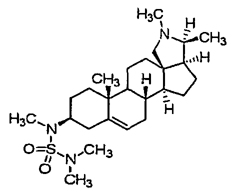

### 1,1-Dimethyl-3-methyl-3-(2,3,11a-trimethyl-2,3,3a,4,5,5a,5b,6,8,9,10,11,11a,11b,12,13-hexadecahydro-1H-2-aza-pentaleno[1,6a-a][phenanthren-9-yl)-sulfamide

The title compound was prepared according to the procedures described in Example 51, except substituting dimethylsulfomoyl chloride for ethanesulfonyl chloride. ¹H NMR (CDCl₃): δ 0.93 (s, 3 H) 1.00-2.50 (m, 25 H) 2.77 (m, 12 H) 2.98 (m, 1 H) 3.56 (m, 1 H) 5.38 (m, 1 H); MS: (M+H)⁺= 450.

### Example 57

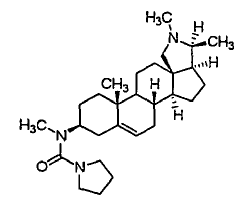

### Pyrrolidine-1-carboxylic acid methyl-(2,3,11a-trimethyl-2,3,3a,4,5,5a,5b,6,8,9,10,11, 11a,11b,12,13-hexadecahydro-1H-2-aza-pentaleno[1,6a-a]phenanthren-9-yl)-amide

A mixture of compound 7B (10.7 mg, 0.031 mmol), 1-pyrrolidinecarbonyl chloride (13 µL, 3.0 eq), triethylamine (26 µL, 6.0 eq) and dichloromethane (1 mL) was stirred at room temperature overnight. The clear solutio n was directly loaded on silica gel column and eluted with 0.5% ammonium hydroxide and 5% methanol in dichloromethane to give the desired product (12 mg, 87% yield).

¹H NMR (CDCl₃): δ 5.30 (m, 1H), 3.65 (m, 2H), 3.30(m, 4H), 2.70 (s, 3H), 0.90 (s, 3H), 2.85-1.00 (m, 32H).
MS: (M+H)⁺= 440

### Example 58

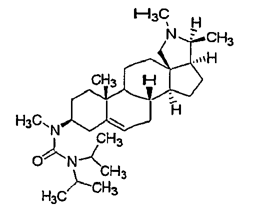

### 1,1-Diisopropyl-3-methyl-3-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-urea

The title compound was prepared according to the procedures described in Example 57, except substituting diisopropylcarbamyl chloride for 1-pyrrolidinecarbonyl chloride. ¹H NMR (CDCl₃): δ 0.91 (s, 3 H) 0.98 (m, 2 H) 1.26 (d, 12 H) 1.30-2.60 (m, 22 H) 2.64 (s, 3 H) 2.80 (d, *J*=4.75 Hz, 3 H) 3.37 (m, 2 H) 3.55 (m, 2 H) 3.72 (m, 1 H) 5.33 (m, 1 H); MS: (M+H)⁺= 470.

### Example 59

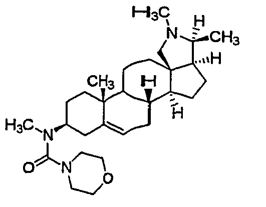

### Morpholine-4-carboxylic acid methyl-(2,3,11a-trimethyl-2,3,3a,4,5,5a,5b,6,8,9,10, 11,11a,11b,12,13-hexadecahydro-1H-2-aza-pentaleno[1,6a-a]phenanthren-9-yl)-amide

The title compound was prepared according to the procedures described in Example 57, except substituting 4-morpholinecarbonyl chloride for 1-pyrrolidinecarbonyl chloride. ¹H NMR (CDCl₃): δ 0.95 (s, 3 H) 1.00-2. 50 (m, 28 H) 2.77 (s, 3 H) 2.97 (m, 1 H) 3.20 (m, 4 H) 3.55 (m, 1 H) 3.69 (m, 4 H) 5.36 (m, 1 H); MS: (M+H)⁺= 456.

### Example 60

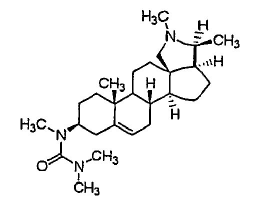

### 1,1,3-Trimethyl-3-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-urea

The title compound was prepared according to the procedures described in Example 57, except substituting dimethylcarbamyl chloride for 1-pyrrolidinecarbonyl chloride. ¹H NMR (CDCl₃): δ 0.95 (s, 3 H) 1.00-2.50 (m, 28 H) 2.72 (s, 3 H) 2.78 (s, 6 H) 2.98 (m, 1 H) 3.47 (m, 1 H) 5.35 (m, 1 H); MS: (M+H)⁺= 414.

### Example 61

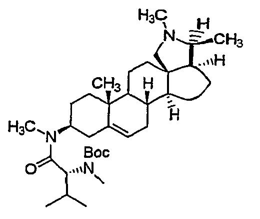

### Methyl-{2-methyl-1-[methyl-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-carbamoyl]-propyl}-carbamic acid tert-butyl ester

A mixture of compound 7B (240 mg, 0.702 mmol), Boc-N-methyl-D-valine (176 mg, 0.762 mmol), 1-[3-(dimethylamino)propyl]-3-ethylcarbodiimide hydrochloride (208 mg, 1.085 mmol), 1-hydroxybenzotriazole (146 mg, 1.085 mmol), dichloromethane (5 mL) and THF (2.5 mL) was stirred at room temperature overnight. The solvent was evaporated under reduced pressure and the residue was dissolved in minimum amount of dichloromethane and purified on silica gel column, which was eluted with 0.5% ammonium hydroxide and 5% methanol in dichloromethane to give the desired product (282 mg, 72% yield).

¹H NMR (CDCl₃): δ 0.89 (m, 12 H) 1.00-1.40 (m, 11 H) 1.46 (m, 9 H) 1.50-2.60 (m, 12 H) 2.74 (d, 3 H) 2.85 (d, 3 H) 2.94 (d, 3 H) 3.91 (m, 1 H) 4.34 (m, 1 H) 4.65 (m, 1 H) 5.38 (m, 1 H).
MS: (M+H)⁺= 556.

### Example 62

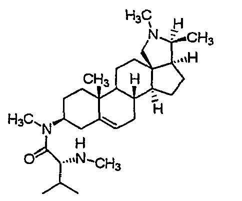

### 3,N-Dimethyl-2-methylamino-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-butyramide

Compound 61 (250 mg, 0.45 mmol) was stirred with a mixture of trifluoroacetic acid and dichloromethane (5 mL / 5 mL) for 3 hours. Solvent was removed under reduced pressure, and the residue was triturated with dichloromethane 3X. The residue was then dissolved in ethyl acetate, stirred with sodium bicarbonate powder for 5 min and filtered. The filtrate was concentrated and purified on sil ica gel column, which was eluted with 0.5% ammonium hydroxide and 5% methanol in dichloromethane to give the desired product (202 mg, 98% yield).

¹H NMR (CDCl₃): δ 0.95 (m, 12 H) 1.00-2.20 (m, 19 H) 2.25 (s, 3 H) 2.29 (s, 3H) 2.40-2.80 (m, 3 H) 2.91 (d, 3 H) 3.05 (m, 3 H) 3.65 (m, 1 H) 4.48 (m, 1 H) 5.38 (m, 1 H).
MS: (M+H)⁺= 456.

### Example 63

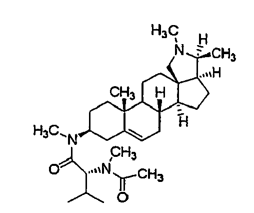

### 2-(Acetyl-methyl-amino)-3,N-dimethyl-N-(2,3,11a-trimethyl-2,3,3a,4,5,5a,5b,6,8,9,10, 11,11a,11b,12,13-hexadecahydro-1H-2-aza-pentaleno[1,6a-a]phenanthren-9-yl)-butyramide

Compound 62 (11 mg, 0.024 mmol) was dissolved in 1 mL dichloromethane. Then triethylamine (20 µL, 6 eq) was added, followed by acetyl chloride (5.2 µL, 3 eq). The mixture was stirred at room temperature overnight. The crude mixture was purified on silica gel column which was eluted with 0.3% ammonium hydroxide and 3% methanol in dichloromethane to give the desired product (6 mg, 50% yi eld).
MS: (M+H)⁺= 498.

### Example 64

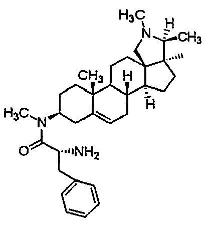

### 2-Amino-N-methyl-3-phenyl-N-(2,3,3a,11a-tetramethyl-2,3,3a,4,5,5a,5b,6,8,9,10, 11,11a,11b,12,13-hexadecahydro-1H-2-aza-pentatenon[1,6a-a]phenanthren-9-yl)-propionamide

The title compound was prepared according to the procedures described in Example 61, except substituting Boc-D-phenylalanine for Boc-N-Methyl-D-Valine and removing the BOC group as described in Example 62. MS: (M+H)⁺= 490.

### Example 65

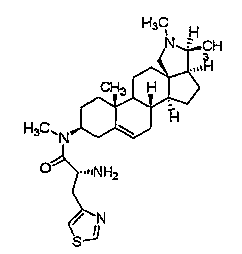

### 2-Amino-N-methyl-3-thiazol-4-yl-N-(2,3,11a-trimethyl-2,3,3a,4,5,5a,5b,6,8,9,10, 11,11a,11b,12,13-hexadecahydro-1H-2-aza-pentaleno[1,6a-a]phenanthren-9-yl)-propionamide

The title compound was prepared according to the procedures described in Example 61, except substituting Boc-D-4-thiazolylalanine for Boc-N-methyl-D-valine and removing the BOC group as described in Example 62. MS: (M+H)⁺= 497.

### Example 66

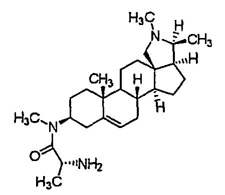

### 2-Amino-N-methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-propionamide

The title compound was prepared according to the procedures described in Example 61, except substituting Boc-D-alanine for Boc-N-methyl-D-valine and removing the BOC group as described in Example 62. MS: (M+H)⁺= 414.

### Example 67

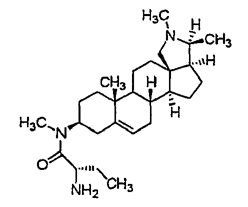

### 2-Amino-N-methyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-butyramide

The title compound was prepared according to the procedures described in Example 61, except substituting Boc-D-ethylglycine for Boc-N-methyl-D-valine and removing the BOC group as described in Example 62. MS: (M+H)⁺= 428.

### Example 68

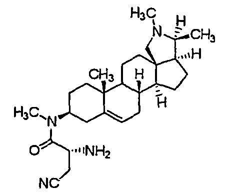

### 2-Amino-3-cyano-N-methyl-N-(2,3,11a-trimethyl-2,3,3a,4,5,5a,5b,6,8,9,10,11,11a,11b, 12,13-hexadecahydro-1H-2-aza-pentaleno[1,6a-a]phenanthren-9-yl)-propionamide

The title compound was prepared according to the procedures described in Example 61, except substituting Boc-β-cyano-D-alanine for Boc-N-methyl-D-valine and removing the BOC group as described in Example 62. MS: (M+H)⁺= 439.

### Example 69

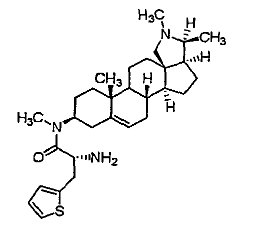

### 2-Amino-N-methyl-3-thiophen-2-yl-N-(2,3,11a-trimethyl-2,3,3a,4,5,5a,5b,6,8,9,10,11, 11a,11b,12,13-hexadecahydro-1H-2-aza-pentaleno[1,6a-a]phenanthren-9-yl)-propionamide

The title compound was prepared according to the procedures described in Example 61, except substituting Boc-β-(2-thienyl)-D-alanine for Boc-N-methyl-D-valine and removing the BOC group as described in Example 62. MS: (M+H)⁺= 496.

### Example 70

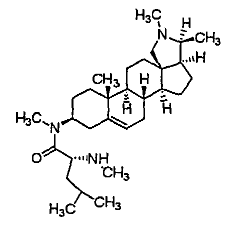

### 4-Methyl-2-methylamino-pentanoic acid methyl-(2,3,11a-trimethyl-2,3,3a,4,5,5a, 5b,6,8,9,10,11,11a,11b,12,13-hexadecahydro-1H-2-aza-pentaleno[1,6a-a]phenanthren-9-yl)-amide

The title compound was prepared according to the procedures described in Example 61, except substituting Boc-N-methyl-D-leucine for Boc-N-methyl-D-valine and removing the BOC group as described in Example 62. ¹H NMR (CDCl₃): δ 0.95 (m, 6 H) 1.00-2.90 (m, 38 H) 2.96 (s, 3 H) 3.37 (m, 1 H) 3.90 (m, 1 H) 4.25 (m, 1 H) 5.39 (m, 1 H); MS: (M+H)⁺= 470.

### Example 71

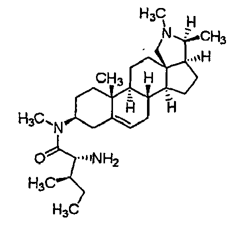

### 2-Amino-3-methyl-pentanoic acid methyl-(2,3,11a-trimethyl-2,3,3a,4,5,5a,5b,6,8,9,10, 11,11a,11b,12,13-hexadecahydro-1H-2-aza-pentaleno[1,6a-a]phenanthren-9-yl)-amide

The title compound was prepared according to the procedures described in Example 61, except substituting Boc-D-isoleucine for Boc-N-methyl-D-valine and removing the BOC group as described in Example 62. ¹H NMR (CDCl₃): δ 0.80-2.85 (m, 38 H) 2.86 (s, 3 H) 2.91 (s, 3 H) 2.99 (m, 1 H) 3.46 (m, 1 H) 3.58 (m, 1 H) 4.42 (m, 1 H) 5.37 (m, 1 H); MS: (M+H)⁺= 456.

### Example 72

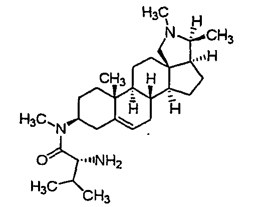

### 2-Amino-3,N-dimethyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-butyramide

The title compound was prepared according to the procedures described in Example 61, except substituting Boc-D-valine for Boc-N-methyl-D-valine and removing the BOC group as described in Example 62. MS: (M+H)⁺= 442.

### Example 73

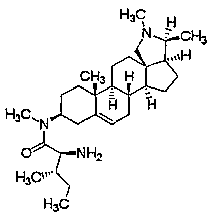

### 2-Amino-3-methyl-pentanoic acid methyl-(2,3,11a-trimethyl-2,3,3a,4,5,5a,5b,6,8,9,10, 11,11a,11b,12,13-hexadecahydro-1H-2-aza-pentaleno[1,6a-a]phenanthren-9-yl)-amide

The title compound was prepared according to the procedures described in Example 61, except substituting Boc-L-isoleucine for Boc-N-methyl-D-valine and removing the BOC group as described in Example 62. MS: (M+H)⁺= 456.

### Example 74

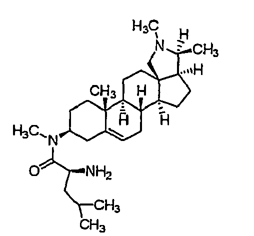

### 2-Amino-4-methyl-pentanoic acid methyl-(2,3,11a-trimethyl-2,3,3a,4,5,5a,5b,6,8,9,10, 11,11a,11b,12,13-hexadecahydro-1H-2-aza-pentateno[1,6a-a]phenanthren-9-yl)-amide

The title compound was prepared according to the procedures described in Example 61, except substituting Boc-L-leucine for Boc-N-methyl-D-valine and removing the BOC group as described in Example 62. MS: (M+H)⁺= 456.

### Example 75

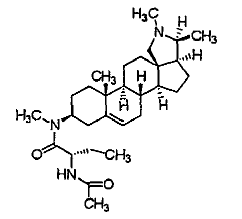

### 2-Acetylamino-N-methyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-butyramide

The title compound was prepared according to the procedures described in Example 61, except substituting Boc-D-2-ethyl-glycine for Boc-N-methyl-D-valine, and further treating according to the procedures described in Example 62 and 63. MS: (M+H)⁺= 470.

### Example 76

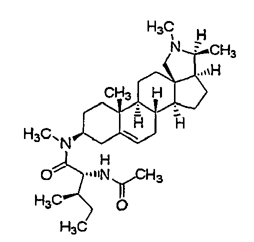

### 2-Acetylamino-3-methyl-pentanoic acid methyl-(2,3,11a-trimethyl-2,3,3a,4,5,5a,5b,6, 8.9,10,11,11a,11b,12,13-hexadecahydro-1H-2-aza-pentaleno[1,6a-a]phenanthren-9-yl)-_ amide

The title compound was prepared according to the procedures described in Example 61, except substituting Boc-D-isoleucine for Boc-N-methyl-D-valine, and further treating according to the procedures described in Example 62 and 63. MS: (M+H)⁺= 470. ¹H NMR (CDCl₃): δ 0.85-2.85 (m, 32 H) 2.00 (d, 3 H) 2.38 (m, 6 H) 2.86 (s, 3 H) 3.00 (s, 3 H) 4.35 (m, 1 H) 4.83 (m, 1 H) 5.36 (m, 1 H) 6.17 (m, 1 H); MS: (M+H)⁺= 498.

### Example 77

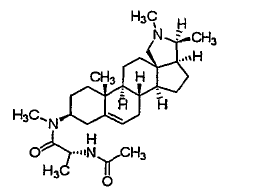

### 2-Acetylamino-N-methyl-N-(2,3,11a-trimethyl-2,3,3a,4,5,5a,5b,6,8,9,10,11,11a,11b, 12,13-hexadecahydro-1H-2-aza-pentaleno[1,6a-a]phenanthren-9-yl)-propionamide

A mixture of compound 7B (15 mg, 0.044 mmol), N-acetyl-D-alanine (7 mg, 0.053 mmol), 1-[3-(dimethylamino)propyl]-3-ethylcarbodiimide hydrochloride (13 mg, 0.068 mmol), 1-hydroxybenzotriazole (9 mg, 0.068 mmol), dichloromethane (1 mL) and THF (0.5 mL) was stirred at room temperature overnight. The solvent was evaporated under reduced pressure and the residue was dissolved in minimum amount of dichloromethane and purified on silica gel column which was eluted with 0.3 % ammonium hydroxide and 3% methanol in dichloromethane to give the desired product (13 mg, 65% yield).

¹H NMR (CDCl₃): δ 0.90-1.95(m, 27 H) 2.00 (s, 3 H) 2.05-2.75 (m, 6 H) 2.87 (s, 3 H) 2.93 (s, 3 H) 4.32 (m, 1 H) 4.83 (m, 1 H) 5.37 (m, 1 H)
MS: (M+H)⁺= 456

### Example 78

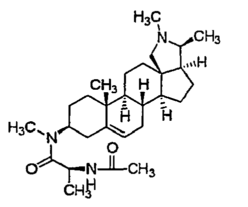

### 2-Acetylamino-N-methyl-N-(2,3,11a-trimethyl-2,3,3a,4,5,5a,5b,6,8,9,10,11,1a,11b, 12,13-hexadecahydro-1H-2-aza-pentaleno[1,6a-a]phenanthren-9-yl)-propionamide

The title compound was prepared according to the procedures described in Example 77, except substituting N-acetyl-L-alanine for N-acetyl-D-alanine. ¹H NMR (CDCl₃): δ 0.95 (m, 3 H) 1.00-2.80 (m, 28H) 2.00 (s, 3 H) 2.87 (s, 3 H) 2.93 (s, 3 H) 3.62 (m, 1 H) 4.32 (m, 1 H) 4.85 (m, 1 H) 5.37 (m, 1 H) 6.68 (m, 1 H); MS: (M+H)⁺= 456.

### Example 79

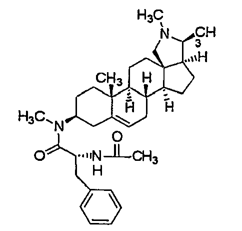

### 2-Acetylamino-N-methyl-3-phenyl-N-(2,3,11a-trimethyl-2,3,3a,4,5,5a,5b,6,8,9,10,11, 11a,11b,12,13-hexadecahydro-1H-2-aza-pentaleno[1,6a-a]phenanthren-9-yl)-propionamide

The title compound was prepared according to the procedures described in Example 77, except substituting N-acetyl-D-phenylalanine for N-acetyl-D-alanine. ¹H NMR (CDCl₃): δ 0.89 (m, 3 H) 1.04 (d, 3 H) 1.05-1.50 (m, 7 H) 1.50-1.90 (m, 9 H) 1.97 (s, 3 H) 2.07 (m, 2 H) 2.20 (s, 3 H) 2.34 (m, 2 H) 2.49 (s, 3 H) 2.78 (d, 2 H) 2.98 (m, 2 H) 3.39 (m, 1 H) 4.27 (m, 1 H) 5.15 (m, 1 H) 5.37 (m, 1 H) 6.41 (m, 1 H) 7.22 (m, 5 H). MS: (M+H)⁺= 532.

### Example 80

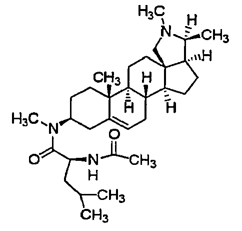

### 2-Acetylamino-4-methyl-pentanoic acid methyl-(2,3,11a-trimethyl-2,3,3a,4,5,5a,5b,6, 8,9,10,11,11a,11b,12,13-hexadecahydro-1H-2-aza-pentaleno[1,6a-a]phenanthren-9-yl)-amide

The title compound was prepared according to the procedures described in Example 77, except substituting N-acetyl-L-leucine for N-acetyl-D-alanine. ¹H NMR (CDCl₃): δ 6.30 (m, 1 H), 5.40 (m, 1 H), 5.00 (m, 1 H), 4.30 (m, 1 H), 3.60 (m, 1 H), 2.95 (s, 3H), 2.83 (s, 3H), 2.60-0.80 (m, 40H); MS: (M+H)⁺= 498.

### Example 81

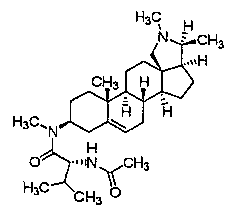

### 2-Acetylamino-3,N-dimethyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-butryamide

The title compound was prepared according to the procedures described in Example 77, except substituting N-acetyl-D-valine for N-acetyl-D-alanine. MS: (M+H)⁺= 484.

### Example 82

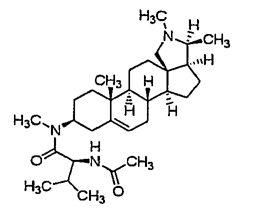

### 2-Acetylamino-3,N-dimethyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-butyramide

The title compound was prepared according to the procedures described in Example 77, except substituting N-acetyl-L-valine for N-acetyl-D-alanine. MS: (M+H)⁺= 484.

### Example 83

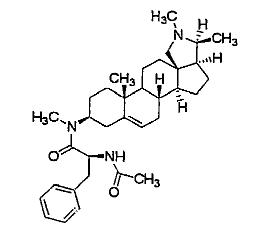

### 2-Acetylamino-N-methyl-3-phenyl-N-(2,3,11a-trimethyl-2,3,3a,4,5,5a,5b,6,8,9,10,11, 11a,11b,12,13-hexadecahydro-1H-2-aza-pentaleno[1,6a-a]phenanthren-9-yl)-propionamide

The title compound was prepared according to the procedures described in Example 77, except substituting N-acetyl-L-phenylalanine for N-acetyl-D-alanine. ¹H NMR (CDCl₃): δ 0.88 (m, 3 H) 0.95-2.50 (m, 26 H) 1.98 (s, 3 H) 2.48 (s, 3 H) 2.78 (s, 3 H) 2.97 (m, 2 H) 4.27 (m, 1 H) 5.15 (m, 1 H) 5.30 (m, 1 H) 6.39 (m, 1 H) 7.20 (m, 5 H); MS: (M+H)⁺= 532.

### Example 84

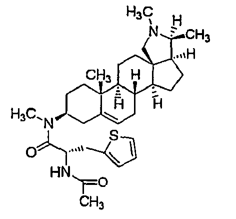

### 2-Acetylamino-N-methyl-3-thiophen-2-yl-N-(2,3,11a-trimethyl-2,3,3a,4,5,5a,5b,6,8,9, 10,11,11a,11b,12,13-hexadecahydro-1H-2-aza-pentaleno[1,6a-a]phenanthren-9-yl)-propionamide

The title compound was prepared according to the procedures described in Example 77, except substituting (S)-N-acetyl-2-(thien-2-yl)-alanine for N-acetyl-D-alanine. ¹H NMR (CDCl₃): δ 0.93 (m, 3 H) 1.04 (d, 3 H) 1.05-1.50 (m, 7 H) 1.50-1.90 (m, 9 H) 1.97 (s, 3 H) 2.07 (m, 2 H) 2.20 (s, 3 H) 2.34 (m, 2 H) 2.49 (s, 3 H) 2.78 (d, 2 H) 2.98 (m, 2 H) 3.39 (m, 1 H) 4.27 (m, 1 H) 5.15 (m, 1 H) 5.37 (m, 1 H) 6.41 (m, 1 H) 7.22 (m, 5 H); MS: (M+H)⁺= 538.

### Example 85

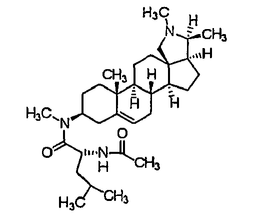

### 2-Acetylamino-4-methyl-pentanoic acid methyl-(2,3,11a-trimethyl-2,3,3a,4,5,5a,5b,6,8,9, 10,11,11a,11b,12,13-hexadecahydro-1H-2-aza-pentaleno[1,6a-a]phenanthren-9-yl)-amide

The title compound was prepared according to the procedures described in Example 77, except substituting N-acetyl-D-leucine for N-acetyl-D-alanine. ¹H NMR (CDCl₃): δ 0.88-2.60 (m, 37 H) 2.03 (s, 3 H) 2.86 (s, 3 H) 2.95 (s, 3 H) 3.62 (m, 1 H) 4.30 (m, 1 H) 4.98 (m, 1 H) 5.40 (m, 1 H) 6.30 (m, 1 H); MS: (M+H)⁺= 498.

### Example 86

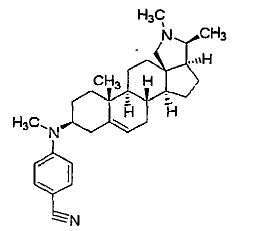

### 4₋[Methyl-(2,3,11a-trimethyl-2,3,3a,4,5,5a,5b,6,8,9,10,11,11a, 11b, 12, 13-hexadecahydro-1H-2-aza-pentaleno[1,6a-a]phenanthren-9-yl)-amino]-benzonitrile

Compound 7B (20 mg, 0.058 mmol), 4-bromobenzonitrile (16 mg, 0.088 mmol), tris(dibenzylideneacetone)dipallidium (2.1 mg, 0.0023 mmol), racemic-2,2'-bis(diphenylphosphino)-1,1'-binaphthyl (BINAP) (2.2 mg, 0.0035 mmol), cesium carbonate (29 mg, 0.088 mmol) and toluene (1 mL) were mixed and heated at 100 °C overnight. The reaction mixture was cooled, quenched with water, and extracted with dichloromethane 3X. Combined organic layer was dried over sodium sulfate, filtered and concentrated to give the crude product, which was then purified on silica gel column which was eluted with 0.3 % ammonium hydroxide and 3% methanol in dichloromethane to give the desired product (12 mg, 46% yield).

¹H NMR (CDCl₃): δ 0.85-2.80(m, 32 H) 2.88 (s, 3 H) 3.63 (m, 1 H) 5.39 (m, 1 H) 6.69 (d, *J*=8.80 Hz, 2 H) 7.44 (d, *J*=8.80 Hz, 2 H)
MS: (M+H)⁺= 444

### Example 87

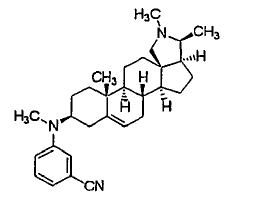

### 3-[Methyl-(2,3,11a-trimethyl-2,3,3a,5,5a,5b,6,8,9,10,11,11a,11b,12,13-hexadecahydro-1H-2-aza-pentaleno[1,6a-a]phenanthren-9-yl)-amino]-benzonitrile

The title compound was prepared according to the procedures described in Example 86, except substituting 3-bromobenzonitrile for 4-bromobenzonitrile. ¹H NMR (CDCl₃): δ 0.99 (s, 3H) 1.05-2.50 (m, 28H) 2.83 (s, 3H) 3.00 (m, 1 H) 3.55(m, 1 H) 5.38 (m, 1 H) 6.92 (m, 2H) 7.25 (m, 2H)
MS: (M+H)⁺= 444

### Example 88

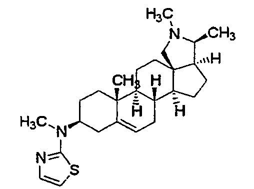

### Methyl-thiazol-2-yl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-amine

The title compound was prepared according to the procedures described in Example 86, except substituting 2-bromothiazole for 4-bromobenzonitrile. ¹H NMR (CDCl₃): δ 0.99 (s, 3H) 1.03-2.55 (m, 29H) 3.00 (s, 3H) 3.85 (m, 1 H) 5.40 (m, 1 H) 6.46 (d, J=3.39 Hz, 1 H) 7.17 (d, *J*=3.39 Hz, 1 H); MS: (M+H)⁺= 426.

### Example 89

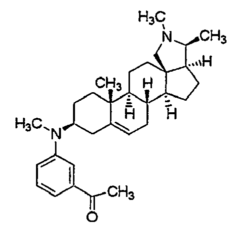

### 1-{3-[Methyl-(2,3,11a-trimethyl-2,3,3a,4,5,5a,5b,6,8,9,10,11,11a, 11b,12,13-hexa decahydro-1H-2-aza-pentaleno[1,6a-a]phenanthren-9-yl)-amino]-phenyl}-ethanone

The title compound was prepared according to the procedures described in Example 86, except substituting 2-bromoacetophenone for 4-bromobenzonitrile. ¹H NMR (CDCl₃): δ 0.97 (s, 3H) 1.04-2.60 (m, 28 H) 2.83 (s, 3H) 2.87 (s, 3H) 2.00 (m, 1H) 3.15 (m, 1 H) 5.40 (m, 1 H) 7.30 (m, 3H) 8.13 (s, 1 H); MS: (M+H)⁺= 461.

### Example 90

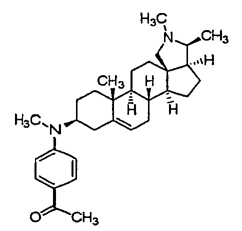

### 1-{4-[Methyl-(2,3,11a-trimethyl-2,3,3a,4,5,5a,5b,6,8,9,10,11,11a,11b,12,13-hexa decahydro-1H-2-aza-pentaleno[1,6a-a]phenanthren-9-yl)-amino]-phenyl}-ethanone

The title compound was prepared according to the procedures described in Example 86, except substituting 4-bromoacetophenone for 4-bromobenzonitrile MS: (M+H)⁺= 461.

### Example 91

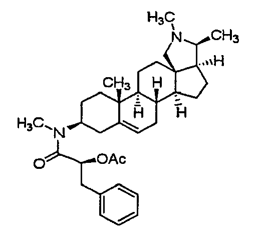

### Acetic acid 1-[methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamoyl]-2-phenylethyl ester

L-3-Phenyllactic acid (0.2 g, 1.2 mmol) was suspended in 3 mL of dichloromethane. Pyridine (0.234 mL, 2.89 mmol) was added and the suspension became a clear solution. Acetyl chloride (0.103 mL, 1.4 mmol) was added dropwise at room temperature, the clear solution became cloudy. The mixture was stirred at room temperature for 4 hours, then quenched with 10% aqueous citric acid, extracted with dichloromethane 3X. The combined organic layers were dried over sodium sulfate, filtered, and the filtrate was concentrated to give the crude L-2-acetoxy-3-phenylpropionic acid, which was used in the next step without purification.

A mixture of compound 7B (40 mg, 0.117 mmol), L-2-acetoxy-3-phenyl-propionic acid (37 mg, 0.175 mmol), 1-[3-(dimethylamino)propyl]-3-ethylcarbodiimide hydrochloride (45 mg, 0.235 mmol), 1-hydroxybenzotriazole (32 mg, 0.235 mmol) and dichloromethane (1.5 mL) was stirred at room temperature overnight. The solvent was evaporated under reduced pressure and the residue was dissolved in minimum amount of dichloromethane and purified on silica gel column which was eluted with 0.3 % ammonium hydroxide and 3% methanol in dichloromethane to give the desired product (50 mg, 81 % yield).

¹H NMR (CDCl₃): δ 0.89 (d, 3 H) 0.95-1.90 (m, 18 H) 2.08 (s, 3 H) 2.18 (m, 3 H) 2.38 (m, 2 H) 2.64 (s, 3 H) 2.79 (s, 3 H) 2.97 (m, 1 H) 3.10 (m, 2 H) 3.37 (m, 1 H) 4.32 (m, 1 H) 5.35 (m, 2 H) 5.52 (m, 1 H) 7.27 (m, 5 H)
MS: (M+H)⁺= 533

### Example 92

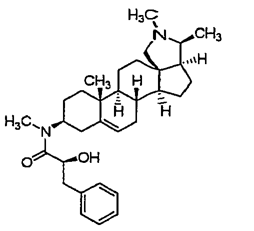

### 2-Hydroxy-N-methyl-3-phenyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-propionamide

Potassium carbonate (51 mg, 0.37 mmol) in 0.5 mL water was added to Compound 91 (36 mg, 0.073 mmol) in 1 mL methanol. The mixture was stirred at room temperature for 4 hours, then quenched with 1 mL water and extracted with dichloromethane to give the crude product which was purified on silica gel column and eluted with 0.3 % ammonium hydroxide and 3% methanol in dichloromethane to give the desired product (25 mg, 76% yield).
¹H NMR (CDCl₃): δ 0.93 (s, 3 H) 1.00-2.60 (m, 25 H) 2.66 (s, 3 H) 2.89 (m, 4 H) 3.36 (m, 1 H) 3.76 (m, 1 H) 4.32 (m, 1 H) 4.57 (m, 2 H) 5.38 (m, 1 H) 7.25 (m, 5 H)
MS: (M+H)⁺= 491

### Example 93

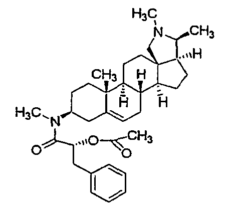

### Acetic acid 1-[methyl-(2,3,11a-trimethyl-2,3,3a,4,5,5a,5b,6,8,9,10,11,1a,11b,12,13-hexadecahydro-1H-2-aza-pentaleno[1,6a-a]phenanthren-9-yl)-carbamoyl]-2-phenylethyl ester

The title compound was prepared according to the procedures described in Example 91, except substituting D-3-phenyllactic acid for L-3-phenyllactic acid. ¹H NMR (CDCl₃): δ 0.90 (d, J=5.09 Hz, 3 H) 1.00-2.40 (m, 25 H) 2.64 (s, 3 H) 2.80 (s, 3 H) 3.10 (m, 4 H) 3.56 (m, 1 H) 4.32 (m, 1 H) 5.19 (m, 1 H) 5.36 (m, 1 H) 5.46 (m, 1 H) 7.26 (m, 5 H)
MS: (M+H)⁺= 533

### Example 94

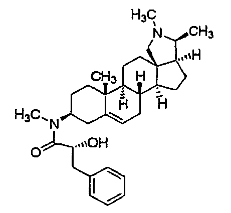

### 2-Hydroxy-N-methyl-3-phenyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-propionamide

The title compound was prepared according to the procedures described in Example 92, except substituting compound 93 for compound 91. ¹H NMR (CDCl₃): δ 0.95 (d, *J=*4.75 Hz, 3 H) 1.03 (m, 3 H) 1.14-2.50 (m, 22H) 2.66 (s, 3 H) 2.92 (m, 5 H) 3.41 (m, 1 H) 3.79 (dd, *J*=8.14, 2.71 Hz, 1 H) 4.31 (m, 1 H) 4.54 (m, 1 H) 5.37 (m, 1 H) 7.26 (m, 5 H)
MS: (M+H)⁺= 491

### Example 95

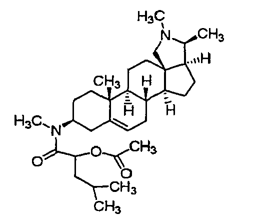

### Acetic acid 3-methyl-1-[methyl-(2,3,11a-trimethyl-2,3,3a,4,5,5a,5b,6,8,9,10,11, 11a,11b,12,13-hexadecahydro-1H-2-aza-pentaleno[1,6a-a]phenanthren-9-yl)-carbamoyl]-butyl ester

The title compound was prepared according to the procedures described in Example 91, except substituting 2-hydroxyisocaproic acid for L-3-phenyllactic acid. MS: (M+H)⁺= 499

### Example 96

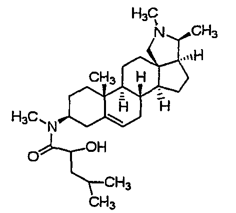

### 2-Hydroxy-4-methyl-pentanoic acid methyl-(2,3,11a-trimethyl-2,3,3a,4,5,5a,5b,6,8,9,10, 11,11a,11b,12, 13-hexadecahydro-1H-2-aza-pentaleno[1,6a-a]phenanthren-9-yl)-amide

The title compound was prepared according to the procedures described in Example 92, except substituting compound 95 for compound 91. MS: (M+H)⁺= 457

### Example 97

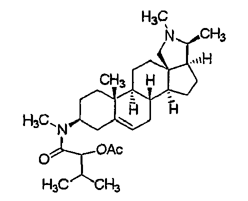

### Acetic acid 2-methyl-1-[methyl-(2,3,11a-trimethyl-2,3,3a,4,5,5a,5b,6,8,9,10,11,11a,11b, 12.13-hexadecahydro-1H-2-aza-pentaleno[1,6a-a]phenanthren-9-yl)-carbamoyl]-propyl ester

The title compound was prepared according to the procedures described in Example 91, except substituting 2-hydroxy-3-methylbutyric acid for L-3-phenyllactic acid. ¹H NMR (CDCl₃): δ 0.95-2.75 (m, 34 H) 2.12 (s, 3 H) 2.86 (s, 3 H) 3.00 (s, 3 H) 3.71 (m, 1H) 4.39 (m, 1 H) 4.97 (m, 1H) 5.08 (m, 1 H) 5.36 (m, 1 H)
MS: (M+H)⁺= 485

### Example 98

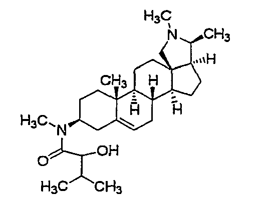

### 2-Hydroxy-3,N-dimethyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-butyramide

The title compound was prepared according to the procedures described in Example 92, except substituting compound 97 for compound 91. MS: (M+H)⁺= 443

### Example 99

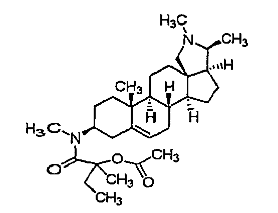

### Acetic acid 1-methyl-1-[methyl-(2,3,11a-trimethyl-2,3,3a,4,5,5a,5b,6,8,9,10,11, 11a,11b,12,13-hexadecahydro-1H-2-aza-pentaleno[1,6a-a]phenanthren-9-yl)-carbamoyl]-propyl ester

The title compound was prepared according to the procedures described in Example 91, except substituting 2-hydroxy-2-methylbutyric acid for L-3-phenyllactic acid. MS: (M+H)⁺= 485

### Example 100

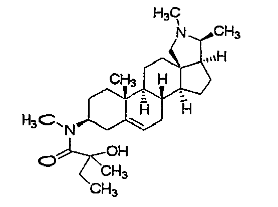

### 2-Hydroxy-2,N-dimethyl-N-(2,3,11a-trimethyl-2,3,3a,4,5,5a,5b,6,8,9,10,11,11a,11b. 12,13-hexadecahydro-1H-2-aza-pentaleno[1,6a-a]phenanthren-9-yl)-butyramide

The title compound was prepared according to the procedures described in Example 92, except substituting compound 99 for compound 91. ¹H NMR (CDCl₃): δ 0.96 (m, 3 H) 1.05-2.60 (m, 33 H) 2.84 (s, 3 H) 2.87 (s, 3 H) 2.98 (m, 1 H) 3.50 (m, 1 H) 4.42 (m, 1 H) 5.37 (m, 1 H)
MS: (M+H)⁺= 443

### Example 101

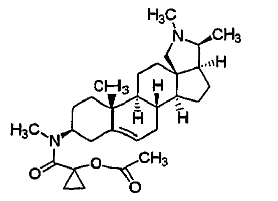

### Acetic acid 1-[methyl-(2,3,11a-trimethyl-2,3,3a,4,5,5a,5b,6,8,9,10,11,11a,11b,12,13a hexadecahydro-1H-2-aza-pentaleno[1,6a-a]phenanthren-9-yl)-carbamoyl]-cyclopropyl ester

The title compound was prepared according to the procedures described in Example 91, except substituting 1-hydroxy-1-cyclopropanecarboxylic acid for L-3-phenyllactic acid. MS: (M+H)⁺= 469

### Example 102

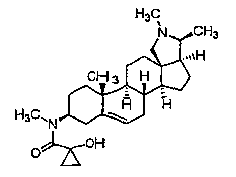

### 1-Hydroxy-cyclopropanecarboxylic acid methyl-(2,3,11a-trimethyl-2,3,3a,4,5,5a,5b,6,8, 9,0,11,11a,11b,12,13-hexadecahydro-1H-2-aza-pentaleno[1,6a-a]phenanthren-9-yl) amide

The title compound was prepared according to the procedures described in Example 92, except substituting compound 101 for compound 91. ¹H NMR (CDCl₃): δ 0.78-2.60 (m, 31 H) 2.87 (s, 3 H) 2.91 (s, 3 H) 3.01 (m, 1 H) 3.72 (m, 1 H) 4.21 (m, 1 H) 4.35 (m, 1 H) 5.38 (m, 1 H)
MS: (M+H)⁺= 427

### Example 103

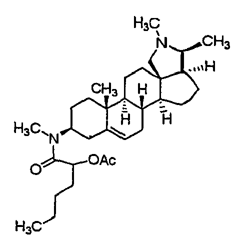

### Acetic acid 1-[methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamoyl]-pentyl ester

The title compound was prepared according to the procedures described in Example 91, except substituting 2-hydroxycaproic acid for L-3-phenyllactic acid. ¹H NMR (CDCl₃): δ 0.91-2.60 (m, 37 H) 2.12 (s, 3 H) 2.86 (s, 3 H) 2.95 (s, 3 H) 3.58 (m, 1 H) 4.35 (m, 1 H) 5.24 (m, 1 H) 5.36 (m, 1 H)
MS: (M+H)⁺= 499

### Example 104

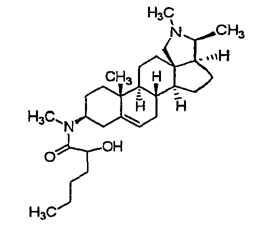

### 2-Hydroxy-hexanoic acid methyl-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-amide

The title compound was prepared according to the procedures described in Example 92, except substituting compound 103 for compound 91. ¹H NMR (CDCl₃): δ 0.88-2.60 (m, 37 H) 2.85 (s, 3 H) 2.90 (s, 3 H) 2.99 (m, 1 H) 3.35 (m, 1 H) 3.83 (m, 1 H) 4.33 (m, 1 H) 5.38 (m, 1 H)
MS: (M+H)⁺= 457

### Example 105

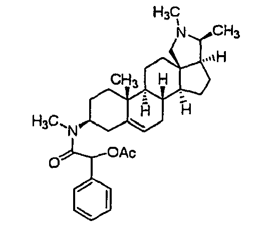

### Acetic acid [methyl-(2,3,11a-trimethyl-2,3,3a,4,5,5a,5b,6,8,9,10,11,11a,11b,12,13-hexa decahydro-1H-2-aza-pentaleno[1,6a-a]phenanthren-9-yl)-carbamoyl]-phenyl-methyl ester

The title compound was prepared according to the procedures described in Example 91, except substituting DL-mandelic acid for L-3-phenyllactic acid. MS: (M+H)⁺= 519

### Example 106

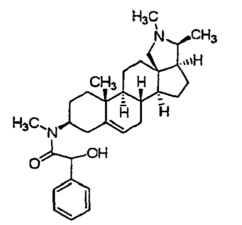

### 2-Hydroxy-N-methyl-2-phenyl-N-(2,3,11a-trimethyl-2,3,3a,4,5,5a,5b,6,8,9,10,11,11a, 11b,12,13-hexadecahydro-1H-2-aza-pentalenon[1,6a-a]phenanthren-9-yl)-acetamide

The title compound was prepared according to the procedures described in Example 92, except substituting compound 105 for compound 91. MS: (M+H)⁺= 477

### Example 107

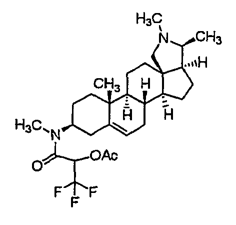

### Acetic acid 2,2,2-trifluoro-1-[methyl-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-carbamoyl]-ethyl ester

The title compound was prepared according to the procedures described in Example 91, except substituting 3,3,3-trifluoro-2-hydroxyprop anoic acid for L-3-phenyllactic acid. MS: (M+H)⁺= 511, (M+NH⁴)⁺=528

### Example 108

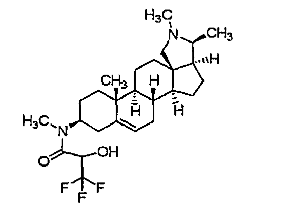

### 3,3,3-Trifluoro-2-hydroxy-N-methyl-N-(2,3,11a-trimethyl-2,3,3a,4,5,5a,5b,6,8,9,10,11. 11a,11b,12,13-hexadecahydro-1H-2-aza-pentaleno[1,6a-a]phenanthren-9-yl)-propionamide

The title compound was prepared according to the procedures described in Example 92, except substituting compound 107 for compound 91. ¹H NMR (CDCl₃): δ 0.97 (s, 3 H) 1.05-2.60 (m, 26H) 2.95 (s, 3 H) 2.99 (s, 3 H) 3.42 (m, 1 H) 4.43 (m, 1 H) 4.76 (m, 1 H) 5.40 (m, 1 H)
MS: (M+H)⁺= 469, (M+NH⁴)+=486

### Example 109

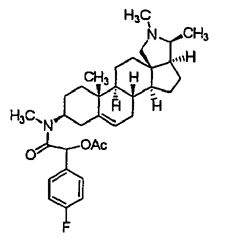

### Acetic acid (4-fluoro-phenyl)-[methyl-(2,3,11a-trimethyl-2,3,3a,4,5,5a,5b,6,8,9,10,11, 11a,11b,12,13-hexadecahydro-1H-2-aza-pentaleno[1,6a-a]phenanthren-9-yl)-carbamoyl]-methyl ester

The title compound was prepared according to the procedures described in Example 91, except substituting 4-fluoromandelic acid for L-3-phenyllactic acid. ¹H NMR (CDCl₃): δ 0.92 (m, 3 H) 1.00-2.60 (m, 23 H) 2.16 (s, 3 H) 2.79 (s, 3 H) 2.85 (s, 3 H) 3.00 (m, 1 H) 3.52 (m, 1 H) 4.32 (m, 1 H) 4.90 (m, 1 H) 5.38 (m, 1 H) 6.18 (m, 1 H) 7.08 (m, 2 H) 7.43 (m, 2 H)
MS: (M+H)⁺= 537

### Example 110

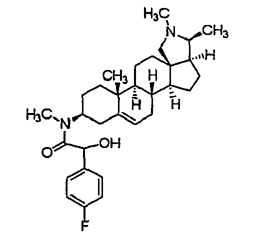

### 2-(4-Fluoro-phenyl)-2-hydroxy-N-methyl-N-(2,3,11a-trimethyl-2,3,3a,4,5,5a,5b,6,8,9. 10,11,11a,11b,12,13-hexadecahydro-1H-2-aza-pentaleno[1,6a-a]phenanthren-9-yl)-acetamide

The title compound was prepared according to the procedures described in Example 92, except substituting compound 109 for compound 91. ¹H NMR (CDCl₃): δ 0.86 (s, 3 H) 1.00-2.50 (m, 25 H) 2.62 (s, 3 H) 2.91 (s, 3 H) 3.30 (m, 1 H) 4.82 (m, 1 H) 4.97 (m, 1 H) 5.15 (m, 1 H) 5.38 (m, 1 H) 7.04 (m, 2 H) 7.28 (m, 2 H)
MS: (M+H)⁺= 495

### Example 111

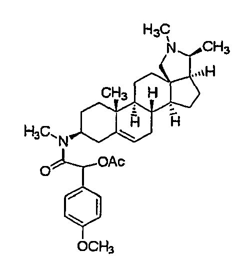

### Acetic acid (4-methoxy-phenyl)-[methyl-(2,3,11a-trimethyl-2,3,3a,4,5,5a,5b,6,8,9,10,11, 11a,11b,12,13-hexadecahydro-1H-2-aza-pentaleno[1,6a-a]phenanthren-9-yl)-carbamoyl]-methyl ester

The title compound was prepared according to the procedures described in Example 91, except substituting 4-methoxymandelic acid for L-3-phenyllactic acid. ¹H NMR (CDCl₃): δ 0.89 (d, *J*=9.83 Hz, 3 H) 1.00-2.60 (m, 24 H) 2.15 (s, 3 H) 2.76 (s, 3H) 2.85 (s, 3 H) 2.96 (m, 1 H) 3.55 (m, 1 H) 3.82 (s, 3H) 4.39 (m, 1 H) 5.40 (m, 1 H) 6.14 (m, 1 H) 6.90 (m, 2 H) 7.36 (m, 2 H)
MS: (M+H)⁺= 549

### Example 112

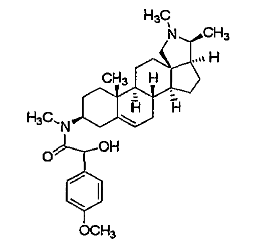

### 2-Hydroxy-2-(4-methoxy-phenyl)-N-methyl-N-(2,3,11a-trimethyl-2,3,3a,4,5,5a,5b,6,8,9. 10,11,11a,11b,12,13-hexadecahydro-1H-2-aza-pentaleno[1,6a-a]phenanthren-9-yl)-acetamide

The title compound was prepared according to the procedures described in Example 92, except substituting compound 111 for compound 91. ¹H NMR (CDCl₃): δ 0.85 (s, 3 H) 0.92 (s, 3 H) 1.04-2.60 (m, 21 H) 2.62 (s, 3 H) 2.90 (s, 3 H) 2.97 (m, 1 H) 3.42 (m, 1 H) 3.81 (s, 3 H) 4.77 (m, 1 H) 4.92 (m, 1 H) 5.13 (m, 1 H) 5.38 (m, 1 H) 6.88 (m, 2 H) 7.23 (m, 2 H)
MS: (M+H)⁺= 507

### Example 113

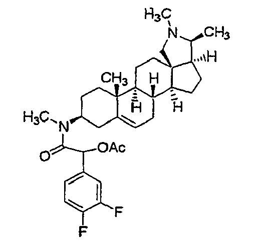

### Acetic acid (3,4-difluoro-phenyl)-[methyl-(2,3,11a-trimethyl-2,3,3a,4,5,5a,5b,6,8,9,10, 11,11a,11b,12,13-hexadecahydro-1H-2-aza-entaleno[1,6a-a]phenanthren-9-yl)-carbamoyl]-methyl ester

The title compound was prepared according to the procedures described in Example 91, except substituting 3,3-difluoromandelic acid for L-3-phenyllactic acid. ¹H NMR (CDCl₃): δ 0.91 (d, *J*=6.10 Hz, 3 H) 1.00-2.60 (m, 28 H) 2.16 (s, 3 H) 2.85 (m, 3 H) 2.98 (m, 1 H) 4.34 (m, 1 H) 5.38 (m, 1 H) 6.14 (m, 1 H) 7.18 (m, 2 H) 7.34 (m, 1 H)
MS: (M+H)⁺= 555

### Example 114

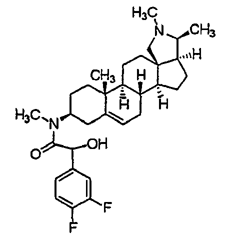

### 2-(3,4-Difluoro-phenyl)-2-hydroxy-N-methyl-N-(2,3,11a-trimethyl-2,3,3a,4,5,5a,5b,6,8. 9,10,11,11a,11b,12,13-hexadecahydro-1H-2-aza-pentaleno[1,6a-a]phenanthren-9-yl)-acetamide

The title compound was prepared according to the procedures described in Example 92, except substituting compound 113 for compound 91. ¹H NMR (CDCl₃): δ 0.87 (s, 3 H) 0.93 (s, 3 H) 1.05-2.55 (m, 20 H) 2.65 (s, 3 H) 2.91 (s, 3 H) 2.98 (m, 1 H) 3.33 (m, 1 H) 4.38 (m, 1 H) 4.82 (m, 1 H) 4.98 (m, 1 H) 5.13 (m, 1 H) 5.39 (m, 1 H) 7.14 (m, 3 H)
MS: (M+H)⁺=513

### Example 115

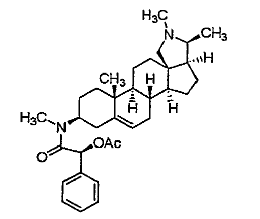

### Acetic acid [methyl-(2,3,11a-trimethyl-2,3,3a4,5,5a,5b,6,8,9,10,11,11a,11b,12,13-hexadecahydro-1H-2-aza-pentaleno[1,6a-a]phenanthren-9-yl)-carbamoyl]-phenylmethyl ester

The title compound was prepared according to the procedures described in Example 91, except substituting (S)-(+)-mandelic acid for L-3-phenyllactic acid. ¹H NMR (CDCl₃): δ 0.88-2.50 (m, 29 H) 2.78 (s, 3 H) 2.85 (s,3 H) 2.95 (t, *J*=9.05 Hz, 2 H) 3.56 (m, 1 H) 4.37 (m, 1 H) 5.38 (m, 1 H) 6.20 (m, 1 H) 7.41 (m, 5 H)
MS: (M+H)⁺=519

### Example 116

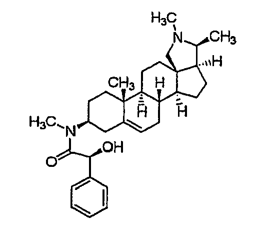

### 2-Hydroxy-N-methyl-2-phenyl-N-(2,3,11a-trimethyl-2,3,3a,4,5,5a,5b,6,8,9,10,11, 11a,11b,12,13-hexadecahydro-1H-2-aza-pentaleno[1,6a-a]phenanthren-9-yl)-acetamide

The title compound was prepared according to the procedures described in Example 92, except substituting compound 115 for compound 91. ¹H NMR (CDCl₃): δ 0.85 (s, 3 H) 1.03 (d, *J*=6.44 Hz, 3 H) 1.05-2.50 (m, 19 H) 2.62 (s, 3 H) 2.90 (s, 3 H) 2.95 (m, 2 H) 3.42 (m, 1 H) 4.41 (m, 1 H) 4.75 (m, 1 H) 4.96 (m, 1 H) 5.18 (m, 1 H) 5.40 (m, 1 H) 7.32 (m, 5 H)
MS: (M+H)⁺= 477

### Example 117

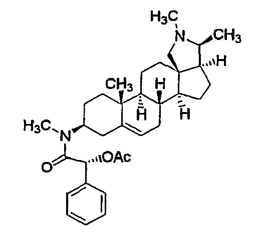

### Acetic acid [methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamoyl]-phenylmethyl ester

The title compound was prepared according to the procedures described in Example 91, except substituting (R)-(-)-mandelic acid for L-3-phenyllactic acid. ¹H NMR (CDCl₃): δ 0.88 (d, *J*=11.19 Hz, 3 H) 1.00-2.60 (m, 24H) 2.17 (s, 3 H) 2.78 (s, 3 H) 2.85 (s, 3 H) 3.26 (m, 1 H) 3.62 (m, 1 H) 4.37 (m, 1 H) 5.37 (m, 1 H) 6.16 (d, *J*=4.07 Hz, 1 H) 7.41 (m, 5 H)
MS: (M+H)⁺= 519

### Example 118

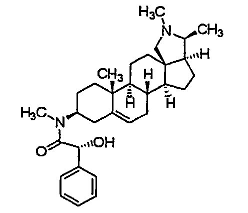

### 2-Hydroxy-N-methyl-2-phenyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-acetamide

The title compound was prepared according to the procedures described in Example 92, except substituting compound 117 for compound 91. ¹H NMR (CDCl₃): δ 0.85 (s, 3 H) 1.03 (d, *J*=6.24 Hz, 3 H) 1.10-2.50 (m, 16H) 2.19 (s, 3 H) 2.62 (s, 3 H) 2.90 (s, 3 H) 2.95 (m, 2 H) 3.42 (m, 1 H) 4.41 (m, 1 H) 4.74 (m, 1 H) 4.98 (m,1 H) 5.19 (m, 1 H) 5.39 (m, 1 H) 7.32 (m, 5 H)
MS: (M+H)⁺=477

### Example 119

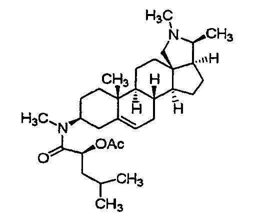

### Acetic acid 3-methyl-1-[methyl-(2,3,11a-trimethyl-2,3,3a,4,5,5a,5b,6,8,9,10,11,11a 11b,12,13-hexadecahydro-1H-2-aza-pentaleno[1,6a-a]phenanthren-9-yl)-carbamoyl]-butyl ester

The title compound was prepared according to the procedures described in Example 91, except substituting (S)-(-)-2-hydroxyisocaproic acid for L-3-phenyllactic acid. ¹H NMR (CDCl₃): δ 0.90-2.60 (m, 33 H) 2.11 (s, 3 H) 2.41 (m, 3 H) 2.85 (s, 3 H) 2.93 (s, 3 H) 2.94 (m, 1 H) 3.53 (m, 1 H) 4.34 (m, 1 H) 5.33 (m, 2 H)
MS: (M+H)⁺= 499

### Example 120

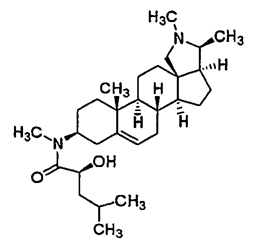

### 2-Hydroxy-4-methyl-pentanoic acid methyl-(2,3,11a-trimethyl-2,3,3a,4,5,5a,5b,6,8,9,10, 11,11a,11b,12,13-hexadecahydro-1H-2-aza-pentaleno[1,6a-a]phenanthren-9-yl)-amide

The title compound was prepared according to the procedures described in Example 92, except substituting compound 119 for compound 91. ¹H NMR (CDCl₃): δ 0.92-2.60 (m, 38 H) 2.84 (s, 3 H) 2.90 (s, 3 H) 2.95 (m, 1 H) 3.75 (m, 1 H) 4.34 (m, 1 H) 5.38 (m, 1 H)
MS: (M+H)⁺= 457

### Example 121

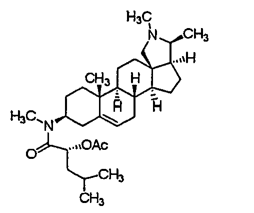

### Acetic acid 3-methyl-1-[methyl-(2,3,11a-trimethyl-2,3,3a,4,5,5a,5b,6,8,9,10,11,11a 11b,12,13-hexadecahydro-1H-2-aza-pentaleno[1,6a-a]phenanthren-9-yl-carbamoyl]-butyl ester

The title compound was prepared according to the procedures described in Example 91, except substituting (R)-(+)-2-hydroxyisocaproic acid for L-3-phenyllactic acid. ¹H NMR (CDCl₃): δ 0.92-2.65 (m, 33 H) 2.11 (s, 3 H) 2.42 (m, 3 H) 2.85 (s, 3 H) 2.94 (s, 3 H) 3.02 (m, 1 H) 3.60 (m, 1 H) 4.34 (m, 1 H) 5.31 (m, 2 H)
MS: (M+H)⁺= 499

### Example 122

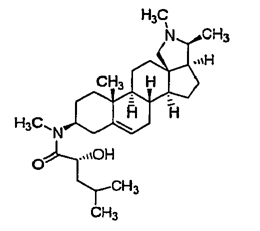

### 2-Hydroxy-4-methyl-pentanoic acid methyl-(2,3,11a-trimethyl-2,3,3a,4,5,5a,5b,6,8,9,10 11,11a,11b,12,13-hexadecahydro-1H-2-aza-pentaleno[1,6a-a]phenanthren-9-yl)-amide

The title compound was prepared according to the procedures described in Example 92, except substituting compound 121 for compound 91. ¹H NMR (CDCl₃): δ 0.94-2.60 (m, 38 H) 2.84 (s, 3 H) 2.89 (s, 3 H) 2.95 (m, 1 H) 3.75 (m, 1 H) 4.34 (m, 1 H) 5.40 (m, 1 H)
MS: (M+H)⁺= 457

### Example 123

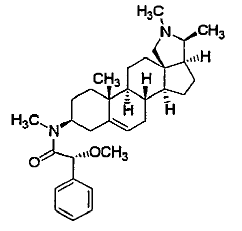

### 2-Methoxy-N-methyl-2-phenyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-acetamide

A mixture of compound 7B (30 mg, 0.088 mmol), R-(-)-α-methoxyphenylacetic acid (22 mg, 0.132 mmol), 1-[3-(dimethylamino)propyl]-3-ethylcarbodiimide hydrochloride (34 mg, 0.177 mmol), 1-hydroxybenzotriazole (24 mg, 0.177 mmol) and dichloromethane (1 mL) was stirred at room temperature overnight. The solvent was evaporated under reduced pressure and the residue was dissolved in minimum amount of dichloromethane and purified on silica gel column which was eluted with 0.3% ammonium hydroxide and 3% methanol in dichloromethane to give the desired product (40 mg, 93% yield).

¹H NMR (CDCl₃): δ 0.86 (s, 3 H) 0.91 (s, 3 H) 1.06-2.50 (m, 20 H) 2.74 (s, 3 H) 2.84 (s, 3 H) 3.04 (m, 1 H) 3.45 (s, 3 H) 3.78 (m, 1 H) 4.41 (m, 1 H) 5.02 (d, *J*=13.22 Hz, 2 H) 5.37 (m, 1 H) 7.36 (m, 5 H)
MS: (M+H)⁺= 491

### Example 124

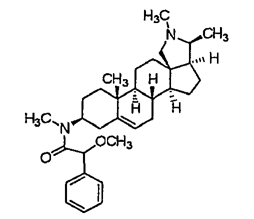

### 2-Methoxy-N-methyl-2-phenyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-acetamide

The title compound was prepared according to the procedures described in Example 123, except substituting 2-methoxyphenylacetic acid for R-(-)-α-methoxyphenylacetic acid. ¹H NMR (CDCl₃): δ 0.86 (s, 3 H) 0.91 (s, 3H) 1.00-2.40 (m, 20 H) 2.74 (s, 3 H) 2.84 (s, 3H) 2.96 (m, 1 H) 3.74 (m, 3H) 3.78 (m, 1H) 4.40 (m, 1 H) 5.02 (d, *J*=11.87 Hz, 2 H) 5.37 (m, 1 H) 7.36 (m, 5 H); MS: (M+H)⁺= 491.

### Example 125

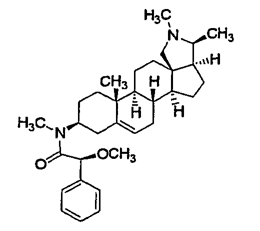

### 2-Methoxy-N-methyl-2-phenyl-N-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl-acetamide

The title compound was prepared according to the procedures described in Example 123, except substituting (S)-(+)-α-methoxyphenylacetic acid for R-(-)- α - methoxyphenylacetic acid. ¹H NMR (CDCl₃): δ 0.86 (s, 3 H) 0.91 (s, 3 H) 1.06-2.50 (m, 20 H) 2.74 (s, 3 H) 2.84 (s, 3 H) 3.01 (m, 1 H) 3.46 (s, 3 H) 3.76 (m, 1 H) 4.39 (m, 1 H) 5.02 (d, *J*=11.87 Hz, 2 H) 5.37 (m, 1 H) 7.36 (m, 5 H); MS: (M+H)⁺= 491.

### Example 126

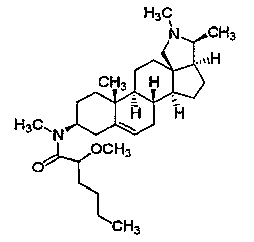

### 2-Methoxy-hexanoic acid methyl-(2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl)-amide

2-Hydroxy caproic acid (152 mg, 1.15 mmol) was dissolved in 3 mL anhydrous THF. Sodium hydride (95%, 61 mg, 2.41 mmol) was added and the mixture was stirred at room temperature for 40 min. lodomethane (0.7 mL) was added and the mixture was stirred at room temperature overnight. It was quenched with water, stirred for 4 hours to hydrolyze the methyl ester, and then adjusted to pH 4 with 10% citric acid. The mixture was extracted with 5%methanol in dichloromethane, organic layer dried over sodium sulfate, filtered, and the filtrate was concentrated to give the crude 2-methoxy caproic acid which was used in next step without further purification.

A mixture of compound 7B (30 mg, 0.088 mmol), 2-methoxy caproic acid (20 mg, 0.137 mmol), 1-[3-(dimethylamino)propyl]-3-ethylcarbodiimide hydrochloride (34 mg, 0.177 mmol), 1-hydroxybenzotriazole (24 mg, 0.177 mmol) and dichloromethane (1 mL) was stirred at room temperature overnight. The solvent was evaporated under reduced pressure and the residue was dissolved in minimum amount of dichloromethane and purified on silica gel column, which was eluted with 0.3% ammonium hydroxide and 3% methanol in dichloromethane to give the desired product (38 mg, 92% yield).

¹H NMR (CDCl₃): δ 0.88-2.75 (m, 37 H) 2.87 (s, 3 H) 2.98 (s, 3 H) 3.31 (s, 3 H) 3.99 (m, 2 H) 4.40 (m, 1 H) 5.37 (m, 1 H)
MS: (M+H)⁺= 471

### Example 127

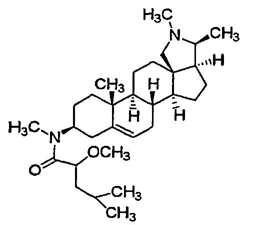

### 2-Methoxy-4-methyl-pentanoic acid methyl-(2,3,11a-trimethyl-2,3,3a,4,5,5a,5b,6,8,9,10, 11,11a,11b,12,13-hexadecahydro-1H-2-aza-pentaleno[1,6a-a]phenanthren-9-yl)-amide

The title compound was prepared according to procedures described in Example 126, except substituting 2-hydroxy isocaproic acid for 2-hydroxy caproic acid. ¹H NMR (CDCl₃): δ 0.91-2.60 (m, 37 H) 2.86 (s, 3 H) 2.97 (s, 3 H) 3.32 (d, *J*=5.43 Hz, 3 H) 3.91 (m, 1 H) 4.06 (dd, *J*=9.32, 4.24 Hz, 1 H) 4.40 (m, 1 H) 5.37 (m, 1 H); MS: (M+H)⁺= 471.

### Example 128

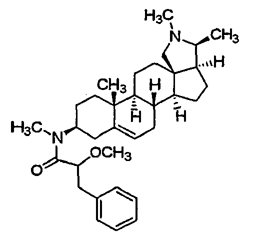

### 2-Methoxy-N-methyl-3-phenyl-N-(2,3,11a-trimethyl-2,3,3a,4,5,5a,5b,6,8,9,10,11,11a. 11b,12,13-hexadecahydro-1H-2-aza-pentaleno[1,6a-a]phenanthren-9-yl)-propionamide

The title compound was prepared according to procedures described in Example 126, except substituting L-3-phenyllactic acid for 2-hydroxy caproic acid. ¹H NMR (CDCl₃): δ 0.93 (m,3 H) 1.04 (d, *J*=6.24 Hz, 3 H) 1.10-2.60 (m, 18 H) 2.72 (s, 3 H) 2.83 (s, 3 H) 3.02 (m, 6 H) 3.31 (d, *J*=3.43 Hz, 3 H) 3.61 (m,1 H) 4.29 (m, 1 H) 4.40 (m,1H) 5.35 (m, 1 H) 7.24 (m, 5 H); MS: (M+H)⁺= 505.

### Example 129

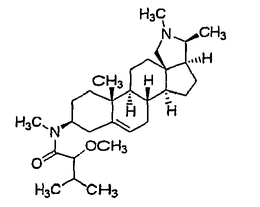

### 2-Methoxy-3,N-dimethyl-N-(2,3,11a-trimethyl-2,3,3a,4,5,5a,5b,6,8,9,10,11,11a,11b. 12,13-hexadecahydro-1H-2-aza-pentaleno[1,6a-a]phenanthren-9-yl)-butyramide

The title compound was prepared according to procedures described in Example 126, except substituting 2-hydroxy-3-methylbutyric acid for 2-hydroxy caproic acid. ¹H NMR (CDCl₃) δ 0.89-2.65 (m, 35 H) 2.88 (s, 3 H) 3.00 (s, 3 H) 3.32 (s, 3 H) 3.64 (m, 1 H) 4.01 (m, 1 H) 4.42 (m, 1 H) 5.36 (m, 1 H); MS: (M+H)⁺= 457.

### Example 130

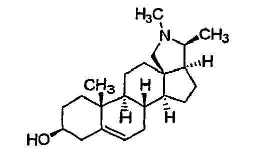

### 130A. 2,3,11a-Trimethyl-2,3,3a,4,5,5a,5b,6,8,9,10,11,11a,11b,12,13-hexadecahydro-1H-2-aza-pentaleno[1,6a-a]phenanthren-9-ol

The title compound was prepared according to the procedure of Hora and Cerny; Collect. Czech. Chem. Commun., 26, 1961, 2217 and Labler et al.; and Collect. Czech. Chem. Commun., 28, 1963, 2015. ¹H NMR (CDCl₃): δ 0.95 (s, 3 H) 1.00-1.50 (m, 12 H) 1.50-2.00 (m, 10 H) 2.07 (m, 1 H) 2.26 (m, 5 H) 2.45 (m, 1 H) 3.05 (m, 1 H) 3.52 (m, 1 H) 5.34 (m, 1 H); MS: (M+H)⁺= 330.

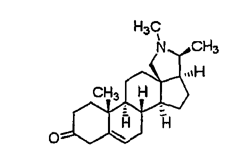

### 130B. 2,3,11a-Trimethyl-1,2,3,3a,4,5,5a,5b,6,8,10,11,11a,11b,12,13-hexadecahydro-2-aza-pentaleno[1,6a-a]phenanthren-9-one

A mixture of Compound 130A (200 mg, 0.607 mmol) Dess-Martin periodinane (386 mg, 0.91 mmol) and dichloromethane (10 mL) was stirred at room temperature for 4.5 hours. The reaction was quenched with 10% sodium thiosulfate and extracted with dichloromethane. Organic layer was dried over sodium sulfate, filtered, and the filtrate was concentrated under reduced pressure to give the crude product, which was purified on silica gel column eluted with 0.3% ammonium hydroxide and 3 % methanol in dichloromethane, to give the desired product (156 mg, 83% yield).

¹H NMR (CDCl₃): δ 1.13 (s, 3H) 1.20-2.80 (m, 23 H) 2.73 (s, 3 H) 3.26 (m, 2 H) 3.88 (m, 1 H) 5.35 (m, 1 H)
MS: (M+H)⁺= 328

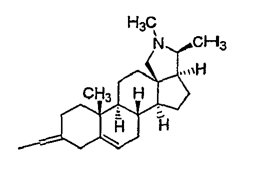

### 130C. 9-Ethylidene-2,3,11a-trimethyl-2,3,3a,4,5,5a,5b,6,8,9,10,11,11a,11b,12,13-hexadecahydro-1H-2-aza-pentaleno[1,6a-a]phenanthrene

Potassium t-butoxide (1.0M in THF, 0.3 mL, 0.30 mmol) was added dropwise to a stirred solution of ethyl triphenylphosphonium bromide (156 mg, 0.42 mmol) in 2 mL anhydrous toluene. A bright orange suspension resulted and was stirred at room temperature for 5 hours. Compound 130B (25 mg, 0.076 mmol) in 1 mL THF was added to the above suspension and stirred overnight. The reaction was quenched with saturated ammonium chloride and extracted with dichloromethane to give the crude product, which was purified on silica gel column eluted with 0.3% ammonium hydroxide and 3 % methanol in dichloromethane to give the desired product (12 mg, 46% yield).

¹H NMR (CDCl₃): δ 0.80-2.60 (m, 34 H) 2.98 (m, 1 H) 5.23 (m, 1 H) 5.71 (m, 1 H)
MS: (M+H)⁺= 340

### Example 131

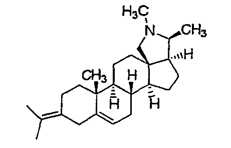

### 9-Isopropylidene-2,3,11a-trimethyl-2,3,3a,4,5,5a,5b,6,8,9,10,11,11a,11b,12,13-hexadecahydro-1H-2-aza-pentaleno[1,6a-a]phenanthrene

The title compound was prepared according to procedures described in Example 130C, except substituting isopropyltriphenylphosphonium iodide for ethyl triphenylphosphonium bromide; MS: (M+H)⁺= 354.

### Reference Example 132

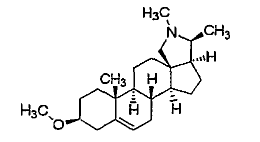

### 9-Methoxy-2,3,11a-trimethyl-2,3,3a,4,5,5a,5b,6,8,9,10,11,11a,11b,12,13-hexadecahydro-1H-2-aza-pentaleno[1,6a-a]phenanthrene

Compound 130A (30 mg, 0.091 mmol) was dissolved in 1.5 mL anhydrous DMF. Sodium hydride (60% dispersion in mineral oil, 6 mg, 0.137 mmol) was added and the mixture was stirred at room temperature for 1 hour before iodomethane (39 mg, 0.274 mmol) was added. The reaction mixture and stirred overnight, quenched with water and extracted with dichloromethane to give the crude product, which was purified on silica gel column eluted with 0.3% ammonium hydroxide and 5 % methanol in dichloromethane to give the desired product (12 mg, 38% yield).

¹H NMR (CDCl₃): δ 0.95 (s, 3 H) 1.01-2.25 (m,263 H) 2.38 (m, 2 H) 3.05 (m, 2 H) 3.36 (s, 3 H) 5.37 (m, 1 H)
MS: (M+H)⁺= 344

### Example 133

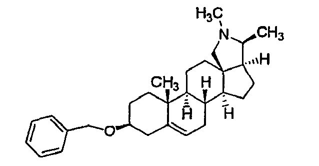

### 9-Benzyloxy-2,3,11a-trimethyl-2,3,3a,4,5,5a,5b,6,8,9,10,11,11a,11b,12,13-hexadecahydro-1H-2-aza-pentaleno[1,6a-a]phenanthrene

The title compound was prepared according to procedures described in Reference Example 132, except substituting benzyl bromide for iodomethane. ¹H NMR (CDCl₃): δ ppm 0.96 (s, 3 H) 1.04 (d, 3 H) 1.06-2.00 (m,18 H) 2.08 (m, 1 H) 2.20 (s, 3 H) 2.36 (m, 3 H) 2.99 (m, 1 H) 3.28 (m, 1 H) 4.55 (s, 2 H) 5.36 (m, 1 H) 7.25 (m, 2 H) 7.34 (m, 3 H); MS: (M+H)⁺= 420.

### Example 134

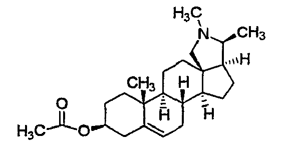

### Acetic acid 2,3,11a-trimethyl-2,3,3a,4,5,5a,5b,6,8,9,10,11,11a,11b,12,13-hexadecahydro-1H-2-aza-pentaleno[1,6a-a]phenanthren-9-yl ester

Compound 130A (15 mg, 0.045 mmol) was dissolved in 1 mL anhydrous dichloromethane. Triethylamine (32 µL, 0.228 mmol) was added followed by acetyl chloride (6.5 µL, 0.09 mmol). The mixture was stirred at room temperature overnight. The crude product was purified on silica gel column eluted with 0.3% ammonium hydroxide and 3 % methanol in dichloromethane to give the desired product (23 mg, 68% yield).

¹H NMR (CDCl₃): δ 0.96 (s, 3 H) 1.03-1.90 (m, 23 H) 2.04 (s, 3 H) 2.20-2.35 (m, 5 H) 2.98 (m, 1 H) 4.59 (m, 1 H) 5.39 m, 1 H)
MS: (M+H)⁺= 372

### Example 135

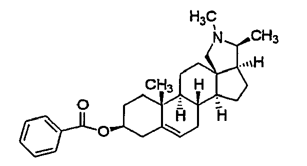

### Benzoic acid 2,3,11a-trimethyl-2,3,3a,4,5,5a,5b,6,8,9,10,11,11a,11b,12,13-hexadecahydro-1H-2-aza-pentaleno[1,6a-a]phenanthren-9-yl ester

The title compound was prepared according to procedures described in Example 134, except substituting benzoyl chloride for acetyl chloride. MS: (M+H)⁺= 434.

### Example 136

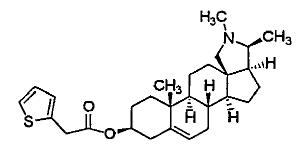

### Thiophen-2-yl-acetic acid 2,3,11a-trimethyl-2,3,3a,4,5,5a,5b,6,8,9,10,11,11a,11b,12,13-hexadecahydro-1H-2-aza-pentaleno[16a-a]phenanthren-9-yl ester

The title compound was prepared according to procedures described in Example 134, except substituting 2-thiopheneacetyl chloride for acetyl chloride. ¹H NMR (CDCl₃): δ 0.90 (s, 3 H) 0.96 (d, *J*=3.73 Hz, 3 H) 1.00-2.40 (m, 27 H) 3.00 (m, 1 H) 4.66 (m, 1H) 5.37 (m, 1 H) 6.94 (m, 2 H) 7.21 (m, 1 H); MS: (M+H)⁺= 454.

### Example 137

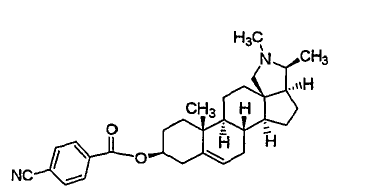

### 4-Cyano-benzoic acid 2,3,11a-trimethyl-2,3,3a,4,5,5a,5b,6,8,9,10,11,11a,11b,12,13 hexadecahydro-1H-2-aza-pentaleno[1,6a-a]phenanthren-9-yl ester

The title compound was prepared according to procedures described in Example 134, except substituting 4-cyanobenzoyl chloride for acetyl chloride. ¹H NMR (CDCl₃): δ 1.01 (s, 3 H) 1.04-2.30 (m, 25 H) 2.47 (d , 3 H) 3.00 (m, 1 H) 4.87 (m, 1 H) 5.44 (m, 1 H) 7.74 (m, 2 H) 8.14 (m, 2 H); MS: (M+H)⁺= 459.

### Example 138

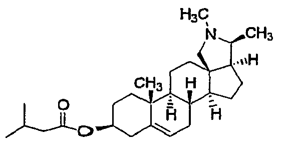

### 3-Methyl-butyric acid 2,3,11a-trimethyl-2,3,3a,4,5,5a,5b,6,8,9,10,11,11a,11b,12,13-hexadecahydro-1H-2-aza-pentaleno[1,6a-a]phenanthren-9-yl ester

The title compound was prepared according to procedures described in Example 134, except substituting isovaleryl chloride for acetyl chloride. ¹H NMR (CDCl₃): δ 0.95 (d, 9 H) 1.00-2.35 (m, 31 H) 3.00 (m, 1 H) 4.62 (m, 1 H) 5.39 (m, 1 H); MS: (M+H)⁺= 414.

### Example 139

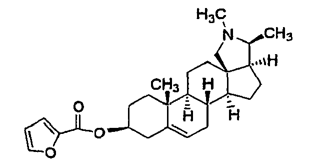

### Furan-2-carboxylic acid 2,3,11a-trimethyl-2,3,3a,4,5,5a,5b,6,8,9,10,11,11a,11b,12,13-hexadecahydro-1H-2-aza-pentaleno[1,6a-a]phenanthren-9-yl ester

The title compound was prepared according to procedures described in Example 134, except substituting 2-furoyl chloride for acetyl chloride. ¹H NMR (CDCl₃): δ 1.00 (s, 3 H) 1.05-2.35 (m, 25 H) 2.45 (d, *J*=7.46 Hz, 3 H) 3.00 (m, 1 H) 4.84 (m, 1 H) 5.42 (m, 1 H) 6.50 (dd, *J*=3.39, 1.70 Hz, 1 H) 7.17 (d, *J*=4.41 Hz, 1 H) 7.57 (s, 1 H); MS: (M+H)⁺= 424.

### Example 140

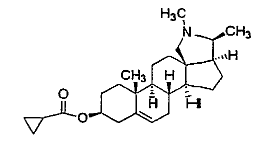

### Cyclopropanecarboxylic acid 2,3,11a-trimethyl-2,3,3a,4,5,5a,5b,6,8,9,10,11,11a 11b,12,13-hexadecahydro-1H-2-aza-pentaleno[1,6a-a]phenanthren-9-yl ester

The title compound was prepared according to procedures described in Example 134, except substituting cyclopropanecarbonyl chloride for acetyl chloride. ¹H NMR (CDCl₃): δ 0.82-2.38 (m, 36 H) 3.01 (m, 1 H) 4.61 (m, 1 H) 5.38 (m, 1 H); MS: (M+H)⁺= 398.

### Example 141

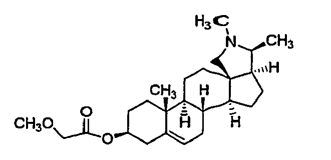

### Methoxy-acetic acid 2,3,11a-trimethyl-2,3,3a,4,5,5a,5b,6,8,9,10,11,11a,11b,12,13-hexadecahydro-1H-2-aza-pentaleno[1,6a-a]phenanthren-9-yl ester

The title compound was prepared according to procedures described in Example 134, except substituting methoxyacetyl chloride for acetyl chloride. ¹H NMR (CDCl₃): δ 0.96 (s, 3 H) 1.00-2.30 (m, 25 H) 2.35 (d, *J*=7.46 Hz, 3 H) 3.01 (m, 1 H) 3.45 (s, 3 H) 4.01 (s, 2 H) 4.71 (m, 1 H) 5.40 (m, 1 H); MS: (M+H)⁺= 402.

### Example 142

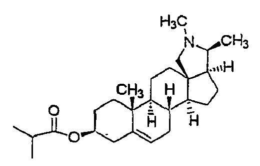

### Isobutyric acid 2,3,11a-trimethyl-2,3,3a,4,5,5a,5b,6,8,9,10,11,11a,11b,12,13-hexadecahydro-1H-2-aza-pentaleno[1,6a-a]phenanthren-9-yl ester

The title compound was prepared according to procedures described in Example 134, except substituting isobutyryl chloride for acetyl chloride. ¹H NMR (CDCl₃): δ 0.97 (s, 3 H) 1.15 (d, *J*=7.12 Hz, 6 H) 1.00-2.30 (m, 26 H) 2.32 (s, 3 H) 3.01 (m, 1 H) 4.63 (m, 1 H) 5.37 (m, 1 H); MS: (M+H)⁺= 400.

### Example 143

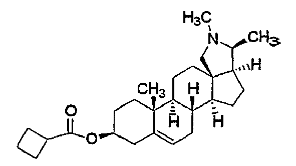

### Cyclobutanecarboxylic acid 2,3,11a-trimethyl-2,3,3a,4,5,5a,5b,6,8,9,10,11,11a. 11b,12,13-hexadecahydro-1H-2-aza-pentaleno[1,6a-a]phenanthren-9-yl ester

The title compound was prepared according to procedures described in Example 134, except substituting cyclobutanecarbonyl chloride for acetyl chloride. ¹H NMR (CDCl₃): δ 0.96 (s, 3 H) 1.00-2.35 (m, 35 H) 3.10 (m, 1 H) 4.61 (m, 1 H) 5.38 (m, 1 H); MS: (M+H)⁺= 412.

### Example 144

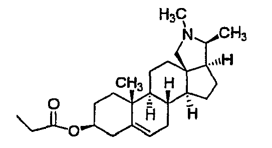

### Propionic acid 2,3,11a-trimethyl-2,3,3a,4,5,5a,5b,6,8,9,10,11,11a,11b,12,13-hexadecahydro-1H-2-aza-pentaleno[1,6a-a]phenanthren-9-yl ester

The title compound was prepared according to procedures described in Example 134, except substituting propionyl chloride for acetyl chloride. ¹H NMR (CDCl₃): δ 0.96 (s, 3 H) 1.08-2.38 (m, 33 H) 3.59 (m, 1 H) 4.61 (m, 1 H) 5.38 (m, 1 H); MS: (M+H)⁺= 386.

### Example 145

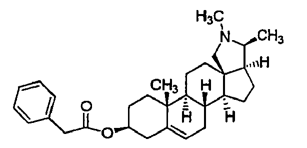

### Phenyl-acetic acid 2,3,11a-trimethyl-2,3,3a,4,5,5a,5b,6,8,9,10,11,11a,11b,12,13-hexadecahydro-1H-2-aza-pentaleno[1,6a-a]phenanthren-9-yl ester

The title compound was prepared according to procedures described in Example 134, except substituting phenylacetyl chloride for acetyl chloride. MS: (M+H)⁺= 448.

### Example 146

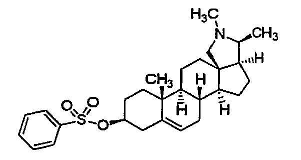

### Benzenesulfonic acid 2,3,11a-trimethyl-2,3,3a,4,5,5a,5b,6,8,9,10,11,11a,11b,12,13-hexadecahydro-1H-2-aza-pentalenor[1,6a-a]phenanthren-9-yl ester

The title compound was prepared according to procedures described in Example 134, except substituting benzenesulfonyl chloride for acetyl chloride. ¹H NMR (CDCl₃): δ 0.92 (m, 3 H) 1.05-2.50 (m, 28 H) 2.94 (m, 1 H) 4.37 (m, 1 H) 5.31 (m, 1 H) 7.54 (m, 2 H) 7.63 (m, 1 H) 7.92 (m, 2 H); MS: (M+H)⁺= 470.

### Example 147

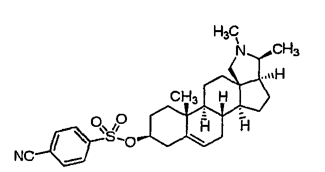

### 4-Cyano-benzenesulfonic acid 2,3,11 a-trimethyl-2,3,3a,4,5,5a,5b,6, 8,9,10,11,11a,11b, 12,13-hexadecahydro-1H-2-aza-pentaleno[1,6a-a]phenanthren-9-yl ester

The title compound was prepared according to procedures described in Example 134, except substituting 4-cyanobenzenesulfonyl chloride for acetyl chloride. ¹H NMR (CDCl₃): δ 0.92 (m, 3 H) 1.05-2.50 (m, 28 H) 2.94 (m, 1 H) 4.37 (m, 1 H) 5.38 (m, 1 H) 7.85 (m, 2 H) 8.02 (m, 2 H); MS: (M+H)⁺= 495.

### Example 148

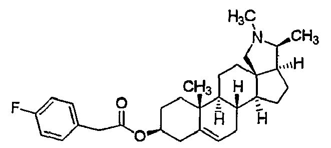

### (4-Fluoro-phenyl)-acetic acid 2,3,11a-trimethyl-2,3,3a,4,5,5a,5b,6,8,9,10,11,11a,11b, 12,13-hexadecahydro-1H-2-aza-pentaleno[1,6a-a]phenanthren-9-yl ester

A mixture of compound 130A (20 mg, 0.06 mmol), 4-fluoroph enylacetic acid (14 mg, 0.09 mmol), 1,3-dicyclohexylcarbodiimide (25 mg, 0.12 mmol), 4-(dimethylamino) pyridine (2 mg, 0.016 mmol) and THF (1 mL) was stirred at room temperature overnight. The crude reaction mixture was loaded directly onto silica gel column and eluted with 0.2% ammonium hydroxide and 2 % methanol in dichloromethane to give the pure desired product (25 mg, 87% yield).

¹H NMR (CDCl₃): δ 0.96 (s, 3H) 1.08-2.38 (m, 27 H) 3.01 (s, 2H) 3.45 (m, 1 H) 4.00 (m, 1 H), 4.60 (m, 1 H) 5.37 (m, 1 H) 7.01 (m, 2H) 7.23 (m, 2H)
MS: (M+H)⁺= 466

### Example 149

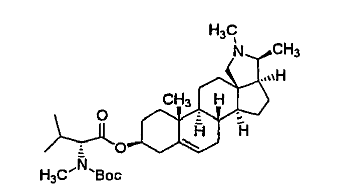

### 2-(tert-Butoxycarbonyl-methyl-amino)-3-methyl-butyric acid 2,3,11a-trimethyl-2,3,3a,4,5,5a,5b,6,8,9,10,11,11a,11b,12,13-hexadecahydro-1H-2-aza-pentaleno [1,6a-a]phenanthren-9-yl ester

The title compound was prepared according to procedures described in Example 148, except substituting Boc-N-methyl-D-valine for 4-fluorophenylacetic acid. MS: (M+H)⁺= 543.

### Example 150

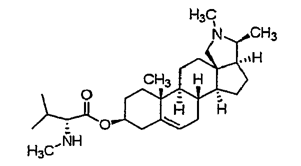

### 3-Methyl-2-methylamino-butyric acid-2,3,11a-trimethyl-2,3,3a,4,5,5a,5b,6,8,9,10,11,11a,11b,12,13-hexadecahydro-1H-2-aza-pentaleno [1,6,a-a]phenanthren-9-yl ester

Compound 149 (10 mg, 0.018 mmol) was stirred with 1:1 mixture of TFA and dichloromethane (1 mL) for 2h. Solvent was evaporated under reduced pressure, and the residue was triturated with ethyl acetate twice and dried on high vacuum to g ive the desired product.
MS: (M+H)⁺= 443

### Example 151

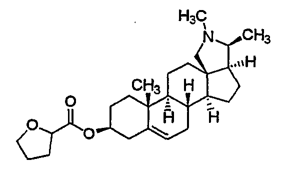

### Tetrahydro-furan-2-carboxylic acid 2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl ester

The title compound was prepared according to procedures described in Example 148, except substituting tetrahydro-2-furoic acid for 4-fluorophenylacetic acid. ¹H NMR (CDCl₃): δ 0.96 (s, 3 H) 1.00-2.60 (m, 30 H) 3.01 (s, 3 H) 3.95 (m, 2 H) 4.42 (m, 1 H) 4.67 (m, 1 H) 5.38 (m, 1 H); MS: (M+H)⁺= 427.

### Example 152

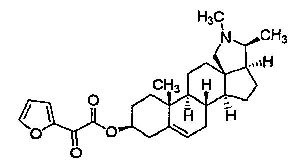

### Furan-2-yl-oxo-acetic acid 2,3,11a-trimethyl-2,3,3a,4,5,5a,5b,6,8,9,10,11,11a,11b,12,13-hexadecahydro-1H-2-aza-pentaleno[1,6a-a]phenanthren-9-yl ester

The title compound was prepared according to procedures described in Example 148, except substituting 2-oxo-2-furanylacetic acid for 4-fluorophenylacetic acid. ¹H NMR (CDCl₃): δ 1.00 (s, 3 H) 1.05-2.60 (m, 26 H) 3.02 (s, 3 H) 4.87 (m, 1 H) 5.43 (m, 1 H) 6.62 (dd, *J=*3.73, 1.70 Hz, 1 H) 7.69 (d, J=3.05 Hz, 1 H) 7.75 (s, 1 H); MS: (M+H)⁺= 452.

### Example 153

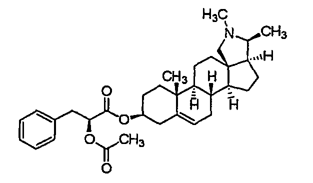

### 2-Acetoxy-3-phenyl-propionic acid 2,3,11a-trimethyl-2,3,3a,4,5,5a,5b,6,8,9,10,11,11a. 11b,12,13-hexadecahydro-1H-2-aza pentaleno[1,6a-a]phenanthren-9-yl ester

The title compound was prepared according to procedures described in Example 148, except substituting L-2-acetoxy-3-phenylpropionic acid for 4-fluorophenylacetic acid and the corresponding carboxylic acid was prepared according to the procedure used in Example 91. ¹H NMR (CDCl₃): δ 0.96 (s, 3 H) 1.08-2.40 (m, 29 H) 2.09 (s, 3 H) 3.11 (m, 2 H) 4.62 (m, 1 H) 5.15 (dd, *J*=8.14, 5.09 Hz, 1 H) 5.36 (m, 1 H) 7.29 (m, 5 H); MS: (M+H)⁺= 520, (M+NH₄)⁺=537.

### Example 154

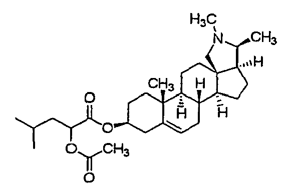

### 2-Acetoxy-4-methyl-pentanoic acid 2,3,11a-trimethyl-2,3,3a,5,5a,5b,6,5,9,10,11,11a, 11b,12,13-hexadecahydro-1H-2-aza-entaleno[1,6a-a]phenanthren-9-yl ester

The title compound was prepared according to procedures described in Example 148, except substituting 2-acetoxyisocaproic acid for 4-fluorophenylacetic acid and the corresponding carboxylic acid was prepared according to the procedure used in Example 91. ¹H NMR (CDCl₃): δ 0.95 (m, 9 H) 1.00-2.00 (m, 28 H) 2.13 (s, 3 H) 2.33 (m, 3 H) 2.78 (m, 1 H) 4.64 (m, 1 H) 4.96 (m, 1 H) 5.38 (m, 1 H); MS: (M+H)⁺= 486.

### Example 155

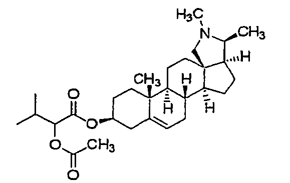

### 2-Acetoxy-3-methyl-butyric acid 2,3,11a-trimethyl-2,3,3a,4,5,5a,5b,6,8,9,10,11,11a,11b, 12,13-hexadecahydro-1H-2-aza-pentaleno[1,6a-a]phenanthren-9-yl ester

The title compound was prepared according to procedures described in Example 148, except substituting 2-acetoxy-3-methylbutyric acid for 4-fluorophenylacetic acid and the corresponding carboxylic acid was prepared according to the procedure used in Example 91. ¹H NMR (CDCl₃): δ 0.99 (m, 12 H) 1.10-2.05 (m, 22 H) 2.14 (s, 3 H) 2.26 (m, 5 H) 4.68 (m, 1 H) 4.78 (d, *J*=4.75 Hz, 1 H) 5.39 (m, 1 H); MS: (M+H)⁺= 472.

### Example 156

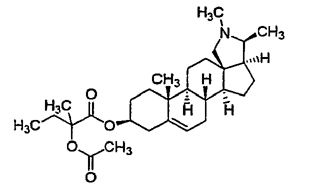

### 2-Acetoxy-2-methyl-butyric acid 2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl ester

The title compound was prepared according to procedures described in Example 148, except substituting 2-hydroxy-2-methylbutyric acid for 4-fluorophenylacetic acid and the corresponding carboxylic acid was prepared according to the procedure used in Example 91. ¹H NMR (CDCl₃): δ 0.92 (m, 9 H) 1.10-1.95 (m, 26 H) 2.03 (d, 3 H) 2.31 (m, 3 H) 2.52 (m, 2 H) 4.62 (m, 1 H) 5.38 (m, 1 H); MS: (M+H)⁺= 472.

### Example 157

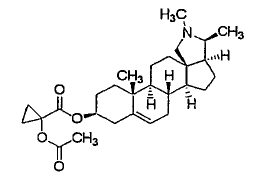

### 1-Acetoxy-cyclopropanecarboxylic acid 2,3,11a-trimethyl-2,3,3a,4,5,5a,5b,6,8,9,10,11, 11a,11 b,12,13-hexadecahydro-1H-2-aza-pentaleno[16a-a]phenanthren-9-yl ester

The title compound was prepared according to procedures described in Example 148, except substituting 1-acetoxy-1-cyclopropanecarboxylic acid for 4-fluorophenylacetic acid and the corresponding carboxylic acid was prepared according to the procedure used in Example 91. ¹H NMR (CDCl₃): δ 0.93 (s, 3 H) 1.15-1.90 (m, 27 H) 2.10 (s, 3 H) 2.15-2.90 (m, 6 H) 4.61 (m, 1 H) 5.37 (m, 1 H); MS: (M+H)⁺= 456.

### Example 158

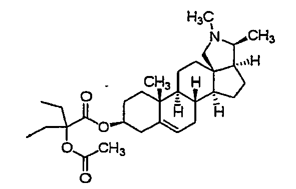

### 2-Acetoxy-2-ethyl-butyric acid 2,3,11a-trimethyl-2,3,3a,4,5,5a,5b,6,8,9 10,11,11 a, 11b,12,13-hexadecahydro-1H-2-aza-pentaleno[1,6a-a]phenanthren-9-yl ester

The title compound was prepared according to procedures described in Example 148, except substituting 2-ethyl-2-acetoxybutyric acid for 4-fluorophenylacetic acid and the corresponding carboxylic acid was prepared according to the procedure used in Example 91. ¹H NMR (CDCl₃): δ 0.85 (t, *J*=7.46 Hz, 6 H) 0.96 (s, 3 H) 1.00-2.00 (m, 27 H) 2.07 (s, 3 H) 2.10-2.35 (m, 6 H) 4.65 (m, 1 H) 5.38 (m, 1 H); MS: (M+H)⁺= 486.

### Example 159

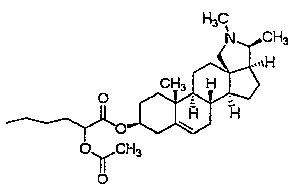

### 2-Acetoxy-hexanoic acid 2,3,11a-trimethyl-2,3,3a,4,5,5a,5b,6,8,9,10,11,11a,11b,12,13-hexadecahydro-1H-2-aza-pentaleno[1,6a-a]phenanthren-9-yl ester

The title compound was prepared according to procedures described in Example 148, except substituting 2-acetoxycaproic acid for 4-fluorophenylacetic acid and the corresponding carboxylic acid was prepared according to the procedure used in Example 91. ¹H NMR (CDCl₃): δ 0.93 (m, 9 H) 1.00-2.00 (m, 26 H) 2.13 (s, 3 H) 2.34 (m, 6 H) 4.66 (m, 1 H) 4.93 (t, *J*=6.44 Hz, 1 H) 5.39 (m, 1 H); MS: (M+H)⁺= 503.

### Example 160

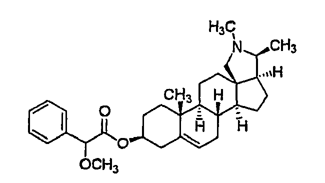

### Methoxy-phenyl-acetic acid 2,3,11a-trimethyl-2,3,3a,4,5,5a,5b,6,8,9,10,11, 11a,11b,12,13-hexadecahydro-1H-2-aza-pentaleno[1,6a-a]phenanthren-9-yl ester

The title compound was prepared according to procedures described in Example 148, except substituting 2-methoxyphenylacetic acid for 4-fluorophenylacetic acid. ¹H NMR (CDCl₃): δ 0.93 (s, 3 H) 1.00-2.30 (m, 27 H) 2.33 (d, *J*=7.80 Hz, 3 H) 3.41 (s, 3 H) 4.68 (m, 1 H) 5.35 (m, 1 H) 7.36 (m, 3 H) 7.44 (m, 2 H); MS: (M+H)⁺= 478, (M+NH₄)⁺=495.

### Example 161

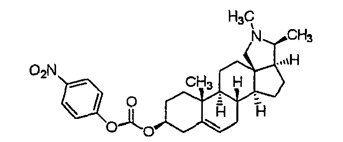

### Carbonic acid 4-nitro-phenyl ester 2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl ester

Compound 130A (250 mg, 0.76 mmol) was dissolved in 10 mL dichloromethane and cooled to 0°C. 4-Nitrophenylchloroformate (170 mg, 0.84 mmol) was added followed by N-methylmorpholine (125 µL, 1.14 mmol). The mixture was stirred overnight while warmed up slowly to room temperature. TLC indicated that the reaction was not completed. 4-Nitrophenylchloroformate (309 mg, 1.52mmol) and N-methylmorpholine (250 µL, 2.28mmol) were added and stirred at room temperature for 3 hours. Reaction was complete. It was loaded on silica gel column and eluted with 0.5% ammonium hydroxide and 5 % methanol in dichloromethane to give the paranitrophenol-carbonate (PNP-carbonate, 260 mg, 69% yield).

¹H NMR (CDCl₃): δ 0.98 (s, 3 H) 1.05-2.60 (m, 26 H) 2.79 (m, 2 H) 3.69 (m, 1 H) 4.60 (m, 1 H) 5.45 (m, 1 H) 7.39 (m, 2 H) 8.28 (m, 2 H)
MS: (M+H)⁺= 495

### Example 162

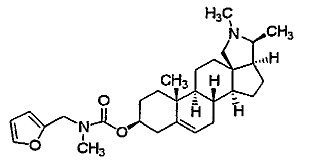

### Furan-2-ylmethyl-methyl-carbamic acid 2,3,11 a-trimethyl-2,3,3a,4,5,5a,5b,6,8,9,10,11 11a,11b,12,13-hexadecahydro-1H-2-aza-pentaleno[1,6a-a]phenanthren-9-yl ester

Compound 161 (25 mg, 0.051 mmol), N-methylfurylamine (7 µL, 0.061 mmol), dichloromethane (0.5 mL) and THF (1 mL) were mixed and stirred at room temperature overnight. The crude reaction mixture was purified on silica gel column, which was eluted with 0.3% ammonium hydroxide and 3 % methanol in dichloromethane to give the desired product (21 mg, 87% yield).
MS: (M+H)⁺= 467

### Example 163

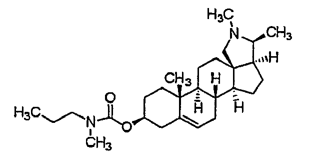

### Methyl-propyl-carbamic acid 2,3,11 a-tdmethyl-2,3,3a,4,5,5a,5b,6,8,9,10,11,11a, 11b,12,13-hexadecahydro-1H-2-aza-pentaleno[16a-a]phenanthren-9-yl ester

The title compound was prepared according to procedures described in Example 162, except substituting methylpropylamine for N-methylfurylamine. MS: (M+H)⁺= 429.

### Example 164

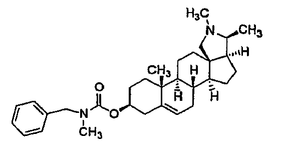

### Benzyl-methyl-carbamic acid 2,3,11a-trimethyl-2,3,3a,4,5,5a,5b,6,8,9,10,11,11a,11,b, 12,13-hexadecahydro-1H-2-aza-pentaleno[1,6a-a]phenanthren-9-yl ester

The title compound was prepared according to procedures described in Example 162, except substituting N-benzylmethylamine for N-methylfurylamine. ¹H NMR (CDCl₃): δ 0.96 (s, 3 H) 1.04-2.42 (m, 27H) 2.87 (m, 4 H) 2.95 (m, 1 H) 4.46 (s, 2 H) 4.57 (m, 1 H) 5.40 (m, 1 H) 7.29 (m, 5 H); MS: (M+H)⁺= 477, (M+NH⁴)⁺ =494.

### Example 165

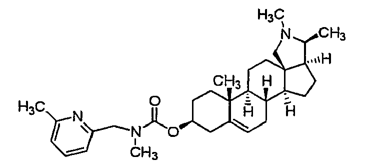

### Methyl-(6-methyl-pyridin-2-ylmethyl)-carbamic acid 2,3,11a-trimethyl-2,3,3a, 4,5,5a,5b,6,8,9,10,11,11a,11b,12,13-hexadecahydro-1H-2-aza-pentaleno[1,6a-a]phenanthren-9-yl ester

The title compound was prepared according to procedures described in Example 162, except substituting 6-methyl-2-picolyl-methylamine for N-methylfurylamine. ¹H NMR (CDCl₃): δ 0.91 (s, 3 H) 0.98 (s, 3 H) 1.15-2.45 (m, 26 H) 2.54 (s, 3 H) 2.97 (m, 4 H) 4.56 (m, 2 H) 5.38 (m, 1 H) 7.03 (m, 2 H) 7.55 (m, 1 H); MS: (M+H)⁺= 492.

### Example 166

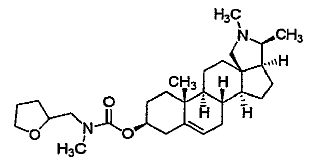

### Methyl-(tetrahydro-furan-2-ylmethyl)-carbamic acid 2,3,11 a-trimethyl-2,3,3a,4,5,5a,5b 6,8,9,10,11,11a,11b,12,13-hexadecahydro-1H-2-aza-pentaleno[1,6a-a]phenanthren-9-yl ester

The title compound was prepared according to procedures described in Example 162, except substituting N-methyltetrahydrofurfurylamine for N-methylfurylamine. ¹H NMR (CDCl₃): δ 0.96 (s, 3 H) 1.03-2.40 (m, 33 H) 2.97 (s, 3 H) 3.10-3.60 (m, 2 H) 3.79 (m, 2 H) 4.05 (m, 1 H) 4.51 (m, 1 H) 5.38 (m, 1 H); MS: (M+H)⁺= 471.

### Example 167

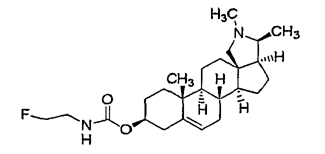

### (2-Fluoro-ethyl)-carbamic acid 2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl ester

The title compound was prepared according to procedures described in Example 162, except substituting 2-fluoroethylaminehydrochloride for N-methylfurylamine. ¹H NMR (CDCl₃): δ 0.96 (s, 3 H) 1.00-1.95 (m, 38 H) 2.05-2.40 (m, 8 H) 3.43 (m, 1 H) 3.53 (m, 1 H) 4.41 (t, *J*=4.75 Hz, 2 H) 4.50 (m, 1 H) 4.57 (t, *J*=4.75 Hz, 2 H) 4.93 (m, 1 H) 5.39 (m, 1 H); MS: (M+H)⁺= 419, (M+NH⁴)⁺ = 436.

### Example 168

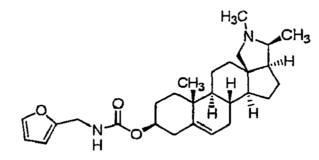

### Furan-2-ylmethyl-carbamic acid 2,3,11a-trimethyl-2,3,3a,4,5,5a,5b,6,8,9,10,11,11a, 11b,12,13-hexadecahydro-1H-2-aza-pentaleno[1,6a-a]phenanthren-9-yl ester

The title compound was prepared according to procedures described in Example 162, except substituting furfurylamine for N-methylfurylamine. ¹H NMR (CDCl₃): δ 0.95 (s, 3 H) 1.00-2.40 (m, 28 H) 3.02 (m, 1 H) 4.34 (d, *J*=5.43 Hz, 2 H) 4.52 (m, 1 H) 4.90 (m, 1H) 5.39 (m, 1 H) 6.22 (d, *J*=2.71 Hz, 1 H) 6.31 (dd, *J*=3.22, 1.86 Hz, 1 H) 7.35 (s, 1 H); MS: (M+H)⁺= 453.

### Example 169

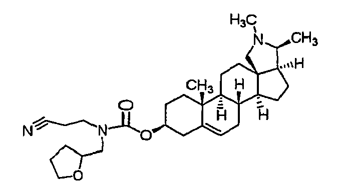

### (2-Cyano-ethyl)-(tetrahydro-furan-2-ylmethyl)-carbamic acid 2,3,11a-trimethyl-2,3, 3a,4,5,5a,5b,6,8,9,10,11,11a,11b,12,13-hexadecahydro-1H-2-aza-pentaleno[1,6a-a]phenanthren-9-yl ester

The title compound was prepared according to procedures described in Example 162, except substituting 3-[(tetrahydrofuran-2-ylmethyl)amino]propane nitrile for N-methylfurylamine. ¹H NMR (CDCl₃): δ 0.95 (s, 3 H) 1.00-2.40 (m, 34 H) 2.71 (m, 2 H) 3.60 (m, 2 H) 3.73 (m, 2 H) 3.85 (m, 1 H) 4.01 (m, 1 H) 4.54 (m, 1 H) 5.38 (m, 1 H); MS: (M+H)⁺= 510.

### Example 170

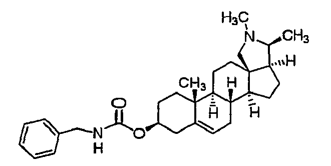

### Benzyl-carbamic acid 2,3,11a-trimethyl-2,3,3a,4,5,5a,5b,6,8,9,10,11,11a,11b,12,13-hexadecahydro-1H-2-aza-pentaleno[1,6a-a]phenanthren-9-yl ester

The title compound was prepared according to procedures described in Example 162, except substituting benzylamine for N-methylfurylamine. MS: (M+H)⁺= 451.

### Example 171

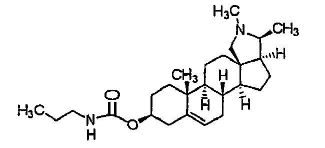

### Propyl-carbamic acid 2,3,11a-trimethyl-2,3,3a,4,5,5a,5b,6,8,9,10,11,11a,11b,12,13-hexadecahydro-1H-2-aza-pentaleno[1,6a-a]phenanthren-9-yl ester

The title compound was prepared according to procedures described in Example 162, except substituting propylamine for N-methylfurylamine. MS: (M+H)⁺= 403.

### Reference Example 172

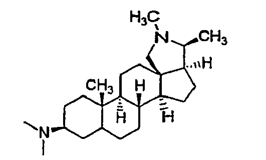

### 172A. Dimethyl-(2,3,11a-trimethyl-octadecahydro-2-aza-pentaleno[1,6a-a]phenanthren-9-yl)-amine

Conessine (50 mg, 0.14 mmol) was dissolved in 3 mL ethyl acetate and 10% Pd/C (20 mg) was added under inert atmosphere. The reaction mixture was hydrogenated under a hydrogen balloon for 24 hours. Reaction was not complete by TLC. The reaction mixture was filtered through diatomaceous earth, washed with ethyl acetate, and the filtrate was concentrated to give the crude product which was purified by silica gel column eluted with 0.3% ammonium hydroxide and 3 % methanol in dichloromethane to give the desired product (15 mg, 30 % yield).

¹H NMR (CDCl₃): δ 0.74 (s, 3 H) 0.95-2.00 (m, 29 H) 2.32 (s, 3 H) 2.43 (s, 6 H) 3.12 (m, 1 H)
MS: (M+H)⁺= 359

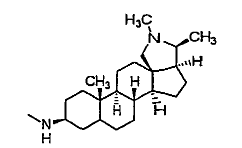

### 172B. Methyl-(2,3,11a-trimethyl-octadecahydro-2-aza-pentaleno[1,6a-a]phenanthren-9-yl)-amine

The title compound was prepared using the procedures described in Example 7A and 7B, except substituting Compound 172A for connessine. ¹H NMR (CDCl₃): δ 0.75 (s, 3 H) 0.90-2.00 (m, 25 H) 2.29 (m, 3 H) 2.51 (s, 3 H) 2.62 (m, 1 H) 2.93 (s, 3 H) 3.09 (m, 1 H) 4.13 (m, 1 H); (M+H)⁺=345.

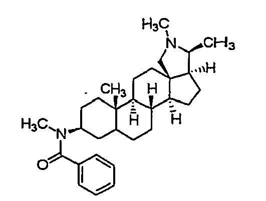

### 172C. N-Methyl-N-(2,3,11a-trimethyl-octadecahydro-2-aza-pentaleno(1,6a-a]henanthren-9-yl)-benzamide

The title compound was prepared according to procedures described in Example 7C, except substituting Compound 172B for Compound 7B and benzoyl chloride for acetyl chloride. ¹H NMR (CDCl₃): δ 0.75 (s, 3 H) 0.85-2.00 (m, 26 H) 2.21 (m, 3 H) 2.80 (s, 3 H) 2.98 (s, 3 H) 3.46 (m, 1 H) 7.39 (m, 5 H); (M+H)⁺=449.

### Example 173

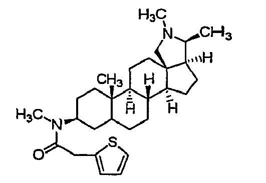

### N-Methyl-2-thiophen-2-yl-N-(2,3,11a-trimethyl-octadecahydro-2-aza-pentaleno[1,6a-a]phenanthren-9-yl)-acetamide

The title compound was prepared according to procedures described in Example 7C, except substituting Compound 172B for Compound 7B and 2-thiopheneacetyl chloride for acetyl chloride. ¹H NMR (CDCl₃): δ 0.74 (s, 3 H) 0.95-2.40 (m, 27 H) 2.83 (s, 3 H) 2.88 (s, 3 H) 3.01 (m, 1 H) 3.69 (m, 1 H) 3.90 (d, *J*=11.53 Hz, 2 H) 4.49 (m, 1 H) 6.92 (m,2 H) 7.19 (m, 1 H); (M+H)⁺=469.

### Example 174

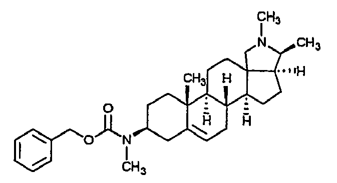

### Methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid benzyl ester

Compound 7B (20 mg, 0.058 mmol) and triethylamine (9-1 µL, 0.064 mmol) were dissolved in dichloromethane (1 mL). Benzyl chloroformate was added to it. The mixture was stirred at room temperature for 2 hours. The crude reaction mixture was purified on silica gel column using 0.5% ammonium hydroxide and 5 % methanol in dichloromethane to give the desired product (18.4 mg, 66.2% yield).

¹H NMR (CDCl₃): δ ppm 0.94 (s, 3 H) 0.98-1.90 (m, 21 H) 2.02 (m, 2 H) 2.21 (s, 3 H) 2.43 (m, 2 H) 2.84 (s, 3 H) 2.98 (m, 1 H) 3.89 (m, 1 H) 5.14 (s, 2 H) 5.35 (d, *J*=5.09 Hz, 1 H) 7.32 (m, 5 H); MS: (M+H)⁺= 477.

### Example 175

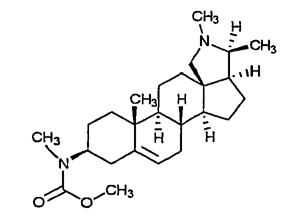

### Methyl-(2,3,11a-trimethyl-2,3,3a,4,5,5a,5b,6,8,9,10,11,11a,11b,12,13-hexadecahydro-1H-2-aza-pental eno[1,6a-a]phenanthren-9-yl)-carbamic acid methyl ester

The title compound was prepared according to procedures described in Example 174, except substituting methyl chloroformate for benzyl chloroformate. MS: (M+H)⁺= 401.

### Example 176

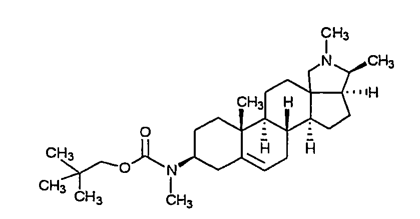

### Methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid 2,2-dimethyl-propyl ester

The title compound was prepared according to procedures described in Example 174, except substituting neopentyl chloroformate for benzyl chloroformate. ¹H NMR (CDCl₃): δ 0.95 (s, 3H) 0.96 (s, 9 H) 0.98-1.70 (m, 17 H) 1.78 (td, 2 H) 1.90 (m, 2 H) 2.05 (td, 2 H) 2.26 (s, 3 H) 2.43 (td, 2 H) 2.83 (s, 3 H) 3.04 (m, 1 H) 3.77 (s, 2 H) 3.92 (m, 1 H) 5.35 (d, *J*=5.09 Hz, 1 H); MS: (M+H)⁺= 457.

### Example 177

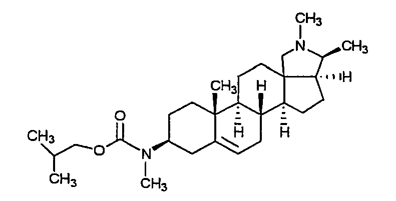

### Methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid isobutyl ester

The title compound was prepared according to procedures described in Example 174, except substituting isobutyl chloroformate for benzyl chloroformate. ¹H NMR (CDCl₃): δ 0.94 (d, *J*=6.78 Hz, 6 H) 0.95 (s, 3H) 1.01-2.10 (m, 24 H) 2.24 (s, 3 H) 2.42 (m, 2 H) 2.82 (s, 3 H) 3.01 (m, 1 H) 3.86 (d, *J*=6.44 Hz, 2 H) 3.94 (m, 1 H) 5.35 (d, *J*=5.10 Hz, 1 H); MS: (M+H)⁺= 443.

### Example 178

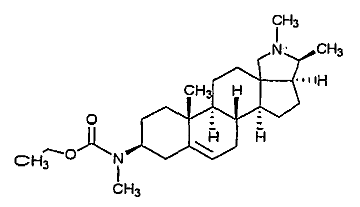

### Methyl-(2,3,11a-trimethyl-2,3,3a,4,5,5a,5b,6,8,9,10,11,11a,11b,12,13-hexadecahydro 1H-2-aza-pentaleno[1,6a-alphenanthren-9-yl)-carbamic acid ethyl ester

The title compound was prepared according to procedures described in Example 174, except substituting ethyl chloroformate for benzyl chloroformate. ¹H NMR (CDCl₃): δ 0.95 (s, 3 H) 1.26 (t, *J*=7.12 Hz, 3 H) 0.97-1.91 (m, 21 H) 2.02 (m, 2 H) 2.19 (s, 3 H) 2.41 (td, 2 H) 2.81 (s, 3 H) 3.00 (m, 1 H) 3.86 (m, 1 H) 4.13 (q, *J*=7.12 Hz, 2 H) 5.36 (d, *J*=5.09 Hz, 1 H); MS: (M+H)⁺= 415.

### Example 179

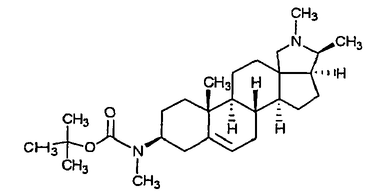

### Methyl-(2,3,11a-trimethyl-2,3,3a,4,5,5a,5b,6,8,9,10,11,11a,11,b,12,13-hexadeca hydro-1H-2-aza-pentaleno[1,6a-a]phenanthren-9-yl)-carbamic acid tert-butyl ester

The title compound was prepared according to procedures described in Example 174, except substituting tert-butyl chloroformate for benzyl chloroformate. ¹H NMR (CDCl₃): δ 0.94 (s, 3 H) 1.04 (d, *J*=6.10 Hz, 3 H) 1.46 (s, 9 H) 1.54 (s, 3 H) 1.10-1.89 (m, 15 H) 2.02 (m, 2 H) 2.21 (s, 3 H) 2.36 (m, 2 H) 2.76 (s, 3 H) 2.99 (d, *J*=10.51 Hz, 1 H) 3.83 (m, 1 H) 5.35 (d, *J*=5.42 Hz, 1 H); MS: (M+H)⁺= 443.

### Example 180

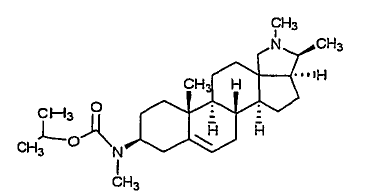

### Methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid isopropyl ester

The title compound was prepared according to procedures described in Example 174, except substituting isopropyl chloroformate for benzyl chloroformate. ¹H NMR (CDCl₃): δ 0.95 (s, 3 H) 1.24 (d, *J*=6.44 Hz, 6 H) 1.79 (m, 23 H) 2.22 (s, 3 H) 2.41 (m, 2 H) 2.80 (s, 3 H) 2.99 (m, 1 H) 3.88 (m, 1 H) 4.92 (m, 1 H) 5.35 (d, *J*=3.00 Hz, 1 H); MS: (M+H)⁺= 429.

### Example 181

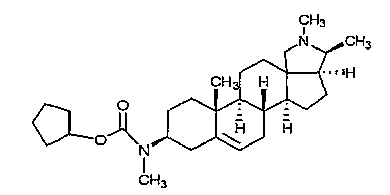

### Methyl-12,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid cyclopentyl ester

The title compound was prepared according to procedures described in Example 174, except substituting cyclopentyl chloroformate for benzyl chloroformate. ¹H NM R (CDClₛ): δ 0.94 (s, 3 H) 1.54 (m, 29 H) 2.00 (m, 2 H) 2.22 (s, 3 H) 2.41 (m, 2 H) 2.79 (s, 3 H) 3.00 (m, 1 H) 3.81 (m, 1 H) 5.11 (m, 1 H) 5.35 (d, *J*=5.42 Hz, 1 H); MS: (M+H)⁺= 455.

### Examp le 182

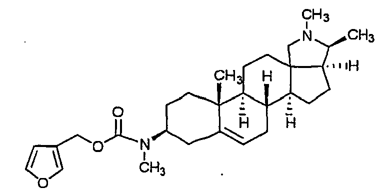

### Methyl-(2,3,11a-trimethyl-2.3,3a,4,5,5a,5b,6,8,9,10,11,11a,11b,12,13-hexadecahydro-1H-2-aza-pentaleno[1,6a-a]phenanthren-9-yl)-carbamic acid furan-3-ylmethyl ester

Compound 7B (10 mg, 0.029 mmol), carbonic acid furan-3-ylmethyl ester 4-nitro-phenyl ester (2.4 mg, 0.032 mmol) and triethylamine (12.0 µL, 0.087 mmol) were dissolved in THF (1 mL) and stirred at room temperature overnight. The crude reaction mixture was purified on silica gel column using 0.5% ammonium hydroxide and 5 % methanol in dichloromethane to give the desired product (10.1 mg, 74.2% yield).

¹H NMR (CDCl₃): δ 0.94 (s, 3 H) 1.04-2.04 (m, 23 H) 2.24 (s, 3 H) 2.42 (m, 2 H) 2.81 (s, 3 H) 3.03 (m, 1 H) 3.85 (m, 1 H) 4.99 (s, 2 H) 5.35 (d, *J*=5.09 Hz, 1 H) 6.43 (s, 1 H) 7.39 (t, *J*=1.70 Hz, 1 H) 7.46 (s, 1 H); MS: (M+H)⁺= 467.

### Example 183

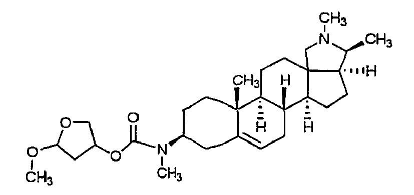

### Methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid 5-methoxy-tetrahydrofuran-3-yl ester

The title compound was prepared according to procedures described in Example 182, except substituting carbonic acid 5-methoxy-tetrahydro-furan-3-yl ester 4-nitro-phenyl ester for carbonic acid furan-3-ylmethyl ester 4-nitro-phenyl ester. ¹H NMR (CDCl₃): δ 0.95 (m, 3 H) 0.99-2.04 (m, 25 H) 2.22 (s, 3 H) 2.40 (t, *J*=11.53 Hz, 2 H) 2.78 (s, 3 H) 2.98 (d, *J*=2.71 Hz, 1 H) 3.35 (s, 3 H) 3.69 (m, 1 H) 3.93 (d, *J*=10.17 Hz, 1 H) 4.03 (dd, *J*=10.50, 4.50 Hz, 1 H) 5.17 (dd, *J*=4.92, 2.88 Hz, 1 H) 5.28 (m, 1 H) 5.35 (d, *J*=5.00 Hz, 1 H); MS: (M+H)⁺= 487.

### Example 184

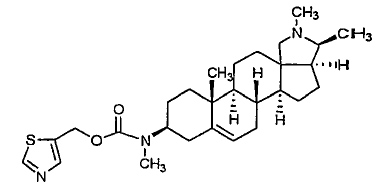

### Methyl-(2,3,11a-trimethyl-2,3,3a,4,5,5a,5b,6,8,9,10,11,11a,11b 12 13-hexadecahydro-1H-2-aza-pentaleno[1,6a-a]phenanthren-9-yl)-carbamic acid thiazol-5-ylmethyl ester

The title compound was prepared according to procedures described in Example 182, except substituting carbonic acid 4-nitro-phenyl ester thiazol-5-ylmethyl ester for carbonic acid furan-3-ylmethyl ester 4-nitro-phenyl ester. ¹H NMR (CDCl₃): δ 0.94 (s, 3 H) 1.05-2.11 (m, 23 H) 2.22 (s, 3 H) 2.40 (m, 2 H) 2.82 (d, 3 H) 2.99 (m, 1 H) 3.83 (m, 1 H) 5.33 (s, 2 H) 5.35 (m, 1 H) 7.87 (s, 1 H) 8.80 (s, 1 H); M S: (M+H)⁺= 484.

### Example 185

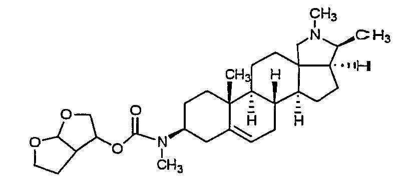

### Methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid hexahydro-furo[2,3-blfuran-3-yl ester

The title compound was prepared according to procedures described in Example 182, except substituting carbonic acid hexahydro-furo[2,3-b]furan-3-yl ester 4-nitro-phenyl ester for carbonic acid furan-3-ylmethyl ester 4-nitro-phenyl ester. ¹H NMR (CDCl₃): δ 0.95 (s, 3 H) 0.99-2.04 (m, 25 H) 2.21 (s, 3 H) 2-43 (m, 2 H) 2.83 (d, 3H) 2.99 (d, *J*=9.83 Hz, 1 H) 3.05 (m, 1 H) 3.73 (m, 1 H) 3.81 (dd, *J*=9.49, 6.44 Hz, 1 H) 3.90 (m, 1 H) 3.98 (m, 1 H) 4.08 (m, 1 H) 5.19 (d, *J*=7.80 Hz, 1 H) 5.37 (d, *J*=4.75 Hz, 1 H) 5.73 (d, *J*=5.43 Hz, 1 H); MS: (M+H)⁺= 499.

### Example 186

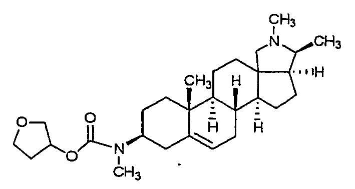

### Methyl-(2,3,11a-trimethyl-2,3,3a,4,5,5a,5b,6,8,9,10,11,11a11b,12,13-hexadecahydro-1H-2-aza-pentaleno[1,6a-a]phenanthren-9-yl)-carbamic acid tetrahydro-furan-3-yl ester

The title compound was prepared according to procedures described in Example 182, except substituting carbonic acid 4-nitro-phenyl ester tetrahydro furan-3-yl ester for carbonic acid furan-3-ylmethyl ester 4-nitro-phenyl ester. ¹H NMR (CDCl₃): δ 0.96 (m, 3 H) 1.01-2.22 (m, 28 H) 2.41 (m, 2 H) 2.80 (s, 3 H) 2.99 (m, 1 H) 3.70 (m, 1 H) 3.87 (m, 4 H) 5.27 (m, 1 H) 5.36 (d, *J*=4.75 Hz, 1 H); MS: (M+H)⁺= 457.

### Example 187

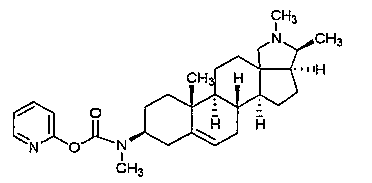

### Methyl-(2,3,11a-trimethyl-2,3,3a,4,5,5a,5b,6,8,9,10,11,11a,11b,12,13-hexadecahydro 1H-2-aza-pentaleno[1,6a-a]phenanthren-9-yl)-carbamic acid pyridin-2-yl ester

The title compound was prepared according to procedures described in Example 182, except substituting carbonic acid dipyridin-2-yl ester for carbonic acid furan-3-ylmethyl ester 4-nitro-phenyl ester. ¹H NMR (CDCl₃): δ 0.97 (s, 3 H) 1.02-2.34 (m, 26 H) 2.53 (m, 2 H) 2.95 (s, 3 H) 3.03 (m, 1 H) 4.02 (m, 1 H) 5.39 (s, 1 H) 7.11 (m, 1 H) 7.17 (dd, *J*=7.12, 5.42 Hz, 1 H) 7.75 (m, 1 H) 8.38 (dd, *J*=5.09, 1.70 Hz, 1 H); MS: (M+H)⁺= 464.

### Example 188

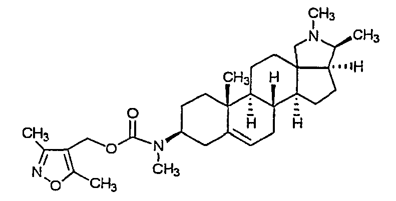

### Methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid 3,5-dimethyl-isoxazol-4-ylmethyl ester

The title compound was prepared according to procedures described in Example 182, except substituting carbonic acid 3,5-dimethyl-isoxazol-4-ylmethyl ester 4-nitro-phenyl ester for carbonic acid furan-3-ylmethyl ester 4-nitro-phenyl ester. ¹H NMR (CDCl₃): δ 0.93 (s, 3 H) 1.00-2.13 (m, 23 H) 2.21 (s, 3H) 2.28 (s, 3 H) 2.37 (m, 2 H) 2.42 (s, 3 H) 2.77 (s, 3 H) 2.99 (m, 1 H) 3.81 (m, 1 H) 4.89 (s, 2 H) 5.35 (m, 1 H) ; MS: (M+H)⁺= 496.

### Example 189

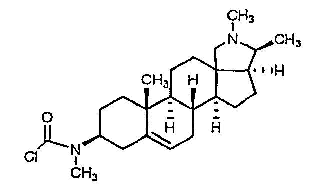

### 189A. Methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid chloride

Compound 7B (125 mg, 0.36 mmol) was dissolved in dichloromethane (5 mL) and cooled to 0 °C. Triethylamine (150 µL, 1.1 mmol) and phosgene (20% in toluene, 0.23 mL, 0.44 mmol) were added to it. The mixture was stirred at 0 °C for 3 hours. The reaction mixture was quenched with water, extracted with dichloromethane. The combined extracts were dried over Na₂SO₄, filtered and concentrated to give crude desired product (144.6 mg, 97.8%), which was used in next step without further purification.
MS: (M+H)⁺= 477.

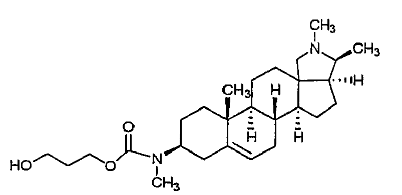

### 189B. Methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-g-yl)-carbamic acid 3-hydroxypropyl ester

Compound 189A (30 mg, 0.074mmol) and 1,3-propanediol (54 µL, 0.061 mmol), were dissolved in pyridine (1 mL). The mixture was stirred at room temperature for 5 days. Stripped of pyridine, the crude reaction mixture was purified on silica gel column using 0.5% ammonium hydroxide and 5% methanol in dichloromethane to give th e desired product (7.3 mg, 22.8% yield).

¹H NMR (CDCl₃): δ 0.96 (m, 3 H) 1.04-2.11 (m, 25 H) 2.22 (s, 3 H) 2.42 (m , 2 H) 2.56 (br s, 1 H) 2.81 (m, 3 H) 3.00 (m, 1 H) 3.65 (m, 2 H) 3.87 (m, 1 H) 4.28 (t, *J*=5.76 Hz, 2 H) 5.36 (m, 1 H); MS: (M+H)⁺= 445.

### Example 190

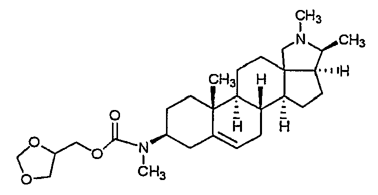

### Methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid[1,3]dioxolan-4-ylmethyl ester

The title compound was prepared according to procedures described in Example 189B, except substituting glycerol formal for 1,3-propanediol. MS: (M+H)⁺= 473.

### Example 191

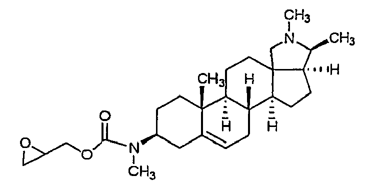

### Methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid oxiranylmethyl ester

The title compound was prepared according to procedures described in Example 189B, except substituting glycidol for 1,3-propanediol. ¹H NMR (CDCl₃): δ 0.95 (m, 3 H) 1.13-2.25 (m, 26 H) 2.43 (m, 2 H) 2.64 (dd, *J*=4.92, 2.54 Hz, 1 H) 2.84 (m, 3 H) 3.03 (m, 1 H) 3.24 (m, 1 H) 3.68 (m, 1 H) 3.90 (m, 2 H) 4.45 (d, *J*=9.0 Hz, 1 H) 5.36 (d, *J*=5.09 Hz, 1 H); MS: (M+H)⁺= 443.

### Example 192

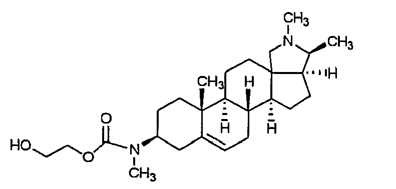

### Methyl-(2,3,11a-trimethyl-2,3,3a,4,5,5a,5b,6,8,910,11,11a,11b,12,13-hexadecahydro-1H-2-aza-pentaleno[1,6a-a]phenanthren-9-yl)-carbamic acid 2-hydroxy-ethyl ester

The title compound was prepared according to procedures described in Example 189B, except substituting ethylene glycol for 1,3-propanediol. ¹H NMR (CDCl₃): δ 0.95 (s, 3 H) 1.03-2.12 (m, 23 H) 2.21 (s, 3 H) 2.41 (m, 2 H) 2.73 (br s, 1 H) 2.84 (s, 3 H) 2.97 (m, 1 H) 3.83 (d, *J*=4.75 Hz, 2 H) 3.94 (m, 1 H) 4.26 (m, 2 H) 5.36 (d, *J*=6.00 Hz, 1 H); MS: (M+H)⁺= 431.

### Example 193

### Determination of Biological-Activity

To determine the effectiveness of representative compounds of this invention as histamine-3 receptor ligands (H₃ receptor ligands), the following tests were conducted according to methods previously described (European Journal of Pharmacology, 188:219-227 (1990); Journal of Pharmacology and Experimental Therapeutics, 275:598-604 (1995); Journal of Pharmacology and Experimental Therapeutics, 276:1009-1015 (1996); and Biochemical Pharmacology, 22:3099-3108 (1973)).

Briefly, male Sprague-Dawley rat brain cortices were homogenized (1g tissue/10 mL buffer) in 50 mM Tris-HCl/5 mM EDTA containing protease inhibitor cocktail (Calbiochem) using a polytron set at 20,500 rpm. Homogenates were centrifuged for 20 minutes at 40,000xg. The supernatant was decanted, and pellets were weighed. The pellet was resuspended by polytron homogenization in 40 mL 50 mM Tris-HCl/5 mM EDTA with protease inhibitors and centrifuged for 20 minutes at 40,000×g. The membrane pellet was resuspended in 6.25 volumes (per gram wet weight of pellet) of 50 mM Tris-HCl/5 mM EDTA with protease inhibitors and aliquots flash frozen in liquid N₂ and stored at -70 °C until used in assays. Rat cortical membranes (12 mg wet weight/tube) were incubated with (³H)-N-α-methylhistamine (~0.6 nM) with or without H₃ receptor antagonists in a total incubation volume of 0.5 mL of 50 mM Tris-HCl/5 mM EDTA (pH 7.7). Test compounds were dissolved in DMSO to provide a 20 mM solution, serially diluted and then added to the incubation mixtures prior to initiating the incubation assay by addition of the membranes. Thioperamide (3 µM) was used to determine nonspecific binding. Binding incubations were conducted for 30 minutes at 25 °C and terminated by addition of 2 mL of ice cold 50 mM Tris-HCl (pH 7.7) and filtration through 0.3% polyethylenimine-soaked Unifilter plates (Packard). These filters were washed 4 additional times with 2 mL of ice-cold 50 mM Tris-HCl and dried for 1 hour. Radioactivity was determined using liquid scintillation counting techniques. Results were analyzed by Hill transformation and Kᵢ values were determined using the Cheng-Prusoff equation.

Representative compounds of the invention bound to histamine-3 receptors with binding affinities from about 810 nM to about 0.12 nM. Preferred compounds of the invention bound to histamine-3 receptors with binding affinities from about 100 nM to about 0.12 nM. More preferred compounds of the invention bound to histamine-3 receptors with binding affinities from about 20 nM to about 0.12 nM.

Compounds of the invention are histamine-3 receptor ligands that modulate function of the histamine-3 receptor by altering the activity of the receptor. These compounds may be inverse agonists that inhibit the basal activity of the receptor or they may be antagonists that completely block the action of receptor-activating agonists. These compounds may also be partial agonists that partially block or partially activate the histamine-3 receptor or they may be agonists that activate the receptor.

## Claims

1. A compound of the formula: 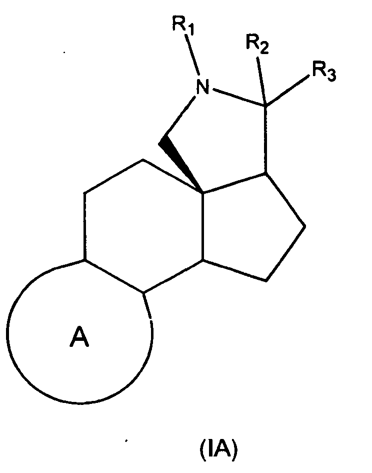 or a pharmaceutically acceptable salt thereof, wherein
R₁ is selected from the group consisting of hydrogen, acetyl, alkyl, fluoroalkyl, and cycloalkyl;
R₂ and R₃ are each independently selected from the group consisting of hydrogen and alkyl, or R₂ and R₃ taken together form a 3- to 6-membered ring;
Ring A of the formula: 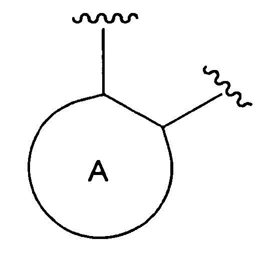 is selected from the following: 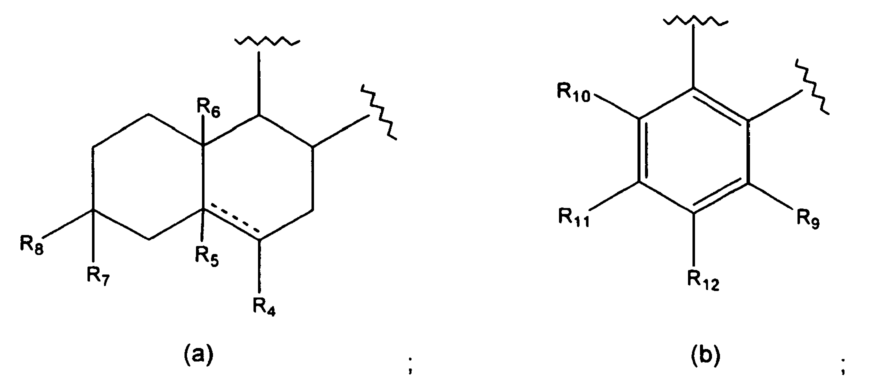 and 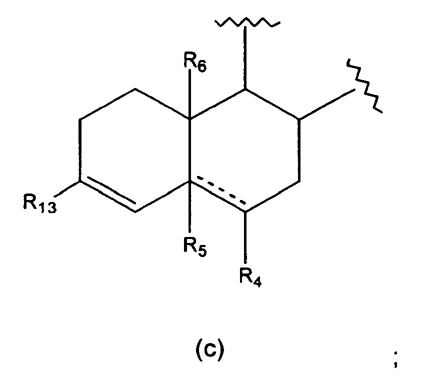 wherein:
the dotted line represents an optional bond;
R₄ and R₅ arse each independently selected from the group consisting of hydrogen and fluorine, provided that R₅ is present only when the bond represented by the dotted line is absent;
R₆ is selected from the group consisting of hydrogen, alkyl, and fluorine;
one of R₇ and R₈ is hydrogen; and the other of R₇ and R₈ is selected from the group consisting of:
a) NR₁₈R₁₉;
b) SR₂₀, or O(C=O)N(R₂₀)(R₂₁), and
c) NR₂₂(C=O)NR₂₆R₂₇, NR₂₂(C=O)CH(NR₂₈R₂₉)R₃₀, or NR₂₂(C=O)C(OR₂₃)R₃₀R_{30b}; and
d) NR₂₂SO₂R₃₁ or NR₂₂SO₂N(R₂₂)(R₂₃);
or R₇ and R₈ taken together with the carbon atom to which each is attached forms a group of the formula -C=C(Rₐ)(R_{b}), wherein Rₐ and R_{b} are each independently selected from the group consisting of hydrogen, alkyl, and cycloalkyl;
R₉ and R₁₀ are each independently selected from the group consisting of hydrogen, halogen, alkyl, and cyano;
R₁₁ and R₁₂ are each independently selected from the group consisting of hydrogen, heteroaryl, heterocycle, aryl, arylalkyl, aryloxy, arylcarbonyl, arylcarbonyloxy, arylalkoxy, alkylsulfonyl, arylsulfonyl, and trifluoromethylsulfonyl; or one of R₁₀ and R₁₁ or R₁₁ and R₁₂ taken together with the atoms to which each is attached form a 5- to 6-membered aromatic or heteroaromatic ring;
R₁₃ is selected from the group consisting of alkyl, cycloalkyl, aryl, and heteroaryl;
R₁₈ is hydrogen or C₁-C₆ alkyl and R₁₉ is selected from the group consisting of aryl and heteroaryl, or R₁₈ and R₁₉ at each occurrence is taken together form a 3- to 8-membered heterocycle;
R₂₀ and R₂₁ at each occurrence are each independently selected from the group consisting of hydrogen, alkyl, cycloalkyl, alkoxyalkyl, cyanoalkyl, hydroxyalkyl, haloalkyl, aryl, aryloxy, arylalkyl, arylsulfonyl, heteroaryl, heteroarylalkyl, heteroarylcarbonyl, heterocycle, and heterocyclealkyl .
R₂₂ at each occurrence is selected from the group consisting of hydrogen and alkyl;
R₂₃ at each occurrence is selected from the group consisting of hydrogen, alkyl, and alkylcarbonyl;
R₂₆ and R₂₇ are each independently selected from the group consisting of alkyl, cycloalkyl, aryl, heteroaryl, and heterocycle, provided that R₂₆ and R₂₇ are not both alkyl, or R₂₆ and R₂₇ taken together with the nitrogen atom to which each is attached forms an aromatic or non-aromatic 5- to 6-membered ring, wherein 0, 1, or 2 carbon atoms in the ring is substituted with a heteroatom selected from O, S, or NR₂₃;
R₂₈ and R₂₉ at each occurrence are each independently selected from the group consisting of hydrogen, alkyl, alkylcarbonyl, alkoxycarbonyl, cycloalkyl, aryl, heteroaryl, and heterocycle, or R₂₈ and R₂₉ taken together with the nitrogen atom to which each is attached forms an aromatic or non-aromatic 5- to 6-membered ring, wherein 0, 1, or 2 carbon atoms in the ring is substituted with a heteroatom selected from O, S, or NR₂₃;
R₃₀ at each occurrence is independently selected from the group consisting of hydrogen, alkyl, alkoxyalkyl, hydroxyalkyl, aryl, arylalkyl, cycloalkyl, cycloalkylalkyl, cyanoalkyl, haloalkyl, heteroaryl, heteroarylalkyl, heteroarylcarbonyl, heterocycle, and heterocyclealkyl;
R_{30b} is selected from the group consisting of hydrogen and alkyl, or when R₃₀ is alkyl and R_{30b} is alkyl, the alkyl groups can be bonded together to form a C₃-C₄ cycloalkyl group; and
R₃₁ is selected from the group consisting of alkyl, cycloalkyl, aryl, heterocycle, and heteroaryl.

2. The compound of claim 1, having the formula: 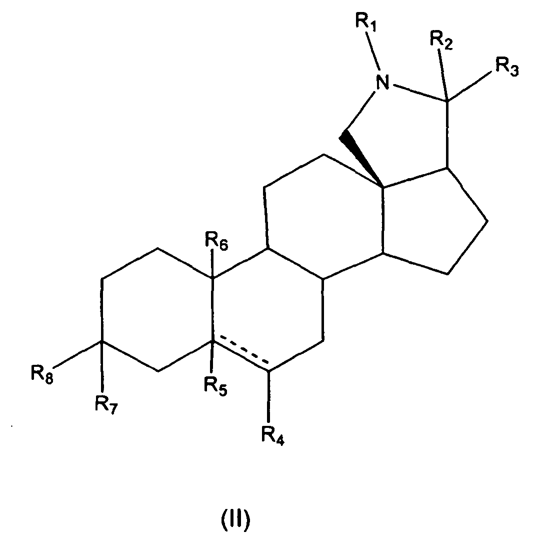 wherein:
the dotted line represents an optional bond;
R₁ is selected from the group consisting of hydrogen, acetyl, alkyl, fluoroalkyl, and cycloalkyl;
R₂ and R₃ are each independently selected from the group consisting of hydrogen and alkyl, or R₂ and R₃ taken together form a 3- to 6-membered ring;
R₄ and R₅ are each independently selected from the group consisting of hydrogen and fluorine, provided that R₅ is present only when the bond represented by the dotted line is absent;
R₆ is selected from the group consisting of hydrogen, alkyl, and fluorine;
one of R₇ and R₈ is hydrogen; and the other of R₇ and R₈ is selected from the group consisting of:
a) NR₁₈R₁₉;
b) SR₂₀, or O(C=O)N(R₂₀)(R₂₁), and
c) NR₂₂(C=O)NR₂₆R₂₇, NR₂₂(C=O)CH(NR₂₈R₂₉)R₃₀, or NR₂₂(C=O)C(OR₂₃)R₃₀R_{30b}; and
d) NR₂₂SO₂R₃₁ or NR₂₂SO₂N(R₂₂)(R₂₃)_{;}
or R₇ and R₈ taken together with the carbon atom to which each is attached forms a group of the formula -C=C(Rₐ)(R_{b}), wherein Rₐ and R_{b} are each independently selected from the group consisting of hydrogen, alkyl, and cycloalkyl;
R₁₈ is hydrogen or C₁-C₆ alkyl and R₁₉ is selected from the group consisting of aryl and heteroaryl, or R₁₈ and R₁₉ at each occurrence is taken together form a 3- to 8-membered heterocycle;
R₂₀ and R₂₁ at each occurrence are each independently selected from the group consisting of hydrogen, alkyl, cycloalkyl, alkoxyalkyl, cyanoalkyl, hydroxyalkyl, haloalkyl, aryl, aryloxy, arylalkyl, arylsulfonyl, heteroaryl, heteroarylalkyl, heteroarylcarbonyl, heterocycle, and heterocyclealkyl ;
R₂₂ at each occurrence is selected from the group consisting of hydrogen and alkyl;
R₂₃ at each occurrence is selected from the group consisting of hydrogen, alkyl, and alkylcarbonyl;
R₂₆ and R₂₇ are each independently selected from the group consisting of alkyl, cycloalkyl, aryl, heteroaryl, and heterocycle, provided that R₂₆ and R₂₇ are not both alkyl, or R₂₆ and R₂₇ taken together with the nitrogen atom to which each is attached forms an aromatic or non-aromatic 5- to 6-membered ring, wherein 0, 1, or 2 carbon atoms in the ring is substituted with a heteroatom selected from O, S, or NR₂₃;
R₂₈ and R₂₉ at each occurrence are each independently selected from the group consisting of hydrogen, alkyl, alkylcarbonyl, alkoxycarbonyl, cycloalkyl, aryl, heteroaryl, and heterocycle, or R₂₈ and R₂₉ taken together with the nitrogen atom to which each is attached forms an aromatic or non-aromatic 5- to 6-membered ring, wherein 0, 1, or 2 carbon atoms in the ring is substituted with a heteroatom selected from O, S, or NR₂₃;
R₃₀ at each occurrence is independently selected from the group consisting of hydrogen, alkyl, alkoxyalkyl, hydroxyalkyl, aryl, arylalkyl, cycloalkyl, cycloalkylalkyl, cyanoalkyl, haloalkyl, heteroaryl, heteroarylalkyl, heteroarylcarbonyl, heterocycle, and heterocyclealkyl;
R_{30b} is selected from the group consisting of hydrogen and alkyl, or when R₃₀ is alkyl and R_{30b} is alkyl, the alkyl groups can be bonded together to form a C₃-C₄ cycloalkyl group; and
R₃₁ is selected from the group consisting of alkyl, cycloalkyl, aryl, heterocycle, and heteroaryl.

3. The compound of claim 2, wherein one of R₇ and R₈ is hydrogen; and the other of R₇ and R₈ is selected from the group consisting of NR₂₂(C=O)CH(NR₂₈R₂₉)R₃₀, and NR₂₂(C=O)C(OR₂₃)R₃₀R_{30b}.

4. The compound of claim 3, wherein R₇ or R₈ is NR₂₂(C=O)CH(NR₂₈R₂₉)R₃₀, wherein R₂₂ is alkyl, R₂₈ and R₂₉ are each hydrogen, and R₃₀ is selected from the group consisting of alkyl and aryl.

5. The compound of claim 4, wherein R₂₂ is methyl.

6. The compound of claim 1, selected from the group consisting of:
trifluoro-methanesulfonic acid 8-methyl-5,6,6a,7,8,9,10,11-octahydro-4bH-benzo[4,5]indeno[1,7a-c]pyrrol-2-yl ester;
1-[3-(8-methyl-5,6,6a,7,8,9,10,11-octahydro-4bH-benzo[4,5]indeno[1,7a-c]pyrrol-2-yl)-phenyl]-ethanone;
1-[3-(8-methyl-5,6,6a,7,8,9,10,11-octahydro-4bH-benzo[4,5]indeno[1,7a-c]pyrrol-2-yl)-phenyl]-ethanone oxime;
1-[3-(8-methyl-5,6,6a,7,8,9,10,11-octahydro-4bH-benzo[4,5]indeno[1,7a-c]pyrrol-2-yl)-phenyl]-ethanone;
N-methyl-2-oxo-2-thiophen-2-yl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
ethanesulfonic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzenesulfonamide;
4-cyano-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzenesulfonamide;
thiophene-2-sulfonic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
4-fluoro-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzenesulfonamide;
1,1-dimethyl-3-methyl-3-(2,3,11a-trimethyl-2,3,3a,4,5,5a,5b,6,8,9,10,11,11a,11b,12,13-hexadecahydro-1H-2-azapentaleno[1,6a-a][phenanthren-9-yl)-sulfamide;
1,1-diisopropyl-3-methyl-3-(2,3,1a-trimethyl-2,3,3a,4,5,5a,5b,6,8,9,10,11,11a,11b,12,13-hexadecahydro-1H-2-azapentaleno[1,6a-a]phenanthren-9-yl)-urea;
1,1,3-trimethyl-3-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-urea;
methyl-{2-methyl-1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-propyl}-carbamic acid tert-butyl ester;
3,N-dimethyl-2-methylamino-N-(2,3,11a-trimethyl-2,3,3a,4,5,5a,5b,6,8,9,10,11,11 a,11b,12,13-hexadecahydro-1H-2-azapentaleno[1,6a-a]phenanthren-9-yl)-butyramide;
2-(acetyl-methyl-amino)-3,N-dimethyl-N-(2,3,11a-trimethyl-2,3,3a,4,5,5a, 5b,6,8,9,10,11,11a,11b,12,13-hexadecahydro-1H-2-azapentaleno[1,6a-a]phenanthren-9-yl)-butyramide;
2-amino-N-methyl-3-phenyl-N-(2,3,3a,11a-tetramethyl-2,3,3a,4,5,5a,5b,6,8,9,10,11,11a,11b,12,13-hexadecahydro-1H-2-azapentaleno[1,6a-a]phenanthren-9-yl)-propionamide;
2-amino-N-methyl-3-thiazol-4-yl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-amino-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-amino-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
2-amino-3-cyano-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-amino-N-methyl-3-thiophen-2-yl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
4-methyl-2-methylamino-pentanoic acid methyl-(2,3,1a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-amino-3-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-amino-3, N-dimethyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
2-amino-3-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-amino-4-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-acetylamino-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
2-acetylamino-3-methyl-pentanoic acid methyl-(2,3,11a-trimethyl-2,3,3a,4,5,5a,5b,6,8,9,10,11,11 a,11b,12,13-hexadecahydro-1H-2-azapentaleno[1,6a-a]phenanthren-9-yl)-amide;
2-acetylamino-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-acetylamino-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-acetylamino-N-methyl-3-phenyl-N-(2,3,11a-trimethyl-2,3,3a,4,5, 5a, 5b,6,8,9,10,11,11 a,11b,12,13-hexadecahydro-1H-2-azapentaleno[1,6a-a]phenanthren-9-yl)-propionamide;
2-acetylamino-4-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-acetylamino-3,N-dimethyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
2-acetylamino-3,N-dimethyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
2-acetylamino-N-methyl-3-phenyl-N-(2,3,11 a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-acetylamino-N-methyl-3-thiophen-2-yl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-acetylamino-4-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
4-[methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-amino]-benzonitrile;
3-[methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-amino]-benzonitrile;
methyl-thiazol-2-yl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amine;
1-{3-[methyl-(2, 3,11 a-trimethyl-2,3,3a,4,5,5a,5b,6,8,9,10,11,11 a,11 b,12,13-hexadecahydro-1 H-2-azapentaleno[1,6a-a]phenanthren-9-yl)-amino]-phenyl}-ethanone;
1-{4-[methyl-(2,3,11 a-trimethyl-2,3,3a,4,5,5a,5b,6,8,9,10,11,11a,11b,12,13-hexadecahydro-1H-2-azapentaleno[1,6a-a]phenanthren-9-yl)-amino]-phenyl}-ethanone;
acetic acid 1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-2-phenyl-ethyl ester;
2-hydroxy-N-methyl-3-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
acetic acid 1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-2-phenyl-ethyl ester;
2-hydroxy-N-methyl-3-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
acetic acid 3-methyl-1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-butyl ester;
2-hydroxy-4-methyl-pentanoic acid methyl-(2,3,11a-trimethyl-2,3,3a,4,5,5a,5b,6,8,9,10,11,11a,11b,12,13-hexadecahydro-1 H-2-azapentaleno[1,6a-a]phenanthren-9-yl)-amide;
acetic acid 2-methyl-1-[methyl-(2,3,11a-trimethyl-2,3,3a,4,5,5a,5b,6,8,9,10,11,11a,11b,12,13-hexadecahydro-1 H-2-azapentaleno[1,6a-a]phenanthren-9-yl)-carbamoyl]-propyl ester;
2-hydroxy-3,N-dimethyl-N-(2,3,1a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
acetic acid 1-methyl-1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-propyl ester;
2-hydroxy-2,N-dimethyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
acetic acid 1-[methyl-(2,3,11 a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-cyclopropyl ester;
1-hydroxy-cyclopropanecarboxylic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
acetic acid 1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-pentyl ester;
2-hydroxy-hexanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
acetic acid [methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-phenyl-methyl ester;
2-hydroxy-N-methyl-2-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
acetic acid 2,2,2-trifluoro-1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-ethyl ester;
3,3,3-trifluoro-2-hydroxy-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
acetic acid (4-fluoro-phenyl)-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-methyl ester;
2-(4-fluoro-phenyl)-2-hydroxy-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
acetic acid (4-methoxy-phenyl)-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-methyl ester;
2-hydroxy-2-(4-methoxy-phenyl)-N-methyl-N-(2,3,11a-trimethyl-2,3,3a,4,5,5a,5b,6,8,9,10,11,11 a,11b,12,13-hexadecahydro-1H-2-azapentaleno[1,6a-a]phenanthren-9-yl)-acetamide;
acetic acid (3,4-difluoro-phenyl)-[methyl-(2,3,11a-trimethyl-2,3,3a,4,5, 5a,5b,6,8,9,10,11,11 a,11b,12,13-hexadecahydro-1H-2-azapentaleno[1 ,6a-a]phenanthren-9-yl)-carbamoyl]-methyl ester;
2-(3,4-difluoro-phenyl)-2-hydroxy-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
acetic acid [methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-phenyl-methyl ester;
2-hydroxy-N-methyl-2-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
acetic acid [methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-phenyl-methyl ester;
2-hydroxy-N-methyl-2-phenyl-N-(2,3,1a-trimethyl-2,3,3a,4,5, 5a,5b,6,8,9,10,11,11 a,11 b,12,13-hexadecahydro-1H-2-azapentaleno[1,6a-a]phenanthren-9-yl)-acetamide;
acetic acid 3-methyl-1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-butyl ester;
2-hydroxy-4-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
acetic acid 3-methyl-1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-butyl ester;
2-hydroxy-4-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-methoxy-N-methyl-2-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
2-methoxy-N-methyl-2-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
2-methoxy-N-methyl-2-phenyl-N-(2,3,11a-trimethyl-2,3,3a,4,5,5a,5b,6,a,9,10,11,11a,11b,12,13-hexadecahydro-1H-2-azapentaleno[1,6a-a]phenanthren-9-yl)-acetamide;
2-methoxy-hexanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-methoxy-4-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-methoxy-N-methyl-3-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-methoxy-3,N-dimethyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
9-ethylidene-2,3,11a-trimethyl-2,3,3a,4,5,5a,5b,6,8,9,10,11,11a,11b,12,13-hexadecahydro-1H-2-azapentaleno[1,6a-a]phenanthrene;
9-isopropylidene-2,3,11a-trimethyl-2,3,3a,4,5,5a,5b,6,8,9,10,11,11a,11b,12,13-hexadecahydro-1H-2-azapentaleno[1,6a-a]phenanthrene;
benzenesulfonic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
4-cyano-benzenesulfonic acid 2,3,11a-trimethyl-2,3,3a,4,5,5a,5b,6,8,9,10,11,11a,11b,12,13-hexadecahydro-1H-2-azapentaleno[1 ,6a-a]phenanthren-9-yl ester;
furan-2-ylmethyl-methyl-carbamic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
methyl-propyl-carbamic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
benzyl-methyl-carbamic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
methyl-(6-methyl-pyridin-2-ylmethyl)-carbamic acid 2,3,11a-trimethyl-2,3,3a, 4,5,5a,5b,6,8,9,10,11,11a,11b,12,13-hexadecahydro-1H-2-azapentateno[1,6a-a]phenanthren-9-yl ester;
methyl-(tetrahydro-furan-2-ylmethyl)-carbamic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
(2-fluoro-ethyl)-carbamic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
furan-2-ylmethyl-carbamic acid 2,3,11 a-trimethyl-2,3,3a,4,5,5a,5b,6,8,9,10,11,11a,11b,12,13-hexadecahydro-1H-2-azapentaleno[1,6a-a]phenanthren-9-yl ester;
(2-cyano-ethyl)-(tetrahydro-furan-2-ylmethyl)-carbamic acid 2,3,11a-trimethyl-2,3,3a,4,5,5a,5b,6,8,9,10,11,11a,11b,12,13-hexadecahydro-1H-2-aza-pentaleno[1,6a-a]phenanthren-9-yl ester;
benzyl-carbamic acid 2,3,11 a-trimethyl-2,3,3a,4,5,5a,5b,6,8,9,10,11,11a,11b, 12,13-hexadecahydro-1H-2-azapentaleno[1,6a-a]phenanthren-9-yl ester; and
propyl-carbamic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester.

7. A compound according to claim 1 for treating conditions and disorders related to histamine-3-receptor modulation in mammals.

8. A compound of the formula: 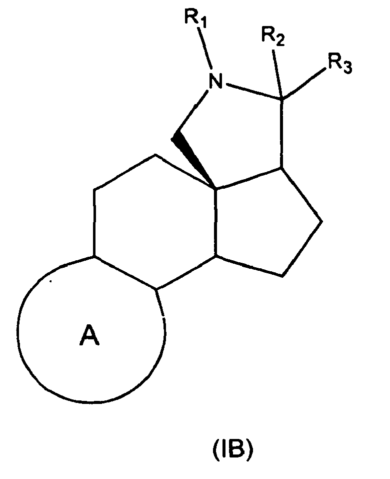 or a pharmaceutically acceptable salt thereof, wherein
R₁ is selected from the group consisting of hydrogen, acetyl, alkyl, fluoroalkyl, and cycloalkyl;
R₂ and R₃ are each independently selected from the group consisting of hydrogen and alkyl, or R₂ and R₃ taken together form a 3- to 6-membered ring;
Ring A of the formula: 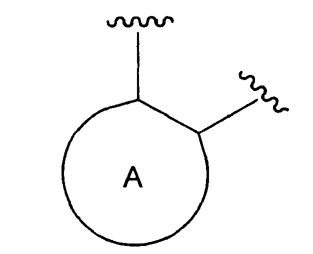 is selected from the following: 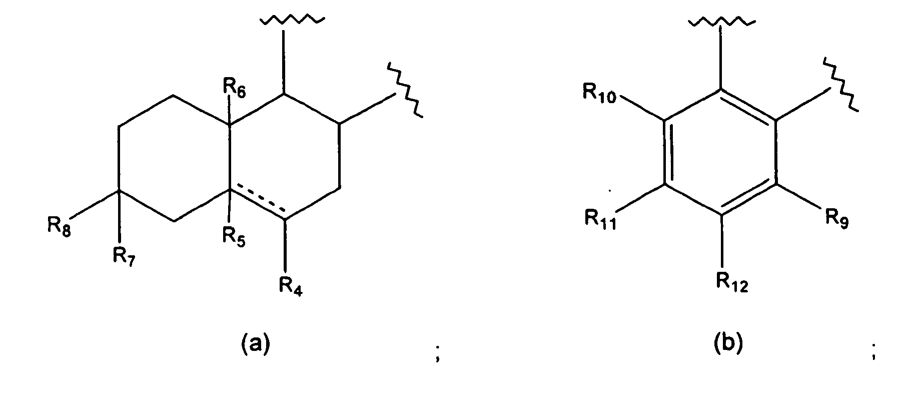 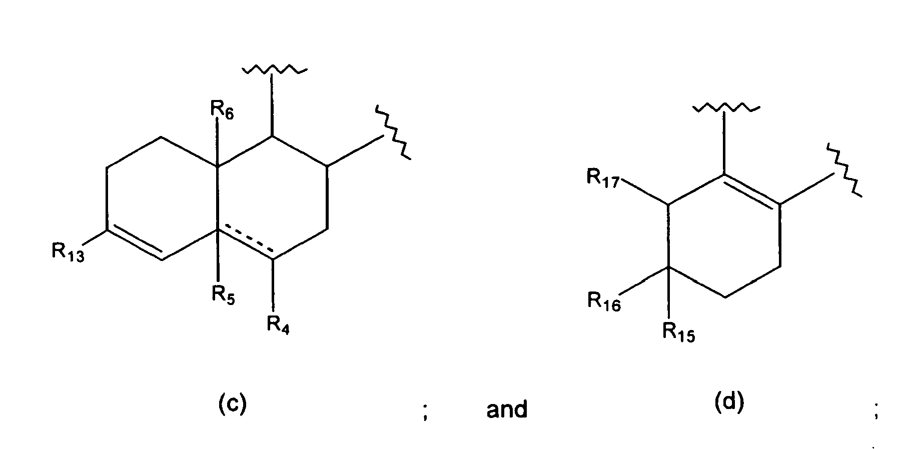 wherein:
the dotted line represents an optional bond;
R₄ and R₅ are each independently selected from the group consisting of hydrogen and fluorine, provided that R₅ is present only when the bond represented by the dotted line is absent;
R₆ is selected from the group consisting of hydrogen, alkyl, and fluorine;
one of R₇ and R₈ is hydrogen; and the other of R₇ and R₈ is selected from the group consisting of:
a) NR₁₈R₁₉;
b) OR₂₀, SR₂₀, O(C=O)OR₂₀, O(C=O)N(R₂₀)(R₂₁), O(C=O)C(R₂₃)(R₂₄)(R_{24b}), or O(C=O)CH(NR₂₈R₂₉)R₂₅; and
c) NR₂₂(C=O)R₂₅, NR₂₂(C=O)NR₂₆R₂₇, NR₂₂(C=O)CH(NR₂₈R₂₉)R₃₀, N(R₂₂) (C=O)OR₂₀, or NR₂₂(C=O)C(OR₂₃)R₃₀R_{30b}; and
d) NR₂₂SO₂R₃₁ or NR₂₂SO₂N(R₂₂)(R₂₃);
or R₇ and R₈ taken together with the carbon atom to which each is attached forms a group of the formula -C=C(Rₐ)(R_{b}), wherein Rₐ and R_{b} are each independently selected from the group consisting of hydrogen, alkyl, and cycloalkyl;
R₉ and R₁₀ are each independently selected from the group consisting of hydrogen, halogen, alkyl, and cyano;
R₁₁ and R₁₂ are each independently selected from the group consisting of hydrogen, heteroaryl, heterocycle, aryl, arylalkyl, aryloxy, arylcarbonyl, arylcarbonyloxy, arylalkoxy, alkylsulfonyl, arylsulfonyl, and trifluoromethylsulfonyl; or one of R₁₀ and R₁₁ or R₁₁ and R₁₂ taken together with the atoms to which each is attached form a 5- to 6-membered aromatic or heteroaromatic ring;
R₁₃ is selected from the group consisting of alkyl, cycloalkyl, aryl, and heteroaryl;
R₁₅ and R₁₆ taken together with the atom to which each is attached forms a 5- to 6-membered ring optionally substituted with an alkylamino group;
R₁₇ is selected from the group consisting of hydrogen, alkyl, and fluoro;
R₁₈ is hydrogen or C₁-C₆ alkyl and R₁₉ is selected from the group consisting of aryl and heteroaryl, or R₁₈ and R₁₉ at each occurrence is taken together form a 3- to 8-membered heterocycle;
R₂₀ and R₂, at each occurrence are each independently selected from the group consisting of hydrogen, alkyl, cycloalkyl, alkoxyalkyl, cyanoalkyl, hydroxyalkyl, haloalkyl, aryl, aryloxy, arylalkyl, arylsulfonyl, heteroaryl, heteroarylalkyl, heteroarylcarbonyl, heterocycle, and heterocyclealkyl;
R₂₂ at each occurrence is selected from the group consisting of hydrogen and alkyl;
R₂₃ at each occurrence is selected from the group consisting of hydrogen, alkyl, and alkylcarbonyl;
R₂₄ and R_{24b} are each independently selected from the group consisting of hydrogen, alkyl, aryl, arylalkyl, and cycloalkyl;
R₂₅ at each occurrence is independently selected from the group consisting of C₁-C₆ alkyl, alkoxyalkyl, hydroxyalkyl, aryl, arylalkyl, cycloalkyl, cycloalkylalkyl, cyanoalkyl, haloalkyl, heteroaryl, heteroarylalkyl, heteroarylcarbonyl, heterocycle, and heterocyclealkyl;
R₂₆ and R₂₇ at each occurrence are each independently selected from the group consisting of alkyl, alkylcarbonyl, alkoxycarbonyl, cycloalkyl, aryl, heteroaryl, and heterocycle, or R₂₆ and R₂₇ taken together with the nitrogen atom to which each is attached forms an aromatic or non-aromatic 5- to 6-membered ring, wherein 0, 1, or 2 carbon atoms in the ring is substituted with a heteroatom selected from O, S, or NR₂₃;
R₂₈ and R₂₉ at each occurrence are each independently selected from the group consisting of hydrogen, alkyl, alkylcarbonyl, alkoxycarbonyl, cycloalkyl, aryl, heteroaryl, and heterocycle, or R₂₈ and R₂₉ taken together with the nitrogen atom to which each is attached forms an aromatic or non-aromatic 5- to 6-membered ring, wherein 0, 1, or 2 carbon atoms in the ring is substituted with a heteroatom selected from O, S, or NR₂₃;
R₃₀ at each occurrence is independently selected from the group consisting of hydrogen, alkyl, alkoxyalkyl, hydroxyalkyl, aryl, arylalkyl, cycloalkyl, cycloalkylalkyl, cyanoalkyl, haloalkyl, heteroaryl, heteroarylalkyl, heteroarylcarbonyl, heterocycle, and heterocyclealkyl;
R_{30b} is selected from the group consisting of hydrogen and alkyl, or when R₃₀ is alkyl and R_{30b} is alkyl, the alkyl groups can be bonded together to form a C₃-C₄ cycloalkyl group; and
R₃₁ is selected from the group consisting of alkyl, cycloalkyl, aryl, heterocycle, and heteroaryl;
or a compound that is benzoic acid 2,3,11a-trimethyl-2,3,3a,4,5,5a,5b,6,8,9,10,11,11a,11b,12,13-hexadecahydro-1H-2-azapentaleno[1 ,6a-a]phenanthren-9-yl ester;
for use in treating a condition modulated by the histamine-3-receptors in a mammal comprising administering said compound to a mammal in need thereof, wherein the condition is related to function in a mammal selected from the group consisting of memory and cognition processes, neurological processes, and regulation of blood sugar.

9. The compound according to claim 8, wherein the condition is related to memory and cognition processes.

10. The compound according to claim 8, wherein the condition is selected from the group consisting of Alzheimer's disease, attention-deficit hyperactivity disorder, bipolar disorder, cognitive enhancement, cognitive deficits in psychiatric disorders, deficits of memory, deficits of learning, dementia drug abuse, diabetes, type II diabetes, depression, epilepsy, insulin resistance syndrome, jet lag, metabolic syndrome, mild cognitive impairment, migraine, mood and attention alteration, motion sickness, narcolepsy, obesity, obsessive compulsive disorder, pain, Parkinson's disease, schizophrenia, seizures, sleep disorders, Syndrome X, Tourette's syndrome, vertigo, and wakefulness.

11. The compound according to claim 8, wherein the compound is selected from the group consisting of:
trifluoro-methanesulfonic acid 8-methyl-5,6,6a,7,8,9,10,11-octahydro-4bH-benzo[4,5]indeno[1,7a-c]pyrrol-2-yl ester;
1-[3-(8-methyl-5,6,6a,7,8,9,10,11-octahydro-4bH-benzo[4,5]indeno[1,7a-c]pyrrol-2-yl)-phenyl]-ethanone;
1-[3-(8-methyl-5,6,6a,7,8,9,10,11-octahydro-4bH-benzo[4,5]indeno[1,7a-c]pyrrol-2-yl)-phenyl]-ethanone oxime;
1-[3-(8-methyl-5,6,6a,7,8,9,10,11-octahydro-4bH-benzo[4,5]indeno[1,7a-c]pyrrol-2-yl)-phenyl]-ethanone;
benzoic acid 8-acetyl-5,6,6a,7,8,9,10,11-octahydro-4bH-benzo[4,5]indeno[1,7a-c]pyrrol-2-yl ester;
1-(2-benzyloxy-5,6,6a,7,10,11-hexahydro-4bH-benzo[4,5]indeno[1,7a-c]pyrrol-8-yl)-ethanone;
2,2,N-trimethyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
furan-2-carboxylic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
4-fluoro-N-methyl-N-(2,3,11a-trimethyl-2,3,3a,4,5,5a,5b,6,8,9,10,11,11,11a,11b,12,13-hexadecahydro-1H-2-azapentaleno[1,6a-a]phenanthren-9-yl)-benzamide;
thiophene-2-carboxylic acid methyl-(2,3,11a-trimethyl-2,3,3a,4,5,5a,5b,6,8,9,10,11,1a,11b,12,13-hexadecahydro-1H-2-azapentaleno[1,6a-a]phenanthren-9-yl)-amide;
isoxazole-5-carboxylic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
N-methyl-N-(2,3,11a-trimethyl-2,3,3a,4,5,5a,5b,6,8,9,10,11,11,11a,11b,12,13-hexadecahydro-1H-2-azapentaleno[1,6a-a]phenanthren-9-yl)-isonicotinamide;
3-chloro-4-fluoro-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzamide;
2-(4-methoxy-phenyl)-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
2,3,4-trifluoro-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzamide;
4-cyano-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzamide;
N-methyl-2-thiophen-2-yl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
3-chloro-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzamide;
3-methoxy-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzamide;
4-methoxy-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzamide;
4,N-dimethyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzamide;
4-chloro-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzamide;
3-cyano-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzamide;
4-bromo-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzamide;
3,N-dimethyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
cyclopropanecarboxylic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-methoxy-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-isopropyl-4-methyl-thiazole-5-carboxylic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-sec-butyl-4-methyl-thiazole-5-carboxylic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-methyl-5-phenyl-furan-3-carboxylic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
N-methyl-2-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
N-methyl-2-thiophen-3-yl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
N-methyl-4-thiophen-2-yl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
2-(3-fluoro-phenyl)-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
N-methyl-2-oxo-2-thiophen-2-yl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
2-indol-1-yl-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
2-benzo[b]thiophen-3-yl-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
N-methyl-2-(3-methyl-benzo[b]thiophen-2-yl)-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
2-furan-2-yl-N-methyl-2-oxo-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
N-methyl-3-thiophen-2-yl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
3-furan-2-yl-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-(3-chloro-phenyl)-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
2-(4-chloro-phenyl)-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
2-(2-fluoro-phenyl)-N-methyl-N-(2, 3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
2-(4-fluoro-phenyl)-N-methyl-N-(2,3,11 a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
N-methyl-2-o-tolyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
ethanesulfonic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzenesulfonamide;
4-cyano-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzenesulfonamide;
thiophene-2-sulfonic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
4-fluoro-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-benzenesulfonamide;
1,1-dimethyl-3-methyl-3-(2,3,11a-trimethyl-2,3,3a,4,5,5a,5b,6,8,9,10,11,11a,11b,12,13-hexadecahydro-1H-2-azapentaleno[1,6a-a][phenanthren-9-yl)-sulfamide;
pyrrolidine-1-carboxylic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
1,1-diisopropyl-3-methyl-3-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-urea;
morpholine-4-carboxylic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
1,1,3-trimethyl-3-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-urea;
methyl-{2-methyl-1-[methyl-(2,3,11a-trimethyl-2,3,3a,4,5,5a,5b,6,8,9,10,11,11 a,11b,12,13-hexadecahydro-1H-2-azapentaleno[1,6a-a]phenanthren-9-yl)-carbamoyl]-propyl}-carbamic acid tert-butyl ester;
3,N-dimethyl-2-methylamino-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
2-(acetyl-methyl-amino)-3, N-dimethyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
2-amino-N-methyl-3-phenyl-N-(2,3,3a,11a-tetramethyl-2,3,3a,4,5,5a,5b,6,8,9,10,11,11a,11b,12,13-hexadecahydro-1H-2-azapentaleno[1,6a-a]phenanthren-9-yl)-propionamide;
2-amino-N-methyl-3-thiazol-4-yl-N-(2,3,11a-trimethyl-2,3,3a,4,5, 5a,5b,6,8,9,10,11,11a,11b,12,13-hexadecahydro-1H-2-azapentaleno[1,6a-a]phenanthren-9-yl)-propionamide;
2-amino-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-amino-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
2-amino-3-cyano-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-amino-N-methyl-3-thiophen-2-yl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
4-methyl-2-methylamino-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-amino-3-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-amino-3,N-dimethyl-N-(2,3,1a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
2-amino-3-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-amino-4-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-acetylamino-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
2-acetylamino-3-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-acetylamino-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-acetylamino-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-acetylamino-N-methyl-3-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-acetylamino-4-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-acetylamino-3, N-dimethyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
2-acetylamino-3,N-dimethyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
2-acetylamino-N-methyl-3-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-acetylamino-N-methyl-3-thiophen-2-yl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-acetylamino-4-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
4-[methyl-(2,3,11a-trimethyl-2,3,3a,4,5,5a,5b,6,8,9,10,11,11a,11 b,12,13-hexadecahydro-1H-2-aza-pentaleno[1,6a-a]phenanthren-9-yl)-amino]-benzonitrile;
3-[methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-amino]-benzonitrile;
methyl-thiazol-2-yl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amine;
1-{3-[methyl-(2,3,11a-trimethyl-2,3,3a,4,5,5a,5b,6,8,9,10,11,11a,11b,12,13-hexa
decahydro-1H-2-aza-pentaleno[1,6a-a]phenanthren-9-yl)-amino]-phenyl}-ethanone;
1-{4-[methyl-(2,3,11a-trimethyl-2,3,3a,4,5,5a,5b,6,8,9,10,11,11 a,11b,12,13-hexadecahydro-1H-2-azapentaleno[1,6a-a]phenanthren-9-yl)-amino]-phenyl}-ethanone;
acetic acid 1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-2-phenyl-ethyl ester;
2-hydroxy-N-methyl-3-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
acetic acid 1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-2-phenyl-ethyl ester;
2-hydroxy-N-methyl-3-phenyl-N-(2,3,11a-trimethyl-2,3,3a,4,5,5a,5b,6,8,9,10,11,11 a,11b,12,13-hexadecahydro-1H-2-azapentaleno[1,6a-a]phenanthren-9-yl)-propionamide;
acetic acid 3-methyl-1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-butyl ester;
2-hydroxy-4-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
acetic acid 2-methyl-1-[methyl-(2,3,11a-trimethyl-2,3,3a,4,5,5a,5b,6,8,9,10,11,1a,11b,12,13-hexadecahydro-1H-2-azapentaleno[1,6a-a]phenanthren-9-yl)-carbamoyl]-propyl ester;
2-hydroxy-3,N-dimethyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
acetic acid 1-methyl-1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-propyl ester;
2-hydroxy-2,N-dimethyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-butyramide;
acetic acid 1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-cyclopropyl ester;
1-hydroxy-cyclopropanecarboxylic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
acetic acid 1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-pentyl ester;
2-hydroxy-hexanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
acetic acid [methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-phenyl-methyl ester;
2-hydroxy-N-methyl-2-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
acetic acid 2,2,2-trifluoro-1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-ethyl ester;
3,3,3-trifluoro-2-hydroxy-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
acetic acid (4-fluoro-phenyl)-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-methyl ester;
2-(4-fluoro-phenyl)-2-hydroxy-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
acetic acid (4-methoxy-phenyl)-[methyl-(2,3,1a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-methyl ester;
2-hydroxy-2-(4-methoxy-phenyl)-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
acetic acid (3,4-difluoro-phenyl)-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-methyl ester;
2-(3,4-difluoro-phenyl)-2-hydroxy-N-methyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
acetic acid [methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-phenyl-methyl ester;
2-hydroxy-N-methyl-2-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
acetic acid [methyl-(2, 3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-phenyl-methyl ester;
2-hydroxy-N-methyl-2-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
acetic acid 3-methyl-1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-butyl ester;
2-hydroxy-4-methyl-pentanoic acid methyl-(2,3,11a-trimethyl-2,3,3a,4,5,5a,5b,6,8,9,10,11,1a,11b,12,13-hexadecahydro-1H-2-azapentaleno[1,6a-a]phenanthren-9-yl)-amide;
acetic acid 3-methyl-1-[methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-carbamoyl]-butyl ester;
2-hydroxyl-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-methoxy-N-methyl-2-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
2-methoxy-N-methyl-2-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
2-methoxy-N-methyl-2-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-acetamide;
2-methoxy-hexanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-methoxy-4-methyl-pentanoic acid methyl-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-amide;
2-methoxy-N-methyl-3-phenyl-N-(2,3,11a-trimethyl-2,3,3a,4,5,5a,5b,6,8,9,10,11,11a,11b,12,13-hexadecahydro-1H-2-azapentaleno[1,6a-a]phenanthren-9-yl)-propionamide;
2-methoxy-3,N-dimethyl-N-(2,3,11a-trimethyl-2,3,3a,4,5, 5a,5b,6,8,9,10,11,11a,11b,12,13-hexadecahydro-1H-2-azapentaleno[1,6a-a]phenanthren-9-yl)-butyramide;
9-ethylidene-2,3,11a-trimethyl-2,3,3a,4,5,5a,5b,6,8,9,10,11,11a,11b,12,13-hexadecahydro-1H-2-azapentaleno[1,6a-a]phenanthrene;
9-isopropylidene-2,3,11a-trimethyl-2,3,3a,4,5,5a,5b,6,8,9,10,11,11a,11b,12,13-hexadecahydro-1H-2-azapentaleno[1,6a-a]phenanthrene;
9-benzyloxy-2,3,11a-trimethyl-2,3,3a,4,5,5a,5b,6,8,9,10,11,11a,11b,12,13-hexadecahydro-1H-2-azapentaleno[1,6a-a]phenanthrene;
acetic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
thiophen-2-yl-acetic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
4-cyano-benzoic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
3-methyl-butyric acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
furan-2-carboxylic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
cyclopropanecarboxylic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
methoxy-acetic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
isobutyric acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
cyclobutanecarboxylic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
propionic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
phenyl-acetic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
benzenesulfonic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
4-cyano-benzenesulfonic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
(4-fluoro-phenyl)-acetic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
2-(tert-butoxycarbonyl-methyl-amino)-3-methyl-butyric acid 2,3,11 a-trimethyl-2,3,3a,4,5,5a,5b,6,8,9,10,11,11a,11b,12,13-hexadecahydro-1H-2-aza-pentaleno[1,6a-a]phenanthren-9-yl ester;
3-methyl-2-methylamino-butyric acid-2,3,11a-trimethyl-2,3,3a,4,5,5a,5b,6,8,9,10,11,11a,11b,12,13-hexadecahydro-1H-2-azapentaleno[1,6,a-a]phenanthren-9-yl ester;
tetrahydro-furan-2-carboxylic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
furan-2-yl-oxo-acetic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
2-acetoxy-3-phenyl-propionic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
2-acetoxy-4-methyl-pentanoic acid 2,3,11a-trimethyl-2,3,3a,4,5,5a,5b,6,8,9,10,11,11a,11b,12,13-hexadecahydro-1H-2-azapentaleno[1 ,6a-a]phenanthren-9-yl ester;
2-acetoxy-3-methyl-butyric acid 2,3,11 a-trimethyl-2,3,3a,4,5,5a,5b,6,8,9,10,11,11a,11b,12,13-hexadecahydro-1H-2-azapentaleno[1,6a-a]phenanthren-9-yl ester;
2-acetoxy-2-methyl-butyric acid 2,3,11 α-trimethyl-2,3,3a,4,5,5a,5b,6,8,9,10,11,11a,11b,12,13-hexadecahydro-1 H-2-azapentaleno[1,6a-a]phenanthren-9-yl ester;
1-acetoxy-cyclopropanecarboxylic acid 2,3,11 a-trimethyl-2,3,3a,4,5,5a,5b,6,8,9,10,11,11a,11b,12,13-hexadecahydro-1H-2-azapentaleno[1,6a-a]phenanthren-9-yl ester;
2-acetoxy-2-ethyl-butyric acid 2,3,11a-trimethyl-2,3,3a,4,5,5a,5b,6,8,9,10,11,11a,11b,12,13-hexadecahydro-1 H-2-azapentaleno[1,6a-a]phenanthren-9-yl ester;
2-acetoxy-hexanoic acid 2,3,11 a-trimethyl-2,3,3a,4,5,5a,5b,6,8,9,10,11,11a,11b,12,13-hexadecahydro-1H-2-azapentaleno[1,6a-a]phenanthren-9-yl ester;
methoxy-phenyl-acetic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
carbonic acid 4-nitro-phenyl ester 2,3,11 a-trimethyl-2,3,3a,4,5,5a,5b,6,8,9,10,11,11a,11b,12,13-hexadecahydro-1H-2-azapentaleno[1,6a-a]phenanthren-9-yl ester;
furan-2-ylmethyl-methyl-carbamic acid 2,3,11 a-trimethyl-2,3,3a,4,5,5a,5b,6,8,9,10,11,11a,11b,12,13-hexadecahydro-1H-2-azapentaleno[1,6a-a]phenanthren-9-yl ester;
methyl-propyl-carbamic acid 2,3,11 a-trimethyl-2,3,3a,4,5,5a,5b,6,8,9,10,11,11a,11b,12,13-hexadecahydro-1 H-2-azapentaleno[1,6a-a]phenanthren-9-yl ester;
benzyl-methyl-carbamic acid 2,3,11a-trimethyl-2,3,3a,4,5,5a,5b,6,8,9,10,11,11a,11b,12,13-hexadecahydro-1H-2-azapentaleno[1,6a-a]phenanthren-9-yl ester;
methyl-(6-methyl-pyridin-2-ylmethyl)-carbamic acid 2,3,11a-trimethyl-2,3,3a, 4.5,5a,5b,6,8,9,10,11,11a,11b,12,13-hexadecahydro-1H-2-azapentaleno[1,6a-a]phenanthren-9-yl ester;
methyl-(tetrahydro-furan-2-ylmethyl)-carbamic acid 2,3,11 a-trimethyl-2,3,3a,4,5,5a,5b,6,8,9,10,11,11a,11b, 12,13-hexadecahydro-1 H-2-azapentaleno[1,6a-a]phenanthren-9-yl ester;
(2-fluoro-ethyl)-carbamic acid 2,3,11 a-trimethyl-2,3,3a,4,5,5a,5b,6,8,9,10,11,11a,11b,12,13-hexadecahydro-1 H-2-azapentaleno[1,6a-a]phenanthren-9-yl ester;
furan-2-ylmethyl-carbamic acid 2,3,11 a-trimethyl-2,3,3a,4,5,5a,5b,6,8,9,10,11,11a,11b,12,13-hexadecahydro-1 H-2-azapentaleno[1,6a-a]phenanthren-9-yl ester;
(2-cyano-ethyl)-(tetrahydro-furan-2-ylmethyl)-carbamic acid 2,3,11 a-trimethyl-2,3,3a,4,5,5a,5b,6,8,9,10,11,11a,11b,12,13-hexadecahydro-1H-2-aza-pentaleno[1,6a-a]phenanthren-9-yl ester;
benzyl-carbamic acid 2,3,11 a-trimethyl-2,3,3a,4,5,5a,5b,6,8,9,10,11,11a,11b,12,13-hexadecahydro-1 H-2-azapentaleno[1,6a-a]phenanthren-9-yl ester;
propyl-carbamic acid 2,3,11a-trimethyl-2,3,3a,4,5,5a,5b,6,8,9,10,11,11a,11b, 12,13-hexadecahydro-1 H-2-azapentaleno[1,6a-a]phenanthren-9-yl ester ;
N-methyl-2-thiophen-2-yl-N-(2,3,11a-trimethyl-octadecahydro-2-azapentaleno[1,6a-a]phenanthren-9-yl)-acetamide;
methyl,-(2,3,1 a-trimethyl-2,3,3a,4,5,5a,5b,6,8,9,10,11,11a,11 b,12,13-hexadecahydro-1H-2-aza-pentaleno[1,6a-a]phenanthren-9-yl)-carbamic acid benzyl ester;
methyl-(2,3,11a-trimethyl-2,3,3a,4,5,5a,5b,6,8,9,10,11,11a,11b,12,13-hexadecahydro-1H-2-aza-pental eno[1,6a-alphenanthren-9-yl)-carbamic acid methyl ester;
methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid 2,2-dimethyl-propyl ester;
methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid isobutyl ester;
methyl-(2,3,11a-trimethyl-2,3,3a,4,5,5a,5b,6,8,9,10,11,11a,11b,12,13-hexadecahydro-1 H-2-aza-pentaleno[1,6a-a]phenanthren-9-yl)-carbamic acid ethyl ester;
methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid tert-butyl ester;
methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid isopropyl ester;
methyl-(2,3,11a-trimethyl-2,3,3a,4,5,5a,5b,6,8,9,10,11,11a,11b,12,13-hexadecahydro-1 H-2-aza-pentaleno[1,6a-a]phenanthren-9-yl)-carbamic acid cyclopentyl ester;
methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid furan-3-ylmethyl ester
methyl-(2,3,11a-trimethyl-2,3,3a,4,5,5a,5b,6,8,9,10,11,11a,11 b,12,13-hexadecahydro-1H-2-aza-pentaleno[1,6a-a]phenanthren-9-yl)-carbamic acid 5-methoxy-tetrahydro-furan-3-yl ester;
methyl-(2,3,11a-trimethyl-2,3,3a,4,5,5a,5b,6,8,9,10,11,11a,11b,12,13-hexadecahydro-1 H-2-aza-pentaleno[1,6a-a]phenanthren-9-yl)-carbamic acid thiazol-5-ylmethyl ester;
methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid hexahydro-furo[2,3-b]furan-3-yl ester;
methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid tetrahydro-furan-3-yl ester;
methyl-(2,3,11a-trimethyl-2,3,3a,4,5,5a,5b,6,8,9,10,11,11a,11b,12,13-hexadecahydro-11H-2-aza-pentaleno[1,6a-a]phenanthren-9-yl)-carbamic acid pyridin-2-yl ester;
methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid 3,5-dimethyl-isoxazol-4-ylmethyl ester;
methyl-(2,3,11a-trimethyl-2,3,3a,4,5,5a,5b,6,8,9,10,11,11a,11b,12,13-hexadecahydro-11H-2-aza-pentaleno[1,6a-a]phenanthren-9-yl)-carbamic acid 3-hydroxy-propyl ester;
methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid [1,3]dioxolan-4-ylmethyl ester;
methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid oxiranylmethyl ester;
methyl-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)-carbamic acid 2-hydroxy-ethyl ester;
N-methyl-N-(2,3,11a-trimethyl-2,3,3a,4,5,5a,5b,6,8,9,10,11,11 a,11b,12,13-hexadecahydro-1H-2-azapentaleno[1,6a-a]phenanthren-9-yl)-acetamide;
N-methyl-N-(2,3,11a-trimethyl-2,3,3a,4,5,5a,5b,6,8,9,10,11,11 a,11b,12,13-hexadecahydro-1H-2-azapentaleno[1,6a-a]phenanthren-9-yl)-benzamide;
9-methoxy-2,3,11a-trimethyl-2,3,3a,4,5,5a,5b,6,8,9,10,11,11 a,11b,12,13-hexadecahydro-1 H-2-azapentaleno[1,6a-a]phenanthrene; and
N-methyl-N-(2,3,11a-trimethyl-octadecahydro-2-aza-pentaleno[1,6a-a]phenanthren-9-yl)-benzamide.

## Patentansprüche

1. Eine Verbindung mit der Formel: 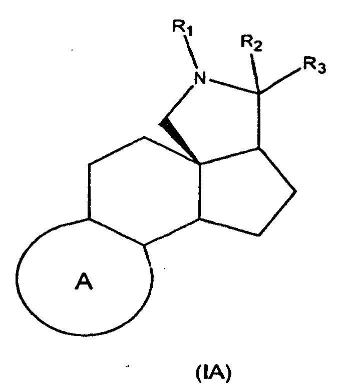 oder ein pharmazeutisch verträgliches Salz davon, worin
R₁ gewählt ist aus der Gruppe bestehend aus Wasserstoff, Acetyl, Alkyl, Fluoralkyl und Cycloalkyl;
R₂ und R₃ sind jeweils unabhängig gewählt aus der Gruppe bestehend aus Wasserstoff und Alkyl, oder R₂ und R₃ bilden zusammengenommen einen 3- bis 6-gliedrigen Ring;
Ring A mit der Formel: 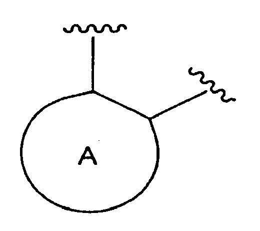 ist gewählt aus folgendem: 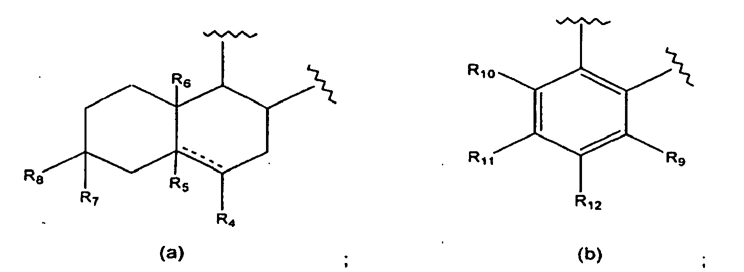 und 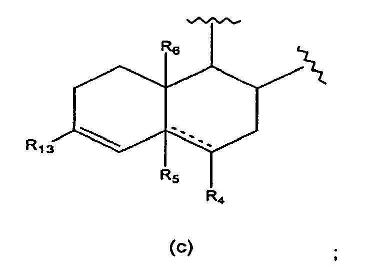 worin:
die gestrichelte Linie eine optionale Bindung darstellt;
R₄ und R₅ sind jeweils unabhängig gewählt aus der Gruppe bestehend aus Wasserstoff und Fluor, vorausgesetzt, dass R₅ nur dann vorhanden ist, wenn die Bindung, die durch die gestrichelte Linie dargestellt ist, vorhanden ist;
R₆ ist gewählt aus der Gruppe bestehend aus Wasserstoff, Alkyl und Fluor;
eines von R₇ und R₈ ist Wasserstoff; und das andere von R₇ und R₈ ist gewählt aus der Gruppe bestehend aus:
a) NR₁₈R₁₉;
b) SR₂₀ oder O(C=O)N(R₂₀) (R₂₁) , und
c) NR₂₂ (C=O)NR₂₆R₂₇, NR₂₂ (C=O) CH (NR₂₈R₂₉) R₃₀ oder NR₂₂ (C=O) C (OR₂₃) R₃₀R_{30b}; und
d) NR₂₂SO₂R₃₁ oder NR₂₂SO₂N(R₂₂) (R₂₃) ;
oder R₇ und R₈ bilden zusammengenommen mit dem Kohlenstoffatom, an welches jedes gebunden ist, eine Gruppe mit der Formel -C=C (Rₐ) (R_{b}) , worin Rₐ und R_{b} jeweils unabhängig gewählt sind aus der Gruppe bestehend aus Wasserstoff, Alkyl und Cycloalkyl;
R₉ und R₁₀ sind jeweils unabhängig gewählt aus der Gruppe bestehend aus Wasserstoff, Halogen, Alkyl und Cyano;
R₁₁ und R₁₂ sind jeweils unabhängig gewählt aus der Gruppe bestehend aus Wasserstoff, Heteroaryl, Heterzyklus, Aryl, Arylalkyl, Aryloxy, Arylcarbonyl, Arylcarbonyloxy, Arylalkoxy, Alkylsulfonyl, Arylsulfonyl und Trifluormethylsulfonyl; oder eines von R₁₀ und R₁₁ oder R₁₁ und R₁₂ bilden zusammengenommen mit den Atomen, an welche sie jeweils gebunden sind, einen 5- bis 6-gliedrigen aromatischen oder heteroaromatischen Ring;
R₁₃ ist gewählt aus der Gruppe bestehend aus Alkyl, Cycloalkyl, Aryl und Heteroaryl;
R₁₈ ist Wasserstoff oder C₁-C₆ Alkyl und R₁₉ ist gewählt aus der Gruppe bestehend aus Aryl und Heteroaryl, oder R₁₈ und R₁₉ werden bei jedem Vorkommen zusammengenommen, um einen 3- bis 8-gliedrigen Heterozyklus zu bilden;
R₂₀ und R₂₁ werden, jedesmal wenn sie vorkommen, jeweils unabhängig gewählt aus der Gruppe bestehend aus Wasserstoff, Alkyl, Cycloalkyl, Alkoxyalkyl, Cyanoalkyl, Hydroxyalkyl, Haloalkyl, Aryl, Aryloxy, Arylalkyl, Arylsulfonyl, Heteroaryl, Heteroarylalkyl, Heteroarylcarbonyl, Heterozyklus und Heterozyklusalkyl;
R₂₂ ist jedesmal wenn es vorkommt, gewählt aus der Gruppe bestehend aus Wasserstoff und Alkyl;
R₂₃ ist jedesmal wenn es vorkommt, gewählt aus der Gruppe bestehend aus Wasserstoff, Alkyl und Alkylcarbonyl;
R₂₆ und R₂₇ sind jeweils unabhängig gewählt aus der Gruppe bestehend aus Alkyl, Cycloalkyl, Aryl, Heteroaryl und Heterozyklus, vorausgesetzt, dass R₂₆ und R₂₇ nicht beide Alkyl sind, oder R₂₆ und R₂₇ zusammengenommen mit dem Stickstoffatom, an welches sie jeweils gebunden sind, einen aromatischen oder nicht aromatischen 5- bis 6-gliedringen Ring bilden, worin 0, 1 oder 2 Kohlenstoffatome in dem Ring durch ein Heteroatom, gewählt aus O, S oder NR₂₃, substituiert sind;
R₂₈ und R₂₉ sind, jedesmal wenn sie vorkommen, jeweils unabhängig gewählt aus der Gruppe bestehend aus Wasserstoff, Alkyl, Alkylcarbonyl, Alkoxycarbonyl, Cycloalkyl, Aryl, Heteroaryl und Heterozyklus, oder R₂₈ und R₂₉ bilden zusammengenommen mit dem Stickstoffatom, an welches jedes gebunden ist, einen aromatischen oder nicht aromatischen 5- bis 6-gliedrigen Ring, worin 0, 1 oder 2 Kohlenstoffatome in dem Ring durch ein Heteroatom, gewählt aus O, S oder NR₂₃, substituiert sind;
R₃₀ ist, jedesmal wenn es vorkommt, unabhängig gewählt aus der Gruppe bestehend aus Wasserstoff, Alkyl, Alkoxyalkyl, Hydroxyalkyl, Aryl, Arylalkyl, Cycloalkyl, Cycloalkylalkyl, Cyanoalkyl, Haloalkyl, Heteroaryl, Heteroarylalkyl, Heteroarylcarbonyl, Heterozyklus und Heterozyklusalkyl;
R_{30b} ist gewählt aus der Gruppe bestehend aus Wasserstoff und Alkyl, oder, wenn R₃₀ Alkyl ist und R_{30b} Alkyl ist, können die Alkylgruppen miteinander verbunden werden, um eine C₃-C₄ Cycloalkylgruppe zu bilden; und
R₃₁ ist gewählt aus der Gruppe bestehend aus Alkyl, Cycloalkyl, Aryl, Heterozyklus und Heteroaryl.

2. Die Verbindung gemäß Anspruch 1, mit der Formel: 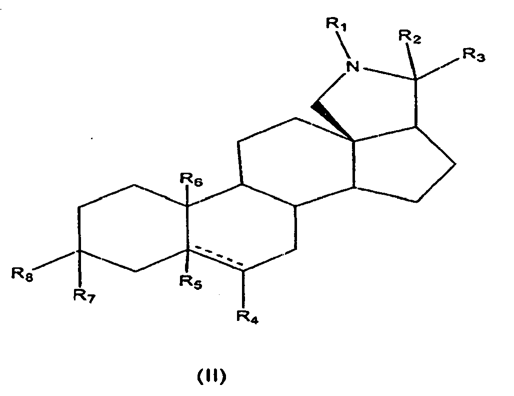 worin:
die gestrichelte Linie eine optionale Bindung darstellt;
R₁ ist gewählt aus der Gruppe bestehend aus Wasserstoff, Acetyl, Alkyl, Fluoralkyl und Cycloalkyl;
R₂ und R₃ sind jeweils unabhängig gewählt aus der Gruppe bestehend aus Wasserstoff und Alkyl, oder R₂ und R₃ zusammengenommen bilden einen 3- bis 6-gliedrigen Ring;
R₄ und R₅ sind jeweils unabhängig gewählt aus der Gruppe bestehend aus Wasserstoff und Fluor, vorausgesetzt, dass R₅ nur anwesend ist, wenn die Bindung, die durch die gestrichelte Linie dargestellt ist, nicht vorhanden ist;
R₆ ist gewählt aus der Gruppe bestehend aus Wasserstoff, Alkyl und Fluor;
eines von R₇ und R₈ ist Wasserstoff; und das andere von R₇ und R₈ ist gewählt aus der Gruppe bestehend aus:
a) NR₁₈R₁₉;
b) SR₂₀ oder O(C=O) N (R₂₀) (R₂₁), und
c) NR₂₂ (C=O) NR₂₆R₂₇, NR₂₂ (C=O) CH (NR₂₈R₂₉) R₃₀ oder NR₂₂ (C=O) C (OR₂₃) R₃₀R_{30b}; und
d) NR₂₂SO₂R₃₁ oder NR₂₂SO₂N (R₂₂) (R₂₃);
oder R₇ und R₈ bilden zusammengenommen mit dem Kohlenstoffatom, an welches jedes gebunden ist, eine Gruppe mit der Formel -C=C(Rₐ) (R_{b}), worin Rₐ und R_{b} jeweils unabhängig gewählt sind aus der Gruppe bestehend aus Wasserstoff, Alkyl und Cycloalkyl;
R₁₈ ist Wasserstoff oder C₁-C₆ Alkyl und R₁₉ ist gewählt aus der Gruppe bestehend aus Aryl und Heteroaryl, oder R₁₈ und R₁₉ bilden, jedesmal wenn sie vorkommen, zusammengenommen einen 3-bis 8-gliedrigen Heterozyklus;
R₂₀ und R₂₁ sind, jedesmal sie vorkommen, jeweils unabhängig gewählt aus der Gruppe bestehend aus Wasserstoff, Alkyl, Cycloalkyl, Alkoxyalkyl, Cyanoalkyl, Hydroxyalkyl, Haloalkyl, Aryl, Aryloxy, Arylalkyl, Arylsulfonyl, Heteroaryl, Heteroarylalkyl, Heteroarylcarbonyl, Heterozyklus und Heterozyklusalkyl;
R₂₂ ist, jedesmal wenn es vorkommt, gewählt aus der Gruppe bestehend aus Wasserstoff und Alkyl;
R₂₃ ist, jedesmal wenn es vorkommt, gewählt aus der Gruppe bestehend aus Wasserstoff, Alkyl und Alkylcarbonyl;
R₂₆ und R₂₇ sind jeweils unabhängig gewählt aus der Gruppe bestehend aus Alkyl, Cycloalkyl, Aryl, Heteroaryl und Heterozyklus, vorausgesetzt, dass R₂₆ und R₂₇ nicht beide Alkyl sind, oder R₂₆ und R₂₇ bilden zusammengenommen mit dem Stickstoffatom, an welches jedes gebunden ist, einen aromatischen oder nicht aromatischen 5- bis 6-gliedrigen Ring, worin 0, 1 oder 2 Köhlenstoffatome in dem Ring durch ein Heteroatom, gewählt aus O, S oder NR₂₃, substituiert sind.
R₂₈ und R₂₉ sind jedesmal wenn sie vorkommen, jeweils unabhängig gewählt aus der Gruppe bestehend aus Wasserstoff, Alkyl, Alkylcarbonyl, Alkoxycarbonyl, Cycloalkyl, Aryl, Heteroaryl und Heterozyklus, oder R₂₈ und R₂₉ bilden zusammengenommen mit dem Stickstoffatom, an welches jedes gebunden ist, einen aromatischen oder nicht-aromatischen 5- bis 6-gliedrigen Ring, worin 0, 1 oder 2 Kohlenstoffatome in dem Ring durch ein Heteroatom, gewählt aus O, S oder NR₂₃, substituiert sind.
R₃₀ ist, jedesmal wenn es vorkommt, unabhängig gewählt aus der Gruppe bestehend aus Wasserstoff, Alkyl, Alkoxyalkyl, Hydroxyalkyl, Aryl, Arylalkyl, Cycloalkyl, Cycloalkylalkyl, Cyanoalkyl, Haloalkyl, Heteroaryl, Heteroarylalkyl, Heteroarylcarbonyl, Heterozyklus und Heterozyklusalkyl;
R_{30b} ist gewählt aus der Gruppe bestehend aus Wasserstoff und Alkyl oder, wenn R₃₀ Alkyl ist und R_{30b} Alkyl ist, können die Alkylgruppen miteinander verbunden werden, um eine C₃-C₄ Cycloalkylgruppe zu bilden; und
R₃₁ ist gewählt aus der Gruppe bestehend aus Alkyl, Cycloalkyl, Aryl, Heterozyklus und Heteroaryl.

3. Die Verbindung gemäß Anspruch 2, worin eines von R₇ und R₈ Wasserstoff ist; und das andere von R₇ und R₈ ist gewählt aus der Gruppe bestehend aus NR₂₂ (C=O) CH (NR₂₈R₂₉) R₃₀ und NR₂₂ (C=O) C (OR₂₃) R₃₀R_{30b}.

4. Die Verbindung gemäß Anspruch 3, worin R₇ oder R₈ NR₂₂ (C=O) CH (NR₂₈R₂₉) R₃₀ ist, worin R₂₂ Alkyl ist, R₂₈ und R₂₉ sind jeweils Wasserstoff, und R₃₀ ist gewählt aus der Grupp bestehend aus Alkyl und Aryl.

5. Die Verbindung gemäß Anspruch 4, worin R₂₂ Methyl ist.

6. Die Verbindung gemäß Anspruch 1, gewählt aus der Gruppe bestehend aus:
Trifluor-methansulfonsäure 8-Methyl-5,6,6a,7,8,9,10,11-octahydro-4bH-benzo[4,5]indeno[1,7a-c]pyrrol-2-yl Ester;
1-[3-(8-Methyl-5,6,6a,7,8,9,10,11-octahydro-4bH-benzo[4,5]indeno[1,7a-c]pyrrol-2-yl)-phenyl]-ethanon;
1-[3-(8-Methyl-5,6,6a,7,8,9,10,11-octahydro-4bH-benzo[4,5]indeno[1,7a-c]pyrrol-2-yl)-phenyl]-ethanonoxim;
1-[3-(8-Methyl-5,6,6a,7,8,9,10,11-octahydro-4bH-benzo[4,5]indeno[1,7a-c]pyrrol-2-yl)-phenyl]-ethanon;
N-Methyl-2-oxo-2-thiophen-2-yl-N-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-acetamid;
Ethansulfonsäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
N-Methyl-N-(2,3,11a-trimethyl-2,3,3a,4,5,5a,6,8,9,10,11,11a,11b,12,13-hexadecahydro-1H-2-azapent.aleno[1,6a-a]phenanthren-9-yl)-benzensulfonamid;
4-Cyano-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzensulfonamid;
Thiophen-2-sulfonsäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
4-Fluor-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzensulfonamid;
1,1-Dimethyl-3-methyl-3-(2,3,11a-trimethyl-2,3,3a,4,5,5a,6,8,9,10,11,11a,11b,12,13-hexadecahydro-1H-2-azapentaleno[1,6a-a]phenanthren-9-yl)-sulfamid;
1,1-Diisopropyl-3-methyl-3-(2,3,11a-trimethyl-2,3,3a,4,5,5a,6,8,9,10,11,11a,11b,12,13-hexadecahydro-1H-2-azapentaleno[1,6a-a]phenanthren-9-yl)-harnstoff;
1,1,3-Trimethyl-3-(2,3,11a-trimethyl-2,3,3a,4,5,5a,6,8,9,10,11,11a,11b,12,13-hexadecahydro-1H-2-azapentaleno[1,6a-a]phenanthren-9-yl)-harnstoff;
Methyl-{2-methyl-1-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-propyl}-carbaminsäure tert-Butylester;
3,N-Dimethyl-2-methylamino-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
2-(Acetyl-methyl-amino)-3,N-dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
2-Amino-N-methyl-3-phenyl-N-(2,3,11a-tetramethyl-2,3,3a,4,5,5a,6,8,9,10,11,11a,11b,12,13-hexadecahydro-1H-2-azapentaleno[1,6a-a]phenanthren-9-yl)-propionamid;
2-Amino-N-methyl-3-thiazol-4-yl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Amino-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Amino-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
2-Amino-3-cyano-N-methyl-N-(2,3,11a-tetramethyl-2,3,3a,4,5,5a,6,8,9,10,11,11a,11b,12,13-hexadecahydro-1H-2-azapentaleno[1,6a-a]phenanthren-9-yl)-propionamid;
2-Amino-N-methyl-3-thiophen-2-yl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
4-Methyl-2-methylamino-pentansäure Methyl-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-amid;
2-Amino-3-methyl-pentansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
2-Amino-3,N-dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
2-Amino-3-methyl-pentansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
2-Amino-4-methyl-pentansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
2-Acetylamino-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
2-Acetylamino-3-methyl-pentansäure Methyl-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-amid;
2-Acetylamino-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Acetylamino-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Acetylamino-N-methyl-3-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Acetylamino-4-methyl-pentansäure Methyl-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-amid;
2-Acetylamino-3,N-dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
2-Acetylamino-3,N-dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
2-Acetylamino-N-methyl-3-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Acetylamino-N-methyl-3-thiophen-2-yl-N-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-propionamid;
2-Acetylamino-4-methyl-pentansäure Methyl-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-amid;
4-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amino]-benzonitril;
3-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amino]-benzonitril;
Methyl-thiazol-2-yl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amin;
1-{3-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amino]-phenyl}-ethanon;
1-{4-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amino]-phenyl}-ethanon;
Essigsäure 1-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-2-phenylethylester;
2-Hydroxy-N-methyl-3-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
Essigsäure 1-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl)-2-phenylethylester;
2-Hydroxy-N-methyl-3-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
Essigsäure 3-Methyl-1-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-butylester;
2-Hydroxy-4-methyl-pentansäure Methyl-(2,3,11a-trimethyl-2,3,:3a,4,5,5a,6,8,9,10,11,11a,11b,12,13-hexadecahydro-1H-2-azapentaleno[1,6a-a]phenanthren-9-yl)-amid;
Essigsäure 2-Methyl-1-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-propylester;
2-Hydroxy-3,N-dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
Essigsäure 1-Methyl-1-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-propylester;
2-Hydroxy-2,N-dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
Essigsäure 1-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-cyclopropylester;
1-Hydroxy-cyclopropancarbonsäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
Essigsäure 1-[Methyl-(2,3,11a-trimethyl-2,3,.3a,4,5,5a,6,8,9,10,11,11a,11b,12,13-hexadecahydro-1H-2-azapentaleno[1,6a-a]phenanthren-9-yl)-carbamoyl]-pentylester;
2-Hydroxy-hexansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
Essigsäure [Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-phenylmethylester;
2-Hydroxy-N-methyl-2-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
Essigsäure 2,2,2-Trifluor-1-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-ethylester;
3,3,3-Trifluor-2-hydroxy-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
Essigsäure (4-Fluor-phenyl)-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-methylester;
2-(4-Fluor-phenyl)-2-hydroxy-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
Essigsäure (4-Methoxy-phenyl)-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-methylester;
2-Hydroxy-2-(4-methoxy-phenyl)-N-methyl-N-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-acetamid;
Essigsäure (3,4-Difluor-phenyl)-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-methylester;
2-(3,4-Difluor-phenyl)-2-hydroxy-N-methyl-N-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-acetamid;
Essigsäure [Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-phenylmethylester;
2-Hydroxy-N-methyl-2-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
Essigsäure [Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-phenylmethylester;
2-Hydroxy-N-methyl-2-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
Essigsäure 3-Methyl-1-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-butylester;
2-Hydroxy-4-methyl-pentansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
Essigsäure 3-Methyl-1-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-butylester;
2-Hydroxy-4-methyl-pentansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
2-Methoxy-N-methyl-2-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
2-Methoxy-N-methyl-2-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
2-Methoxy-N-methyl-2-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
2-Methoxy-hexansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
2-Methoxy-4-methyl-pentansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
2-Methoxy-N-methyl-3-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Methoxy-3,N-dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
9-Ethyliden-2,3,11a-trimethyl-2,3,3a,4,5,5a,6,8,9,10,11,11a,11b,12,13-hexadecahydro-1H-2-azapentaleno[1,6a-a]phenanthren;
9-Isopropyliden-2,3,11a-trimethyl-2,3,3a,4,5,5a,6,8,9,10,11,11a,11b,12,13-hexadecahydro-1H-2-azapentaleno[1,6a-a]phenanthren;
Benzensulfonsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
4-Cyano-benzensulfonsäure 2,3,11a-Trimethyl-2,3,3a,4,5,5a,6,8,9,10,11,11a,11b,12,13-hexadecahydr-1H-2-azapentaleno[1,6a-a]phenanthren-9-yl Ester;
Furan-2-ylmethyl-methyl-carbaminsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Methyl-propyl-carbaminsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Benzyl-methyl-carbaminsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Methyl-(6-methyl-pyridin-2-ylmethyl)-carbaminsäure 2,3,11a-Trimethyl-2,3,3a,4,5,5a,6,8,9,10,11,11a,11b,12,13-hexadecahydro-1H-2-aza-pentaleno[1,6a-a]phenanthren-9-yl Ester;
Methyl-(tetrahydro-furan-2-ylmethyl)carbaminsäure 2,3,11a-Trimethyl-2,3,3a,4,5,5a,6,8,9,10,11,11a,11b,12,13-hexadecahydro-1H-2-aza-pentaleno[1,6a-a]phenanthren-9-yl Ester;
(2-Fluor-ethyl)-carbaminsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Furan-2-ylmethyl-carbaminsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
(2-Cyano-ethyl)-(tetrahydro-furan-2-ylmethyl)carbaminsäure 2,3,11a-Trimethyl-2,3,3a,4,5,5a,6,8,9,10,11,11a,11b,12,13hexadecahydro-1H-2-aza-pentaleno[1,6a-a]phenanthren-9-yl Ester;
Benzyl-carbaminsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester; und
Propyl-carbaminsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester.

7. Eine Verbindung gemäß Anspruch 1 zur Behandlung von Erkrankungen und Störungen, die mit der Histamin-3-Rezeptor-Modulation in Säugetieren in Zusammenhang stehen.

8. Eine Verbindung mit der Formel: 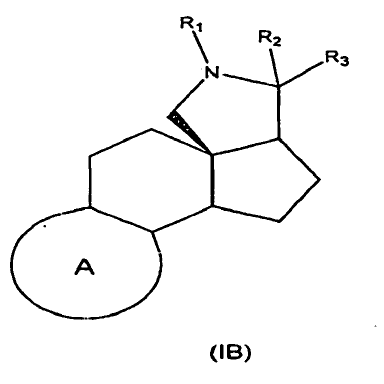 oder ein pharmazeutisch verträgliches Salz davon, worin
R₁ gewählt ist aus der Gruppe bestehend aus Wasserstoff, Acetyl, Alkyl, Fluoralkyl und Cycloalkyl;
R₂ und R₃ sind jeweils unabhängig gewählt aus der Gruppe bestehend aus Wasserstoff und Alkyl, oder R₂ und R₃ bilden zusammengenommen einen 3- bis 6-gliedrigen Ring;
Ring A mit der Formel: 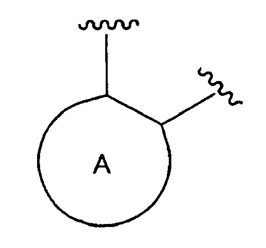 ist gewählt aus folgendem: 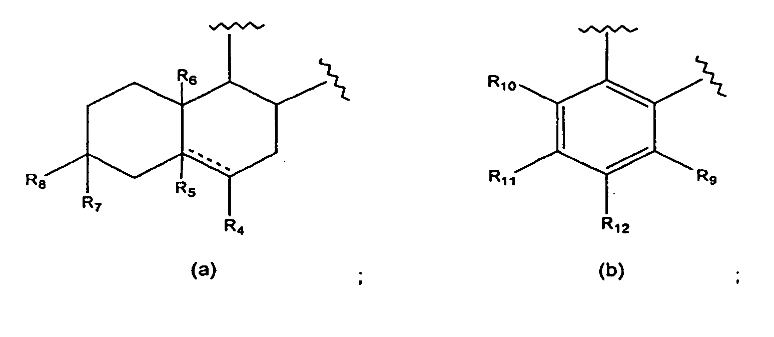 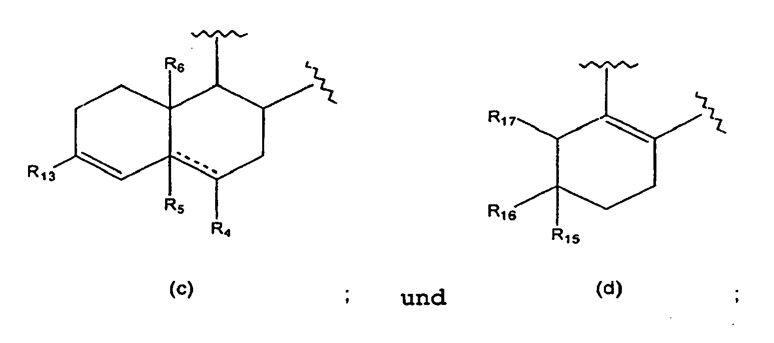 worin:
die gestrichelte Linie eine optionale Bindung darstellt;
R₄ und R₅ sind jeweils unabhängig gewählt aus der Gruppe bestehend aus Wasserstoff und Fluor, vorausgesetzt, dass R₅ nur dann vorhanden ist, wenn die Bindung, die durch die gestrichelte Linie dargestellt ist, nicht vorhanden ist;
R₆ ist gewählt aus der Gruppe bestehend aus Wasserstoff, Alkyl und Fluor;
eines von R₇ und R₈ ist Wasserstoff; und das andere von R₇ und R₈ ist gewählt aus der Gruppe bestehend aus:
a) NR₁₈R₁₉;
b) OR₂₀, SR₂₀, O(C=O)OR₂₀, O(C=O)N(R₂₀) (R₂₁), O(C=O)C(R₂₃) (R₂₄) (R_{24b}) oder O(C=O)CH(NR₂₈R₂₉)R₂₅; und
c) NR₂₂ (C=O)R₂₅, NR₂₂ (C=O)NR₂₆R₂₇, NR₂₂ (C=O)CH(NR₂₈R₂₉ R₃₀, N(R₂₂) (C=O)OR₂₀ oder NR₂₂ (C=O) C (OR₂₃) R₃₀R_{30b}; und
d) NR₂₂SO₂R₃₁ oder NR₂₂SO₂N(R₂₂) (R₂₃) ;
oder R₇ und R₈ bilden zusammengenommen mit dem Kohlenstoffatom, an welches jedes gebunden ist, eine Gruppe mit der Formel -C=C (Rₐ) (R_{b}), worin Rₐ und R_{b} jeweils unabhängig gewählt sind aus der Gruppe bestehend aus Wasserstoff, Alkyl und Cycloalkyl;
R₉ und R₁₀ sind jeweils unabhängig gewählt aus der Gruppe bestehend aus Wasserstoff, Halogen, Alkyl und Cyano;
R₁₁ und R₁₂ sind jeweils unabhängig gewählt aus der Gruppe bestehend aus Wasserstoff, Heteroaryl, Heterzyklus, Aryl, Arylalkyl, Aryloxy, Arylcarbonyl, Arylcarbonyloxy, Arylalkoxy, Alkylsulfonyl, Arylsulfonyl und Trifluormethylsulfonyl; oder eines von R₁₀ und R₁₁ oder R₁₁ und R₁₂ bilden zusammengenommen mit den Atomen, an welche sie jeweils gebunden sind, einen 5- bis 6-gliedrigen aromatischen oder heteroaromatischen Ring;
R₁₃ ist gewählt aus der Gruppe bestehend aus Alkyl, Cycloalkyl, Aryl und Heteroaryl;
R₁₅ und R₁₆ bilden zusammengenommen mit dem Atom, an welches jedes gebunden ist, einen 5- bis 6-gliedrigen Ring, wahlweise substituiert mit einer Alkylaminogruppe;
R₁₇ ist gewählt aus der Gruppe bestehend aus Wasserstoff, Alkyl und Fluoro;
R₁₈ ist Wasserstoff oder C₁-C₆ Alkyl und R₁₉ ist gewählt aus der Gruppe bestehend aus Aryl und Heteroaryl, oder R₁₈ und R₁₉ bilden, jedesmal wenn sie vorkommen, zusammengenommen einen 3-bis 8-gliedrigen Heterozyklus;
R₂₀ und R₂₁ sind, jedesmal wenn sie vorkommen, jeweils unabhängig gewählt aus der Gruppe bestehend aus Wasserstoff, Alkyl, Cycloalkyl, Alkoxyalkyl, Cyanoalkyl, Hydroxyalkyl, Haloalkyl, Aryl, Aryloxy, Arylalkyl, Arylsulfonyl, Heteroaryl, Heteroarylalkyl, Heteroarylcarbonyl, Heterozyklus und Heterozyklusalkyl;
R₂₂ ist jedesmal wenn es vorkommt, gewählt aus der Gruppe bestehend aus Wasserstoff und Alkyl;
R₂₃ ist jedesmal wenn es vorkommt, gewählt aus der Gruppe bestehend aus Wasserstoff, Alkyl und Alkylcarbonyl;
R₂₄ und R_{24b} sind jeweils unabhängig gewählt aus der Gruppe bestehend aus Wasserstoff, Alkyl, Aryl, Arylalkyl und Cycloalkyl;
R₂₅ ist, jedesmal wenn es vorkommt, unabhängig gewählt aus der Gruppe bestehend aus C₁-C₆ Alkyl, Alkoxyalkyl, Hydroxyalkyl, Aryl, Arylalkyl, Cycloalkyl, Cycloalkylalkyl, Cyanoalkyl, Haloalkyl, Heteroaryl, Heterarylalkyl, Heteroarylcarbonyl, Heterozyklus und Heterozyklusalkyl;
R₂₆ und R₂₇ sind, jedesmal wenn sie vorkommen, jeweils unabhängig gewählt aus der Gruppe bestehend aus Alkyl, Alkylcarbonyl, Alkoxycarbonyl, Cycloalkyl, Aryl, Heteroaryl und Heterozyklus, oder R₂₆ und R₂₇ bilden zusammengenommen mit dem Stickstoffatom, an welches sie jeweils gebunden sind, einen aromatischen oder nicht-aromatischen 5- bis 6-gliedringen Ring, worin 0, 1 oder 2 Kohlenstoffatome in dem Ring substituiert sind mit einem Heteroatom, gewählt aus O, S oder NR₂₃;
R₂₈ und R₂₉ sind, jedesmal wenn sie vorkommen, jeweils unabhängig gewählt aus der Gruppe bestehend aus Wasserstoff, Alkyl, Alkylcarbonyl, Alkoxycarbonyl, Cycloalkyl, Aryl, Heteroaryl und Heterozyklus, oder R₂₈ und R₂₉ bilden zusammengenommen mit dem Stickstoffatom, an welches sie gebunden sind, einen aromatischen oder nicht-aromatischen 5- bis 6-gliedrigen Ring, worin 0, 1 oder 2 Kohlenstoffatome in dem Ring mit einem Heteroatom, gewählt aus O, S oder NR₂₃, substituiert sind;
R₃₀ ist, jedesmal wenn es vorkommt, unabhängig gewählt aus der Gruppe bestehend aus Wasserstoff, Alkyl, Alkoxyalkyl, Hydroxyalkyl, Aryl, Arylalkyl, Cycloalkyl, Cycloalkylalkyl, Cyanoalkyl, Haloalkyl, Heteroaryl, Heteroarylalkyl, Heteroarylcarbonyl, Heterozyklus und Heterozyklusalkyl;
R_{30b} ist gewählt aus der Gruppe bestehend aus Wasserstoff und Alkyl oder, wenn R₃₀ Alkyl ist und R_{30b} Alkyl ist, können die Alkylgruppen miteinander verbunden werden, um eine C₃-C₄ Cycloalkylgruppe zu bilden; und
R₃₁ ist gewählt aus der Gruppe bestehend aus Alkyl, Cycloalkyl, Aryl, Heterozyklus und Heteroaryl;
oder eine Verbindung, die Benzoesäure 2,3,11a-Trimethyl-2,3,3a,4,5,5a,5b,6,8,9,10,11,11a,11b,12,13-hexadecahydro-1H-2-aza-pentaleno[1,6a-a]phenanthren-9-yl Ester ist;
für die Verwendung in der Behandlung eines Zustandes, der durch die Histamin-3-Rezeptoren in einem Säugetier moduliert wird, umfassend die Verabreichung dieser Verbindung an ein Säugetier, das dies benötigt, worin der Zustand eine Funktion in einem Säugetier betrifft, die gewählt ist aus der Gruppe bestehend aus Gedächtnis- und kognitiven Prozessen, neurologischen Prozessen und der Regulierung des Blutzuckers.

9. Die Verbindung gemäß Anspruch 8, worin der Zustand das Gedächtnis und kognitive Prozesse betrifft.

10. Die Verbindung gemäß Anspruch 8, worin der Zustand gewählt ist aus der Gruppe bestehend aus der Alzheimer sehen Krankheit, Aufmerksamkeitsdefizit-Hyperaktivitätsstörung, bipolarer Störung, kognitiver Erweiterung, kognitiven Defiziten bei psychiatrischen Erkrankungen, Defiziten des Gedächtnisses, Defiziten des Lernens, Demenz, Drogenmissbrauch, Diabetes, Diabetes vom Typ II, Depression, Epilepsie, Insulinresistenz-Syndrom, Jetlag, metabolischem Syndrom, leichter kognitiver Beeinträchtigung, Migräne, Stimmungs- und Aufmerksamkeitsschwankungen, Reisekrankheit, Narkolepsie, Fettleibigkeit, Zwangsstörungen, Schmerz, Parkinson'scher Krankheit, Schizophrenie, Anfällen, Schlafstörungen, dem X Syndrom, Tourette Syndrom, Schwindel und Schlaflosigkeit.

11. Die Verbindung gemäß Anspruch 8, worin die Verbindung gewählt ist aus der Gruppe bestehend aus:
Trifluor-methansulfonsäure 8-Methyl-5,6,6a,7,8,9,10,11-octahydro-4bH-benzo[4,5]indeno[1,7a-c]pyrrol-2-yl Ester;
1-[3-(8-Methyl-5,6,6a,7,8,9,10,11-octahydro-4bH-benzo[4,5]indeno[1,7a-c]pyrrol-2-yl)-phenyl]-ethanon;
1-[3-(8-Methyl-5,6,6a,7,8,9,10,11-octahydro-4bH-benzo[4,5]indeno[1,7a-c]pyrrol-2-yl)-phenyl]-ethanonoxim;
1-[3-(8-Methyl-5,6,6a,7,8,9,10,11-octahydro-4bH-benzo[4,5]indeno[1,7a-c]pyrrol-2-yl)-phenyl]-ethanon;
Benzoesäure 8-Acetyl-5,6,6a,7,8,9,10,11-octahydro-4bH-benzo[4,5]indeno[1,7a-c]pyrrol-2-yl Ester;
1-(2-Benzyloxy-5,6,6a,7,10,11-hexahydro-4bH-benzo[4,5]indeno[1,7a-c]pyrrol-8-yl)-ethanon;
2,2,N-Trimethyl-N-(2,3,11a-trimethyl-2,3,3a,4,5,5a,5b,6,8,9,10,11,11a,11b,12,13-hexadecahydro-1H-2-aza-pentaleno[1,6a-a]phenanthren-9-yl)propionamid;
Furan-2-carbonsäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
4-Fluor-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzamid;
Thiophen-2-carbonsäure Methyl-(2,3,11a-trimethyl-2,:3,3a,4,5,5a,6,8,9,10,11,11a,11b,12,13-hexadecahydro-1H-2-azapentaleno[1,6a-a]phenanthren-9-yl)-amid;
Isoxazol-5-carbonsäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
N-Methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-isonicotinamid;
3-Chlor-4-fluor-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzamid;
2-(4-Methoxy-phenyl)-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
2,3,4-Trifluor-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzamid;
4-Cyano-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzamid;
N-Methyl-2-t-hiophen-2-yl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
3-Chlor-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzamid;
3-Methoxy-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzamid;
4-Methoxy-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzamid;
4,N-Dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzamid;
4-Chlor-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzamid;
3-Cyano-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzamid;
4-Brom-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzamid;
3,N-Dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
Cyclopropancarbonsäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
2-Methoxy-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
N-Methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Isopropyl-4-methyl-thiazol-5-carbonsäure Methyl-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-amid;
2-sec-Butyl-4-methyl-thiazol-5-carbonsäure Methyl-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-amid;
2-Methyl-5-phenyl-furan-3-carbonsäure Methyl-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-amid;
N-Methyl-2-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
N-Methyl-2-thiophen-3-yl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
N-Methyl-4-thiophen-2-yl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
2-(3-Fluor-phenyl)-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
N-Methyl-2-oxo-2-thiophen-2-yl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
2-Indol-1-yl-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
2-Benzo[b]thiophen-3-yl-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
N-Methyl-2-(3-methyl-benzo[b]thiophen-2-yl)-N-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-acetamid;
2-Furan-2-yl-N-methyl-2-oxo-N-(2,3,11a-trimethyl-2,3,3a,4,5,5a,6,8,9,10,11,11a,11b,12,1.3-hexadecahydro-1H-2-azapentaleno[1,6a-a]phenanthren-9-yl)-acetamid;
N-Methyl-3-thiophen-2-yl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
3-Furan-2-yl-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-(3-Chlor-phenyl)-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
2-(4-Chlor-phenyl)-N-methyl-N-(2,3,11a-trimethyl2,3,3a,4,5,5a,6,8,9,10,11,11a,11b,12,13-hexadecahydro-1H-2-azapentaleno[1,6a-a]phenanthren-9-yl)-acetamid;
2-(2-Fluor-phenyl)-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
2-(4-Fluor-phenyl)-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
N-Methyl-2-o-tolyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
Ethansulfonsäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
N-Methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzensulfonamid;
4-Cyano-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzensulfonamid;
Thiophen-2-sulfonsäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
4-Fluor-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzensulfonamid;
1,1-Dimethyl-3-methyl-3-(2,3,11a-trimethyl-2,3,3a,4,5,5a,6,8,9,10,11,11a,11b,12,13-hexadecahydro-1H-2-azapentaleno[1,6a-a]phenanthren-9-yl)-sulfamid;
Pyrrolidin-1-carbonsäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
1,1-Diisopropyl-3-methyl-3-(2,3,11a-trimethyl-2,3,3a,4,5,5a,6,8,9,10,11,11a,11b,12,13-hexadecahydro-1H-2-azapentaleno[1,6a-a]phenanthren-9-yl)-harnstoff;
Morpholin-4-carbonsäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
1,1,3-Trimethyl-3-(2,3,11a-trimethyl-2,3,3a,4,5,5a,6,8,9,10,11,11a,11b,12,13-hexadecahydro-1H-2-azapentaleno[1,6a-a]phenanthren-9-yl)-harnstoff;
Methyl-{2-methyl-1-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-propyl]-carbaminsäure tert-Butylester;
3,N-Dimethyl-2-methylamino-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
2-(Acetyl-methyl-amino)-3,N-dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
2-Amino-N-methyl-3-phenyl-N-(2,3,3a,11a-tetramethyl-2,3,3a,4,5,5a,6,8,9,10,11,11a,11b,12,13-hexadecahydro-1H-2-azapentaleno[1,6a-a]phenanthren-9-yl)-propionamid;
2-Amino-N-methyl-3-thiazol-4-yl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Amino-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Amino-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
2-Amino-3-cyano-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Amino-N-methyl-3-thiophen-2-yl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
4-Methyl-2-methylamino-pentansäure Methyl-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-amid;
2-Amino-3-methyl-pentansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
2-Amino-3,N-dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
2-Amino-3-methyl-pentansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
2-Amino-4-methyl-pentansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
2-Acetylamino-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
2-Acetylamino-3-methyl-pentansäure Methyl-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-amid;
2-Acetylamino-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Acetylamino-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Acetylamino-N-methyl-3-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Acetylamino-4-methyl-pentansäure Methyl-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-amid;
2-Acetylamino-3,N-dimethyl-N-(2,3,11a-trimethyl-2,3,3a,4,5,5a,6,8,9,10,11,11a,11b,12,1.3-hexadecahydro-1H-2-azapentaleno[1,6a-a]phenanthren-9-yl)-butyramid;
2-Acetylamino-3,N-dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
2-Acetylamino-N-methyl-3-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Acetylamino-N-methyl-3-thiophen-2-yl-N-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-propionamid;
2-Acetylamino-4-methyl-pentansäure Methyl-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-amid;
4-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amino]-benzonitril;
3-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amino]-benzonitril;
Methyl-thiazol-2-yl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amin;
1-{3-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amino]-phenyl}-ethanon;
1-{4-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amino]-phenyl}-ethanon;
Essigsäure 1-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-2-phenyl-ethylester;
2-Hydroxy-N-methyl-3-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
Essigsäure 1-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-2-phenylethylester;
2-Hydroxy-N-methyl-3-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
Essigsäure 3-Methyl-1-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-butylester;
2-Hydroxy-4-methyl-pentansäure Methyl-(2,3,11ä-trimethyl-2,3,3a,4,5,5a,6,8,9,10,11,11a,11b,12,13-hexadecahydro-1H-2-azapentaleno[1,6a-a]phenanthren-9-yl)-amid;
Essigsäure 2-Methyl-1-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-propylester;
2-Hydroxy-3,N-dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
Essigsäure 1-Methyl-1-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-propylester;
2-Hydroxy-2,N-dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
Essigsäure 1-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-cyclopropylester;
1-Hydroxy-cyclopropancarbonsäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
Essigsäure 1-[Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-pentylester;
2-Hydroxy-hexansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
Essigsäure [Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-phenylmethylester;
2-Hydroxy-N-methyl-2-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
Essigsäure 2,2,2-Trifluor-1-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-ethylester;
3,3,3-Trifluor-2-hydroxy-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
Essigsäure (4-Fluor-phenyl)-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-methylester;
2-(4-Fluor-phenyl)-2-hydroxy-N-methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
Essigsäure (4-Methoxy-phenyl)-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-methylester;
2-Hydroxy-2-(4-methoxy-phenyl)-N-methyl-N-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-acetamid;
Essigsäure (3,4-Difluor-phenyl)-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-methylester;
2-(3,4-Difluor-phenyl)-2-hydroxy-N-methyl-N-(2,3,11a-trimethyl-2,3,3a,4,5,5a,6,8,9,10,11,11a,11b,12,13-hexadecahydro-1H-2-aza-pentaleno[1,6a-a]phenanthren-9-yl)-acetamid;
Essigsäure [Methyl-(2,3,11a-trimethyl-2,3,3a,4,5,5a,6,8,9,10,11,11a,11b,12,13-hexadecahydr-o-1H-2-azapentaleno[1,6a-a]phenanthren-9-yl)-carbamoyl]-phenylmethylester;
2-Hydroxy-N-methyl-2-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
Essigsäure [Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-phenylmethylester;
2-Hydroxy-N-methyl-2-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
Essigsäure 3-Methyl-1-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-butylester;
2-Hydroxy-4-methyl-pentansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
Essigsäure 3-Methyl-1-[methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbamoyl]-butylester;
2-Hydroxy-4-methyl-pentansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
2-Methoxy-N-methyl-2-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
2-Methoxy-N-methyl-2-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
2-Methoxy-N-methyl-2-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
2-Methoxy-hexansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
2-Methoxy-4-methyl-pentansäure Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-amid;
2-Methoxy-N-methyl-3-phenyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-propionamid;
2-Methoxy-3,N-dimethyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-butyramid;
9-Ethyliden-2,3,11a-trimethyl-2,3,3a,4,5,5a,6,8,9,10,11,11a,11b,12,13-hexadecahydro-1H-2-azapentaleno[1,6a-a]phenanthren;
9-Isopropyliden-2,3,11a-trimethyl-2,3,3a,4,5,5a,6,8,9,10,11,11a,11b,12,13-hexadecahydro-H-2-azapentaleno[1,6a-a]phenanthren;
9-Benzyloxy-2,3,11a-trimethyl-2,3,3a,4,5,5a,6,8,9,10,11,11a,11b,12,13-hexadecahydro-1H-2-azapentaleno[1,6a-a]phenanthren;
Essigsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Thiophen-2-yl-essigsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
4-Cyano-benzoesäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
3-Methyl-buttersäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Furan-2-carbonsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Cyclopropancarbonsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Methoxy-essigsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Isobuttersäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Cyclobutancarbonsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Propionsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Phenyl-essigsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Benzensulfonsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
4-Cyano-benzensulfonsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
(4-Fluor-phenyl)-essigsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
2-(tert-Butoxycarbonyl-methyl-amino)-3-methyl-buttersäure 2,3,11a-Trimethyl-2,3,3a,4,5,5a,6,8,9,10,11,11a,11b,12,13-hexadecahydro-1H-2-aza-pentaleno[1,6a-a]phenanthren-9-yl Ester;
3-Methyl-2-methylamino-buttersäure-2,3,11a-trimethyl-2,3,3a,4,5,5a,6,8,9,10,11,11a,11b,12,13-hexadecahydro-1H-2-azapentaleno[1,6a-a]phenanthren-9-yl Ester;
Tetrahydro-furan-2-carbonsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Furan-2-yl-oxo-essigsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
2-Acetoxy-3-phenyl-propionsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
2-Acetoxy-4-methyl-pentansäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
2-Acetoxy-3-methyl-buttersäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
2-Acetoxy-2-methyl-buttersäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
1-Acetoxy-cyclopropancarbonsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
2-Acetoxy-2-ethyl-buttersäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
2-Acetoxy-hexansäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Methoxy-phenyl-essigsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Carbonsäure 4-Nitro-phenylester 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Furan-2-ylmethyl-methyl-carbaminsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Methyl-propyl-carbaminsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Benzyl-methyl-carbaminsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Methyl-(6-methyl-pyridin-2-ylmethyl)carbaminsäure 2,3,11a-Trimethyl-2,3,3a,4,5,5a,6,8,9,10,11,11a,11b,12,13-hexadecahydro-1H-2-aza-pentaleno[1,6a-a]phenanthren-9-yl Ester;
Methyl-(tetrahydro-furan-2-ylmethyl)-carbaminsäure 2,3,11a-Trimethyl-2,3,3a,4,5,5a,6,8,9,10,11,11a,11b,12,13-hexadecahydro-1H-2-aza-pentaleno[1,6a-a]phenanthren-9-yl Ester;
(2-Fluor-ethyl)-carbaminsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Furan-2-ylmethyl-carbaminsäure 2,3,11a-Trimethyl-2,3,3a,4,5,5a,6,8,9,10,11,11a,11b,12,1.3-hexadecahydro-1H-2-azapentaleno[1,6a-a]phenanthren-9-yl Ester;
(2-Cyano-ethyl)-(tetrahydro-furan-2-ylmethyl)-carbaminsäure 2,3,11a-Trimethyl-2,3,3a,4,5,5a,6,8,9,10,11,11a,11b,12,13-hexadecahydro-1H-2-aza-pentaleno[1,6a-a]phenahthren-9-yl Ester;
Benzyl-carbaminsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
Propyl-carbaminsäure 2,3,11a-Trimethyl-2,3,3a,4,5,5a,6,8,9,10,11,11a,11b,12,13-hexadecahydro-1H-2-azapentaleno[1,6a-a]phenanthren-9-yl Ester;
N-Methyl-2-thiophen-2-yl-N-(2,3,11a-trimethyloctadecahydro-2-aza-pentaleno[1,6a-a]phenanthren-9-yl)-acetamid;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure Benzylester;
Methyl-(2,3,11a-trimethyl2,3,3a,4,5,5a,6,8,9,10,11,11a,11b,12,13-hexadecahydro-1H-2-azapentaleno[1,6a-a]phenanthren-9-yl)-carbaminsäure Methylester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure 2,2-Dimethylpropylester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure Isobutylester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure Ethylester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure tert-Butylester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure Isopropylester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure Cyclopentylester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure Furan-3-ylmethylester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure 5-Methoxy-tetrahydro-furan-3-yl Ester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure Thiazol-5-ylmethylester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure Hexahydrofuro[2,3-b]furan-3-yl Ester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure Tetrahydrofuran-3-yl Ester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure Pyridin-2-yl Ester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure 3,5-Dimethylisoxazol-4-ylmethylester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure 3-Hydroxypropylester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure [1,3]Dioxolan-4-ylmethylester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure Oxiranylmethylester;
Methyl-(2,3,11a-trimethyl-2,3,3a,4,5,5a,6,8,9,10,11,11a,11b,12,13-hexadecahydro-1H-2-azapentaleno[1,6a-a]phenanthren-9-yl)-carbaminsäure 2-Hydroxyethylester;
N-Methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-acetamid;
N-Methyl-N-(2,3,11a-trimethyl-2,3,3a,4,5,5a,6,8,9,10,11,11a,11b,12,13-hexadecahydro-1H-2-azapentaleno[1,6a-a]phenanthren-9-yl)-benzamid;
9-Methoxy-2,3,11a-trimethyl-2,3,3a,4,5,5a,6,8,9,10,11,11a,11b,12,13-hexadecahydro-1H-2-azapentaleno[1,6a-a]phenanthren; und
N-Methyl-N-(2,3,11a-trimethyl-octadecahydro-2-azapentaleno[1,6a-a]phenanthren-9-yl)-benzamid.

## Revendications

1. Composé de formule : 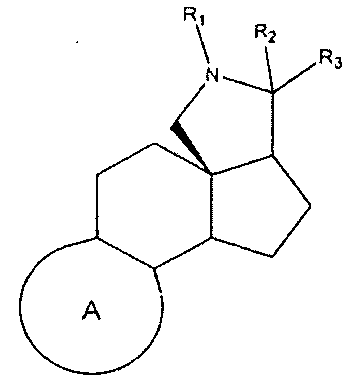 ou un sel pharmaceutiquement acceptable de celui-ci, formule dans laquelle
R₁ est choisi dans le groupe constitué par l'hydrogène, acétyle, alkyle, fluoroalkyle, et cycloalkyle ;
chacun de R₂ et R₃ est indépendamment choisi dans le groupe constitué par l'hydrogène et alkyle, ou bien R₂ et R₃, pris ensemble, forment un cycle à 3 à 6 chaînons ;
le cycle A de formule : 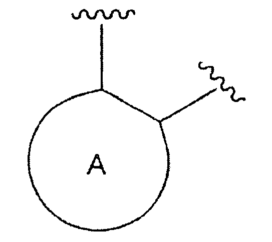 est choisi parmi les suivants : 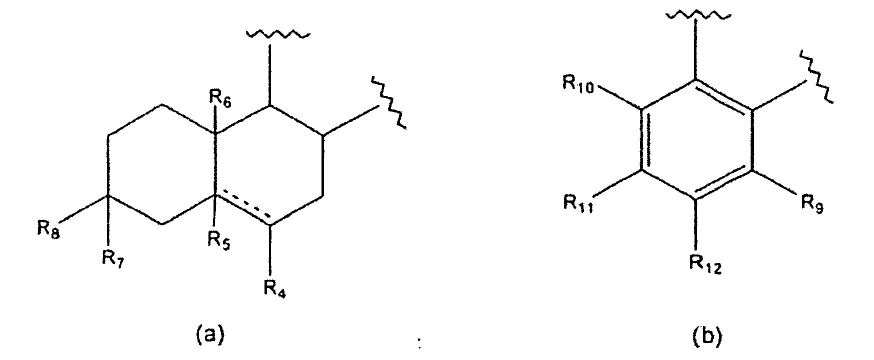 et 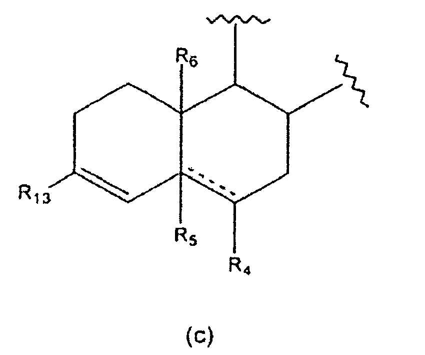 où :
la ligne de tirets représente une liaison optionnelle ;
chacun de R₄ et R₅ est indépendamment choisi dans le groupe constitué par l'hydrogène et le fluor, à la condition que R₅ soit présent uniquement quand la liaison représentée par la ligne de tirets est absente ;
R₆ est choisi dans le groupe constitué par l'hydrogène, alkyle et le fluor ;
l'un de R₇ et R₈ est l'hydrogène ; et l'autre de R₇ et R₈ est choisi dans le groupe constitué par :
a) NR₁₈R₁₉ ;
b) SR₂₀ ou O(C=O)N(R₂₀)(R₂₁), et
c) NR₂₂(C=O)NR₂₆R₂₇, NR₂₂(C=O)CH(NR₂₈R₂₉)R₃₀, ou
NR₂₂(C=O)C(OR₂₃)R₃₀R_{30b} ; et
d) NR₂₂SO₂R₃₁ ou NR₂₂SO₂N(R₂₂)(R₂₃) ;
ou bien R₇ et R₈, pris ensemble avec l'atome de carbone auquel chacun est rattaché, forment un groupe de formule -C=C(Rₐ)(R_{b}) dans laquelle chacun de Rₐ et R_{b} est indépendamment choisi dans le groupe constitué par l'hydrogène, alkyle et cycloalkyle ;
chacun de R₉ et R₁₀ est indépendamment choisi dans le groupe constitué par l'hydrogène, un halogène, alkyle, et cyano ;
chacun de R₁₁ et R₁₂ est indépendamment choisi dans le groupe constitué par l'hydrogène, hétéroaryle, un hétérocycle, aryle, arylalkyle, aryloxy, arylcarbonyle, arylcarbonyloxy, arylalcoxy, alkylsulfonyle, arylsulfonyle, et trifluorométhylsulfonyle ;
ou bien l'un de R₁₀ et R₁₁ ou de R₁₁ et R₁₂, pris ensemble avec les atomes auxquels chacun est rattaché, forment un cycle aromatique ou hétéroaromatique à 5 ou 6 chaînons ;
R₁₃ est choisi dans le groupe constitué par alkyle, cycloalkyle, aryle et hétéroaryle ;
R₁₈ est l'hydrogène ou un alkyle en C₁ à C₆ et R₁₉ est choisi dans le groupe constitué par aryle et hétéroaryle, ou bien R₁₈ et R₁₉, à chaque occurrence, sont choisis de façon à former ensemble un hétérocycle à 3 à 8 chaînons ;
chacun de R₂₀ et R₂₁, à chaque occurrence, est indépendamment choisi dans le groupe constitué par l'hydrogène, alkyle, cycloalkyle, alcoxyalkyle, cyanoalkyle, hydroxyalkyle, halogénoalkyle, aryle, aryloxy, arylalkyle, arylsulfonyle, hétéroaryle, hétéroarylalkyle, hétéroarylcarbonyle, un hétérocycle, et hétérocycloalkyle ;
R₂₂, à chaque occurrence, est choisi dans le groupe constitué par l'hydrogène et
alkyle ;
R₂₃, à chaque occurrence, est choisi dans le groupe constitué par l'hydrogène, alkyle et alkylcarbonyle ;
chacun de R₂₆ et R₂₇ est indépendamment choisi dans le groupe constitué par alkyle, cycloalkyle, aryle, hétéroaryle, et un hétérocycle, à la condition que R₂₆ et R₂₇ ne soient pas tous deux alkyle, ou bien R₂₆ et R₂₇, pris ensemble avec l'atome d'azote auquel chacun est rattaché, forment un cycle aromatique ou non aromatique à 5 ou 6 chaînons, dans lequel 0, 1 ou 2 atomes de carbone du cycle sont remplacés par un hétéroatome choisi parmi O, S et NR₂₃ ;
chacun de R₂₈ et R₂₉, à chaque occurrence, est indépendamment choisi dans le groupe constitué par l'hydrogène, alkyle, alkylcarbonyle, alcoxycarbonyle, cycloalkyle, aryle, hétéroaryle et un hétérocycle, ou bien R₂₈ et R₂₉, pris ensemble avec l'atome d'azote auquel chacun est rattaché, forment un cycle aromatique ou non aromatique à 5 ou 6 chaînons, dans lequel 0, 1 ou 2 atomes de carbone du cycle sont remplacés par un hétéroatome choisi parmi O, S et NR₂₃ ;
R₃₀, à chaque occurrence, est indépendamment choisi dans le groupe constitué par l'hydrogène, alkyle, alcoxyalkyle, hydroxyalkyle, aryle, arylalkyle, cycloalkyle, cycloalkylalkyle, cyanoalkyle, halogénoalkyle, hétéroaryle, hétéroarylalkyle, hétéroarylcarbonyle, un hétérocycle, et hétérocycloalkyle ;
R_{30b} est choisi dans le groupe constitué par l'hydrogène et alkyle, ou bien, quand R₃₀ est un alkyle et R_{30b} est un alkyle, les groupes alkyle peuvent être liés ensemble pour former un groupe cycloalkyle en C₃ à C₄ ; et
R₃₁ est choisi dans le groupe constitué par alkyle, cycloalkyle, aryle, un hétérocycle,
et hétéroaryle.

2. Composé selon la revendication 1, de formule : 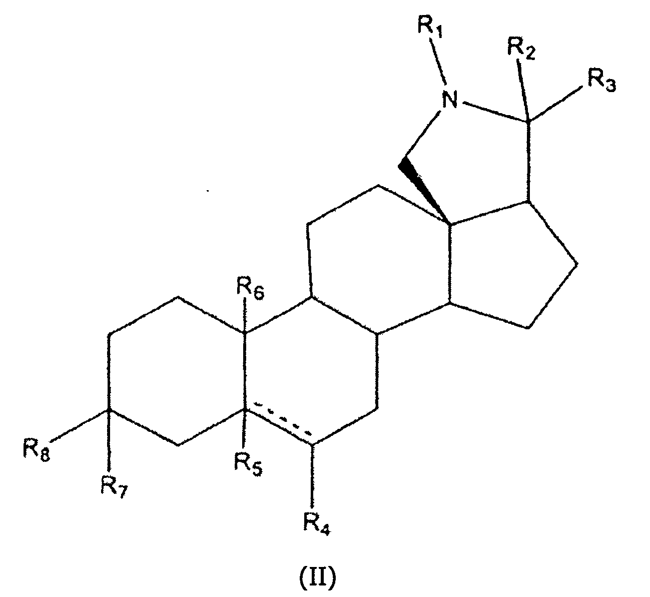 dans laquelle
la ligne de tirets représente une liaison optionnelle,
R₁ est choisi dans le groupe constitué par l'hydrogène, acétyle, alkyle, fluoroalkyle, et cycloalkyle ;
chacun de R₂ et R₃ est indépendamment choisi dans le groupe constitué par l'hydrogène et alkyle, ou bien R₂ et R₃, pris ensemble, forment un cycle à 3 à 6 chaînons ;
chacun de R₄ et R₅ est indépendamment choisi dans le groupe constitué par l'hydrogène et le fluor, à la condition que R₅ soit présent uniquement quand la liaison représentée par la ligne de tirets est absente ;
R₆ est choisi dans le groupe constitué par l'hydrogène, alkyle, et le fluor ;
l'un de R₇ et R₈ est l'hydrogène ; et l'autre de R₇ et R₈ est choisi dans le groupe constitué par :
a) NR₁₈R₁₉ ;
b) SR₂₀ ou O(C=O)N(R₂₀)(R₂₁), et
c) NR₂₂(C=O)NR₂₆R₂₇, NR₂₂(C=O)CH(NR₂₈R₂₉)R₃₀, ou
NR₂₂(C=O)C(OR₂₃)R₃₀R_{30b} ; et
d) NR₂₂SO₂R₃₁ ou NR₂₂SO₂N(R₂₂)(R₂₃) ;
ou bien R₇ et R₈, pris ensemble avec l'atome de carbone auquel chacun est rattaché, forment un groupe de formule -C=C(Rₐ)(R_{b}) dans laquelle chacun de Rₐ et R_{b} est indépendamment choisi dans le groupe constitué par l'hydrogène, alkyle et cycloalkyle ;
R₁₈ est l'hydrogène ou un alkyle en C₁ à C₆ et R₁₉ est choisi dans le groupe constitué par aryle et hétéroaryle, ou bien R₁₈ et R₁₉, à chaque occurrence, sont choisis de façon à former ensemble un hétérocycle à 3 à 8 chaînons ;
chacun de R₂₀ et R₂₁, à chaque occurrence, est indépendamment choisi dans le groupe constitué par l'hydrogène, alkyle, cycloalkyle, alcoxyalkyle, cyanoalkyle, hydroxyalkyle, halogénoalkyle, aryle, aryloxy, arylalkyle, arylsulfonyle, hétéroaryle, hétéroarylalkyle, hétéroarylcarbonyle, un hétérocycle, et hétérocycloalkyle ;
R₂₂, à chaque occurrence, est choisi dans le groupe constitué par l'hydrogène et alkyle ;
R₂₃, à chaque occurrence, est choisi dans le groupe constitué par l'hydrogène, alkyle et alkylcarbonyle ;
chacun de R₂₆ et R₂₇ est indépendamment choisi dans le groupe constitué par alkyle, cycloalkyle, aryle, hétéroaryle, et un hétérocycle, à la condition que R₂₆ et R₂₇ ne soient pas tous deux alkyle, ou bien R₂₆ et R₂₇, pris ensemble avec l'atome d'azote auquel chacun est rattaché, forment un cycle aromatique ou non aromatique à 5 ou 6 chaînons, dans lequel 0, 1 ou 2 atomes de carbone du cycle sont remplacés par un hétéroatome choisi parmi O, S et NR₂₃ ;
chacun de R₂₈ et R₂₉, à chaque occurrence, est indépendamment choisi dans le groupe constitué par l'hydrogène, alkyle, alkylcarbonyle, alcoxycarbonyle, cycloalkyle, aryle, hétéroaryle et un hétérocycle, ou bien R₂₈ et R₂₉, pris ensemble avec l'atome d'azote auquel chacun est rattaché, forment un cycle aromatique ou non aromatique à 5 ou 6 chaînons, dans lequel 0, 1 ou 2 atomes de carbone du cycle sont remplacés par un hétéroatome choisi parmi O, S et N₂₃ ;
R₃₀, à chaque occurrence, est indépendamment choisi dans le groupe constitué par l'hydrogène, alkyle, alcoxyalkyle, hydroxyalkyle, aryle, arylalkyle, cycloalkyle, cycloalkyl-alkyle, cyanoalkyle, halogénoalkyle, hétéroaryle, hétéroarylalkyle, hétéroarylcarbonyle, un hétérocycle, et hétérocycloalkyle ;
R_{30b} est choisi dans le groupe constitué par l'hydrogène et alkyle, ou bien, quand R₃₀ est un alkyle et R_{30b} est un alkyle, les groupes alkyle peuvent être liés ensemble pour former un groupe cycloalkyle en C₃ à C₄ ; et
R₃₁ est choisi dans le groupe constitué par alkyle, cycloalkyle, aryle, un hétérocycle, et hétéroaryle.

3. Composé selon la revendication 2, dans lequel l'un de R₇ et R₈ est l'hydrogène ; et l'autre de R₇ et R₈ est choisi dans le groupe constitué par NR₂₂(C=O)CH(NR₂₈R₂₉)R₃₀ et NR₂₂(C=O)C(OR₂₃)R₃₀R_{30b}.

4. Composé selon la revendication 3, dans lequel R₇ ou R₈ est NR₂₂(C=O)CH(NR₂₈R₂₉)R₃₀ où R₂₂ est un alkyle, et chacun de R₂₈ et R₂₉ est l'hydrogène, et R₃₀ est choisi dans le groupe constitué par alkyle et aryle.

5. Composé selon la revendication 4, dans lequel R₂₂ est méthyle.

6. Composé selon la revendication 1, choisi dans le groupe constitué par les suivants :
ester 8-méthyl-5,6,6a,7,8,9,10,11-octahydro-4bH-benzo[4,5]indéno[1,7a-c]pyrrol-2-ylique d'acide trifluorométhanesulfonique ;
1-[3-(8-méthyl-5,6,6a,7,8,9,10,11-octahydro-4bH-benzo[4,5]indéno[1,7a-c]pyrrol-2-yl)-phényl]-éthanone ;
1-[3-(8-méthyl-5,6,6a,7,8,9,10,11-octahydro-4bH-benzo[4,5]indéno[1,7a-c]pyrrol-2-yl)-phényl]-éthanone-oxime ;
1-[3-(8-méthyl-5,6,6a,7,8,9,10,11-octahydro-4bH-benzo[4,5]indéno[1,7a-c]pyrrol-2-yl)-phényl]-éthanone ;
N-méthyl-2-oxo-2-thiophén-2-yl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide ;
N-méthyl-N-(2,3,11a-triméthyl-2,3,3a,4,5,5a,5b,6,8,9,10,11,11a,11 b,12,13-hexadécahydro-1H-2-aza-pentaléno[1,6a-a]phénanthrén-9-yl)-benzènesulfonamide ;
4-cyano-N-méthyl-N-(2,3,11a-triméthyl-2,3,3a,4,5,5a,5b,6,8,9,10,11,11a,11 b,12,13-hexadécahydro-1H-2-azapentaléno[1,6a-a]phénanthrén-9-yl)-benzènesulfonamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide thiophène-2-sulfonique ;
4-fluoro-N-méthyl-N-(2,3,11a-triméthyl-2,3,3a,4,5,5a,5b,6,8,9,10,11,11a,116,12,13-hexadécahydro-1H-2-azapentaléno[1,6a-a]phénanthrén-9-yl)-benzènesulfonamide ;
1,1-diméthyl-3-méthyl-3-(2,3,11a-tri méthyl2,3,3a,4,5,5a,5b,6,8,9,10,11,11a,11b,12,13-hexadécahydro-1H-2-azapentaléno[1,6a-a][phénanthrén-9-yl)-sulfamide ;
1,1-diisopropyl-3-méthyl-3-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-urée ;
1,1,3-triméthyl-3-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-urée ;
ester tert-butylique d'acide méthyl-{2-méthyl-1-[méthyl-(2,3,11a-triméthy)-2,3,3a,4,5,5a,5b,6,8,9,10,11,11a,11b,12,13-hexadécahydro-1H-2-azapentaléno[1,6a-a]phénanthrén-9-yl)-carbamoyl]-propyl}-carbamique;
3,N-diméthyl-2-méthylamino-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
2-(acétyl-méthyl-amino)-3,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
2-amino-N-méthyl-3-phényl-N-(2,3,3a,11a-tétraméthyl2,3,3a,4,5,5a,5b,6,8,9,10,11,11a,11b,12,13-hexadécahydro-1H-2-azapentaléno[1,6a-a]phénanthrén-9-yl)-propionamide ;
2-amino-N-méthyl-3-thiazol-4-yl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
2-amino-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
2-amino-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
2-amino-3-cyano-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
2-amino-N-méthyl-3-thiophén-2-yl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 4-méthyl-2-méthylamino-pentanoïque ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-amino-3-méthyl-pentanoïque ;
2-amino-3,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-amino-3-méthyl-pentanoïque ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-amino-4-méthyl-pentanoïque ;
2-acétylamino-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-acétylamino-3-méthyl-pentanoïque ;
2-acétylamino-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
2-acétylamino-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
2-acétylamino-N-méthyl-3-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-acétylamino-4-méthyl-pentanoïque ;
2-acétylamino-3,N-diméthyl-N-(2,3,11a-triméthyl2,3,3a,4,5,5a,5b,6,8,9,10,11,11a,11b,12,13-hexadécahydro-1H-2-azapentaléno[1,6a-a]phénanthrén-9-yl)-butyramide ;
2-acétylamino-3,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
2-acétylamino-N-méthyl-3-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
2-acétylamino-N-méthyl-3-thiophén-2-yl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-acétylamino-4-méthyl-pentanoïque ;
4-[méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amino]-benzonitrile ;
3-[méthyl-(2,3,11a-triméthyl-2,3,3a,4,5,5a,5b,6,8,9,10,11,11a,11b12,13-hexadécahydro-1H-2-aza-pentaléno[1,6a-a]phénanthrén-9-yl)-amino]-benzonitrile ;
méthyl-thiazol-2-yl-(2,3,11a-triméthyl-2,3,3a,4,5,5a,5b,6,8,940,11,11a,11b,12,13-hexadécahydro-1H-2-azapentaléno[1,6a-a]phénanthrén-9-yl)-amine ;
1-{3-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-amino]-phényl}-éthanone ;
1-{4-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-amino]-phényl}-éthanone ;
ester 1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-2-phényl-éthylique d'acide acétique ;
2-hydroxy-N-méthyl-3-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
ester 1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-2-phényl-éthylique d'acide acétique ;
2-hydroxy-N-méthyl-3-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
ester 3-méthyl-1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-butylique d'acide acétique ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-hydroxy-4-méthyl-pentanoïque ;
ester 2-méthyl-1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-propylique d'acide acétique ;
2-hydroxy-3,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
ester 1-méthyl-1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-propylique d'acide acétique ;
2-hydroxy-2,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
ester 1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-cyclopropylique d'acide acétique ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 1-hydroxy-cyclopropanecarboxylique ;
ester 1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-pentylique d'acide acétique ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-hydroxy-hexanoïque ;
ester [méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-phényl-méthylique d'acide acétique ;
2-hydroxy-N-méthyl-2-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
ester 2,2,2-trifluoro-1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-éthylique d'acide acétique ;
3,3,3-trifluoro-2-hydroxy-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
ester (4-fluoro-phényl)-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-méthylique d'acide acétique ;
2-(4-fluoro-phényl)-2-hydroxy-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
ester (4-méthoxy-phényl)-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-méthylique d'acide acétique ;
2-hydroxy-2-(4-méthoxy-phényl)-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
ester (3,4-difluoro-phényl)-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-méthylique d'acide acétique ;
2-(3,4-difluoro-phényl)-2-hydroxy-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
ester [méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-phényl-méthylique d'acide acétique ;
2-hydroxy-N-méthyl-2-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
ester [méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-phényl-méthylique d'acide acétique ;
2-hydroxy-N-méthyl-2-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
ester 3-méthyl-1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-butylique d'acide acétique ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-hydroxy-4-méthyl-pentanoïque ;
ester 3-méthyl-1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-butylique d'acide acétique ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-hydroxy-4-méthyl-pentanoïque ;
2-méthoxy-N-méthyl-2-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
2-méthoxy-N-méthyl-2-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
2-méthoxy-N-méthyl-2-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-méthoxy-hexanoïque ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-méthoxy-4-méthyl-pentanoïque ;
2-méthoxy-N-méthyl-3-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
2-méthoxy-3,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
9-éthylidène-2,3,11a-triméthyl-2,3,3a,4,5,5a,5b,6,8,9,10,11,11a,11b,12,13-hexadécahydro-1H-2-aza-pentaléno[1,6a-a]phénanthrène ;
9-isopropylidène-2,3,11a-triméthyl-2,3,3a,4,5,5a,5b,6,8,9,10,11,11a,11b,12,13-hexadécahydro-1H-2-azapentaléno[1,6a-a]phénanthrène ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide benzènesulfonique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide 4-cyano-benzènesulfonique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide furan-2-ylméthyl-méthyl-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide méthylpropyl-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide benzylméthyl-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide méthyl-(6-méthyl-pyridin-2-ylméthyl)-carbamique ;
ester 2,3,11a-triméthyl-2,3,3a,4,5,5a,5b,6,8,9,10,11,1ia,11b,12,13-hexadécahydro-1H-2-aza-pentaléno[1,6a-a]phénanthrén-9-ylique d'acide méthyl-(tétrahydro-furan-2-ylméthyl)-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide (2-fluoro-éthyl)-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide furan-2-ylméthyl-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide (2-cyano-éthyl)-(tétrahydro-furan-2-ylméthyl)-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide benzylcarbamique ; et
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide propylcarbamique.

7. Composé selon la revendication 1, pour le traitement d'états et de troubles liés à une modulation du récepteur d'histamine 3 chez les mammifères.

8. Composé de formule : 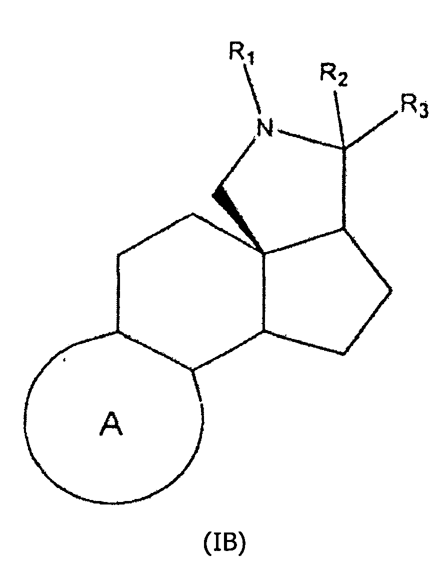 ou un sel pharmaceutiquement acceptable de celui-ci,
formule dans laquelle
R₁ est choisi dans le groupe constitué par l'hydrogène, acétyle, alkyle, fluoroalkyle, et cycloalkyle ;
chacun de R₂ et R₃ est indépendamment choisi dans le groupe constitué par l'hydrogène et alkyle, ou bien R₂ et R₃, pris ensemble, forment un cycle à 3 à 6 chaînons ;
le cycle A de formule : 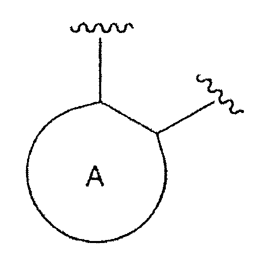 est choisi parmi les suivants : 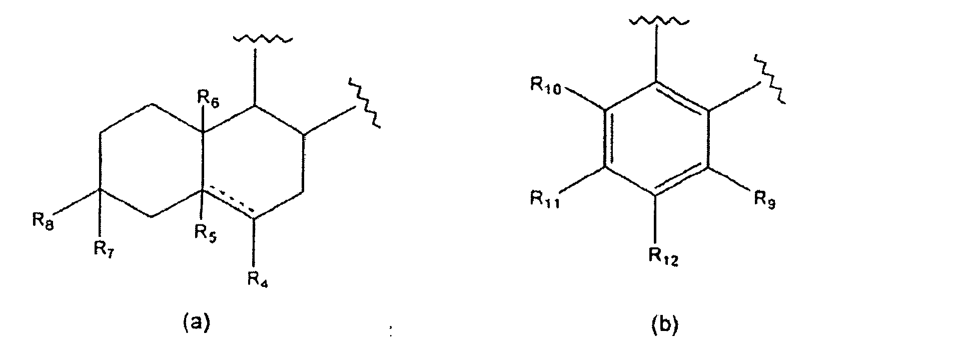 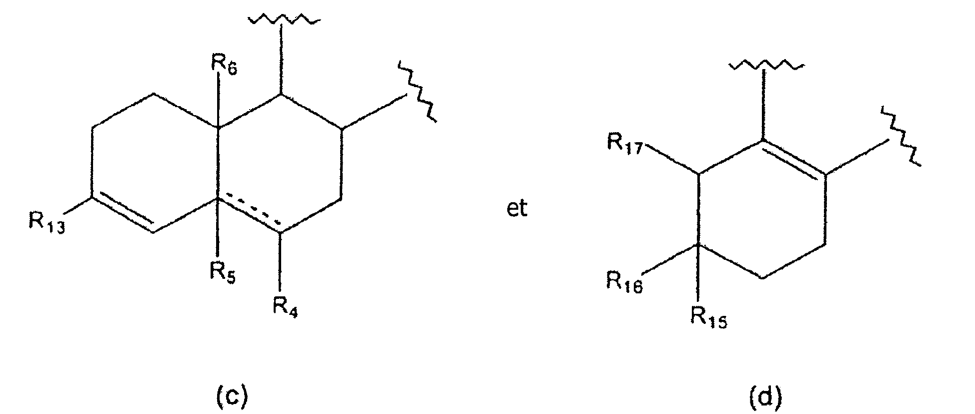 où :
la ligne de tirets représente une liaison optionnelle ;
chacun de R₄ et R₅ est indépendamment choisi dans le groupe constitué par l'hydrogène et le fluor, à la condition que R₅ soit présent uniquement quand la liaison représentée par la ligne de tirets est absente ;
R₆ est choisi dans le groupe constitué par l'hydrogène, alkyle et le fluor ;
l'un de R₇ et R₈ est l'hydrogène ; et l'autre de R₇ et R₈ est choisi dans le groupe constitué par :
a) NR₁₈R₁₉ ;
b) OR₂₀, SR₂₀, O(C=O)OR₂₀, O(C=O)N(R₂₀)(R₂₁),
O(C=O)C(R₂₃)(R₂₄)(R_{24b}), ou O(C=O)CH(NR₂₈R₂₉)R₂₅ ; et
c) NR₂₂(C=O)R₂₅, NR₂₂(C=O)NR₂₆R₂₇, NR₂₂(C=O)CH(NR₂₈R₂₉)R₃₀, N(R₂₂)(C=O)OR₂₀, ou NR₂₂(C=O)C(OR₂₃)R₃₀R_{30b} ; et
d) NR₂₂SO₂R₃₁ ou NR₂₂SO₂N(R₂₂)(R₂₃) ;
ou bien R₇ et R₈, pris ensemble avec l'atome de carbone auquel chacun est rattaché, forment un groupe de formule -C=C(Rₐ)(R_{b}) dans laquelle chacun de Rₐ et R_{b} est indépendamment choisi dans le groupe constitué par l'hydrogène, alkyle et cycloalkyle ;
chacun de R₉ et R₁₀ est indépendamment choisi dans le groupe constitué par l'hydrogène, un halogène, alkyle, et cyano ;
chacun de R₁₁ et R₁₂ est indépendamment choisi dans le groupe constitué par l'hydrogène, hétéroaryle, un hétérocycle, aryle, arylalkyle, aryloxy, arylcarbonyle, arylcarbonyloxy, arylalcoxy, alkylsulfonyle, arylsulfonyle, et trifluorométhylsulfonyle ;
ou bien l'un de R₁₀ et R₁₁ ou de R₁₁ et R₁₂, pris ensemble avec les atomes auxquels chacun est rattaché, forment un cycle aromatique ou hétéroaromatique à 5 ou 6 chaînons ;
R₁₃ est choisi dans le groupe constitué par alkyle, cycloalkyle, aryle et hétéroaryle ;
R₁₅ et R₁₆, pris ensemble avec l'atome auquel chacun est rattaché, forment un cycle à 5 ou 6 chaînons éventuellement substitué par un groupe alkylamino ;
R₁₇ est choisi dans le groupe constitué par l'hydrogène, alkyle, et fluoro ;
R₁₈ est l'hydrogène ou un alkyle en C₁ à C₆ et R₁₉ est choisi dans le groupe constitué par aryle et hétéroaryle, ou bien R₁₈ et R₁₉, à chaque occurrence, sont choisis de façon à former ensemble un hétérocycle à 3 à 8 chaînons ;
chacun de R₂₀ et R₂₁, à chaque occurrence, est indépendamment choisi dans le groupe constitué par l'hydrogène, alkyle, cycloalkyle, alcoxyalkyle, cyanoalkyle, hydroxyalkyle, halogénoalkyle, aryle, aryloxy, arylalkyle, arylsulfonyle, hétéroaryle, hétéroarylalkyle, hétéroarylcarbonyle, un hétérocycle, et hétérocycloalkyle ;
R₂₂, à chaque occurrence, est choisi dans le groupe constitué par l'hydrogène et alkyle ;
R₂₃, à chaque occurrence, est choisi dans le groupe constitué par l'hydrogène, alkyle et alkylcarbonyle ;
chacun de R₂₄ et R_{24b} est indépendamment choisi dans le groupe constitué par l'hydrogène, alkyle, aryle, arylalkyle, et cycloalkyle ;
R₂₅, à chaque occurrence, est indépendamment choisi dans le groupe constitué par alkyle en C₁ à C₆, alcoxyalkyle, hydroxyalkyle, aryle, arylalkyle, cycloalkyle, cycloalkylalkyle, cyanoalkyle, halogénoalkyle, hétéroaryle, hétéroarylalkyle, hétéroarylcarbonyle, un hétérocycle, et hétérocycloalkyle ;
chacun de R₂₆ et R₂₇, à chaque occurrence, est indépendamment choisi dans le groupe constitué par alkyle, alkylcarbonyle, alcoxycarbonyle, cycloalkyle, aryle, hétéroaryle, et un hétérocycle, ou bien R₂₆ et R₂₇, pris ensemble avec l'atome d'azote auquel chacun est rattaché, forment un cycle aromatique ou non aromatique à 5 ou 6 chaînons, dans lequel 0, 1 ou 2 atomes de carbone du cycle sont remplacés par un hétéroatome choisi parmi O, S et NR₂₃ ;
chacun de R₂₈ et R₂₉, à chaque occurrence, est indépendamment choisi dans le groupe constitué par l'hydrogène, alkyle, alkylcarbonyle, alcoxycarbonyle, cycloalkyle, aryle, hétéroaryle et un hétérocycle, ou bien R₂₈ et R₂₉, pris ensemble avec l'atome d'azote auquel chacun est rattaché, forment un cycle aromatique ou non aromatique à 5 ou 6 chaînons, dans lequel 0, 1 ou 2 atomes de carbone du cycle sont remplacés par un hétéroatome choisi parmi O, S et N₂₃ ;
R₃₀, à chaque occurrence, est indépendamment choisi dans le groupe constitué par l'hydrogène, alkyle, alcoxyalkyle, hydroxyalkyle, aryle, arylalkyle, cycloalkyle, cycloalkylalkyle, cyanoalkyle, halogénoalkyle, hétéroaryle, hétéroarylalkyle, hétéroarylcarbonyle, un hétérocycle, et hétérocycloalkyle ;
R_{30b} est choisi dans le groupe constitué par l'hydrogène et alkyle, ou bien, quand R₃₀ est un alkyle et R₃₀ est un alkyle, les groupes alkyle peuvent être liés ensemble pour former un groupe cycloalkyle en C₃ à C₄ ; et
R₃, est choisi dans le groupe constitué par alkyle, cycloalkyle, aryle, un hétérocycle, et hétéroaryle ;
ou un composé qui est l'ester 2,3,11a-triméthyl-2,3,3a,4,5,5a,5b,6,8,9,10,11,11a,11b,12,13-hexadécahydro-1H-2-azapentaléno[1,6a-a]phénanthrén-9-ylique d'acide benzoïque ;
pour une utilisation dans le traitement d'un état modulé par les récepteurs d'histamine 3 chez un mammifère comprenant l'administration dudit composé à un mammifère en ayant besoin, dans lequel l'état est lié à une fonction chez un mammifère choisie dans le groupe constitué par les processus mémoriels et cognitifs, les processus neurologiques, et la régulation du sucre dans le sang.

9. Composé selon la revendication 8, dans lequel l'état est lié à des processus mémoriels et cognitifs.

10. Composé selon la revendication 8, dans lequel l'état est choisi dans le groupe constitué par la maladie d'Alzheimer, le trouble déficitaire de l'attention avec hyperactivité, le trouble bipolaire, l'amplification cognitive, les déficits cognitifs dans des troubles psychiatriques, les déficits mémoriels, les déficits d'apprentissage, la démence, la toxicomanie, le diabète, le diabète de type II, la dépression, l'épilepsie, le syndrome de résistance à l'insuline, le décalage horaire, le syndrome métabolique, l'altération cognitive légère, la migraine, l'altération de l'humeur et de l'attention, le mal des transports, la narcolepsie, l'obésité, le trouble compulsif obsessionnel, la douleur, la maladie de Parkinson, la schizophrénie, l'épilepsie, les troubles du sommeil, le syndrome X, le syndrome de Tourette, le vertige, et l'insomnie.

11. Composé selon la revendication 8, lequel composé est choisi dans le groupe constitué par les suivants :
ester 8-méthyl-5,6,6a,7,8,9,10,11-octahydro-4bH-benzo[4,5]indéno[1,7a-c]pyrrol-2-ylique d'acide trifluorométhanesulfonique ;
1-[3-(8-méthyl-5,6,6a,7,8,9,10,11-octahydro-4bH-benzo[4,5]indéno[1,7a-c]pyrro1-2-yl)-phényl]-éthanone ;
1-[3-(8-méthyl-5,6,6a,7,8,9,10,11-octahydro-4bH-benzo[4,5]indéno[1,7a-c]pyrrol-2-yl)-phényl]-éthanone-oxime ;
1-[3-(8-méthyl-5,6,6a,7,8,9,10,11-octahydro-4bH-benzo[4,5]indéno[1,7a-c]pyrro1-2-yl)-phényl]-éthanone ;
ester 8-acétyl-5,6,6a,7,8,9,10,11-octahydro-4bH-benzo[4,5]indéno[1,7a-c]pyrrol-2-ylique d'acide benzoïque ;
1-(2-benzyloxy-5,6,6a,7,10,11-hexahydro-4bH-benzo[4,5]indéno[1,7a-c]pyrrol-8-yl)-éthanone ;
2,2,N-triméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide furane-2-carboxylique ;
4-fluoro-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-benzamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide thiophène-2-carboxylique ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide isoxazole-5-carboxylique ;
N-méthyl-N-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-isonicotinamide ;
3-chloro-4-fluoro-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-benzamide ;
2-(4-méthoxy-phényl)-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
2,3,4-trifluoro-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-benzamide ;
4-cyano-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-benzamide ;
N-méthyl-2-thiophén-2-yl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
3-chloro-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-benzamide ;
3-méthoxy-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-benzamide ;
4-méthoxy-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-benzamide ;
4,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-benzamide ;
4-chloro-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-benzamide ;
3-cyano-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-benzamide ;
4-bromo-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-benzamide ;
3,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
méthyl-(2,3,11a-triméthyl-2,3,3a,4,5,5a,5b,6,8,9,10,1,11a,11b,12,13-hexadécahydro-1H-2-aza-pentaléno[1,6a-a]phénanthrén-9-yl)-amide d'acide cyclopropanecarboxylique ;
2-méthoxy-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
N-méthyl-N-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-propionamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-isopropyl-4-méthyl-thiazole-5-carboxylique ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-sec-butyl-4-méthyl-thiazole-4-carboxylique ;
méthyl-(2,3,11a-triméthyl-2,3,3a,4,5,5a,5b,6,8,9,10,11,11a,11b,12,13-hexadécahydro-1H-2-aza-pentaléno[1,6-a-a]phénanthrén-9-yl)-amide d'acide 2-méthyl-5-phényl-furane-3-carboxylique ;
N-méthyl-2-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
N-méthyl-2-thiophén-3-yl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
N-méthyl4-thiophén-2-yl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
2-(3-fluoro-phényl)N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
N-méthyl-2-oxo-2-thiophén-2-yl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
2-indol-1-yl-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
2-benzo[b]thiophén-3-yl-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
N-méthyl-2-(3-méthyl-benzo[b]thiophén-2-yl)-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
2-furan-2-yl-N-méthyl-2-oxo-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
N-méthyl-3-thiophén-2-yl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
3-furan-2-yl-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
2-(3-chloro-phényl)-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
2-(4-chloro-phényl)-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
2-(2-fluoro-phényl)-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
2-(4-fluoro-phényl)-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
N-méthyl-2-o-tolyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide éthanesulfonique ;
N-méthyl-N-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-benzènesulfonamide ;
4-cyano-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-benzènesulfonamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide thiophène-2-sulfonique ;
4-fluoro-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-benzènesulfonamide ;
1,1-diméthyl-3-méthyl-3-(2,3,11a-triméthyl-2,3,3a,4,5,5a,5b,6,8,9,10,11,11a,11b,12,13-hexadécahydro-1H-2-azapentaléno[1,6a-a][phénanthrén-9-yl)-sulfamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide pyrrolidine-1-carboxylique ;
1,1-diisopropyl-3-méthyl-3-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-urée ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide morpholine-4-carboxylique ;
1,1,3-triméthyl-3-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-urée ;
ester tert-butylique d'acide méthyl-{2-méthyl-1-[méthyl-(2,3,11a-triméthyl2,3,3a,4,5,5a,5b,6,8,9,10,11,11a,11b,12,13-hexadécahydro-1H-2-azapentaléno[1,6a-a]phénanthrén-9-yl)-carbamoyl]-propyl}-carbamique ;
3,N-diméthyl-2-méthylamino-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
2-(acétyl-méthyl-amino)-3,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
2-amino-N-méthyl-3-phényl-N-(2,3,3a,11a-tétraméthyl-2,3,3a,4,5,5a,5b,6,8,9,10,11,11a,11b,12,13-hexadécahydro-1H-2-azapentaléno[1,6a-a]phénanthrén-9-yl)-propionamide ;
2-amino-N-méthyl-3-thiazol-4-yl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
2-amino-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
2-amino-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
2-amino-3-cyano-N-méthyl-N-(2,3,11a-triméthyl2,3,3a,4,5,5a,5b,6,8,9,10,11,11a,11b,12,13-hexadécahydro-1H-2-azapentaléno[1,6a-a]phénanthrén-9-yl)-propionamide ;
2-amino-N-méthyl-3-thiophén-2-yl-N-(2,3,11a-triméthyl2,3,3a,4,5,5a,5b,6,8,9,10,11,11a,11b,12,13-hexadécahydro-1H-2-azapentaléno[1,6a-a]phénanthrén-9-yl)-propionamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 4-méthyl-2-méthylamino-pentanoïque ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-amino-3-méthyl-pentanoïque ;
2-amino-3,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-amino-3-méthyl-pentanoïque ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-amino-4-méthyl-pentanoïque ;
2-acétylamino-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-acétylamino-3-méthyl-pentanoïque ;
2-acétylamino-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
2-acétylamino-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
2-acétylamino-N-méthyl-3-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-acétylamino-4-méthyl-pentanoïque ;
2-acétylamino-3,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
2-acétylamino-3,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
2-acétylamino-N-méthyl-3-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
2-acétylamino-N-méthyl-3-thiophén-2-yl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-acétylamino-4-méthyl-pentanoïque ;
4-[méthyl-(2,3,11a-triméthyl-2,3,3a,4,5,5a,5b,6,8,9,10,11,11a,11b, 12,13-hexadécahydro-1H-2-aza-pentaléno[1,6a-a]phénanthrén-9-yl)-amino]-benzonitrile ;
3-[méthyl-(2,3,11a-triméthyl-2,3,3a,4,5,5a,5b,6,8,9,10,11,11a,11b, 12,13-hexadécahydro-1H-2-aza-pentaléno[1,6a-a]phénanthrén-9-yl)-amino]-benzonitrile ;
méthyl-thiazol-2-yl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-amine ;
1-{3-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-amino]-phényl}-éthanone ;
1-{4-[méthyl-(2,3,11a-triméthyl-2,3,3a5,5a,5b,6,8,9,10,11,11a,1b,12,13hexadécahydro-1H-2-aza-pentaléno[1,6a-a]phénanthrén-9-yl)-amino]-phényl}-éthanone ;
ester 1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-2-phényl-éthylique d'acide acétique ;
2-hydroxy-N-méthyl-3-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
ester 1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-2-phényl-éthylique d'acide acétique ;
2-hydroxy-N-méthyl-3-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
ester 3-méthyl-1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-butylique d'acide acétique ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-hydroxy-4-méthyl-pentanoïque ;
ester 2-méthyl-1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-propylique d'acide acétique ;
2-hydroxy-3,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
ester 1-méthyl-1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-propylique d'acide acétique ;
2-hydroxy-2,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
ester 1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-cyclopropylique d'acide acétique ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 1-hydroxy-cyclopropanecarboxylique ;
ester 1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-pentylique d'acide acétique ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-hydroxy-hexanoïque ;
ester [méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-phényl-méthylique d'acide acétique ;
2-hydroxy-N-méthyl-2-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
ester 2,2,2-trifluoro-1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-éthylique d'acide acétique ;
3,3,3-trifluoro-2-hydroxy-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-propionamide ;
ester (4-fluoro-phényl)-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-méthylique d'acide acétique ;
2-(4-fluoro-phényl)-2-hydroxy-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
ester (4-méthoxy-phényl)-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-méthylique d'acide acétique ;
2-hydroxy-2-(4-méthoxy-phényl)-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
ester (3,4-difluoro-phényl)-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-méthylique d'acide acétique ;
2-(3,4-difluoro-phényl)-2-hydroxy-N-méthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
ester [méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-phényl-méthylique d'acide acétique ;
2-hydroxy-N-méthyl-2-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
ester [méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-phényl-méthylique d'acide acétique ;
2-hydroxy-N-méthyl-2-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
ester 3-méthyl-1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-butylique d'acide acétique ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-hydroxy-4-méthyl-pentanoïque ;
ester 3-méthyl-1-[méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamoyl]-butylique d'acide acétique ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-hydroxy-4-méthyl-pentandique ;
2-méthoxy-N-méthyl-2-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
2-méthoxy-N-méthyl-2-phényl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-acétamide ;
2-méthoxy-N-méthyl-2-phényl-N-(2,3,11a-triméthyl-2,3,3a,4,5,5a,5b,6,8,9,10, 11,11a,11b,12,13-hexadécahydro-1H-2-azapentaléno[1,6a-a]phénanthrén-9-yl)-acétamide ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-méthoxy-hexanoïque ;
méthyl-(2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-amide d'acide 2-méthoxy-4-méthyl-pentanoïque ;
2-méthoxy-N-méthyl-3-phényl-N-(2,3, 11a-triméthyl-2,3,3a,4,5,5a,5b,6,8,9,10,11,11a, 11b,12,13-hexadécahydro-1H-2-azapentaléno[1,6a-a]phénanthrén-9-yl)-propionamide ;
2-méthoxy-3,N-diméthyl-N-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-butyramide ;
9-éthylidène-2,3,11a-triméthyl-2,3,3a,4,5,5a,5b,6,8,9,10,11,11a,11b, 12,13-hexadécahydro-1H-2-aza-pentaléno[1,6a-a]phénanthrène ;
9-isopropylidène-2,3,11a-triméthyl-2,3,3a,4,5,5a,5b,6,8,9,10,11,11a 11b,12,13-hexadécahydro-1H-2-aza-pentaléno[1,6a-a]phénanthrène ;
9-benzyloxy-2,3,11a-triméthyl-2,3,3a,4,5,5a,5b,6,8,9,10,11,11a11b,12,13-hexadécahydro-1H-2-aza-pentaléno[1,6a-a]phénanthrène ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide acétique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide thiophén-2-yl-acétique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide 4-cyano-benzoïque ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide 3-méthyl-buyrique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide furane-2-carboxylique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide cyclopropanecarboxylique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide méthoxy-acétique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide isobutyrique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide cyclobutanecarboxylique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide propionique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide phényl-acétique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide benzènesulfonique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide 4-cyano-benzènesulfonique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide (4-fluoro-phényl)-acétique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide 2-(tert-butoxycarbonyl-méthyl-amino)-3-méthyl-butyrique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide 3-méthyl-2-méthylamino-butyrique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide tétrahydro-furane-2-carboxylique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide furan-2-yl-oxo-acétique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide 2-acétoxy-3-phényl-propionique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide 2-acétoxy-4-méthyl-pentanoïque ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide 2-acétoxy-3-méthyl-butyrique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide 2-acétoxy-2-méthyl-butyrique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide 1-acétoxy-cyclopropanecarboxylique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide 2-acétoxy-2-éthyl-butyrique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide 2-acétoxy-hexanoïque ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide méthoxy-phényl-acétique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'ester 4-nitrophénylique d'acide carbonique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide furan-2-ylméthyl-méthyl-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide méthylpropyl-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide benzylméthyl-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide méthyl-(6-méthyl-pyridin-2-ylméthyl)-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide méthyl-(tétrahydro-furan-2-ylméthyl)-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide (2-fluoro-éthyl)-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide furan-2-ylméthyl-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide (2-cyano-éthyl)-(tétrahydro-furan-2-ylméthyl)-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide benzyl-carbamique ;
ester 2,3,11a-triméthyl-2,3,3a,4,5,5a,5b,6,8,9,10,11,11a,11b,12,13-hexadécahydro-1H-2-aza-pentaléno[1,6a-a]phénanthrén-9-ylique d'acide propyl-carbamique ;
N-méthyl-2-thiophén-2-yl-N-(2,3,11a-triméthyl-octadécahydro-2-azapentaléno[1,6a-a]phénanthrén-9-yl)-acétamide ;
ester benzylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester méthylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester 2,2-diméthyl-propylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester isobutylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester éthylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester tert-butylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester isopropylique d'acide méthyl-(2,3,11a-triméthyl-2,3,3a,4,5,5a,5b,6,8,9,10,11,11a,16,12,13-hexadécahydro-1H-2-azapentaléno[1,6a-a]phénanthrén-9-yl)-carbamique ;
ester cyclopentylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester furan-3-ylméthylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester 5-méthoxy-tétrahydro-furan-3-ylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester thiazol-5-ylméthylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester hexahydro-furo[2,3-b]furan-3-ylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester tétrahydro-furan-3-ylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester pyridin-2-ylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester 3,5-diméthyl-isoxazol-4-ylméthylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester 3-hydroxy-propylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester [1,3]dioxolan-4-ylméthylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester oxiranylméthylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
ester 2-hydroxy-éthylique d'acide méthyl-(2,3,11a-triméthyl-2,3,3a,4,5,5a,5b,6,8,9,10,11,11a,11b,12,13-hexadécahydro-1H-2-azapentaléno[1,6a-a]phénanthrén-9-yl)-carbamique ;
N-méthyl-N-2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-acétmide ;
N-méthyl-N-2,3,11a-triméthyl-2,3,3a,4,5,5a,5b,6,8,9,10,11,11a,11b,12,13-hexadécahydro-1H-2-aza-pentaléno[1,6a-a]phénanthrén-9-yl)-benzamide ;
9-méthoxy-2,3,11a-triméthyl-2,3,3a,4,5,5a,5b,6,8,9,10,11,11a,11b,12,13-hexadécahydro-1H-2-aza-pentaléno[1,6a-a]phénanthrène ; et
N-méthyl-N-2,3,11a-triméthyl-octadécahydro-2-aza-pentaléno[1,6a-a]phénanthrén-9-yl)-benzamide.
